# EUROPÄISCHE PATENTSCHRIFT

(11) **EP 1 280 901 B1**
(45) Veröffentlichungstag und Bekanntmachung des Hinweises auf die Patenterteilung: **26.01.2005**
(21) Anmeldenummer: 01929476.8
(22) Anmeldetag: 29.03.2001
(51) Int. Cl.: C12N 15/12, C07K 14/47, A61K 38/17, G01N 33/50, G06F 17/50

(54) **RAUMFORM VON TPR-STRUKTURMOTIV ENTHALTENDEN POLYPEPTIDEN MIT CHAPERON-BINDUNGSFUNKTION, DEREN KRISTALLE UND VERBINDUNGEN ZUR INHIBIERUNG DERARTIGER POLYPEPTIDE**
3D STRUCTURE OF POLYPEPTIDES CONTAINING A TPR-STRUCTURE MOTIF WITH CHAPERONE-BINDING FUNCTION, CRYSTALS THEREOF AND COMPOUNDS FOR INHIBITION OF SAID PEPTIDES
FORME SPATIALE DE POLYPEPTIDES CONTENANT UN MOTIF A STRUCTURE TPR, A FONCTION DE LIAISON DE TYPE CHAPERON, SES CRISTAUX ET SES COMPOSES POUR INHIBER DES POLYPEPTIDES DE CE TYPE

(30) Priorität: 29.03.2000 DE 10015748; 13.04.2000 DE 10018335
(43) Veröffentlichungstag der Anmeldung: 05.02.2003
(73) Patentinhaber: MAX-PLANCK-GESELLSCHAFT, 80539 München (DE)
(72) Erfinder: MOAREFI, Ismail, 80337 München (DE); SCHEUFLER, Clemens, 81375 München (DE); HARTL, Ulrich, 82288 Kottgeisering (DE); BRINKER, Achim, 45731 Waltrop (DE)
(74) Vertreter: Graf von Stosch, Andreas, Dr. rer. nat.
(86) Internationale Anmeldenummer: PCT/EP2001/003617
(87) Internationale Veröffentlichungsnummer: WO 2001/073019

(56) Entgegenhaltungen:
- EP-A- 0 927 757
- WO-A-00/12702
- WO-A-00/14105
- WO-A-97/12975
- WO-A-99/27080
- YOUNG JASON C ET AL: "Specific binding of tetratricopeptide repeat proteins to the C-terminal 12-kDa domain of hsp90." JOURNAL OF BIOLOGICAL CHEMISTRY, Bd. 273, Nr. 29, 17. Juli 1998 (1998-07-17), Seiten 18007-18010, XP002174788 ISSN: 0021-9258 in der Anmeldung erwähnt
- CHEN SHIYING ET AL: "Interactions of p60, a mediator of progesterone receptor assembly, with heat shock proteins Hsp90 and Hsp70." MOLECULAR ENDOCRINOLOGY, Bd. 10, Nr. 6, 1996, Seiten 682-693, XP001013471 ISSN: 0888-8809 in der Anmeldung erwähnt
- DAS AMIT K ET AL: "The structure of the tetratricopeptide repeats of protein phosphatase 5: Implications for TPR-mediated protein-protein interactions." EMBO (EUROPEAN MOLECULAR BIOLOGY ORGANIZATION) JOURNAL, Bd. 17, Nr. 5, 2. März 1998 (1998-03-02), Seiten 1192-1199, XP002174789 ISSN: 0261-4189 in der Anmeldung erwähnt
- OWENS-GRILLO JANET K ET AL: "A model of protein targeting mediated by immunophilins and other proteins that bind to hsp90 via tetratricopeptide repeat domains." JOURNAL OF BIOLOGICAL CHEMISTRY, Bd. 271, Nr. 23, 1996, Seiten 13468-13475, XP002174790 ISSN: 0021-9258 in der Anmeldung erwähnt
- DATABASE SWALL [Online] AC P53804, 1. Oktober 1996 (1996-10-01) OHIRA M. ET AL.: "TTC3" XP002174797
- SU GUANFANG ET AL: "TTC4, a novel human gene containing the tetratricopeptide repeat and mapping to the region of chromosome 1p31 that is frequently delected in sporadic breast cancer." GENOMICS, Bd. 55, Nr. 2, 15. Januar 1999 (1999-01-15), Seiten 157-163, XP002174791 ISSN: 0888-7543
- KORDES ELISABETH ET AL: "Isolation and characterization of human SGT and identification of homologues in Saccharomyces cerevisiae and Caenorhabditis elegans." GENOMICS, Bd. 52, Nr. 1, 15. August 1998 (1998-08-15), Seiten 90-94, XP002174792 ISSN: 0888-7543
- NAGASE T ET AL: "PREDICTION OF THE CODING SEQUENCES OF UNIDENTIFIED HUMAN GENES. XI. THE COMPLETE SEQUENCES OF 100 NEW CDNA CLONES FROM BRAIN WHICH CODE FOR LARGE PROTEINS IN VITRO" DNA RESEARCH, UNIVERSAL ACADEMY PRESS, JP, Bd. 5, 1998, Seiten 277-286, XP000853885 ISSN: 1340-2838
- CHEN SHIYING ET AL: "Differential interactions of p23 and the TPR-containing proteins Hop, Cyp40, FKBP52 and FKBP51 with Hsp90 mutants." CELL STRESS & CHAPERONES, Bd. 3, Nr. 2, Juni 1998 (1998-06), Seiten 118-129, XP002174793 ISSN: 1355-8145
- LIU FU-HWA ET AL: "Specific interaction of the 70-kDa heat shock cognate protein with the tetratricopeptide repeats." JOURNAL OF BIOLOGICAL CHEMISTRY, Bd. 274, Nr. 48, 26. November 1999 (1999-11-26), Seiten 34425-34432, XP002174794 ISSN: 0021-9258
- CARRELLO AMERIGO ET AL: "The common tetratricopeptide repeat acceptor site for steroid receptor-associated immunophilins and Hop is located in the dimerization domain of hsp90." JOURNAL OF BIOLOGICAL CHEMISTRY, Bd. 274, Nr. 5, 29. Januar 1999 (1999-01-29), Seiten 2682-2689, XP002174795 ISSN: 0021-9258 in der Anmeldung erwähnt
- SCHEUFLER CLEMENS ET AL: "Structure of TPR domain-peptide complexes: Critical elements in the assembly of the Hsp70-Hsp90 multichaperone machine." CELL, Bd. 101, Nr. 2, 14. April 2000 (2000-04-14), Seiten 199-210, XP002174796 ISSN: 0092-8674

### SEQUENZPROTOKOLL

<110> Max-Planck-Gesellschaft e. V.
<120> TPR-HITZESCHOCKPROTEIN
<130> M1984-001-DEPDESk
<140> 100 18 335.2
   <141> 2000-04-13
<160> 30
<170> PatentIn Ver. 2.1
<210> 1
   <211> 122
   <212> PRT
   <213> Homo sapiens
<220>
   <223> Diese Sequenz entspricht der Sequenz TPR1 in Fig.3. Die im Sequenzprotokoll an erster Stelle genannte Aminosäure entspricht der Aminosäure 4 der TPR1-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85) .. (97)
   <223> Helix 3B
<220>
   <221> HELIX
   <222> (105)..(117)
   <223> Helix C
<400> 1
   Val Asn Glu Leu Lys Glu Lys Gly Asn Lys Ala Leu Ser Val Gly Asn
    1               5                  10                  15
   Ile Asp Asp Ala Leu Gln Cys Tyr Ser Glu Ala Ile Lys Leu Asp Pro
               20                  25                  30
   His Asn His Val Leu Tyr Ser Asn Arg Ser Ala Ala Tyr Ala Lys Lys
           35                  40                  45
   Gly Asp Tyr Gln Lys Ala Tyr Glu Asp Gly Cys Lys Thr Val Asp Leu
       50                  55                  60
   Lys Pro Asp Trp Gly Lys Gly Tyr Ser Arg Lys Ala Ala Ala Leu Glu
   65                  70                  75                  80
   Phe Leu Asn Arg Phe Glu Glu Ala Lys Arg Thr Tyr Glu Glu Gly Leu
                   85                  90                  95
   Lys His Glu Ala Asn Asn Pro Gln Leu Lys Glu Gly Leu Gln Asn Met
               100                 105                 110
   Glu Ala Arg Leu Ala Glu Arg Lys Phe Met
           115                 120
<210> 2
   <211> 122
   <212> PRT
   <213> Homo sapiens
<220>
   <223> Diese Sequenz entspricht der Sequenz CHIP in Fig.3. Die im Sequenzprotokoll an erster Stelle genannte Aminosäure entspricht der Aminosäure 26 der CHIP-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105)..(117)
   <223> Helix c
<400> 2
   Ala Gln Glu Leu Lys Glu Gln Gly Asn Arg Leu Phe Val Gly Arg Lys
   1               5                   10                  15
   Tyr Pro Glu Ala Ala Ala Cys Tyr Gly Arg Val Ile Thr Arg Asn Pro
               20                  25                  30
   Leu Val Ala Val Tyr Tyr Thr Asn Arg Ala Leu Cys Tyr Leu Lys Met
           35                  40                  45
   Gln Gln His Glu Gln Ala Leu Ala Asp Cys Arg Arg Ala Leu Glu Leu
           50                  55                  60
   Asp Gly Gln Ser Val Lys Ala His Phe Phe Leu Gly Gln Cys Gln Leu
   65                  70                  75                  80
   Glu Met Glu Ser Tyr Asp Glu Ala Ile Ala Asn Leu Gln Arg Ala Tyr
                   85                  90                  95
   Ser Leu Ala Lys Glu Gln Arg Leu Asn Phe Gly Asp Asp Ile Pro Ser
               100                 105                 110
   Ala Leu Arg Ile Ala Lys Lys Lys Arg Trp
           115                 120
<210> 3
   <211> 128
   <212> PRT
   <213> Homo sapiens
<220>
   <223> Diese sequenz entspricht der Sequenz TPR2A in Fig.3. Die im Sequenzprotokoll an erster Stelle genannte Aminosäure entspricht der Aminosäure 225 der TPR2A-Sequenz in Fig.3.
<220>
   <221> HELIX
   <222> (1) .. (12)
   <223> Helix 1A
<220>
   <221> HELIX
   <222> (17)..(29)
   <223> Helix 1B
<220>
   <221> HELIX
   <222> (35) .. (46)
   <223> Helix 2A
<220>
   <221> HELIX
   <222> (51)..(63)
   <223> Helix 2B
<220>
   <221> HELIX
   <222> (76) .. (87)
   <223> Helix 3A
<220>
   <221> HELIX
   <222> (92)..(104)
   <223> Helix 3B
<220>
   <221> HELIX
   <222> (111)..(123)
   <223> Helix C
<400> 3
   Ala Leu Lys Glu Lys Glu Leu Gly Asn Asp Ala Tyr Lys Lys Lys Asp
   1 5 10 15
   Phe Asp Thr Ala Leu Lys His Tyr Asp Lys Ala Lys Glu Leu Asp Pro
   20 25 30
   Thr Asn Met Thr Tyr Ile Thr Asn Gln Ala Ala Val Tyr Phe Glu Lys
   35 40 45
   Gly Asp Tyr Asn Lys Cys Arg Glu Leu Cys Glu Lys Ala Ile Glu Val
   50 55 60
   Gly Arg Glu Asn Arg Glu Asp Tyr Arg Gln Ile Ala Lys Ala Tyr Ala
   65 70 75 80
   Arg Ile Gly Asn Ser Tyr Phe Lys Glu Glu Lys Tyr Lys Asp Ala Ile
   85 90 95
   His Phe Tyr Asn Lys Ser Leu Ala Glu His Arg Thr Pro Asp Val Leu
   100 105 110
   Lys Lys Cys Gln Gln Ala Glu Lys Ile Leu Lys Glu Gln Glu Arg Leu
   115 120 125
<210> 4
   <211> 122
   <212> PRT
   <213> Homo sapiens
<220>
   <223> Diese Sequenz entspricht der Sequenz TPR2B in Fig.3. Die im Sequenzprotokoll an erster Stelle genannte Aminosäure entspricht der Aminosäure 360 der TPR2B-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105)..(117)
   <223> Helix C
<400> 4
   Ala Leu Glu Glu Lys Asn Lys Gly Asn Glu Cys Phe Gln Lys Gly Asp
    1               5                  10                  15
   Tyr Pro Gln Ala Met Lys His Tyr Thr Glu Ala Ile Lys Arg Asn Pro
               20                  25                  30
   Lys Asp Ala Lys Leu Tyr Ser Asn Arg Ala Ala Cys Tyr Thr Lys Leu
           35                  40                  45
   Leu Glu Phe Gln Leu Ala Leu Lys Asp Cys Glu Glu Cys Ile Gln Leu
       50                  55                  60
   Glu Pro Thr Phe Ile Lys Gly Tyr Thr Arg Lys Ala Ala Ala Leu Glu
   65                  70                  75                  80
   Ala Met Lys Asp Tyr Thr Lys Ala Met Asp Val Tyr Gln Lys Ala Leu
                   85                  90                  95
   Asp Leu Asp Ser Ser Cys Lys Glu Ala Ala Asp Gly Tyr Gln Arg Cys
               100                 105                 110
   Met Met Ala Gln Tyr Asn Arg His Asp Ser
           115                 120
<210> 5
   <211> 122
   <212> PRT
   <213> Homo sapiens
<220>
   <223> Diese Sequenz entspricht der Sequenz PP5 in Fig.3. Die erste Aminosäure der vorliegenden Sequenz entspricht der Aminosäure 28 der PP5-Sequenz in Fig.3.
<220>
   <221> HELIX
   <222> (1) .. (12)
   <223> Helix 1A
<220>
   <221> HELIX
   <222> (17)..(29)
   <223> Helix 1B
<220>
   <221> HELIX
   <222> (35) .. (46)
   <223> Helix 2A
<220>
   <221> HELIX
   <222> (51) .. (63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105)..(117)
   <223> Helix C
<400> 5
   Ala Glu Glu Leu Lys Thr Gln Ala Asn Asp Tyr Phe Lys Ala Lys Asp
   1 5 10 15
   Tyr Glu Asn Ala Ile Lys Phe Tyr Ser Gln Ala Ile Glu Leu Asn Pro
   20 25 30
   Ser Asn Ala Ile Tyr Tyr Gly Asn Arg Ser Leu Ala Tyr Leu Arg Thr
   35 40 45
   Glu Cys Tyr Gly Tyr Ala Leu Gly Asp Ala Thr Arg Ala Ile Glu Leu
   50 55 60
   Asp Lys Lys Tyr Ile Lys Gly Tyr Tyr Arg Arg Ala Ala Ser Asn Met
   65 70 75 80
   Ala Leu Gly Lys Phe Arg Ala Ala Leu Arg Asp Tyr Glu Thr Val Val
   85 90 95
   Lys Val Lys Pro His Asp Lys Asp Ala Lys Met Lys Tyr Gln Glu Cys
   100 105 110
   Asn Lys Ile Val Lys Gln Lys Ala Phe Glu
   115 120
<210> 6
   <211> 137
   <212> PRT
   <213> Homo sapiens
<220>
   <223> Diese Sequenz entspricht der Sequenz FKBP51 in Fig.3. Die im Sequenzprotokoll an erster Stelle genannte Aminosäure entspricht der Aminosäure 268 der FKBP51-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (50) .. (61)
   <223> Helix 2A
<220>
   <221> HELIX
   <222> (66)..(78)
   <223> Helix 2B
<220>
   <221> HELIX
   <222> (84)..(95)
   <223> Helix 3A
<220>
   <221> HELIX
   <222> (100) .. (112)
   <223> Helix 3B
<220>
   <221> HELIX
   <222> (120)..(132)
   <223> Helix C
<400> 6
   Ala Ala Ile Val Lys Glu Lys Gly Thr Val Tyr Phe Lys Gly Gly Lys
   1               5                   10                  15
   Tyr Met Gln Ala Val Ile Gln Tyr Gly Lys Ile Val Ser Trp Leu Glu
               20                  25                  30
   Met Glu Tyr Gly Leu Ser Glu Lys Glu Ser Lys Ala Ser Glu Ser Phe
           35                  40                  45
   Leu Leu Ala Ala Phe Leu Asn Leu Ala Met Cys Tyr Leu Lys Leu Arg
       50                  55                  60
   Glu Tyr Thr Lys Ala Val Glu Cys Cys Asp Lys Ala Leu Gly Leu Asp
   65                  70                  75                  80
   Ser Ala Asn Glu Lys Gly Leu Tyr Arg Arg Gly Glu Ala Gln Leu Leu
                   85                  90                  95
   Met Asn Glu Phe Glu Ser Ala Lys Gly Asp Phe Glu Lys Val Leu Glu
               100                 105                 110
   Val Asn Pro Gln Asn Lys Ala Ala Arg Leu Gln Ile Ser Met Cys Gln
           115                 120                 125
   Lys Lys Ala Lys Glu His Asn Glu Arg
       130                 135
<210> 7
   <211> 137
   <212> PRT
   <213> Homo sapiens
<220>
   <223> Diese Sequenz entspricht der Sequenz FKBP52 in Fig.3. Die im Sequenzprotokoll an erster Stelle genannte Aminosäure entspricht der Aminosäure 270 der FKBP52-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (50)..(61)
   <223> Helix 2A
<220>
   <221> HELIX
   <222> (66)..(78)
   <223> Helix 2B
<220>
   <221> HELIX
   <222> (84) .. (95)
   <223> Helix 3A
<220>
   <221> HELIX
   <222> (100)..(112)
   <223> Helix 3B
<220>
   <221> HELIX
   <222> (120)..(132)
   <223> Helix c
<400> 7
   Ser Thr Ile Val Lys Glu Arg Gly Thr Val Tyr Phe Lys Glu Gly Lys
   1               5                   10                  15
   Tyr Lys Gln Ala Leu Leu Gln Tyr Lys Lys Ile Val Ser Trp Leu Glu
               20                  25                  30
   Tyr Glu Ser Ser Phe Ser Asn Glu Glu Ala Gln Lys Ala Gln Ala Leu
           35                  40                  45
   Arg Leu Ala Ser His Leu Asn Leu Ala Met Cys His Leu Lys Leu Gln
       50                  55                  60
   Ala Phe Ser Ala Ala Ile Glu Ser Cys Asn Lys Ala Leu Glu Leu Asp
   65                  70                  75                  80
   Ser Asn Asn Glu Lys Gly Leu Phe Arg Arg Gly Glu Ala His Leu Ala
                   85                  90                  95
   Val Asn Asp Phe Glu Leu Ala Arg Ala Asp Phe Gln Lys Val Leu Gln
               100                 105                 110
   Leu Tyr Pro Asn Asn Lys Ala Ala Lys Thr Gln Leu Ala Val Cys Gln
           115                 120                 125
   Gln Arg Ile Arg Arg Gln Leu Ala Arg
       130                 135
<210> 8
   <211> 138
   <212> PRT
   <213> Homo sapiens
<220>
   <223> Diese Sequenz entspricht der Sequenz CYP40 in Fig.3. Die im Sequenzprotokoll an erster Stelle genannte Aminosäure entspricht der Aminosäure 223 in der CYP40-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (51)..(62)
   <223> Helix 2A
<220>
   <221> HELIX
   <222> (67) .. (79)
   <223> Helix 2B
<220>
   <221> HELIX
   <222> (85) .. (96)
   <223> Helix 3A
<220>
   <221> HELIX
   <222> (101)..(113)
   <223> Helix 3B
<220>
   <221> HELIX
   <222> (121)..(133)
   <223> Helix C
<400> 8
   Thr Glu Asp Leu Lys Asn Ile Gly Asn Thr Phe Phe Lys Ser Gln Asn
   1               5                   10                  15
   Trp Glu Met Ala Ile Lys Lys Tyr Ala Glu Val Leu Arg Tyr Val Asp
               20                  25                  30
   Ser Ser Lys Ala Val Ile Glu Thr Ala Asp Arg Ala Lys Leu Gln Pro
           35                  40                  45
   Ile Ala Leu Ser Cys Val Leu Asn Ile Gly Ala Cys Lys Leu Lys Met
       50                  55                  60
   Ser Asn Trp Gln Gly Ala Ile Asp Ser Cys Leu Glu Ala Leu Glu Leu
   65                  70                  75                  80
   Asp Pro Ser Asn Thr Lys Ala Leu Tyr Arg Arg Ala Gln Gly Trp Gln
                   85                  90                  95
   Gly Leu Lys Glu Tyr Asp Gln Ala Leu Ala Asp Leu Lys Lys Ala Gln
               100                 105                 110
   Gly Ile Ala Pro Glu Asp Lys Ala Ile Gln Ala Glu Leu Leu Lys Val
           115                 120                 125
   Lys Gln Lys Ile Lys Ala Gln Lys Asp Lys
       130                 135
<210> 9
   <211> 117
   <212> PRT
   <213> Homo sapiens
<220>
   <223> Diese Sequenz entspricht der Sequenz TOM34 in Fig.3. Die im Sequenzprotokoll an erster Stelle genannte Aminosäure entspricht der Aminosäure 193 der TOM34-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105)..(117)
   <223> Helix C
<400> 9
   Ala Arg Val Leu Lys Glu Glu Gly Asn Glu Leu Val Lys Lys Gly Asn
   1 5 10 15
   His Lys Lys Ala Ile Glu Lys Tyr Ser Glu Ser Leu Leu Cys Ser Asn
   20 25 30
   Leu Glu Ser Ala Thr Tyr Ser Asn Arg Ala Leu Cys Tyr Leu Val Leu
   35 40 45
   Lys Gln Tyr Thr Glu Ala Val Lys Asp Cys Thr Glu Ala Leu Lys Leu
   50 55 60
   Asp Gly Lys Asn Val Lys Ala Phe Tyr Arg Arg Ala Gln Ala His Lys
   65 70 75 80
   Ala Leu Lys Asp Tyr Lys Ser Ser Phe Ala Asp Ile Ser Asn Leu Leu
   85 90 95
   Gln Ile Glu Pro Arg Asn Gly Pro Ala Gln Lys Leu Arg Gln Glu Val
   100 105 110
   Lys Gln Asn Leu His
   115
<210> 10
   <211> 121
   <212> PRT
   <213> Homo sapiens
<220>
   <223> Diese Sequenz entspricht der Sequenz TOM70 in Fig.3. Die im Sequenzprotokoll an erster Stelle genannte Aminosäure entspricht der Aminosäure 136 der TOM70-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(45)
   <223> Helix 2A
<220>
   <221> HELIX
   <222> (50)..(62)
   <223> Helix 2B
<220>
   <221> HELIX
   <222> (68) .. (79)
   <223> Helix 3A
- <220>
   <221> HELIX
   <222> (84).. (96)
   <223> Helix 3B
<220>
   <221> HELIX
   <222> (104)..(116)
   <223> Helix C
<400> 10
   Ala Ala Lys Leu Lys Glu Leu Gly Asn Lys Ala Tyr Gly Ser Lys Asp
   1               5                   10                  15
   Phe Asn Lys Ala Ile Asp Leu Tyr Ser Lys Ala Ile Ile Cys Lys Pro
               20                  25                  30
   Asp Pro Val Tyr Tyr Ser Asn Arg Ala Ala Cys His Asn Ala Leu Ala
           35                  40                  45
   Gln Trp Glu Gln Val Val Ala Asp Thr Thr Ala Ala Leu Lys Leu Asp
       50                  55                  60
   Pro His Tyr Val Lys Ala Leu Asn Arg Arg Ala Asn Ala Tyr Asp Gln
   65                  70                  75                  80
   Leu Ser Arg Tyr Arg His Ala Leu Leu Asp Phe Thr Ala Ser Cys Ile
                   85                  90                  95
   Ile Asp Gly Phe Arg Asn Glu Gln Ser Ala Gln Ala Val Glu Arg Leu
               100                 105                 110
   Leu Lys Lys Phe Ala Glu Asn Lys Ala
           115                 120
<210> 11
   <211> 126
   <212> PRT
   <213> Homo sapiens
<220>
   <223> Diese Sequenz entspricht der Sequenz CNS1_ sc in Fig.3. Die im Sequenzprotokoll an erster Stelle genannte Aminosäure entspricht der Aminosäure 83 der CNS1 _sc-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (39)..(50)
   <223> Helix 2A
<220>
   <221> HELIX
   <222> (55)..(67)
   <223> Helix 2B
<220>
   <221> HELIX
   <222> (73)..(84)
   <223> Helix 3A
<220>
   <221> HELIX
   <222> (89)..(101)
   <223> Helix 3B
<220>
   <221> HELIX
   <222> (109)..(121)
   <223> Helix C
<400> 11
   Ala Glu Asn Phe Lys Lys Gln Gly Asn Glu Leu Tyr Lys Ala Lys Arg
    1               5                   10                  15
   Phe Lys Asp Ala Arg Glu Leu Tyr Ser Lys Gly Leu Ala Val Glu Cys
               20                  25                  30
   Glu Asp Lys Ser Ile Asn Glu Ser Leu Tyr Ala Asn Arg Ala Ala Cys
           35                  40                  45
   Glu Leu Glu Leu Lys Asn Tyr Arg Arg Cys Ile Glu Asp Cys Ser Lys
       50                  55                  60
   Ala Leu Thr Ile Asn Pro Lys Asn Val Lys Cys Tyr Tyr Arg Thr Ser
   65                  70                  75                  80
   Lys Ala Phe Phe Gln Leu Asn Lys Leu Glu Glu Ala Lys Ser Ala Ala
                   85                  90                  95
   Thr Phe Ala Asn Gln Arg Ile Asp Pro Glu Asn Lys Ser Ile Leu Asn
               100                 105                 110
   Met Leu Ser Val Ile Asp Arg Lys Glu Gln Glu Leu Lys Ala
           115                 120                 125
<210> 12
   <211> 127
   <212> PRT
   <213> Homo sapiens
<220>
   <223> Diese Sequenz entspricht der Sequenz TTC1 in Fig.3. Die im Sequenzprotokoll an erster Stelle genannte Aminosäure entspricht der Aminosäure 116 der Sequenz TTC1 in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (90)..(51)
   <223> Helix 2A
<220>
   <221> HELIX
   <222> (56)..(68)
   <223> Helix 2B
<220>
   <221> HELIX
   <222> (74)..(85)
   <223> Helix 3A
<220>
   <221> HELIX
   <222> (90)..(102)
   <223> Helix 3B
<220>
   <221> HELIX
   <222> (110)..(122)
   <223> Helix C
<400> 12
   Ser Thr Arg Leu Lys Glu Glu Gly Asn Glu Gln Phe Lys Lys Gly Asp
    1               5                  10                  15
   Tyr Ile Glu Ala Glu Ser Ser Tyr Ser Arg Ala Leu Glu Met Cys Pro
               20                  25                  30
   Ser Cys Phe Gln Lys Glu Arg Ser Ile Leu Phe Ser Asn Arg Ala Ala
           35                  40                  45
   Ala Arg Met Lys Gln Asp Lys Lys Glu Met Ala Ile Asn Asp Cys Ser
       50                  55                  60
   Lys Ala Ile Gln Leu Asn Pro Ser Tyr Ile Arg Ala Ile Leu Arg Arg
   65                  70                  75                  80
   Ala Glu Leu Tyr Glu Lys Thr Asp Lys Leu Asp Glu Ala Leu Glu Asp
                   85                  90                  95
   Tyr Lys Ser Ile Leu Glu Lys Asp Pro Ser Ile His Gln Ala Arg Glu
               100                 105                 110
   Ala Cys Met Arg Leu Pro Lys Gln Ile Glu Glu Arg Asn Glu Arg
           115                 120                 125
<210> 13
   <211> 126
   <212> PRT
   <213> Homo sapiens
<220>
   <223> Diese Sequenz entspricht der Sequenz TTC2 in Fig.3. Die im Sequenzprotokoll an erster Stelle genannte Aminosäure entspricht der Aminosäure 246 der TTC2-Sequenz in Fig.3.
<220>
   <221> HELIX
   <222> (1) .. (12)
   <223> Helix 1A
<220>
   <221> HELIX
   <222> (17) .. (29)
   <223> Helix 1B
<220>
   <221> HELIX
   <222> (39)..(50)
   <223> Helix 2A
<220>
   <221> HELIX
   <222> (55)..(67)
   <223> Helix 2B
<220>
   <221> HELIX
   <222> (73)..(84)
   <223> Helix 3A
<220>
   <221> HELIX
   <222> (89)..(101)
   <223> Helix 3B
<220>
   <221> HELIX
   <222> (109)..(121)
   <223> Helix C
<400> 13
   Leu Lys Ala Lys Lys Glu Asp Gly Asn Lys Ala Phe Lys Glu Gly Asn
   1 5 10 15
   Tyr Lys Leu Ala Tyr Glu Leu Tyr Thr Glu Ala Leu Gly Ile Asp Pro
   20 25 30
   Asn Asn Ile Lys Thr Asn Ala Lys Leu Tyr Cys Asn Arg Gly Thr Val
   35 40 45
   Asn Ser Lys Leu Arg Lys Leu Asp Asp Ala Ile Glu Asp Cys Thr Asn
   50 55 60
   Ala Val Lys Leu Asp Asp Thr Tyr Ile Lys Ala Tyr Leu Arg Arg Ala
   65 70 75 80
   Gln Cys Tyr Met Asp Thr Glu Gln Tyr Glu Glu Ala Val Arg Asp Tyr
   85 90 95
   Glu Lys Val Tyr Gln Thr Glu Lys Thr Lys Glu His Lys Gln Leu Leu
   100 105 110
   Lys Asn Ala Gln Leu Glu Leu Lys Lys Ser Lys Arg Lys Asp
   115 120 125
<210> 14
   <211> 122
   <212> PRT
   <213> Homo sapiens
<220>
   <223> Diese Sequenz entspricht der Sequenz TTC3 in Fig.3. Die im sequenzprotokoll an erster Stelle genannte Aminosäure entspricht der Aminosäure 231 der TTC3-Sequenz in Fig.3.
<220>
   <221> HELIX
   <222> (1)..(12)
   <223> Helix 1A
<220>
   <221> HELIX
   <222> (17).. (29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85) .. (97)
   <223> Helix 3B
<220>
   <221> HELIX
   <222> (105) .. (117)
   <223> Helix C
<400> 14
   Gly Glu Leu Met Lys Met Lys Gly Asn Glu Glu Phe Ser Lys Glu Arg
   1               5                   10                  15
   Phe Asp Ile Ala Ile Ile Tyr Tyr Thr Arg Ala Ile Glu Tyr Arg Pro
               20                  25                  30
   Glu Asn Tyr Leu Leu Tyr Gly Asn Arg Ala Leu Cys Phe Leu Arg Thr
           35                  40                  45
   Gly Gln Phe Arg Asn Ala Leu Gly Asp Gly Lys Arg Ala Thr Ile Leu
       50                  55                  60
   Lys Asn Thr Trp Pro Lys Gly His Tyr Arg Tyr Cys Asp Ala Leu Ser
   65                  70                  75                  80
   Met Leu Gly Glu Tyr Asp Trp Ala Leu Gln Ala Asn Ile Lys Ala Gln
                   85                  90                  95
   Lys Leu Cys Lys Asn Asp Pro Glu Gly Ile Lys Asp Leu Ile Gln Gln
               100                 105                 110
   His Val Lys Leu Gln Lys Gln Ile Glu Asp
           115                 120
<210> 15
   <211> 126
   <212> PRT
   <213> Homo sapiens
<220>
   <223> Diese Sequenz entspricht der Sequenz TTC4 in Fig.3. Die im Sequenzprotokoll an erster Stelle genannte Aminosäure der vorliegenden Sequenz entspricht der Aminosäure 79 der TTC4-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (39) .. (50)
   <223> Helix 2A
<220>
   <221> HELIX
   <222> (55)..(67)
   <223> Helix 2B
<220>
   <221> HELIX
   <222> (73)..(84)
   <223> Helix 3A
<220>
   <221> HELIX
   <222> (89)..(101)
   <223> Helix 3B
<220>
   <221> HELIX
   <222> (109)..(121)
   <223> Helix C
<400> 15
   Ala Lys Thr Tyr Lys Asp Glu Gly Asn Asp Tyr Phe Lys Glu Lys Asp
   1               5                   10                  15
   Tyr Lys Lys Ala Val Ile Ser Tyr Thr Glu Gly Leu Lys Lys Lys Cys
               20                  25                  30
   Ala Asp Pro Asp Leu Asn Ala Val Leu Tyr Thr Asn Arg Ala Ala Ala
           35                  40                  45
   Gln Tyr Tyr Leu Gly Asn Phe Arg Ser Ala Leu Asn Asp Val Thr Ala
       50                  55                  60
   Ala Arg Lys Leu Lys Pro Cys His Leu Lys Ala Ile Ile Arg Gly Ala
   65                  70                  75                  80
   Leu Cys His Leu Glu Leu Ile His Phe Ala Glu Ala Val Asn Trp Cys
                   85                  90                  95
   Asp Glu Gly Leu Gln Ile Asp Ala Lys Glu Lys Lys Leu Leu Glu Met
               100                 105                 110
   Arg Ala Lys Ala Asp Lys Leu Lys Arg Ile Glu Gln Arg Asp
           115                 120                 125
<210> 16
   <211> 122
   <212> PRT
   <213> Homo sapiens
<220>
   <223> Diese Sequenz entspricht der Sequenz IRSP in Fig.3. Die im Protokoll an erster Stelle genannte Aminosäure entspricht der Aminosäure 225 der IRSP-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105)..(117)
<223> Helix c
<400> 16
   Phe Lys Ala Leu Lys Glu Glu Gly Asn Gln Cys Val Asn Asp Lys Asn
   1               5                   10                  15
   Tyr Lys Asp Ala Leu Ser Lys Tyr Ser Glu Cys Leu Lys Ile Asn Asn
               20                  25                  30
   Lys Glu Cys Ala Ile Tyr Thr Asn Arg Ala Leu Cys Tyr Leu Lys Leu
           35                  40                  45
   Cys Gln Phe Glu Glu Ala Lys Gln Asp Cys Asp Gln Ala Leu Gln Leu
       50                  55                  60
   Ala Asp Gly Asn Val Lys Ala Phe Tyr Arg Arg Ala Leu Ala His Lys
   65                  70                  75                  80
   Gly Leu Lys Asn Tyr Gln Lys Ser Leu Ile Asp Leu Asn Lys Val Ile
                   85                  90                  95
   Leu Leu Asp Pro Ser Ile Ile Glu Ala Lys Met Glu Leu Glu Glu Val
               100                 105                 110
   Thr Arg Leu Leu Asn Leu Lys Asp Lys Thr
           115                 120
<210> 17
   <211> 122
   <212> PRT
   <213> Homo sapiens
<220>
   <223> Diese Sequenz entspricht der Sequenz SGT in Fig.3. Die im Sequenzprotokoll an erster Stelle genannte Aminosäure entspricht der Aminosäure 89 der SGT-Sequenz in Fig.3.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105) .. (117)
   <223> Helix C
<400> 17
   Ala Glu Arg Leu Lys Thr Glu Gly Asn Glu Gln Met Lys Val Glu Asn
   1 5 10 15
   Phe Glu Ala Ala Val His Phe Tyr Gly Lys Ala Ile Glu Leu Asn Pro
   20 25 30
   Ala Asn Ala Val Tyr Phe Cys Asn Arg Ala Ala Ala Tyr Ser Lys Leu
   35 40 45
   Gly Asn Tyr Ala Gly Ala Val Gln Asp Cys Glu Arg Ala Ile Cys Ile
   50 55 60
   Asp Pro Ala Tyr Ser Lys Ala Tyr Gly Arg Met Gly Leu Ala Leu Ser
   65 70 75 80
   Ser Leu Asn Lys His Val Glu Ala Val Ala Tyr Tyr Lys Lys Ala Leu
   85 90 95
   Glu Leu Asp Pro Asp Asn Glu Thr Tyr Lys Ser Asn Leu Lys Ile Ala
   100 105 110
   Glu Leu Lys Leu Arg Glu Ala Pro Ser Pro
   115 120
<210> 18
   <211> 127
   <212> PRT
   <213> Homo sapiens
<220>
   <223> Die vorliegende Sequenz entspricht der Sequenz KIAA0719 in Fig.3. Die im Sequenzprotokoll an erster Stelle genannte Aminosäure entspricht der Aminosäure 114 der KIAA0719-Sequenz in Fig.3.
<220>
   <221> HELIX
   <222> (1)..(12)
   <223> Helix 1A
<220>
   <221> HELIX
   <222> <17)..(29)
   <223> Helix 1B
<220>
   <221> HELIX
   <222> (40)..(51)
   <223> Helix 2A
<220>
   <221> HELIX
   <222> (56)..(68)
   <223> Helix 2B
<220>
   <221> HELIX
   <222> (74) .. (85)
   <223> Helix 3A
<220>
   <221> HELIX
   <222> (90)..(102)
   <223> Helix 3B
<220>
   <221> HELIX
   <222> (110)..(122)
   <223> Helix C
<400> 18
   Ala Gln Ala Ala Lys Asn Lys Gly Asn Lys Tyr Phe Lys Ala Gly Lys
   1               5                   10                  15
   Tyr Glu Gln Ala Ile Gln Cys Tyr Thr Glu Ala Ile Ser Leu Cys Pro
               20                  25                  30
   Thr Glu Lys Asn Val Asp Leu Ser Thr Phe Tyr Gln Asn Arg Ala Ala
           35                  40                  45
   Ala Phe Glu Gln Leu Gln Lys Trp Lys Glu Val Ala Gln Asp Cys Thr
       50                  55                  60
   Lys Ala Val Glu Leu Asn Pro Lys Tyr Val Lys Ala Leu Phe Arg Arg
   65                  70                  75                  80
   Ala Lys Ala His Glu Lys Leu Asp Asn Lys Lys Glu Cys Leu Glu Asp
                   85                  90                  95
   Val Thr Ala Val Cys Ile Leu Glu Gly Phe Gln Asn Gln Gln Ser Met
               100                 105                 110
   Leu Leu Ala Asp Lys Val Leu Lys Leu Leu Gly Lys Glu Lys Ala
           115                 120                 125
<210> 19
   <211> 34
   <212> PRT
   <213> Homo sapiens
<220>
   <223> Diese Sequenz entspricht der Sequenz (1) in Fig.3A. Die im Sequenzprotokoll an erster Stelle genannte Aminosäure entspricht der Aminosäure 4 der Sequenz (1) in Fig. 3A.
<220>
   <221> HELIX
   <222> (1)..(12)
   <223> Helix 1A
<220>
   <221> HELIX
   <222> (17)..(29)
   <223> Helix 1B
<400> 19
   Val Asn Glu Leu Lys Glu Lys Gly Asn Lys Ala Leu Ser Val Gly Asn
   1               5                   10                  15
   Ile Asp Asp Ala Leu Gln Cys Tyr Ser Glu Ala Ile Lys Leu Asp Pro
               20                  25                  30
   His Asn
<210> 20
   <211> 34
   <212> PRT
   <213> Homo sapiens
<220>
   <223> Diese Sequenz entspricht der Sequenz (2) in Fig. 3A. Die im Sequenzprotokoll an erster stelle genannte Aminosäure entspricht der Aminosäure 225 der Sequenz (2) in Fig.3A.
<220>
   <221> HELIX
   <222> (1)..(12)
   <223> Helix 1A
<220>
   <221> HELIX
   <222> (17)..(29)
   <223> Helix 1B
<400> 20
   Ala Leu Lys Glu Lys Glu Leu Gly Asn Asp Ala Tyr Lys Lys Lys Asp
   1               5                   10                  15
   Phe Asp Thr Ala Leu Lys His Tyr Asp Lys Ala Lys Glu Leu Asp Pro
               20                  25                  30
   Thr Asn
<210> 21
   <211> 34
   <212> PRT
   <213> Homo sapiens
<220>
   <223> Diese Sequenz entspricht der Sequenz (3) in Fig.3A. Die im Sequenzprotokoll an erster Stelle genannte Aminosäure entspricht der Aminosäure 360 der sequenz (3) in Fig. 3A.
<220>
   <221> HELIX
   <222> (1)..(12)
   <223> Helix 1A
<220>
   <221> HELIX
   <222> (17)..(29)
   <223> Helix 1B
<400> 21
   Ala Leu Glu Glu Lys Asn Lys Gly Asn Glu Cys Phe Gln Lys Gly Asp
    1               5                  10                  15
   Tyr Pro Gln Ala Met Lys His Tyr Thr Glu Ala Ile Lys Arg Asn Pro
               20                  25                  30
   Lys Asp
<210> 22
   <211> 34
   <212> PRT
   <213> Homo sapiens
<220>
   <223> Diese Sequenz entspricht der Sequenz (4) in Fig.3A.
<220>
   <221> HELIX
   <222> (1)..(12)
   <223> Helix 2A
<220>
   <221> HELIX
   <222> (17) .. (29)
   <223> Helix 2B
<400> 22
   His Val Leu Tyr Ser Asn Arg Ser Ala Ala Tyr Ala Lys Lys Gly Asp
    1               5                  10                  15
   Tyr Gln Lys Ala Tyr Glu Asp Gly Cys Lys Thr Val Asp Leu Lys Pro
               20                  25                  30
   Asp Trp
<210> 23
   <211> 41
   <212> PRT
   <213> Homo sapiens
<220>
   <223> Diese Sequenz entspricht der Sequenz (5) in Fig.3A.
<220>
   <221> HELIX
   .<222> (1)..(12)
   <223> Helix 2A
<220>
   <221> HELIX
   <222> (17)..(29)
   <223> Helix 2B
<400> 23.
   Met Thr Tyr Ile Thr Asn Gln Ala Ala Val Tyr Phe Glu Lys Gly Asp
   1               5                   10                  15
   Tyr Asn Lys Cys Arg Glu Leu Cys Glu Lys Ala Ile Glu Val Gly Arg
               20                  25                  30
   Glu Asn Arg Glu Asp Tyr Arg Gln Ile
           35                  40
<210> 24
   <211> 34
   <212> PRT
   <213> Homo sapiens
<220>
   <223> Diese Sequenz entspricht der Sequenz (6) in Fig.3A.
<220>
   <221> HELIX
   <222> (1) .. (12)
   <223> Helix 2A
<220>
   <221> HELIX
   <222> (17) .. (29)
   <223> Helix 2B
<400> 24
   Ala Lys Leu Tyr Ser Asn Arg Ala Ala Cys Tyr Thr Lys Leu Leu Glu
   1 5 10 15
   Phe Gln Leu Ala Leu Lys Asp Cys Glu Glu Cys Ile Gln Leu Glu Pro
   20 25 30
   Thr Phe
<210> 25
   <211> 32
   <212> PRT
   <213> Homo sapiens
<220>
   <223> Diese Sequenz entspricht der Sequenz (7) in Fig.3A. Die im Sequenzprotokoll an erster Stelle genannte Aminosäure entspricht der Aminosäure 72 in Sequenz (7) in Fig.3A.
<220>
   <221> HELIX
   <222> (1) .. (12)
   <223> Helix 3A
<220>
   <221> HELIX
   <222> (17)..(29)
   <223> Helix 3B
<400> 25
   Gly Lys Gly Tyr Ser Arg Lys Ala Ala Ala Leu Glu Phe Leu Asn Arg
   1 5 10 15
   Phe Glu Glu Ala Lys Arg Thr Tyr Glu Glu Gly Leu Lys His Glu Ala
   20 25 30
<210> 26
   <211> 32
   <212> PRT
   <213> Homo sapiens
<220>
   <223> Diese Sequenz entspricht der Sequenz (8) in Fig.3A. Die im Sequenzprotokoll an erster Stelle genannte Aminosäure entspricht der Aminosäure 300 in Sequenz (8) in Fig.3A.
<220>
   <221> HELIX
   <222> (1)..(12)
   <223> Helix 3A
<220>
   <221> HELIX
   <222> (17).. (29)
   <223> Helix 3B
<400> 26
   Ala Lys Ala Tyr Ala Arg Ile Gly Asn Ser Tyr Phe Lys Glu Glu Lys
   1 5 10 15
   Tyr Lys Asp Ala Ile His Phe Tyr Asn Lys Ser Leu Ala Glu His Arg
   20 25 30
<210> 27
   <211> 32
   <212> PRT
   <213> Homo sapiens
<220>
   <223> Diese Sequenz entspricht der Sequenz (9) in Fig.3A. Die im Sequenzprotokoll an erster Stelle genannte Aminosäure entspricht der Aminosäure 428 in Sequenz (9) in Fig.3A.
<220>
   <221> HELIX
   <222> (1)..(12)
   <223> Helix 3A
<220>
   <221> HELIX
   <222> (17)..(29)
   <223> Helix 3B
<400> 27
   Ile Lys Gly Tyr Thr Arg Lys Ala Ala Ala Leu Glu Ala Met Lys Asp
   1 5 10 15
   Tyr Thr Lys Ala Met Asp Val Tyr Gln Lys Ala Leu Asp Leu Asp Ser
   20 25 30
<210> 28
   <211> 122
   <212> PRT
   <213> Homo sapiens
<220>
   <223> Diese Sequenz entspricht der Sequenz TPR1 in Fig.3B. Die im Sequenzprotokoll an erster Stelle genannte Aminosäure entspricht der Aminosäure 4 der TPR1-Sequenz in Fig.3B.
<220>
   <221> HELIX
   <222> (1) .. (12)
   <223> Helix 1A
<220>
   <221> HELIX
   <222> (17) .. (29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105)..(117)
   <223> Helix C
<400> 28
   Val Asn Glu Leu Lys Glu Lys Gly Asn Lys Ala Leu Ser Val Gly Asn
   1               5                   10                  15
   Ile Asp Asp Ala Leu Gln Cys Tyr Ser Glu Ala Ile Lys Leu Asp Pro
               20                  25                  30
   His Asn His Val Leu Tyr Ser Asn Arg Ser Ala Ala Tyr Ala Lys Lys
           35                  40                  45
   Gly Asp Tyr Gln Lys Ala Tyr Glu Asp Gly Cys Lys Thr Val Asp Leu
       50                  55                  60
   Lys Pro Asp Trp Gly Lys Gly Tyr Ser Arg Lys Ala Ala Ala Leu Glu
   65                  70                  75                  80
   Phe Leu Asn Arg Phe Glu Glu Ala Lys Arg Thr Tyr Glu Glu Gly Leu
                   85                  90                  95
   Lys His Glu Ala Asn Asn Pro Gln Leu Lys Glu Gly Leu Gln Asn Met
               100                 105                 110
   Glu Ala Arg Leu Ala Glu Arg Lys Phe Met
           115                 120
<210> 29
   <211> 128
   <212> PRT
   <213> Homo sapiens
<220>
   <223> Die vorliegende Sequenz entspricht der Sequenz TPR2A in Fig.3B. Die im Sequenzprotokoll an erster Stelle genannte Aminosäure entspricht der Aminosäure 225 der TPR2A-Sequenz in Fig.3B.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35)..(46)
   <223> Helix 2A
<220>
   <221> HELIX
   <222> (51)..(63)
   <223> Helix 2B
<220>
   <221> HELIX
   <222> (76)..(87)
   <223> Helix 3A
<220>
   <221> HELIX
   <222> (92)..(104)
   <223> Helix 3B
<220>
   <221> HELIX
   <222> (111)..(123)
   <223> Helix C
<400> 29
   Ala Leu Lys Glu Lys Glu Leu Gly Asn Asp Ala Tyr Lys Lys Lys Asp
    1               5                  10                  15
   Phe Asp Thr Ala Leu Lys His Tyr Asp Lys Ala Lys Glu Leu Asp Pro
                   20                  25                  30
   Thr Asn Met Thr Tyr Ile Thr Asn Gln Ala Ala Val Tyr Phe Glu Lys
               35                  40                  45
   Gly Asp Tyr Asn Lys Cys Arg Glu Leu Cys Glu Lys Ala Ile Glu Val
           50                  55                  60
   Gly Arg Glu Asn Arg Glu Asp Tyr Arg Gln Ile Ala Lys Ala Tyr Ala
   65                  70                  75                  80
   Arg Ile Gly Asn Ser Tyr Phe Lys Glu Glu Lys Tyr Lys Asp Ala Ile
                   85                  90                  95
   His Phe Tyr Asn Lys Ser Leu Ala Glu His Arg Thr Pro Asp Val Leu
               100                 105                 110
   Lys Lys Cys Gln Gln Ala Glu Lys Ile Leu Lys Glu Gln Glu Arg Leu
           115                 120                 125
<210> 30
   <211> 122
   <212> PRT
   <213> Homo sapiens
<220>
   <223> Diese Sequenz entspricht der Sequenz TPR2B in Fig.3B. Die im Sequenzprotokoll an erster Stelle genannte Aminosäure entspricht der Aminosäure 360 der Sequenz TPR2B in Fig.3B.
<220>
   <221> HELIX
   <222> (1)..(12)
   <223> Helix 1A
<220>
   <221> HELIX
   <222> (17)..(29)
   <223> Helix 1B
<220>
   <221> HELIX
   <222> (35) .. (46)
   <223> Helix 2A
<220>
   <221> HELIX
   <222> (51)..(63)
   <223> Helix 2B
<220>
   <221> HELIX
   <222> (69)..(80)
   <223> Helix 3A
<220>
   <221> HELIX
   <222> (85)..(97)
   <223> Helix 3B
<220>
   <221> HELIX
   <222> (105)..(117)
   <223> Helix C
<400> 30
   Ala Leu Glu Glu Lys Asn Lys Gly Asn Glu Cys Phe Gln Lys Gly Asp
   1               5                   10                  15
   Tyr Pro Gln Ala Met Lys His Tyr Thr Glu Ala Ile Lys Arg Asn Pro
               20                  25                  30
   Lys Asp Ala Lys Leu Tyr Ser Asn Arg Ala Ala Cys Tyr Thr Lys Leu
           35                  40                  45
   Leu Glu Phe Gln Leu Ala Leu Lys Asp Cys Glu Glu Cys Ile Gln Leu
       50                  55                  60
   Glu Pro Thr Phe Ile Lys Gly Tyr Thr Arg Lys Ala Ala Ala Leu Glu
   65                  70                  75                  80
   Ala Met Lys Asp Tyr Thr Lys Ala Met Asp Val Tyr Gln Lys Ala Leu
                   85                  90                  95
   Asp Leu Asp Ser Ser Cys Lys Glu Ala Ala Asp Gly Tyr Gln Arg Cys
               100                 105                 110
   Met Met Ala Gln Tyr Asn Arg His Asp Ser
           115                 120

## Beschreibung

Die vorliegende Erfindung betrifft Verfahren zur Herstellung von Kristalle sowie Kristalle mit einer TPR-Strukturmotivsequenz, Verfahren zur Strukturaufklärung von derartigen Polypeptiden, Verbindungen mit der Fähigkeit, hochaffin an durch derartige Raumformen vorgegebene Strukturbereiche zu binden, Verwendungen derartiger Verbindungen zur Herstellung eines Arzneimittels sowie für Arzneimittel für bestimmte medizinische Indikationen, Verfahren zur Ermittlung derartiger Verbindungen und Verwendungen der mittels vorgenannter Verfahren erhältlichen Verbindungen.

Aus dem Stand der Technik ist bekannt, daß bei einer Anzahl von zellulären Signalproteinen für deren Faltung und konformationelle Regulation eine Koordination der Wirkungen von Hsp70- und Hsp90-Chaperon- Proteinen erforderlich ist. Hierbei wären unter den Signal-proteinen insbesondere nukleäre Rezeptoren von Steroidhormonen und mehrere Serin/Threonin- und Tyrosin-Kinasen mit Proto-Onkogen-Eigenschaften, wie z.B. Raf oder Src, zu nennen (Buchner, 1999, Trends Biochem. Sci. 24, 136-141; Caplan, 1999, Trends Cell. Biol. 9, 262-268; Pratt, 1997, Endocr. Rev. 18, 306-360).

Viele Polypeptidketten interagieren co-translational mit Chaperon-Proteinen der Hsp70-Familie, die dadurch ihre Wirkung entfalten, daß sie die Mißfaltung und Aggregation von naszierenden Proteinketten verhindern. Gerade an den Ribosomen synthetisierte Polypeptidketten werden entweder für die Auffaltung in ihre native Konformation freigegeben, und zwar ohne die Hilfe von weiteren Chaperon-Proteinen, oder aber zu einem diesbezüglich spezialisierten Chaperon-System weitergereicht (Hartl, 1996, Nature 381, 571-579; Johnson und Craig, 1997, Cell 90, 201-204). Unter diesen weiter stromabwärts wirkenden Systemen im eukaryotischen Cytosol sind insbesondere das Chaperonin-System TriC/CCT und der Hsp90-Multi-Chaperon-Apparat charakterisiert worden. Hsp90 erhält seine Substrate von Hsp70 in einer Reaktion, die in kritischer Weise von der Funktion des Hop-Proteins (Hsp70 und Hsp90 organisierendes Protein, auch als p60 oder Stilp bekannt) abhängig ist. In verschiedenen Arbeiten (Chang et al., 1997, Mol. Cell. Biol. 17, 318-325; Chen und Smith, 1998, J. Biol. Chem. 273, 35194-35200; Frydman und Höhfeld, 1997, Trends Biochem. Sci. 22, 1718-1720; Johnson et al., 1998, J. Biol. Chem. 273, 3679-3686) wurde gezeigt, daß das Hop-Protein als Adapterprotein spezifische Bindungsstellen für diese beiden vorgenannten Haupt-Chaperon-Proteine zur Verfügung stellt.

Honore et al. (1992 J. Biol. Chem. 267, 8485-8491) und Smith et al. (1993 Mol. Cell. Biol. 13, 869-876) konnten zeigen, daß das Hop-Protein fast ausschließlich aus TPR (Tetratrico-Peptid-"Repeats")-Domänen aufgebaut ist, selbständig aber keine Aktivität als Chaperon-Protein entfalten kann (Bose et al., 1996, Science 274, 1715-177; Freeman et al., 1996, Science 274, 1718-1720). Aus der Arbeit von Lamb et al. (1995, Trends Biochem. Sci. 20, 257-259) ist bekannt, daß TPR-Domänen aus drei oder mehr TPR-Strukturmotiven (mit ca. 34 AS Länge) bestehen, so daß sich die TPR-Domänen also durch degenerierte "Repeats" von einer Länge von 34 Aminosäuren auszeichnen.

Aufgrund der Primärsequenzdaten vom Hop-Protein wurde in der Literatur vorhergesagt, daß das Hop-Protein neun TPR-Strukturmotive enthält, die wiederum zwei TPR-Domänen bilden. Als Ergebnis der Analyse von Deletionsmutanten hatte sich nämlich ergeben, daß die N-terminale TPR-Domäne von Hop (TPR1 (drei TPR-Strukturmotive)) für die Wechselwirkung mit dem C-Terminus von Hsp70 verantwortlich ist, während eine C-terminale TPR2-Domäne (sechs TPR-Strukturmotive) im wesentlichen die Wechselwirkung des Hop-Proteins mit Hsp90 sicherstellt (Chen et al., 1996, Mol. Endocrinol. 10, 682-693; Demand et al., 1998, Mol. Cell. Biol. *18*, 2023-2028; Lassle et al., 1997, J. Biol. Chem. 272, 1876-1884). Prodromou et al. (1999, EMBO J. *18*, 754-762) konnten zeigen, daß das Hop-Protein die Hsp90-ATPase inhibiert und den Zugang von ATP oder des Inhibitors Geldanamycin zu ihrer Bindungstasche in der N-terminalen. Domäne von Hsp90 blockiert (Stebbins et al., 1997, Cell 89, 239-250; Prodromou et al., 1997, Cell 90, 65-75 und 1999, EMBO J. *18*, 754-762). Carrello et al. (1999, J. Biol. Chem. 274, 2682-2689) und Young et al. (1998, J. Biol. Chem. 273, 18007-18010) haben, genauso wie beim Hsp70-Chaperon, die Bindungsstelle des Hop-Proteins der C-terminalen Domäne des Hsp90-Chaperons zugeordnet. Hierbei konnten Chen et al. (1998, Cell Stress Chaperones 3, 118-129) zeigen, daß die Integrität des konservierten C-terminalen EEVD-Motivs bei Hsp90 eine wesentliche Komponente für die Wechselwirkung darstellen könnte. Auch ist aus dem Stand der Technik bekannt, daß die C-terminale Domäne von Hsp90 eine Anzahl von anderen TPR-enthaltenden Co-Chaperons bindet, wobei insbesondere die großen Immunophiline Cyp-40, FKBP51 und FKBP52 und die Serin-Threonin-Phosphotase PP5 zu erwähnen sind (Buchner, 1999, Trends Biochem. Sci 24, 136-141; Dolinski et al., 1998, Mol. Cell. Biol. 18, 7344-7352; Marsh et al., 1998, Mol. Cell. Biol. 18, 7353-7359; Pratt und Toft, 1997, Endocr. Rev. 18, 306-360). Entsprechende Experimente von Owens-Grillo et al. (1996, J. Biol. Chem. 271, 13468-13575) und Young et al. (1998, J. Biol. Chem. 273, 18007-18010) ließen vermuten, daß nur ein TPR-Akzeptorplatz in dieser Region vorhanden ist. Aus der Arbeit von Ballinger et al. (1999, Mol. Cell. Biol. 19, 4535-4545) läßt sich entnehmen, daß, genauso wie die TPR1-Domäne von Hop-Proteinen, auch das TPR-Protein CHIP an den C-Terminus von Hsp70 bindet.

Aus den multiplen Sequenzvergleichen von TPR-Domänen verschiedener Proteine ist zu erkennen, daß es keine streng konservierten Aminosäurereste in den 34 Aminosäuren langen TPR-Strukturmotiven gibt. Lamb et al. (1995, Trends Biochem. Sci. 20, 257-259) stellten fest, daß es gleichwohl eine starke Präferenz für kleine hydrophobe Aminosäuren an gewissen Positionen der Strukturmotive gibt.

Außerdem ist eine Kristallstruktur einer TPR-Domäne, und zwar des Hsp90-bindenden Proteins Phosphotase 5 (PP5), jedoch ohne einen (physiologischen oder unphysiologischen) Peptidliganden, gelöst worden, wobei sich herausstellte, daß jedes TPR-Strukturmotiv ein Helix-Turn-Helix-Motiv ausbildet (Das et al., 1998, EMBO J. 17, 1192-1199). Benachbarte TPR-Motive werden dabei in eine geordnete Folge von antiparallelen α-Helices gepackt. Das et al. (1998, EMBO J. 17, 1192-1199) haben daher vorgeschlagen, daß die TPR-Domänen spezifische Strukturelemente höherer Ordnung, z.B. Sekundär- oder Tertiärstrukturen, erkennen, um ihre biologische Funktion erfüllen können. Aus den Publikationen und Erkenntnissen des Standes der Technik ist gleichwohl nicht zu erkennen, auf welcher strukturellen Basis die Wechselwirkung zwischen Proteinen mit TPR-Domänen und beispielsweise Chaperon-Proteinen, wie z.B. Hsp70 und/oder Hsp90, beruht.

Aufgabe der vorliegenden Erfindung ist es daher, auf der Basis von biophysikalischen Methoden Erkenntnisse über die Wechselwirkung zwischen TPR-Domänen und ihren Bindungspartnern zu gewinnen und diese Erkenntnisse beispielsweise bei der Modellierung von Inhibitoren einzusetzen, die in Form eines molekularen Mimikry eine hohe Bindungsfähigkeit an die TPR-Domäne oder umgekehrt das Chaperon-Protein besitzen und derart kompetitiv die biologische Funktion der physiologischen Bindungspartner blockieren.

Erfindungsgemäß werden daher Kristalle mit Kristallstrukturen von TPR-Domänen des Hop-Proteins (TPR1, TPR2A) in An- und Abwesenheit von Peptiden, die das Bindungsverhalten von Hopund Chaperon-Proteinen simulieren, offenbart. Diese Strukturen erlauben eine Aussage über die Art und Weise der Interaktion zwischen TPR-Domänen enthaltenden Co-Chaperons und Chaperon-Proteinen, wie z.B. Hap70 und Hsp90.

Ein weiterer Gegenstand der vorliegenden Erfindung sind Verfahren zur Herstellung eines Kristalls mit Einheitszellen, enthaltend in der asymmetrischen Einheit mindestens eine Raumform der erfindungsgemäßen Art, also eines Polypeptids und ggf. mindestens einer weiteren Verbindung, wobei (a) das Polypeptid in einem Expressionssystem überexprimiert wird, (b) das überexprimierte Polypeptid gereinigt und aufkonzentriert wird, (c) das gemäß (b) erhaltene Polypeptidkonzentrat in einem geeigneten Puffersystem, ggf. unter Hinzufügung mindestens einer weiteren Verbindung, gelöst wird und (d) die Kristallisierung beispielsweise durch Dampfdiffusionsverfahren eingeleitet wird.

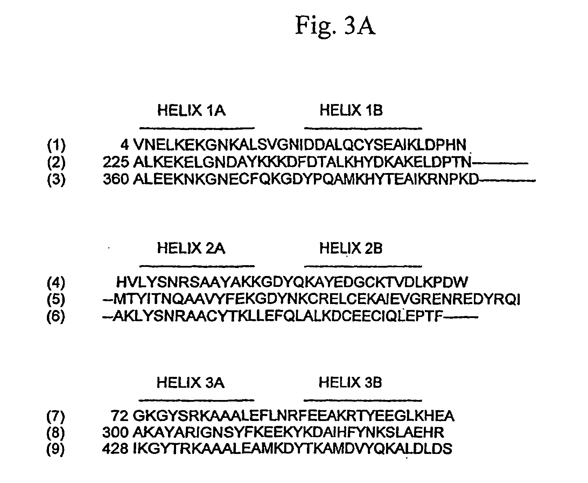

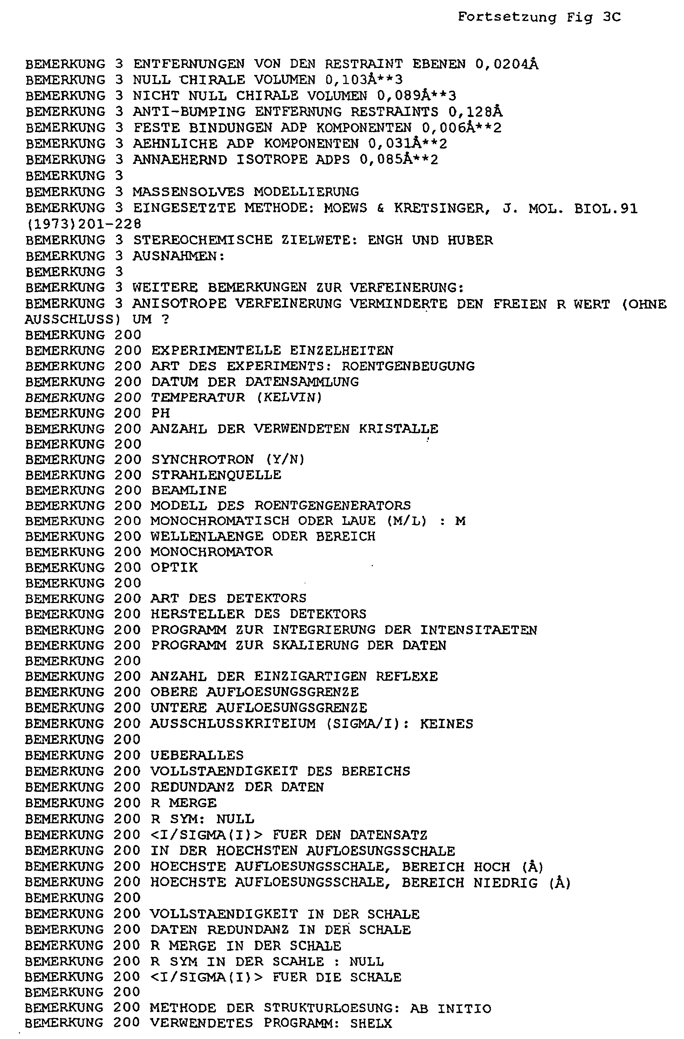

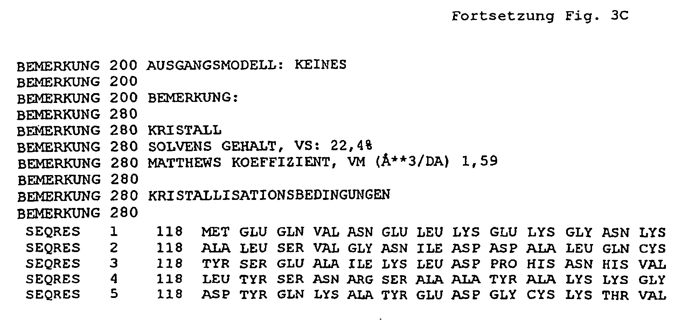

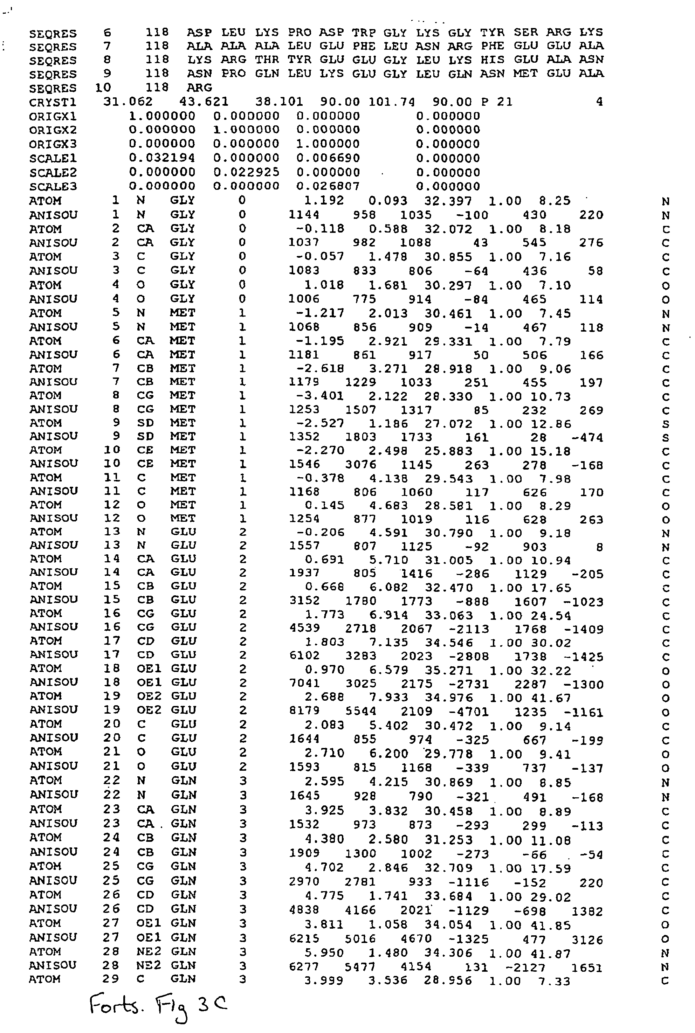

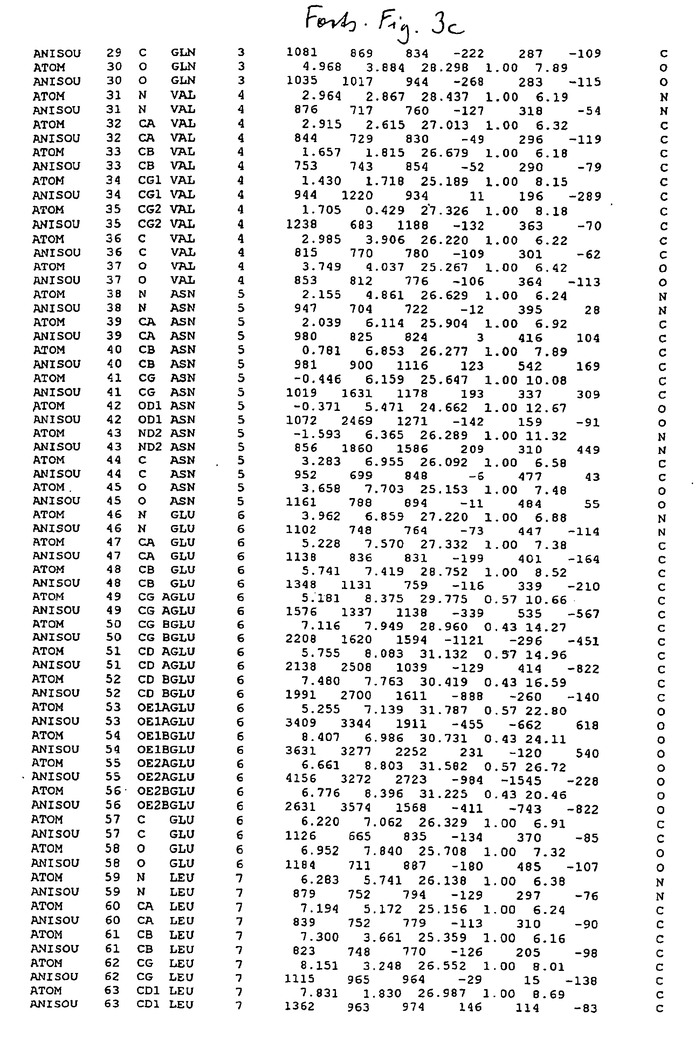

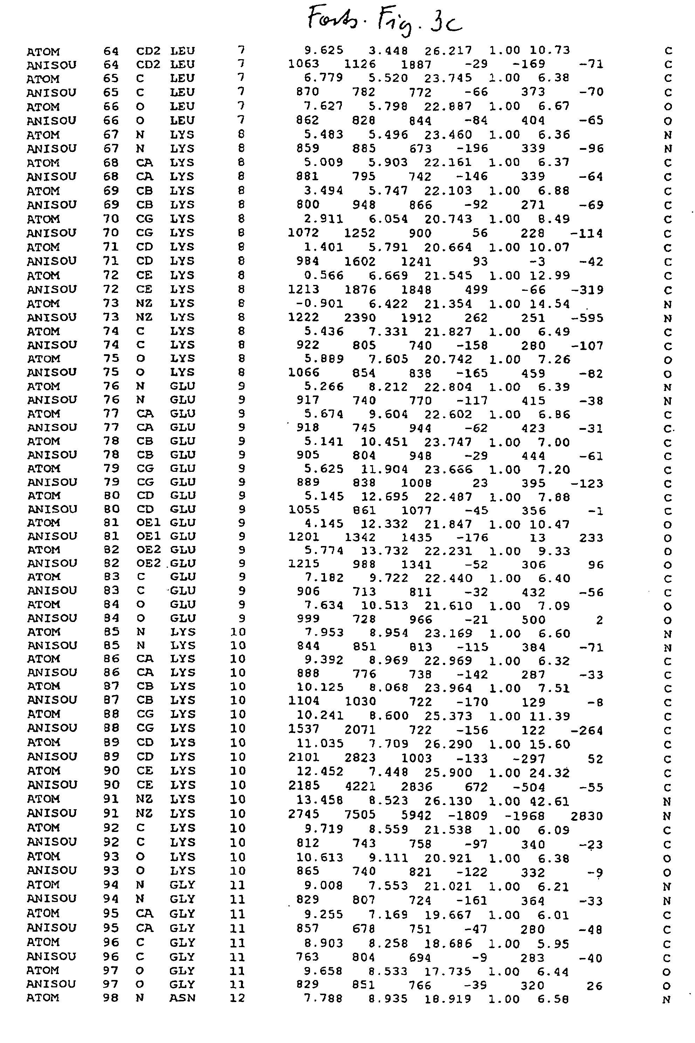

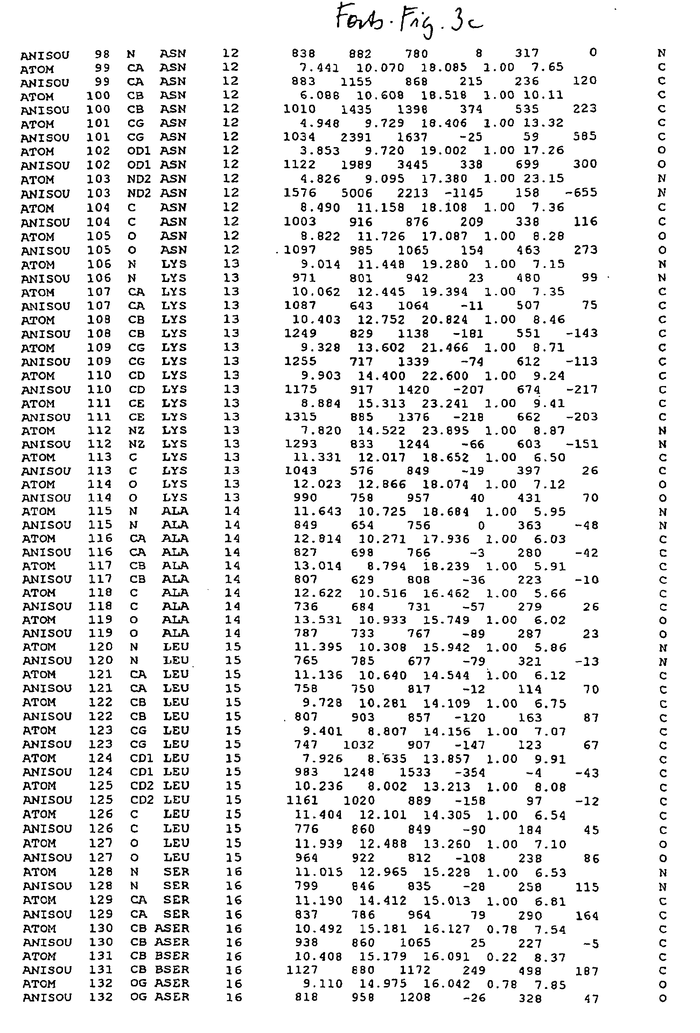

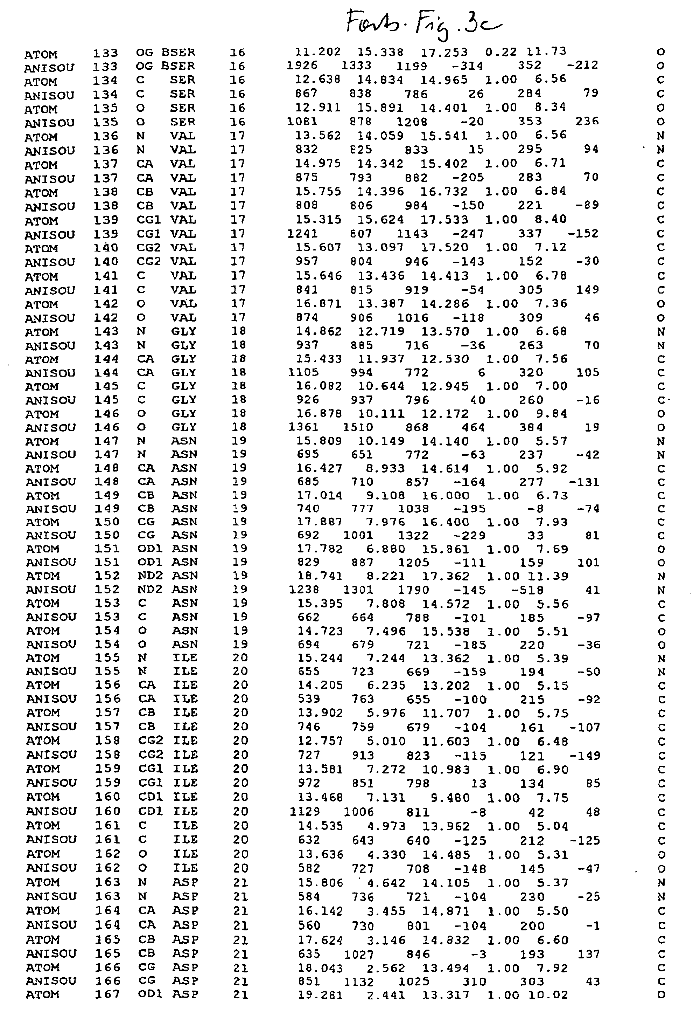

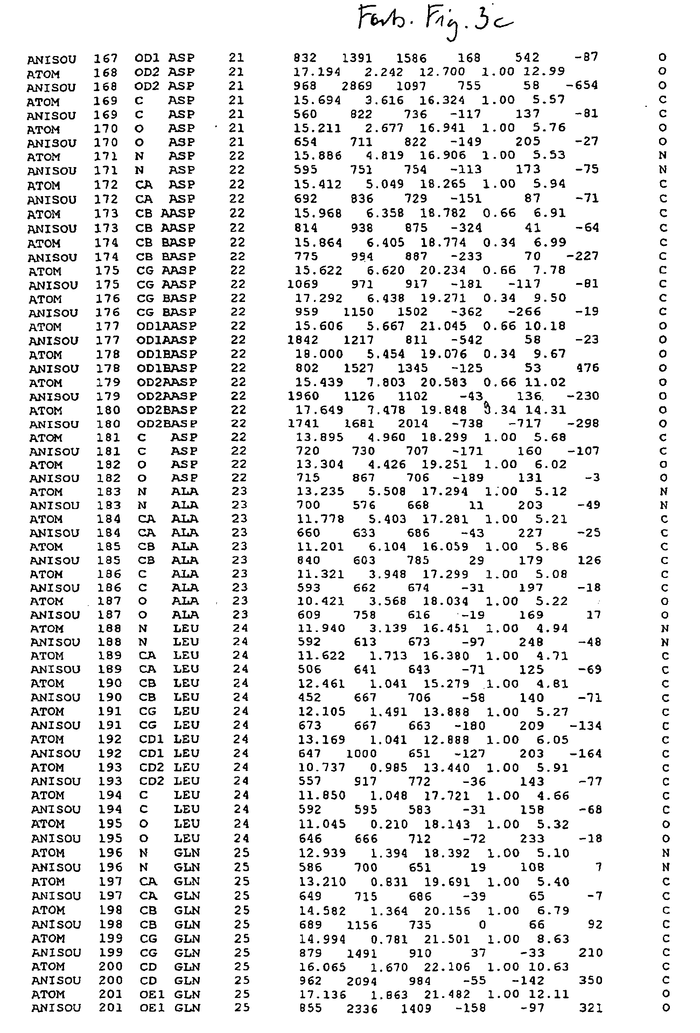

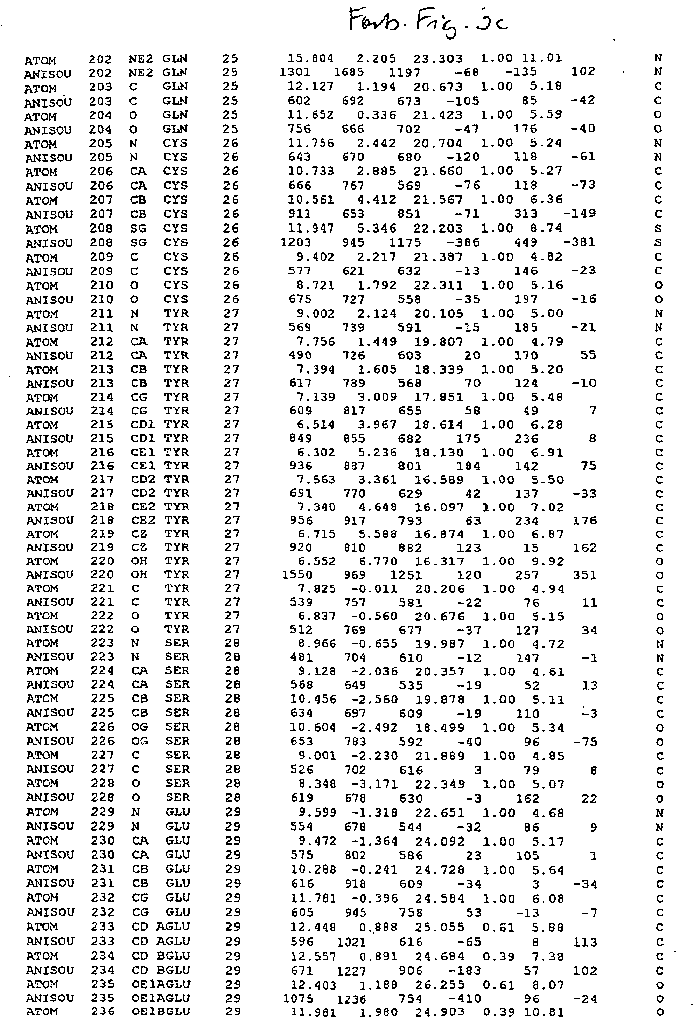

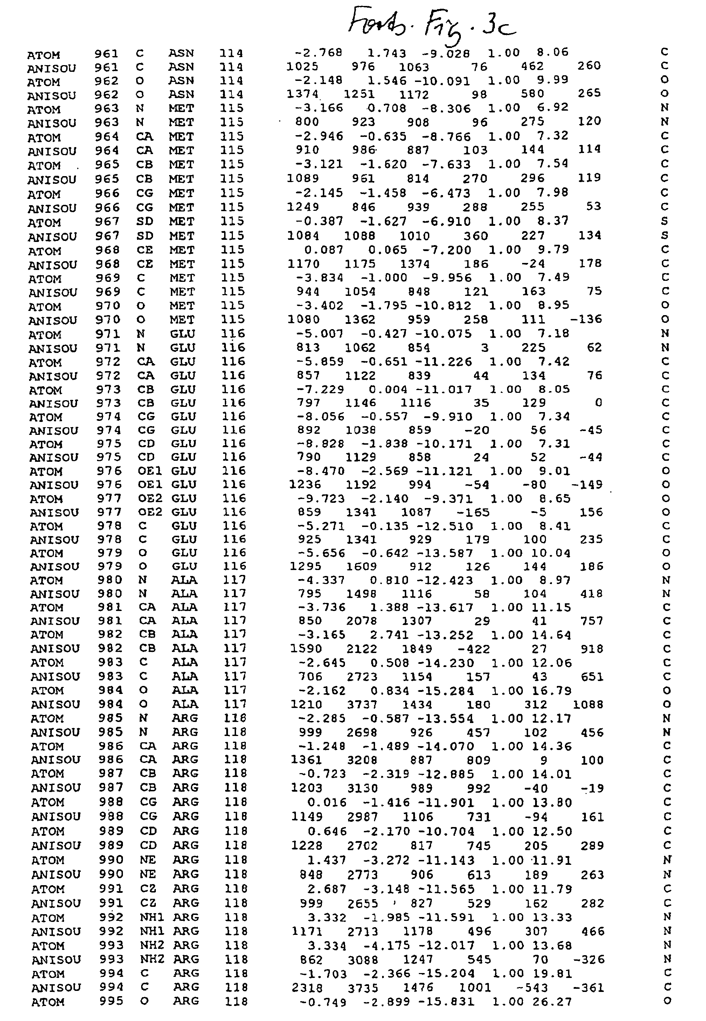

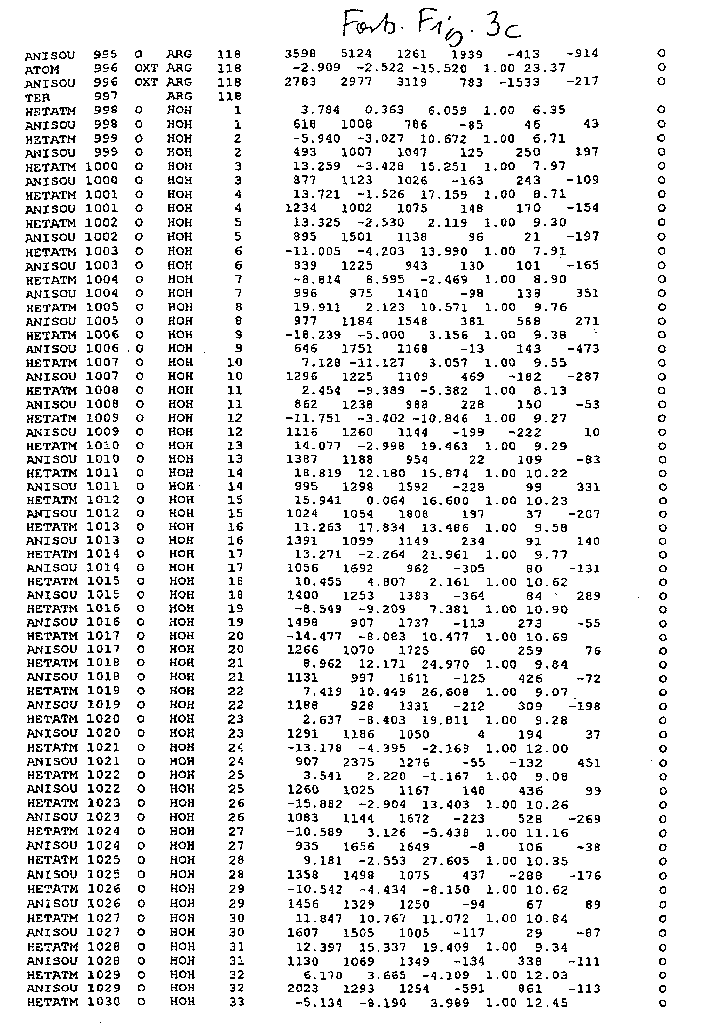

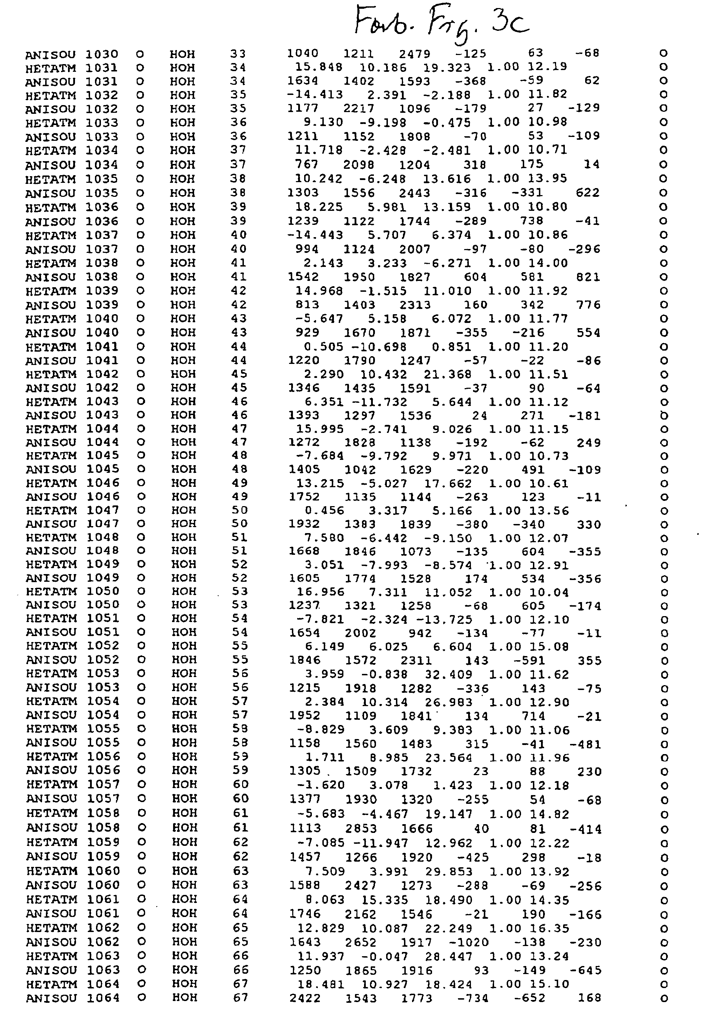

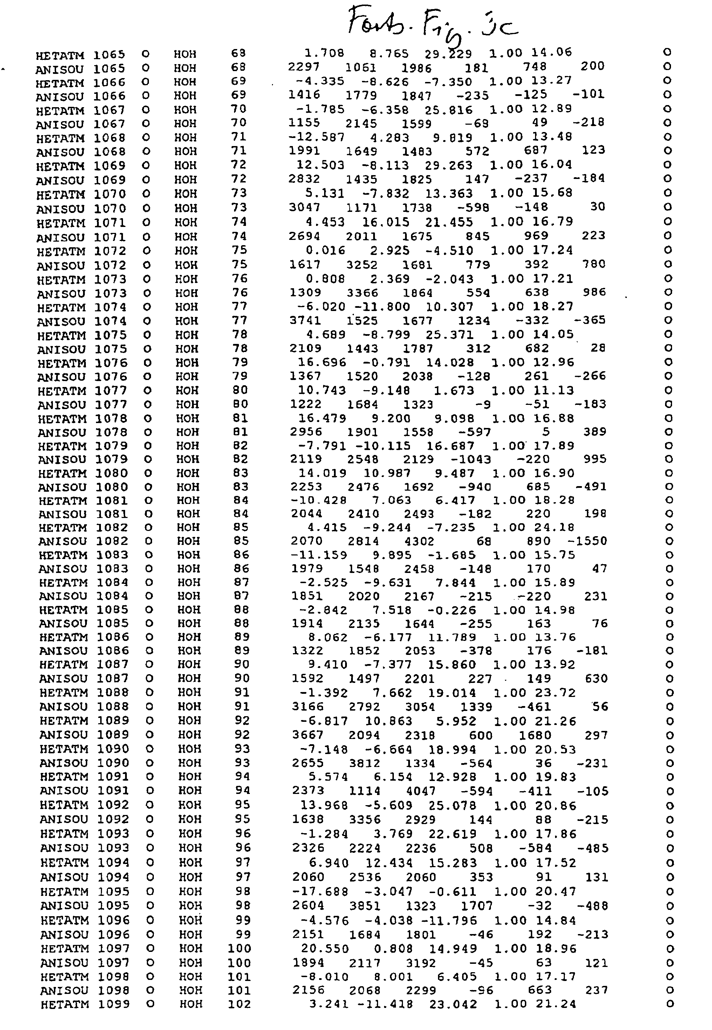

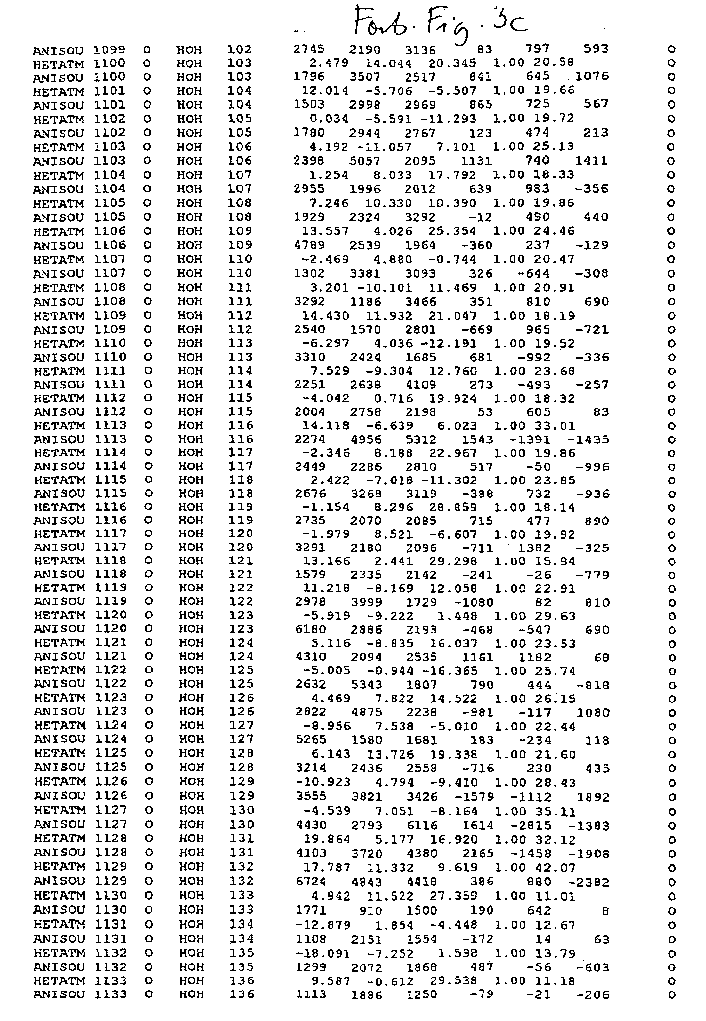

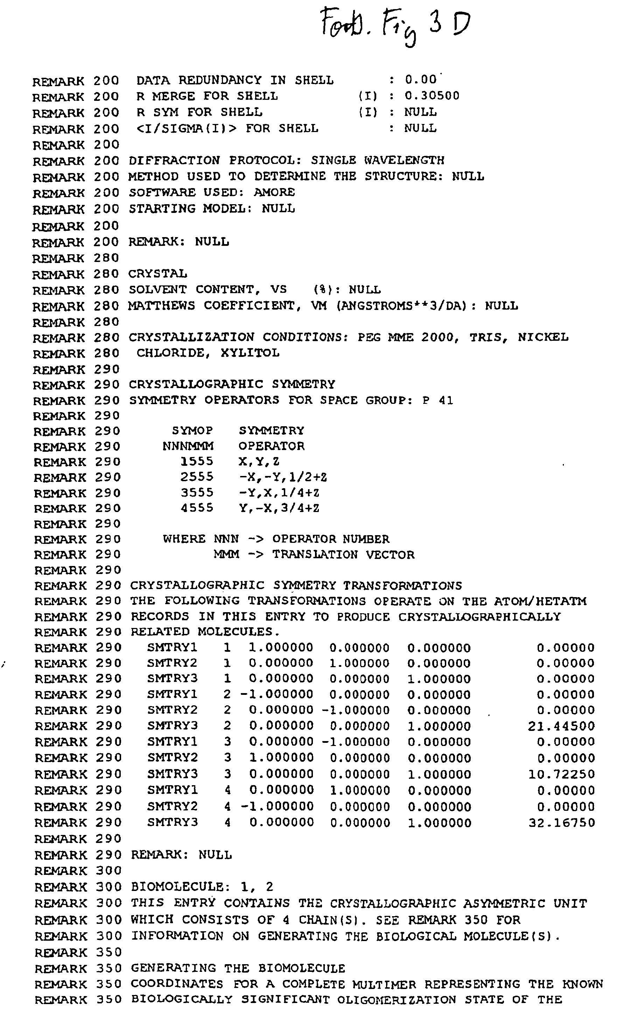

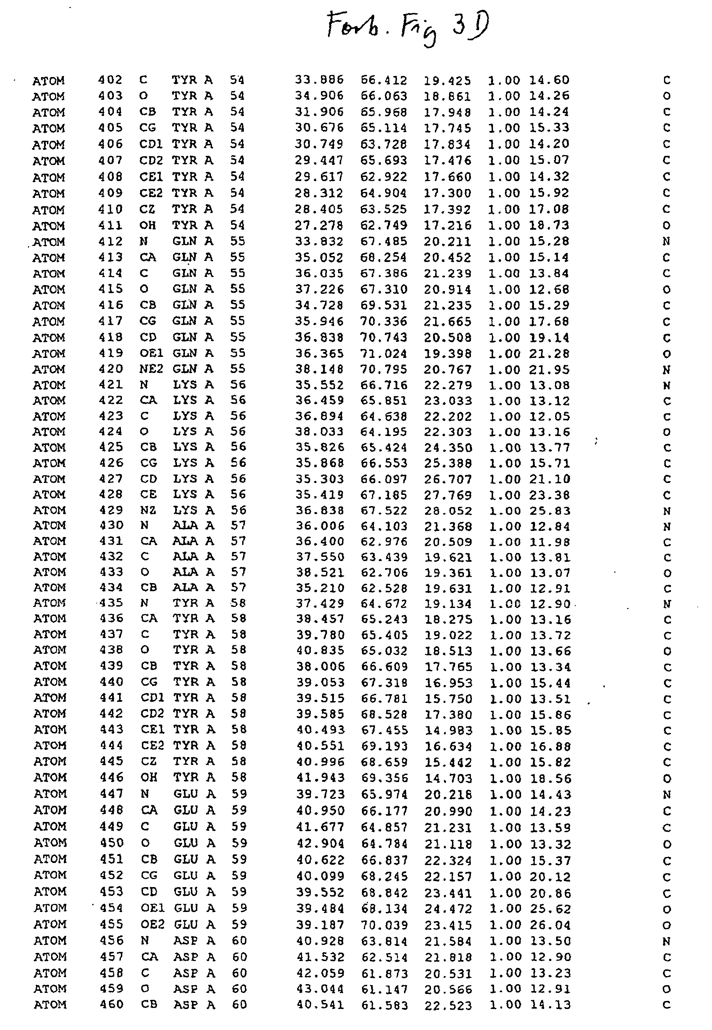

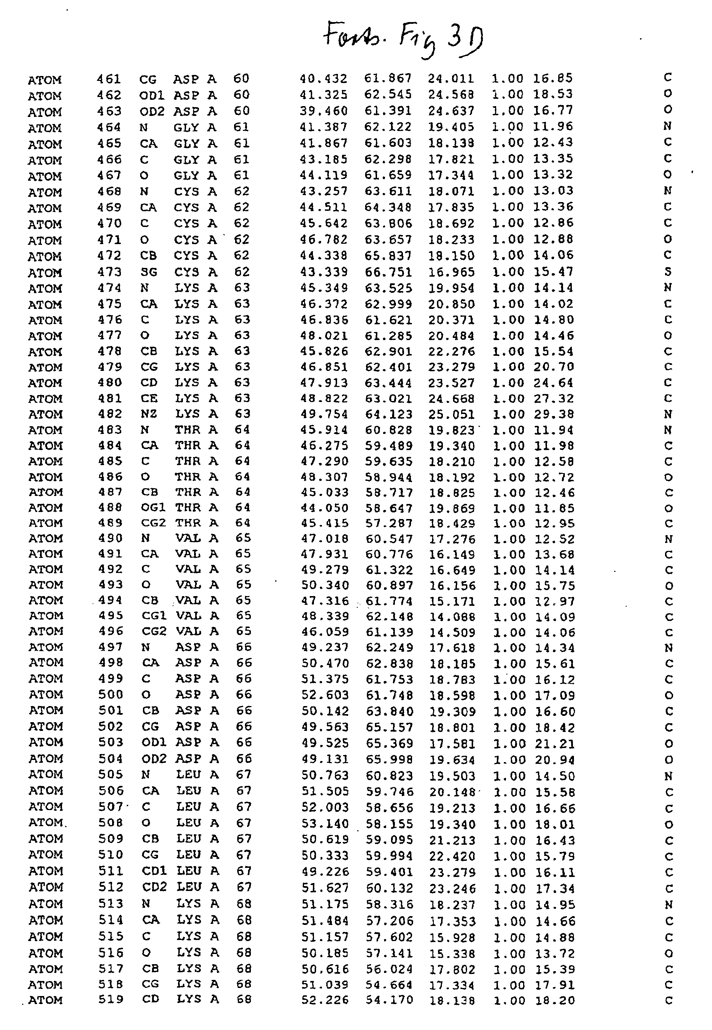

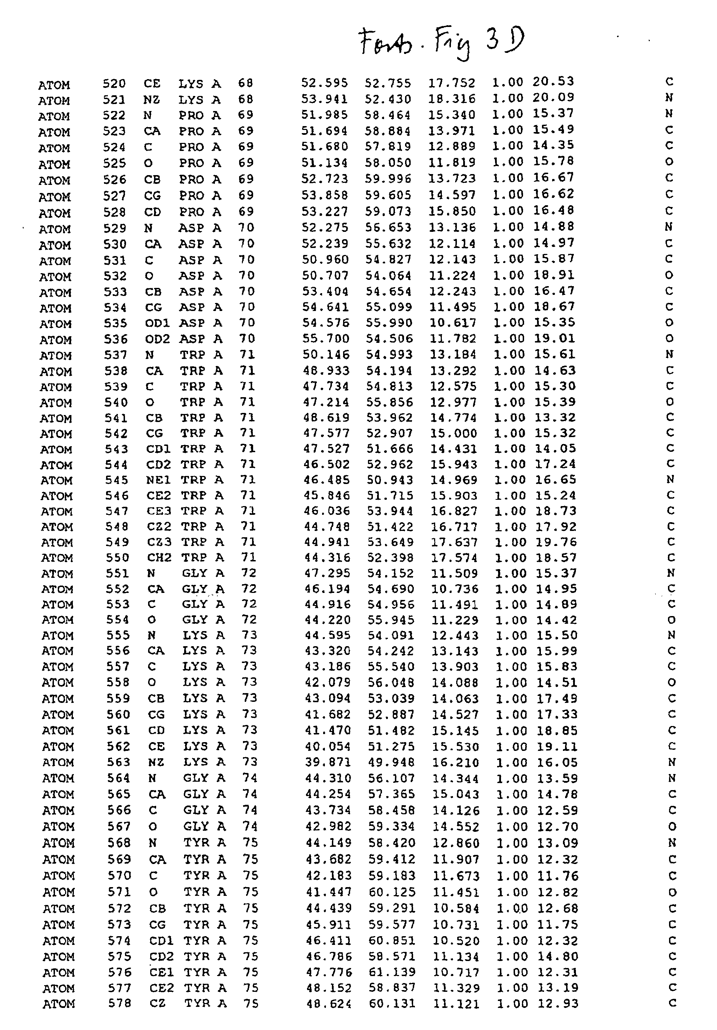

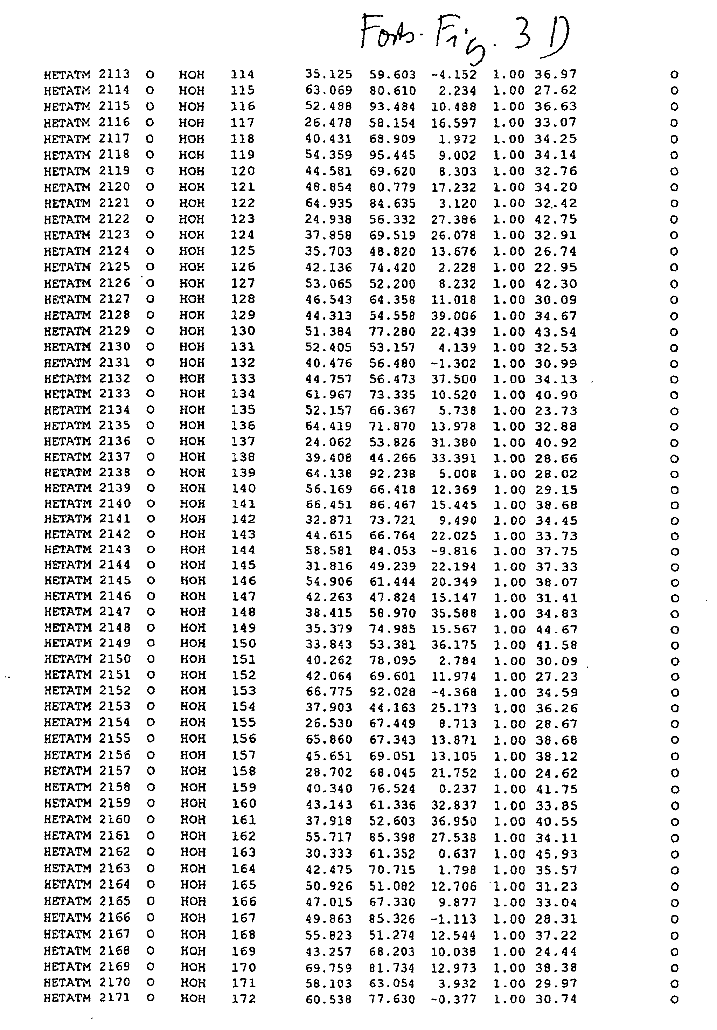

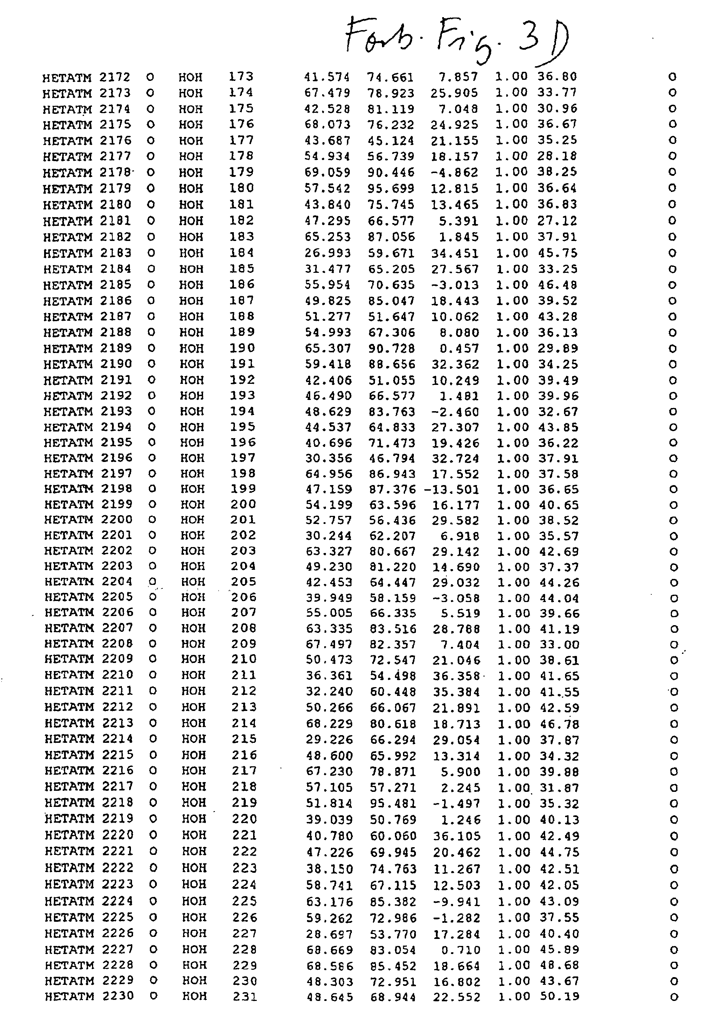

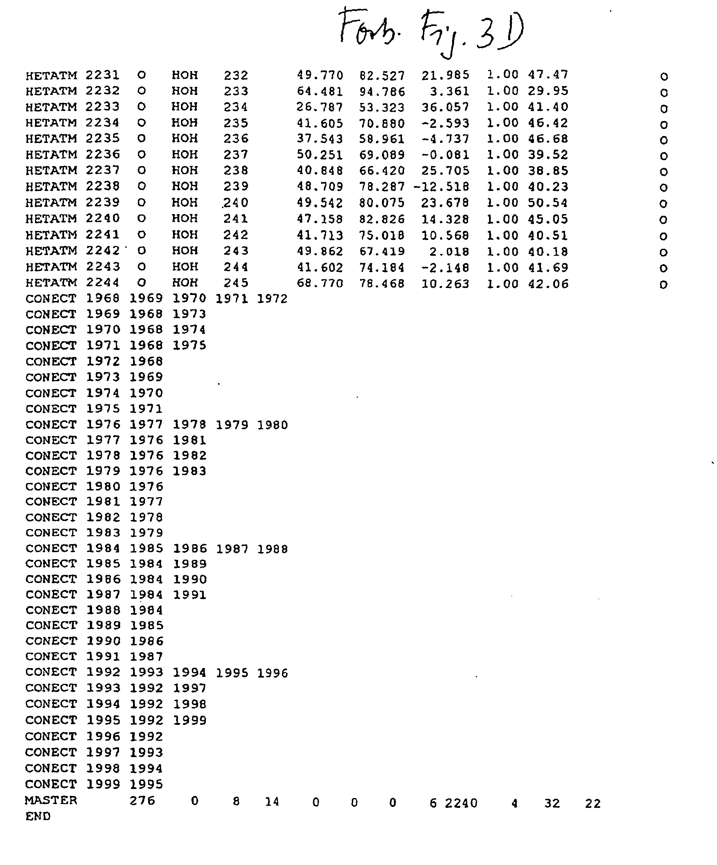

Gemäß Anspruch 2 der vorliegenden Erfindung einer Verfahrensgemäß hergestellter Kristall beruhend auf einer Raumform eines Polypeptids beansprucht, wobei das Poly peptid in der Raumform mindestens eine Aminosäuresequenz eines TPR-Strukturmotivs eines Hop-Proteins oder ein Derivat bzw. ein Fragment einer Aminosäuresequenz eines solchen TPR-Strukturmotivs eines Hop-Proteins enthält. Bei diesen Aminosäuresequenzen eines TPR-Strukturmotivs eines Hop-Proteins kann es sich beispielsweise um die in Figur 3A dargestellten Aminosäuresequenzen, die dort fortlaufend mit (1), (2), (3), (4), (5), (6), (7), (8) und (9) bezeichnet werden, handeln. Derartige Polypeptide können bspw. die vorgenannten Aminosäuresequenzen allein oder in Kombination, z.B. als Kombination der Sequenzen (1), (4) und (7), oder in jeder anderen Kombination von 2, 4, 5, 6, 7, 8 oder 9 der vorgenannten Sequenzen, beispielsweise (2) mit (6) oder (3) mit (4), oder auch als Kombination aller neun vorgenannten Aminosäuresequenzen isoliert oder eingebettet bzw. verbunden durch andere ("Linker")-Aminosäuresequenzen in beliebiger Reihenfolge aufweisen.

Vörzugsweise wird der Kristall Polypeptide in einer Raumform aufweisen, wobei mindestens eine der Aminosäuresequenzen der TPR-Domänen TPR1, TPR2A und/oder TPR2B eines Hop-Proteins, eines Abschnitts/e oder Derivats/e der vorgenannten TPR-Domänen enthalten. Dies bedeutet, daß das Polypeptid in der Raumform mehrere TPR-Strukturmotive in Form der physiologischen-TPR-Domänen eines Hop-Proteins enthalten kann. Bei der TPR-Domäne kann es sich aber auch um nicht-physiologisch auftretende Kombinationen von TPR-Strukturmotiven handeln. Beispielsweise fallen hierunter auch Raumformen von Polypeptiden, die mindestens eine TPR-Domäne enthalten, wobei die mindestens eine TPR-Domäne aus beispielsweise drei TPR-Strukturmotiven in artifizieller Kombination aufgebaut sein kann. Hierbei kann es sich entweder um Kombinationen von bereits zuvor genannten, in Figur 3A wiedergegebenen Hop-TPR-Strukturmotiven aus verschiedenen TPR-Domänen von Hop-Protein oder aber auch um eine beliebige Kombination von Hop-TPR-Strukturmotiven mit TPR-Strukturmotiven anderer TPR-Domänen enthaltender Proteine, insbesondere von TPR-Domänen enthaltenden Co-Chaperons, handeln.

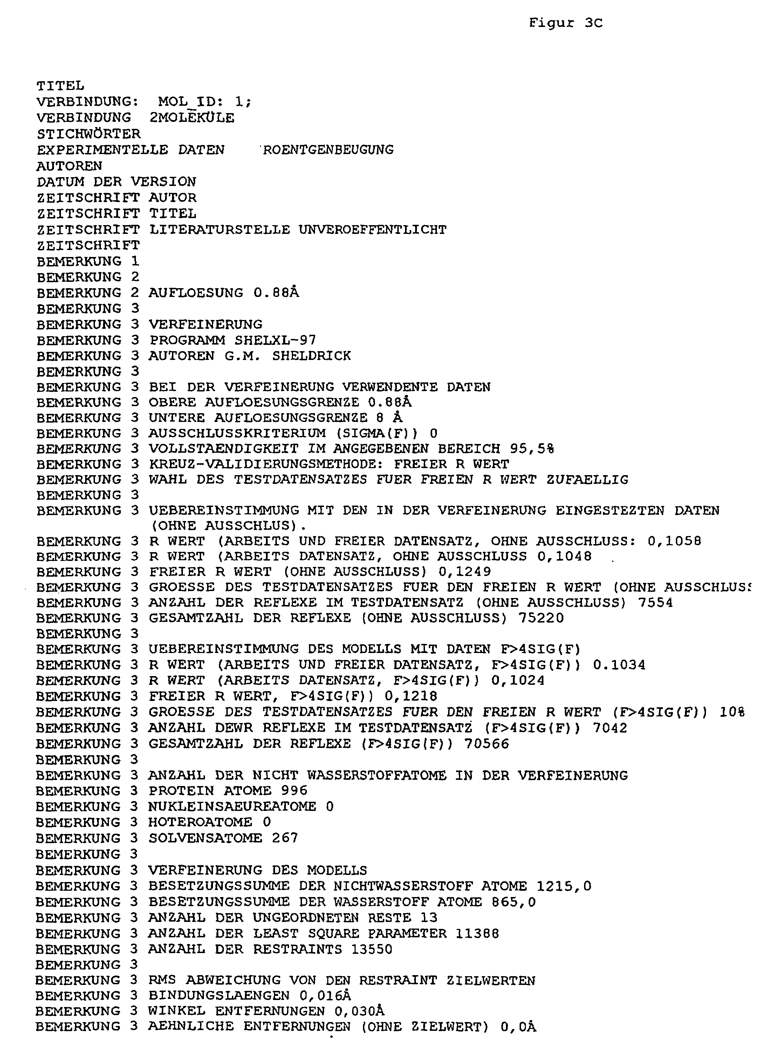

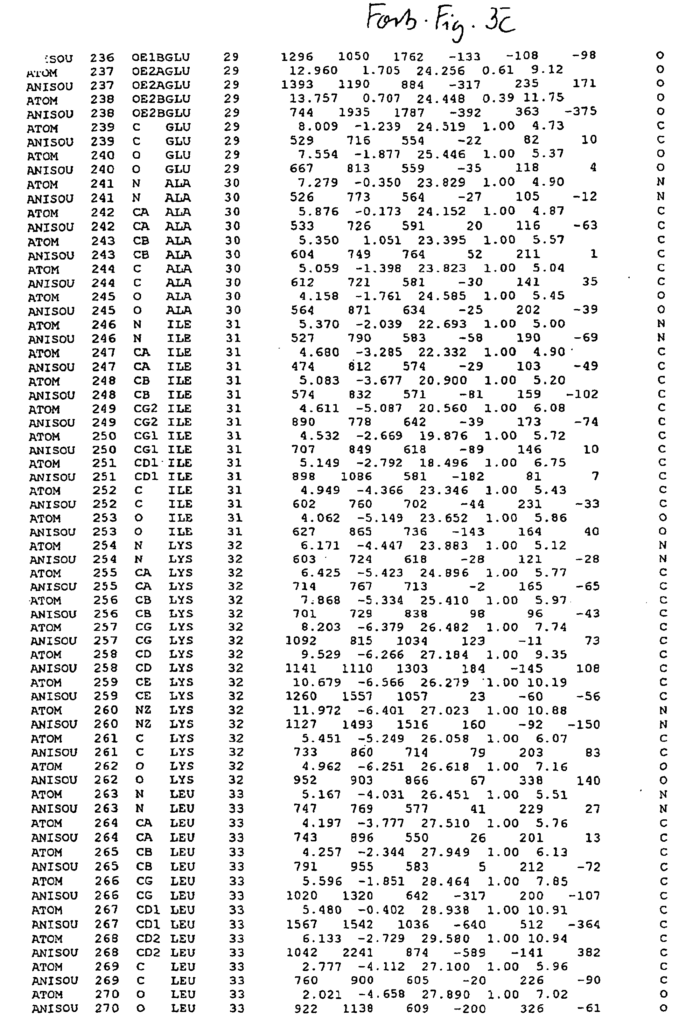

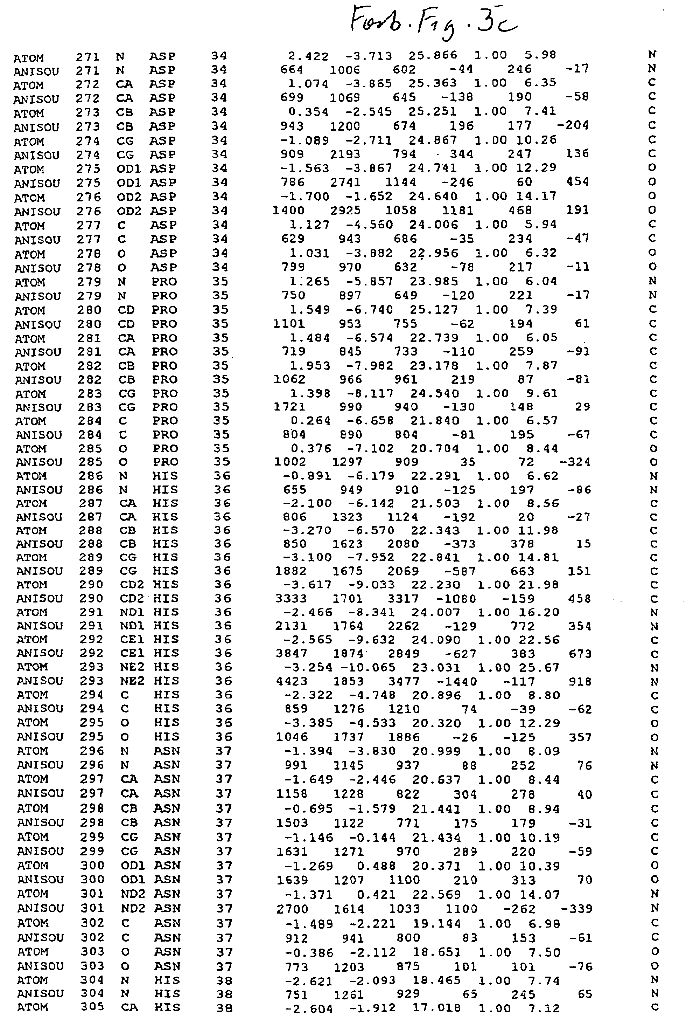

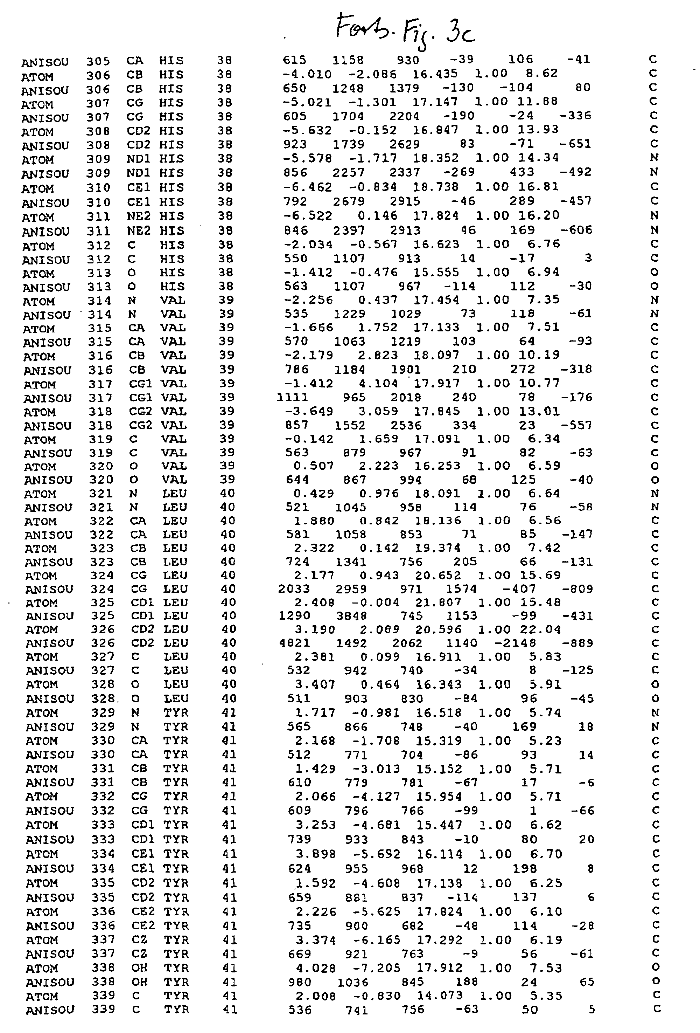

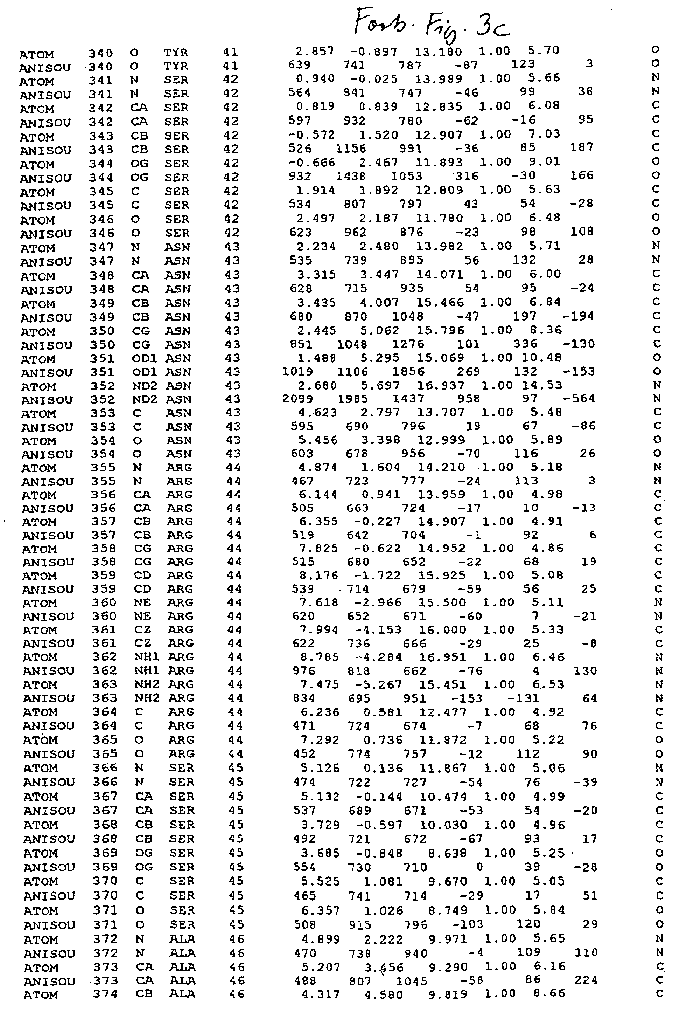

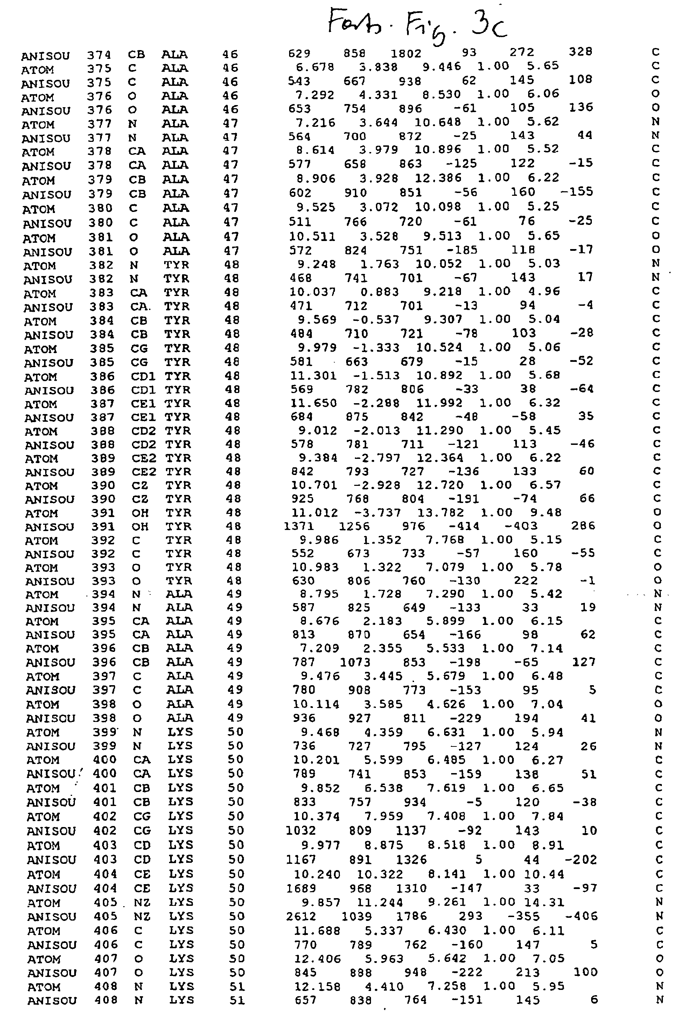

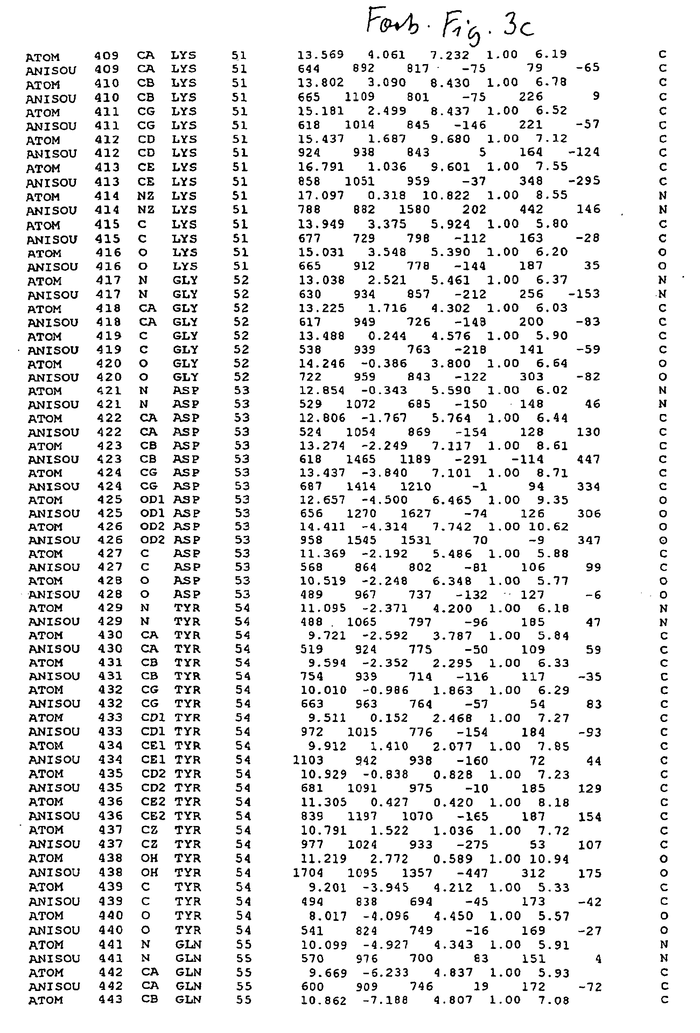

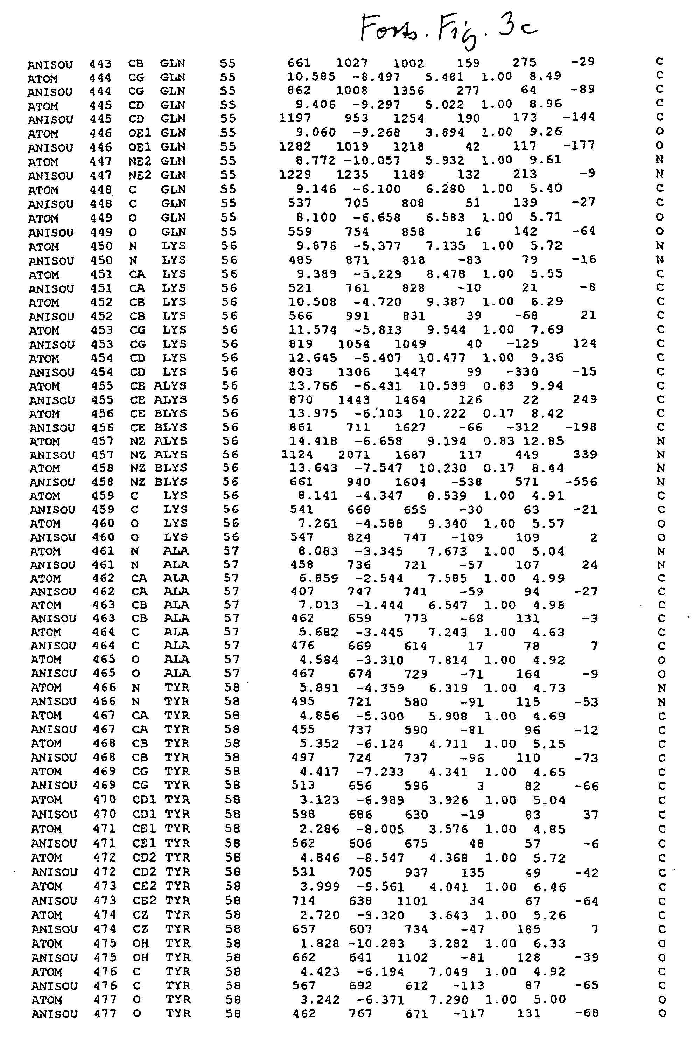

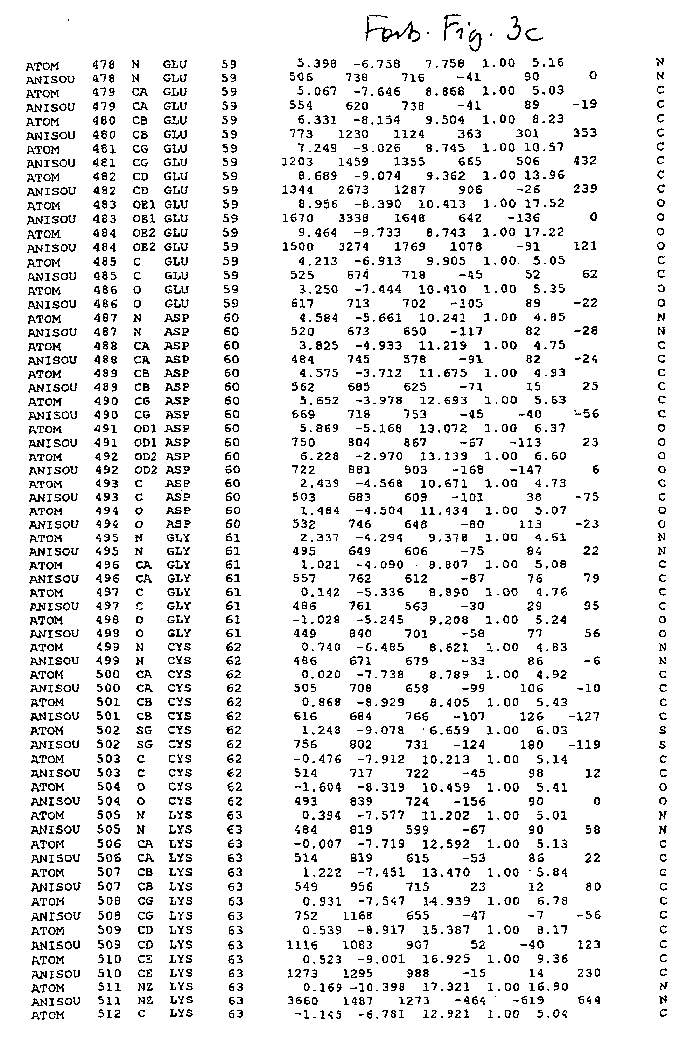

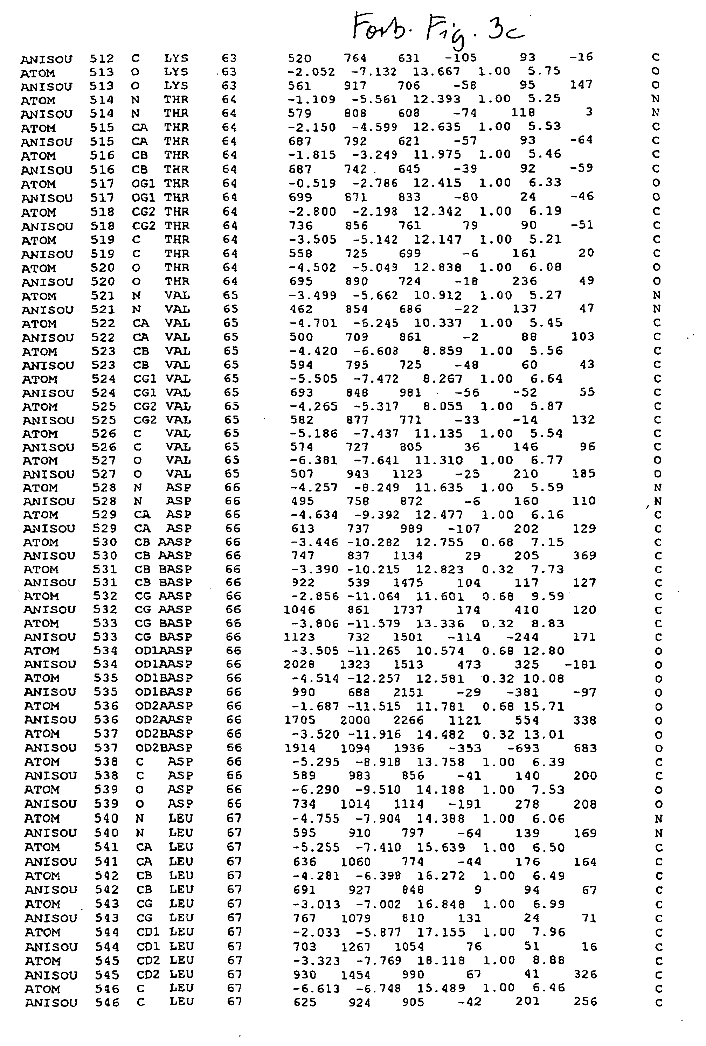

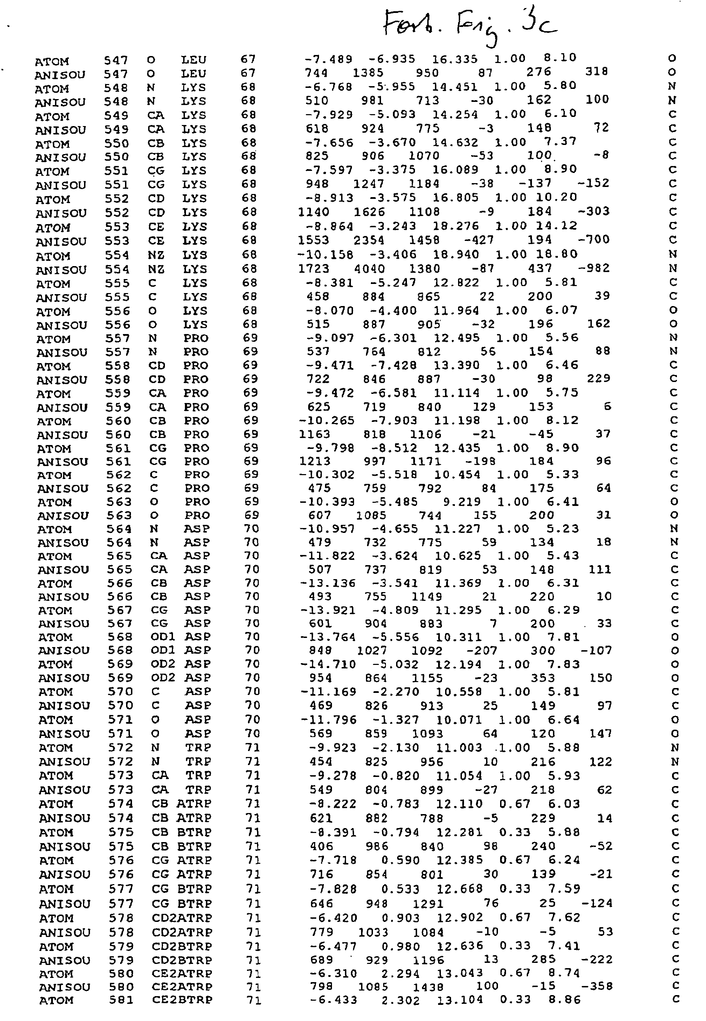

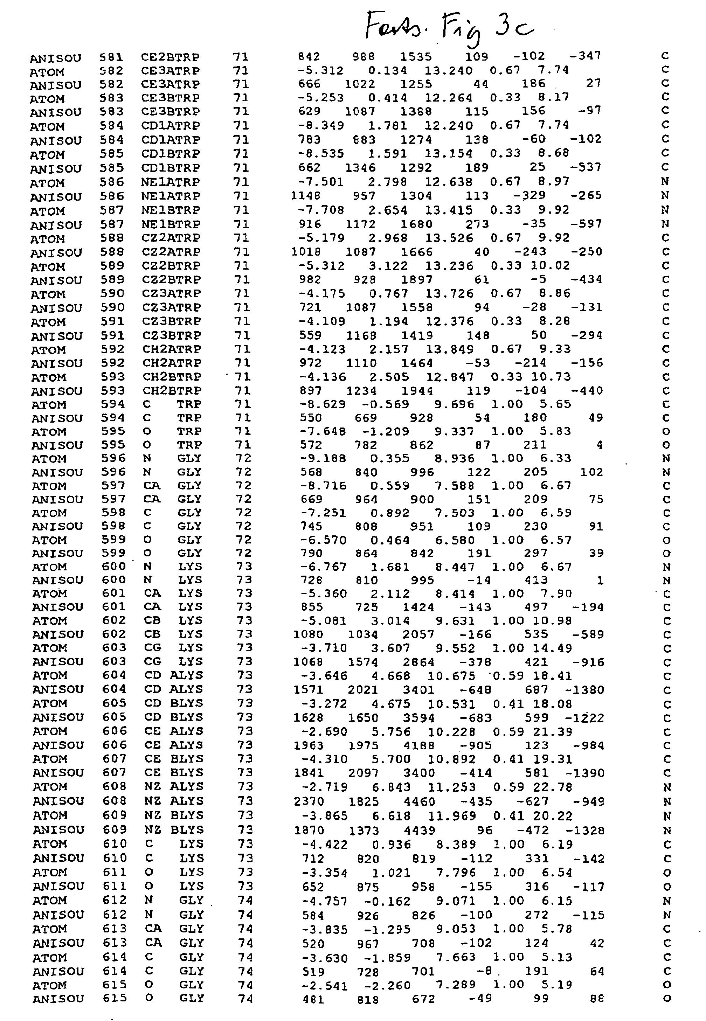

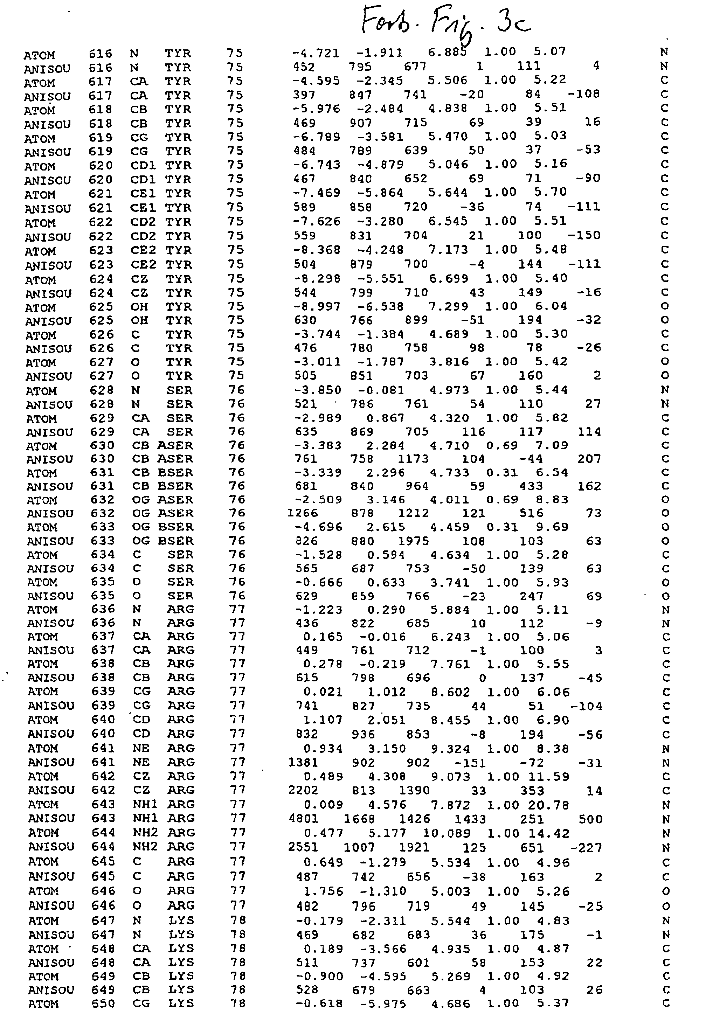

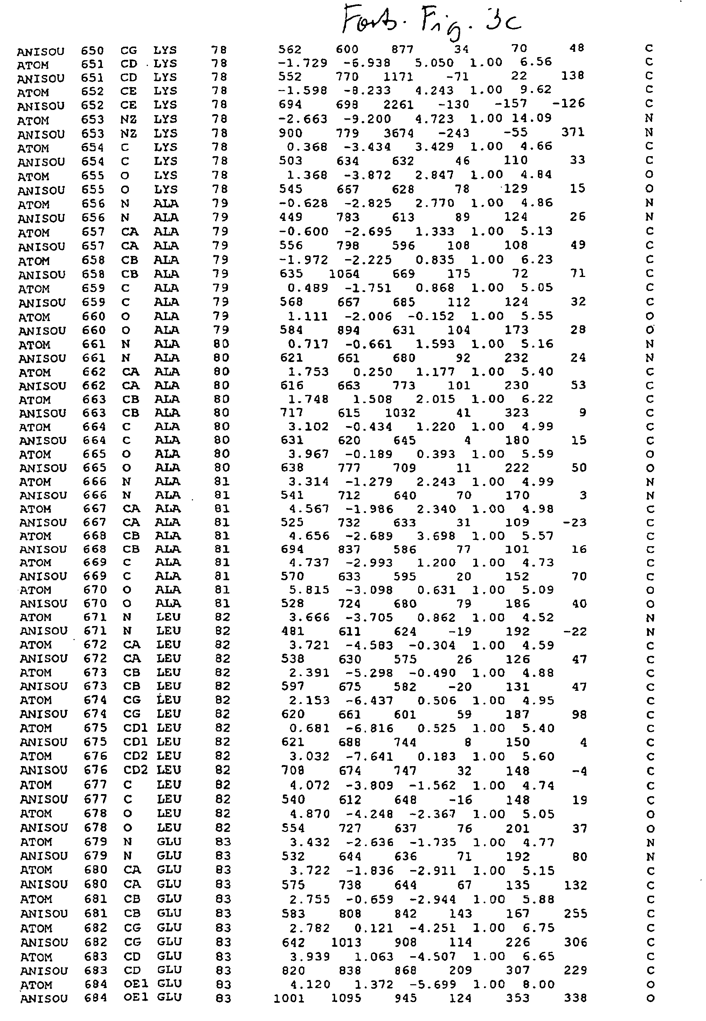

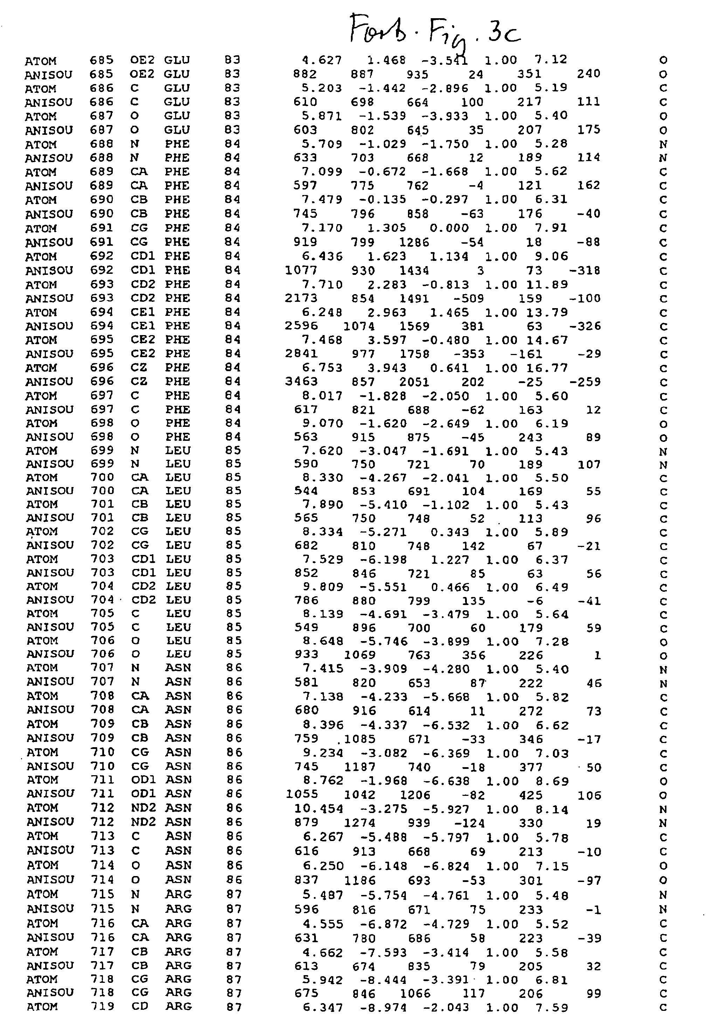

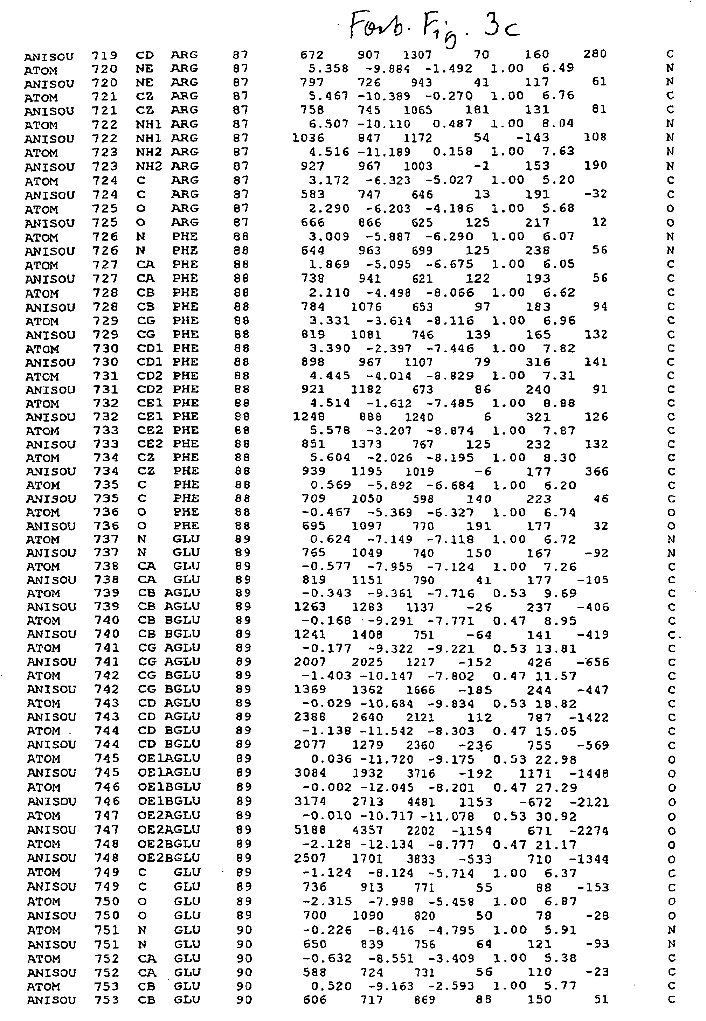

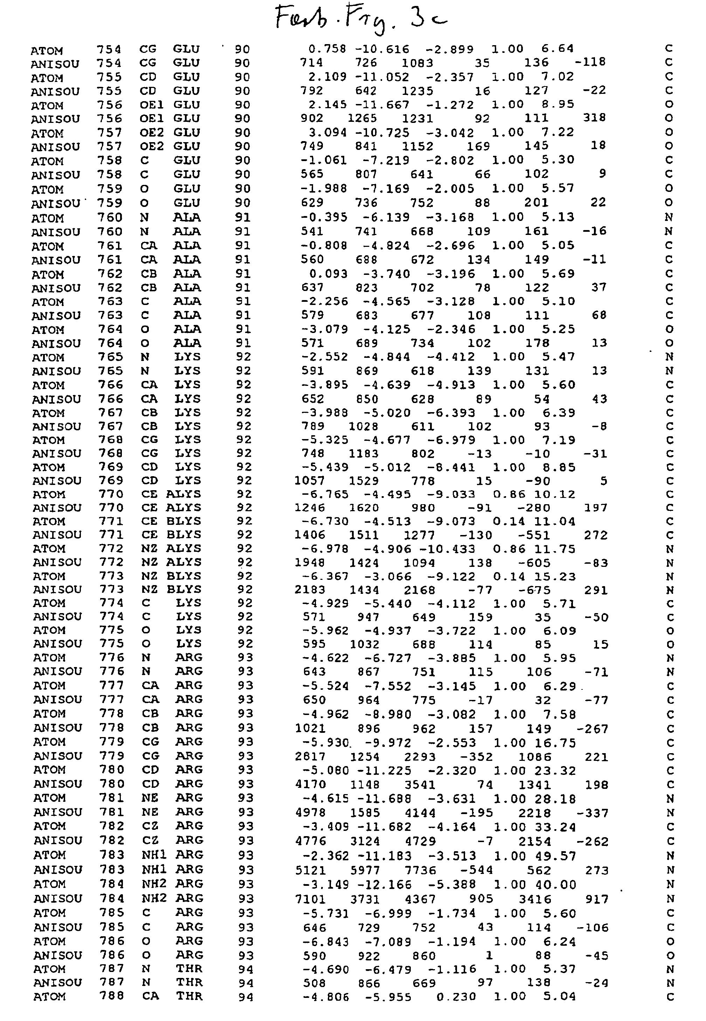

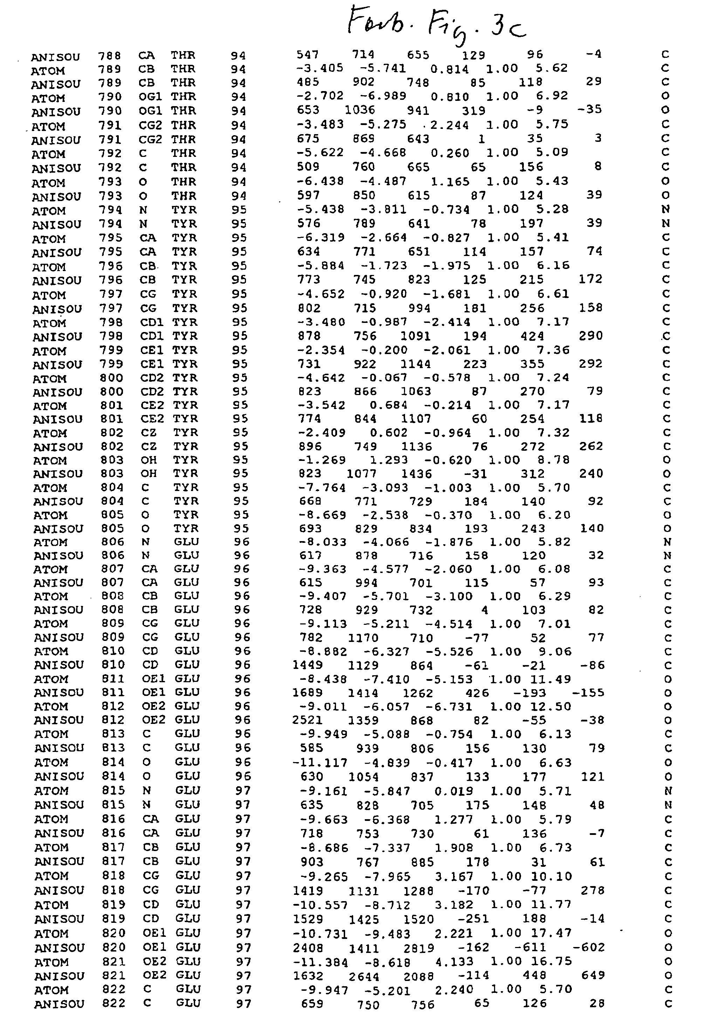

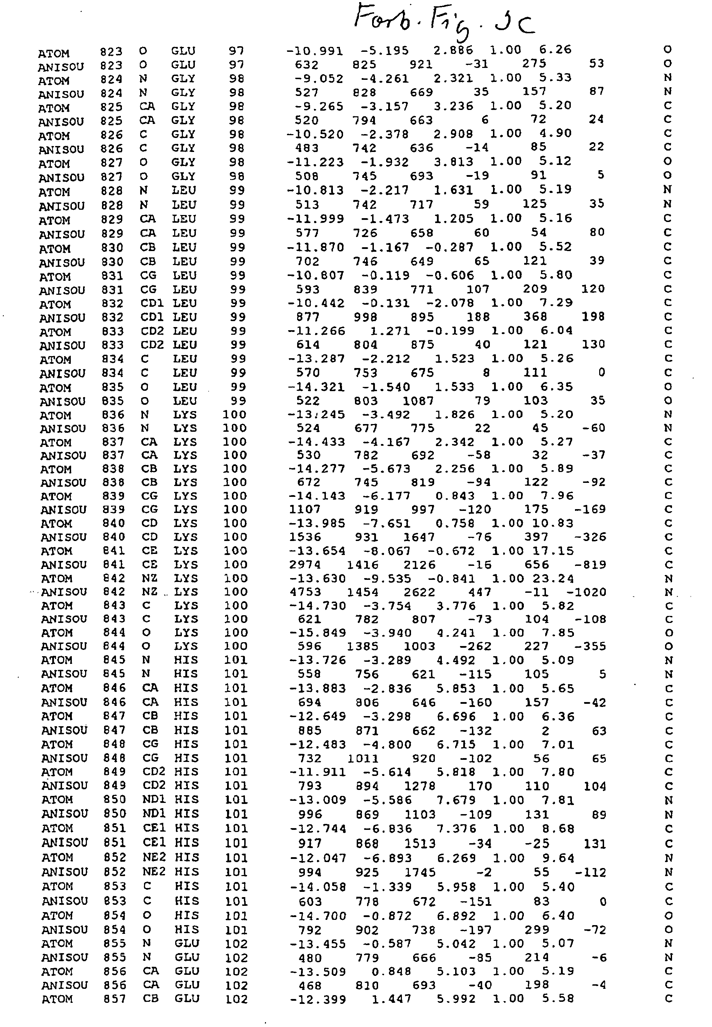

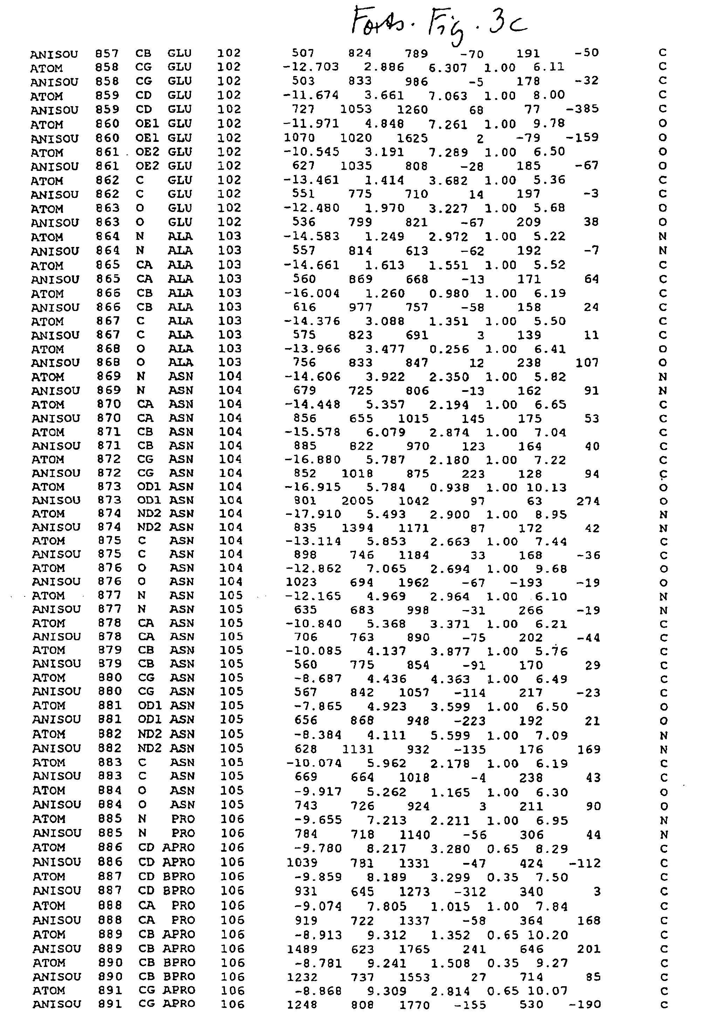

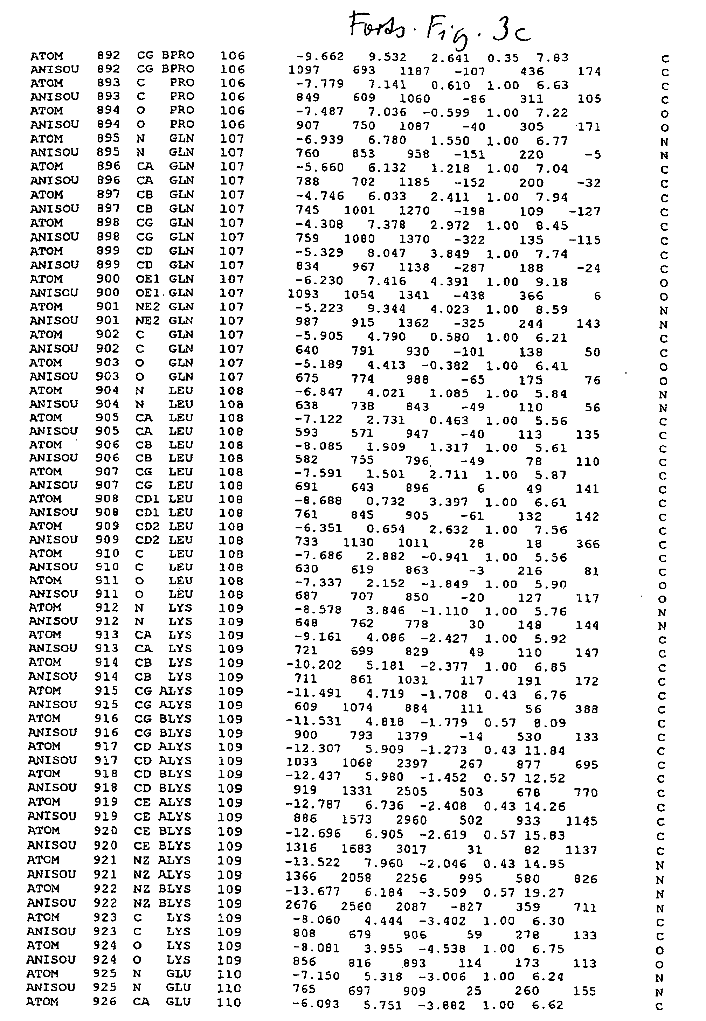

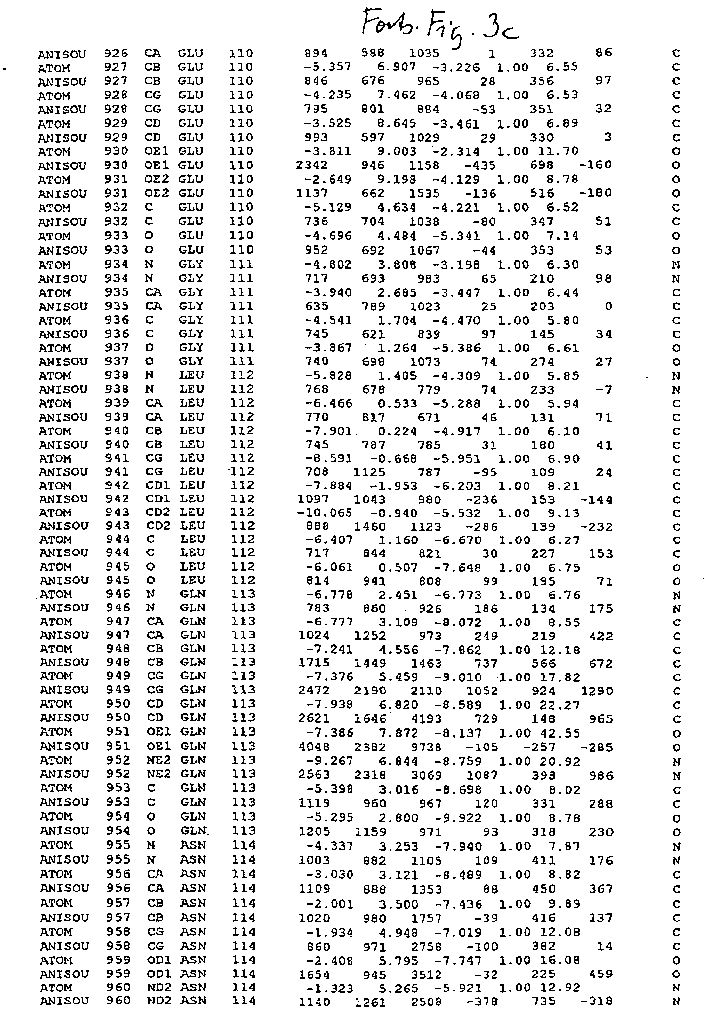

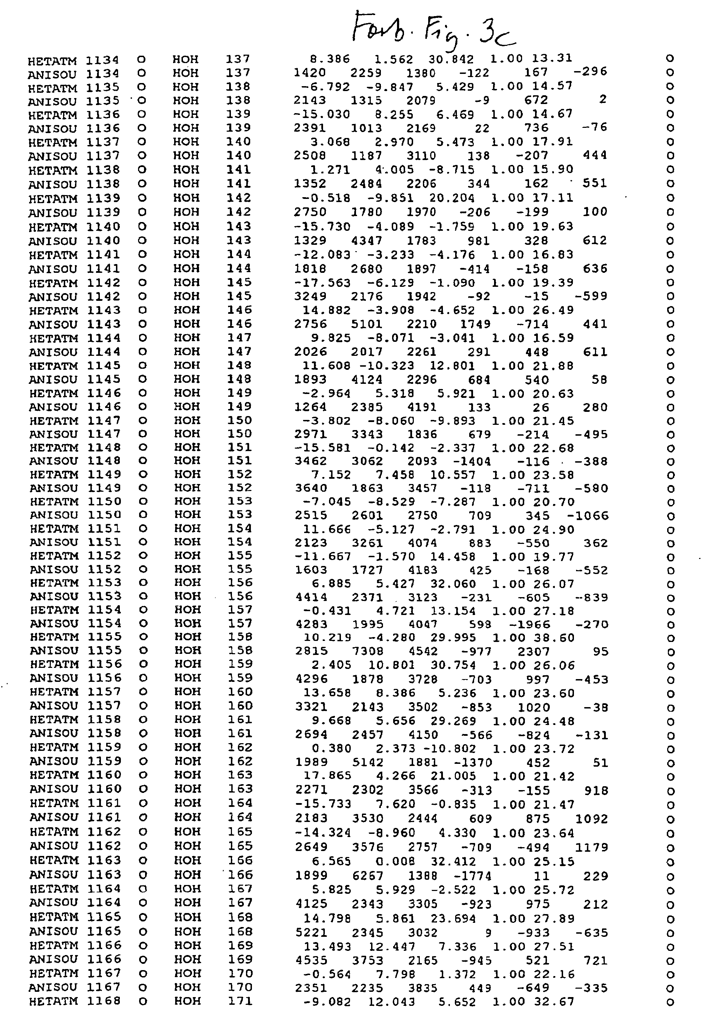

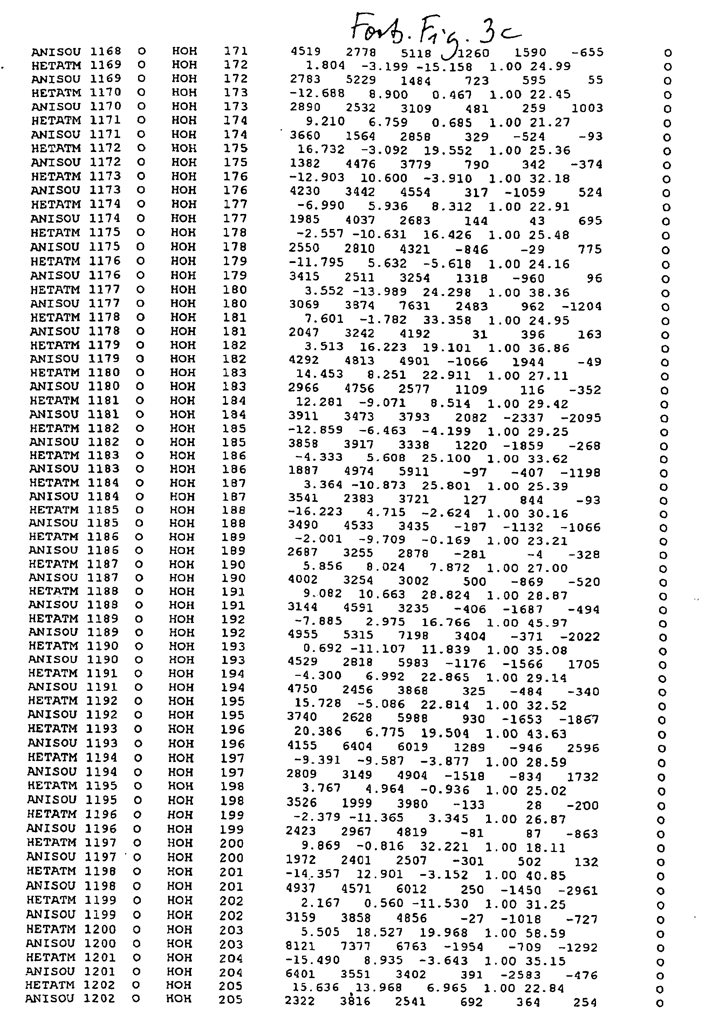

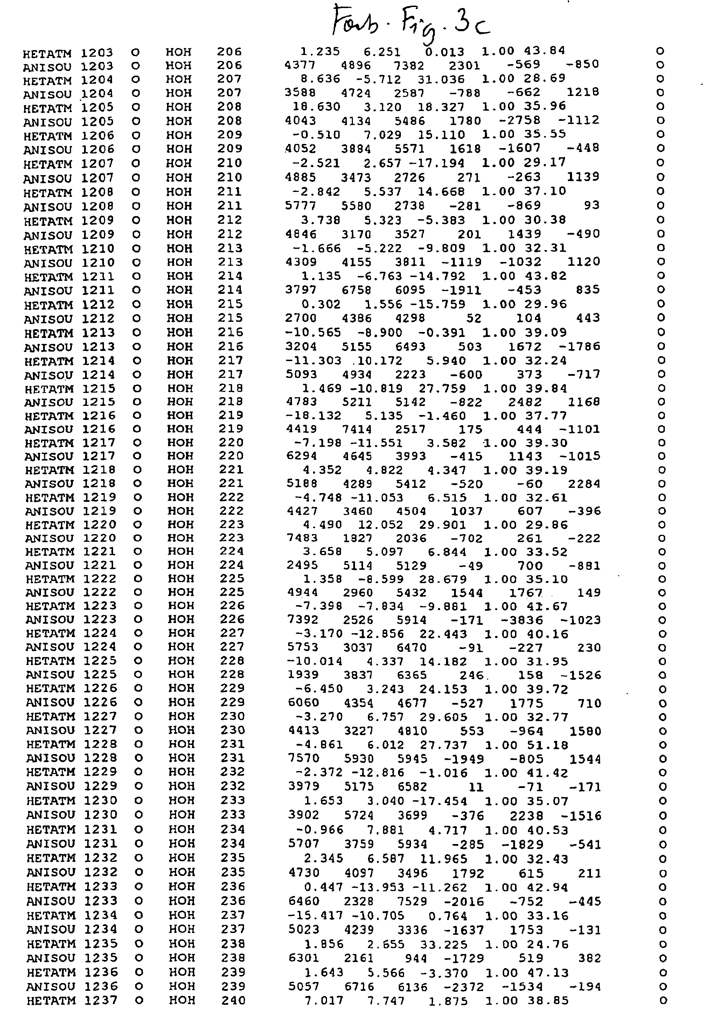

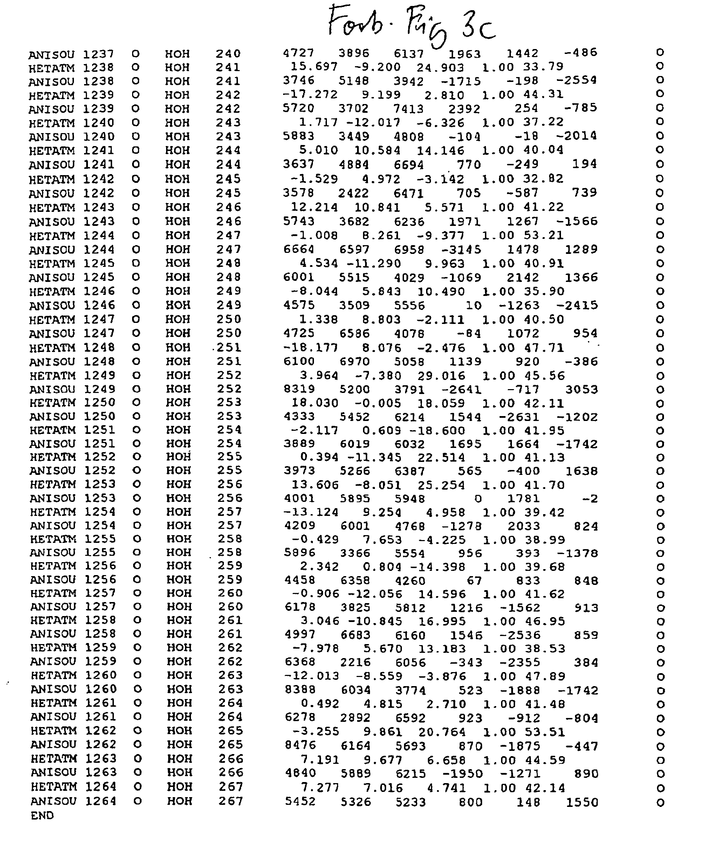

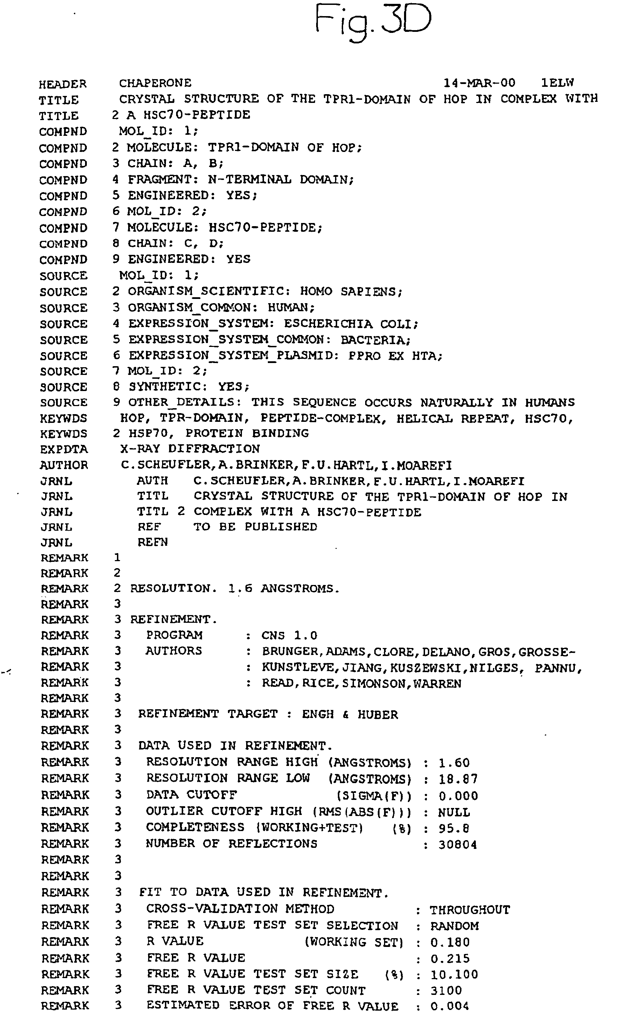

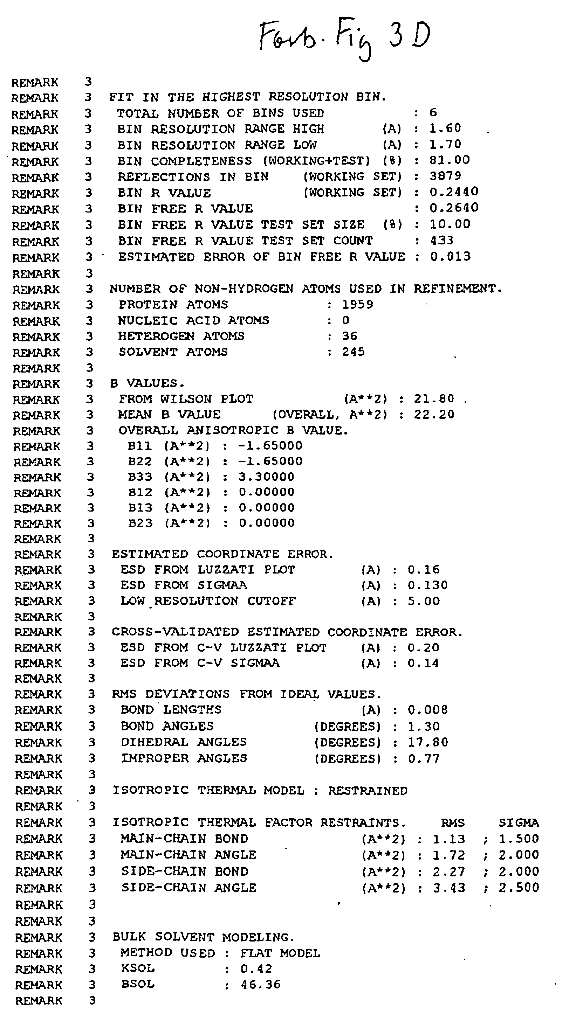

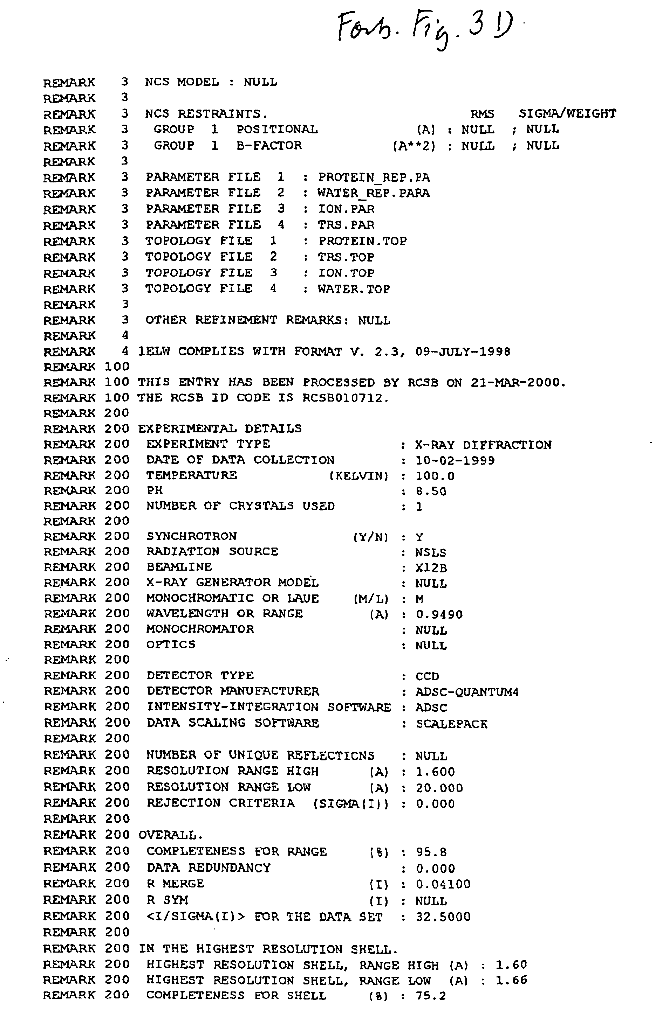

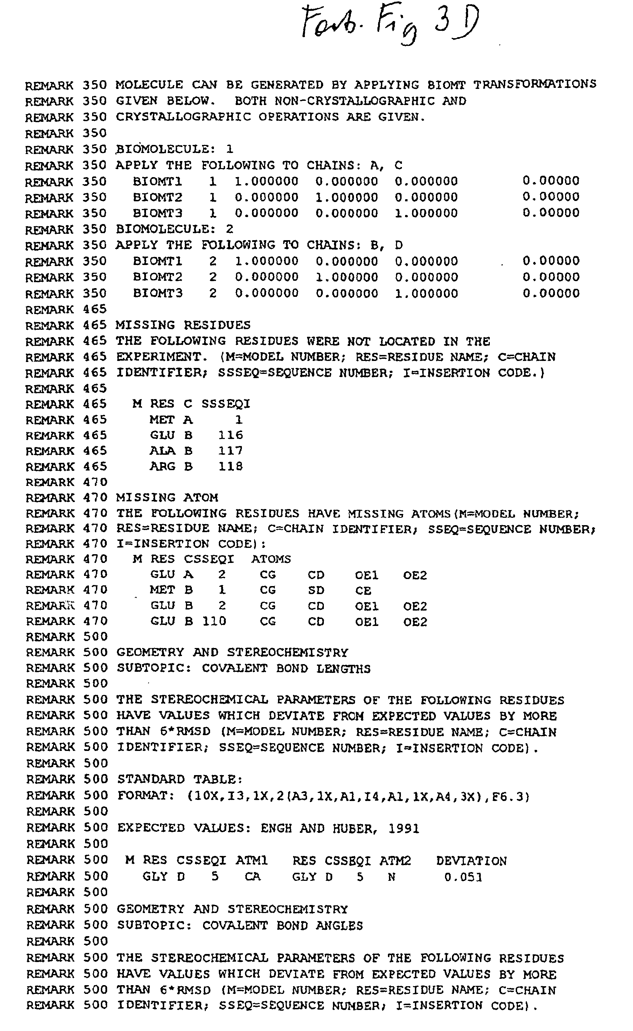

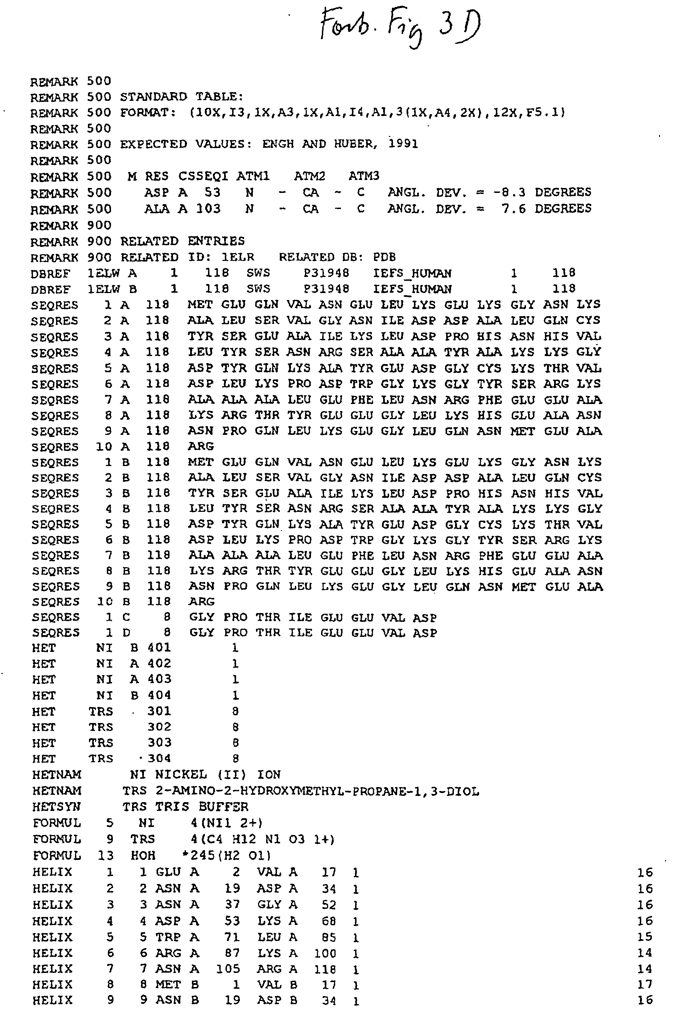

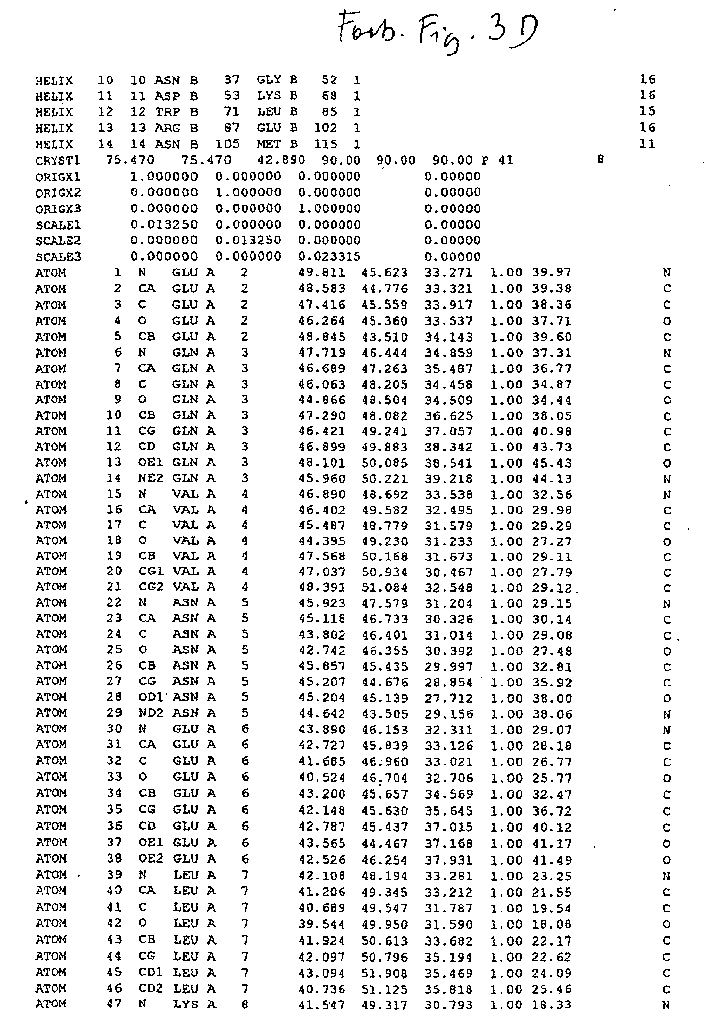

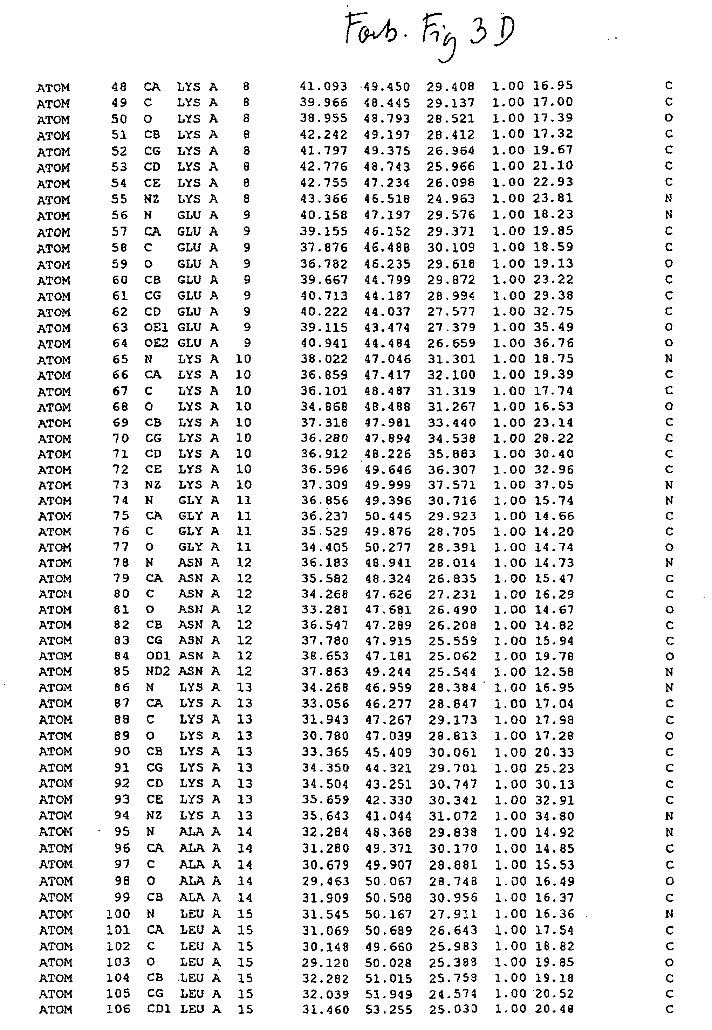

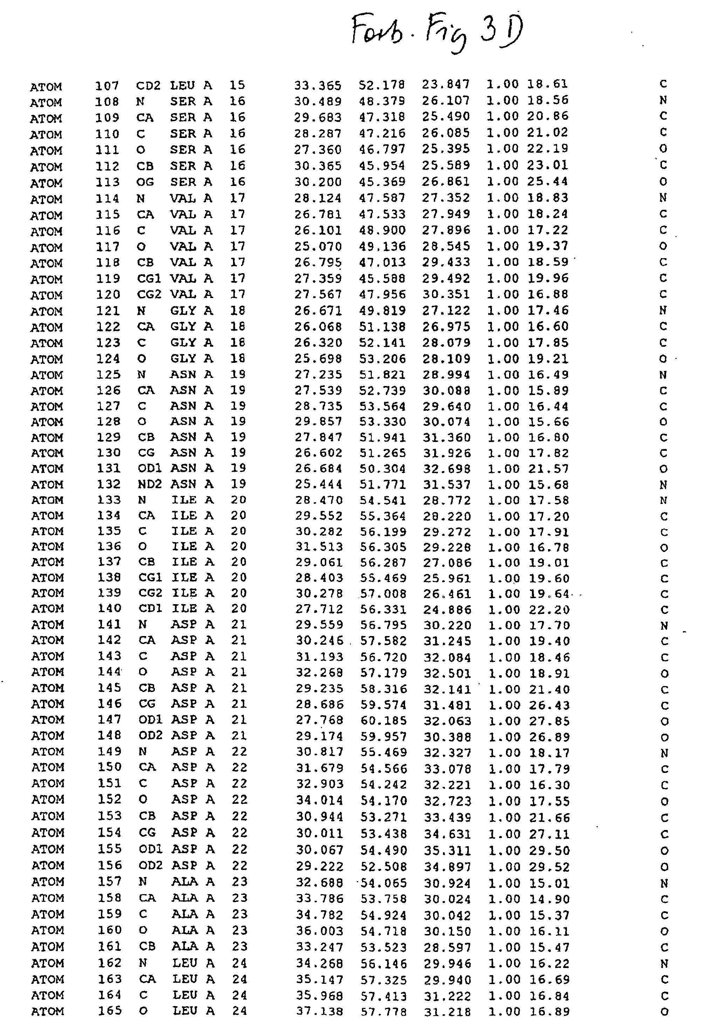

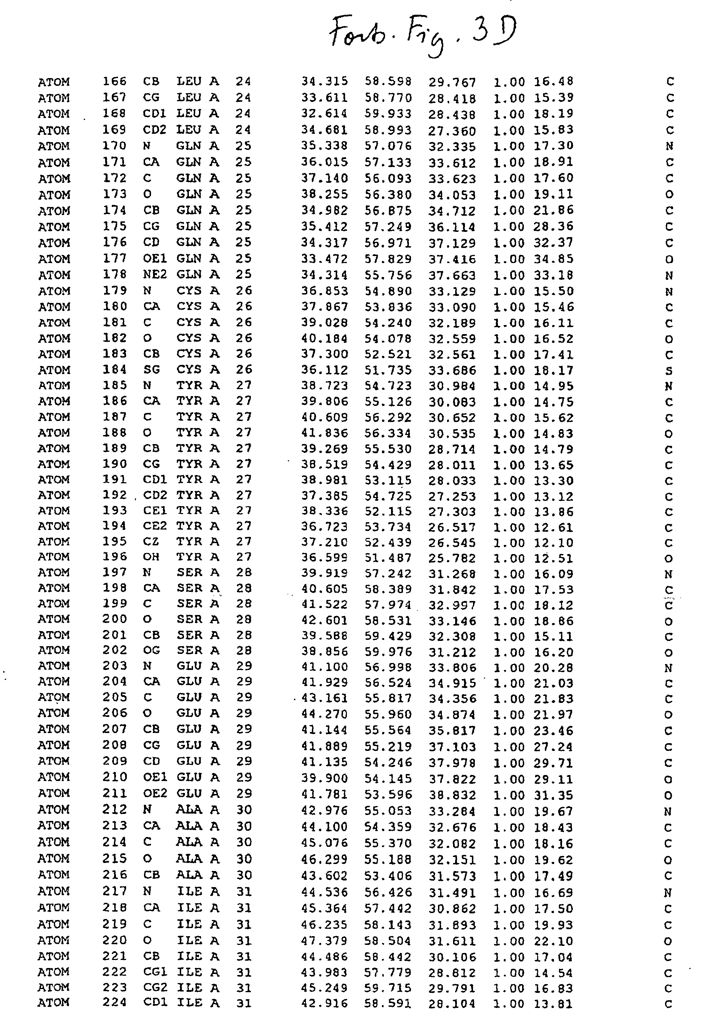

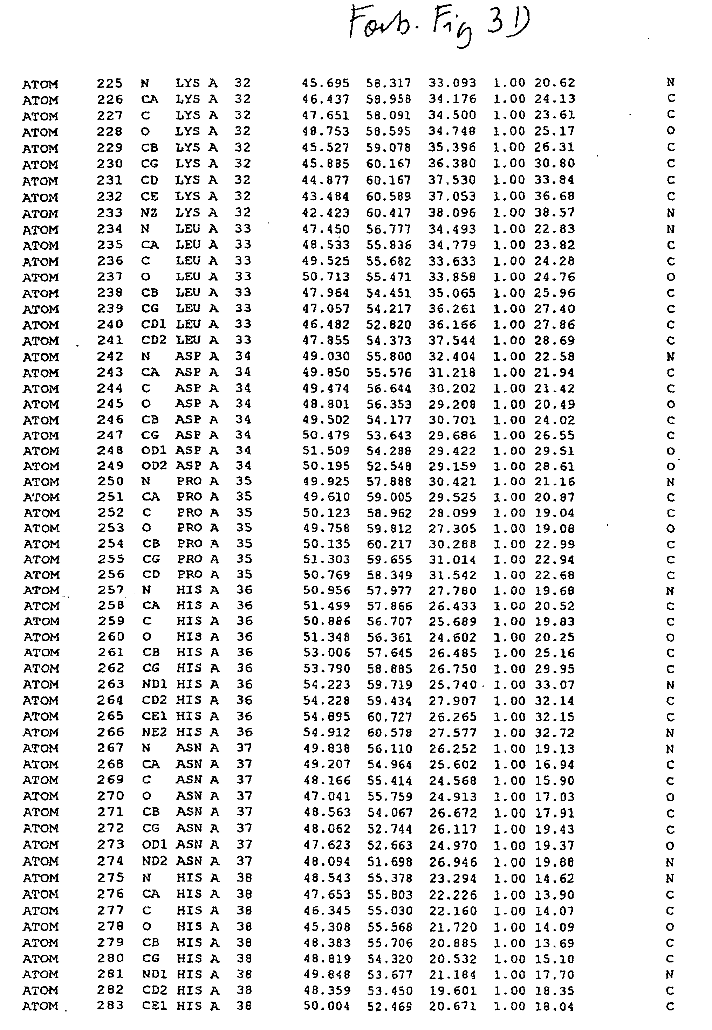

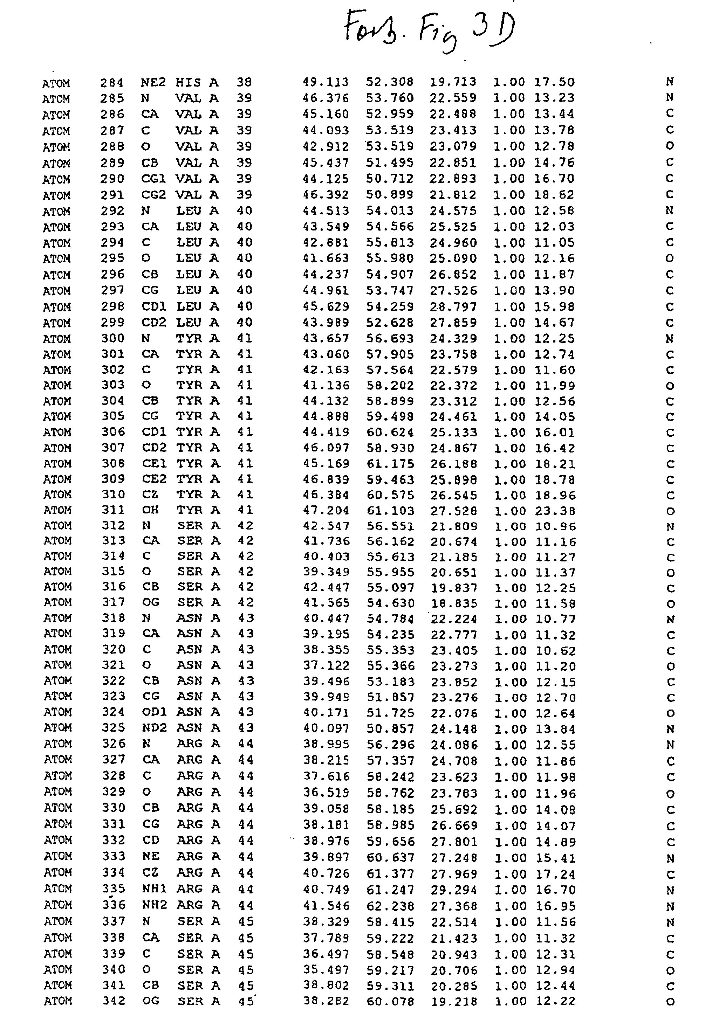

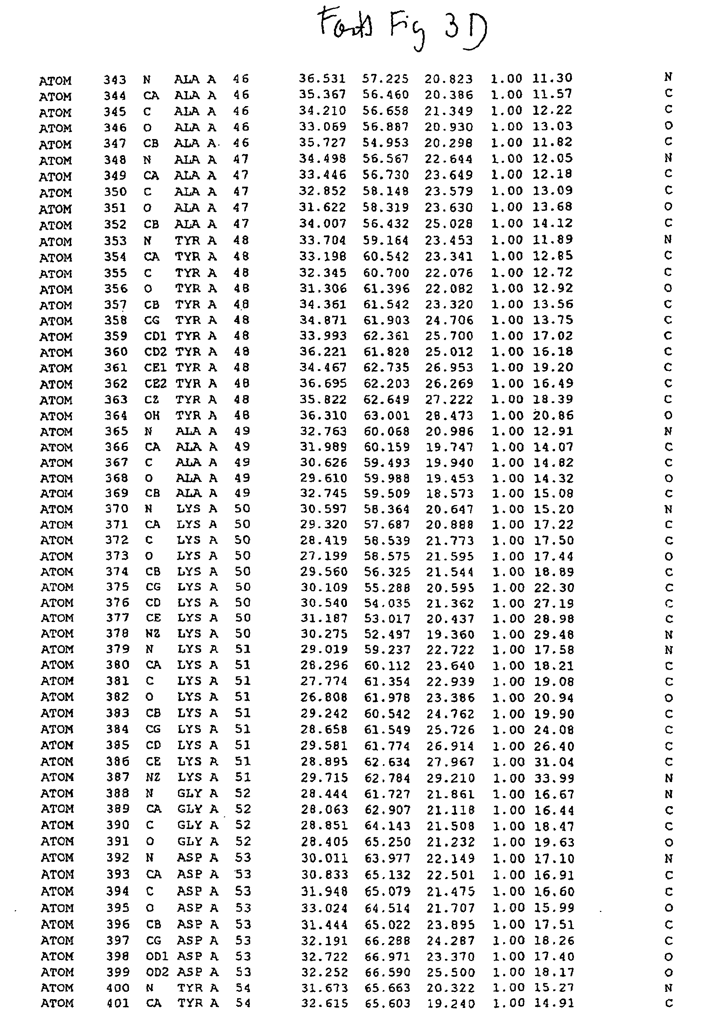

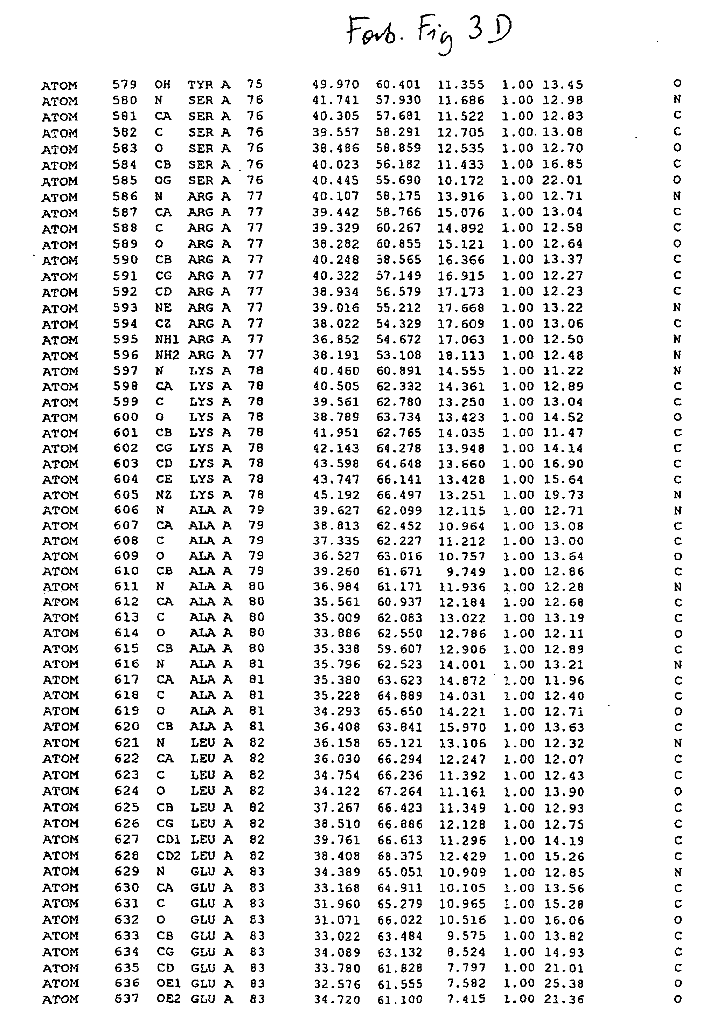

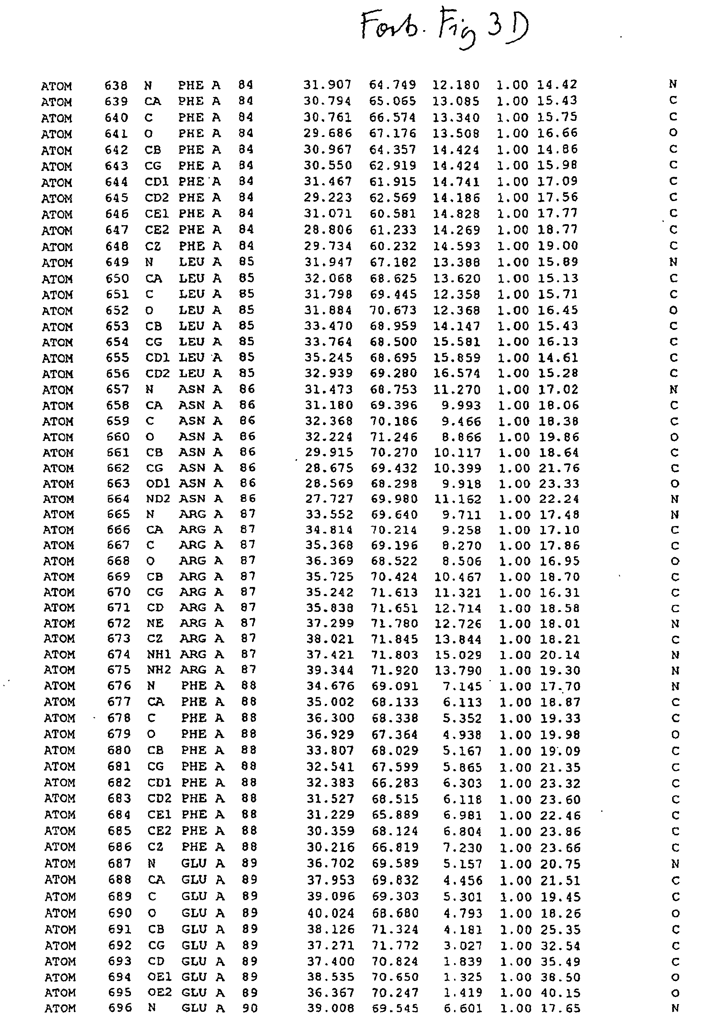

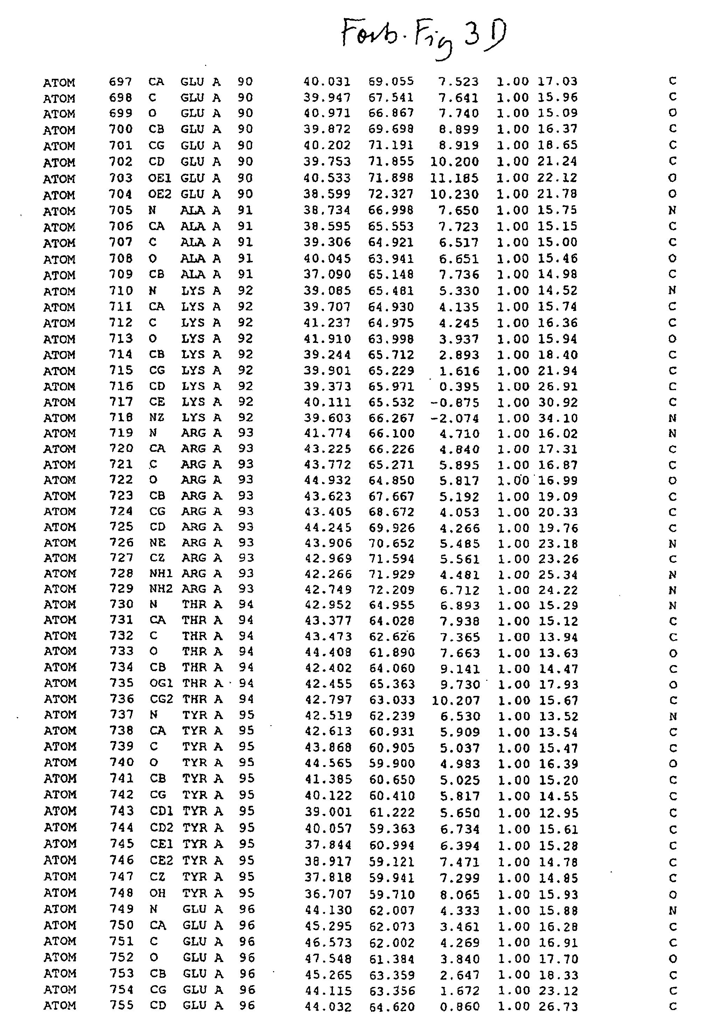

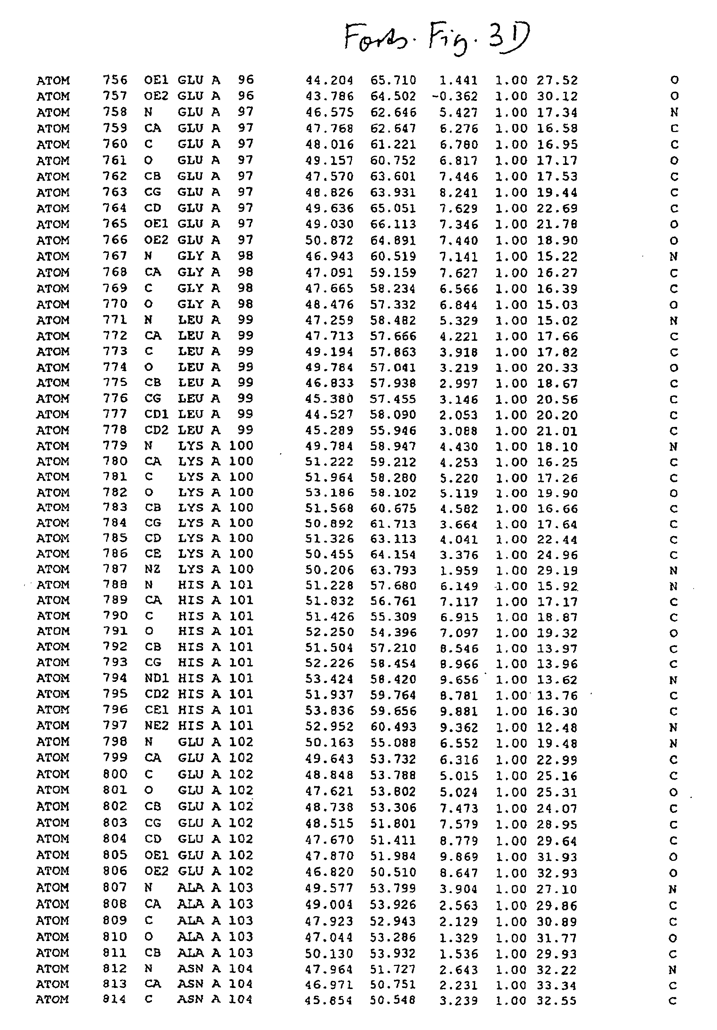

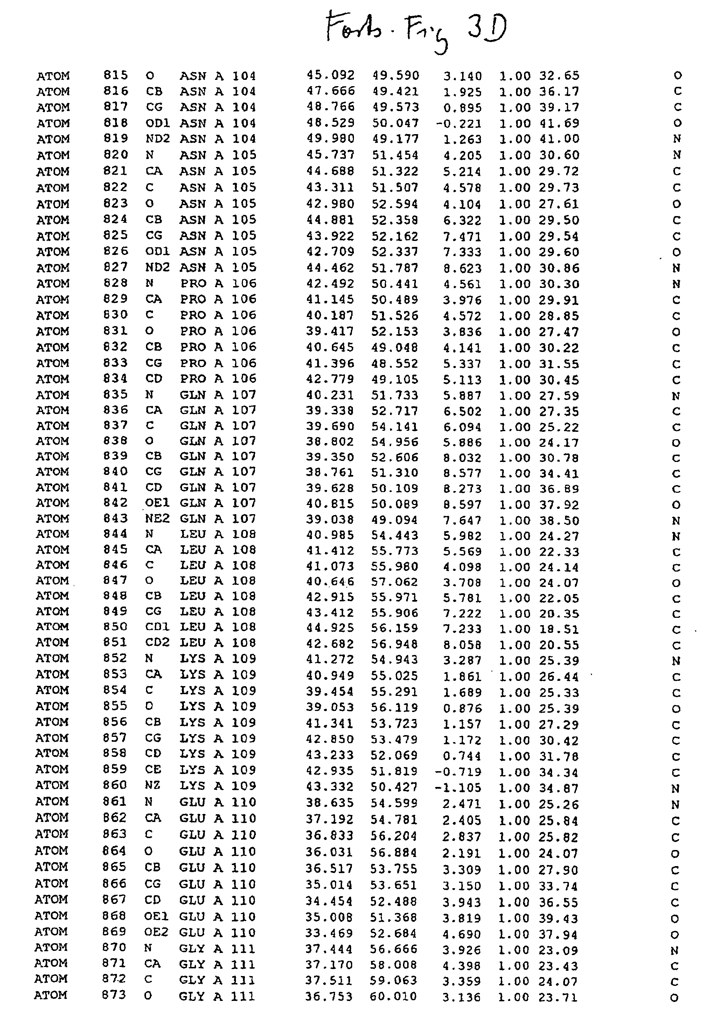

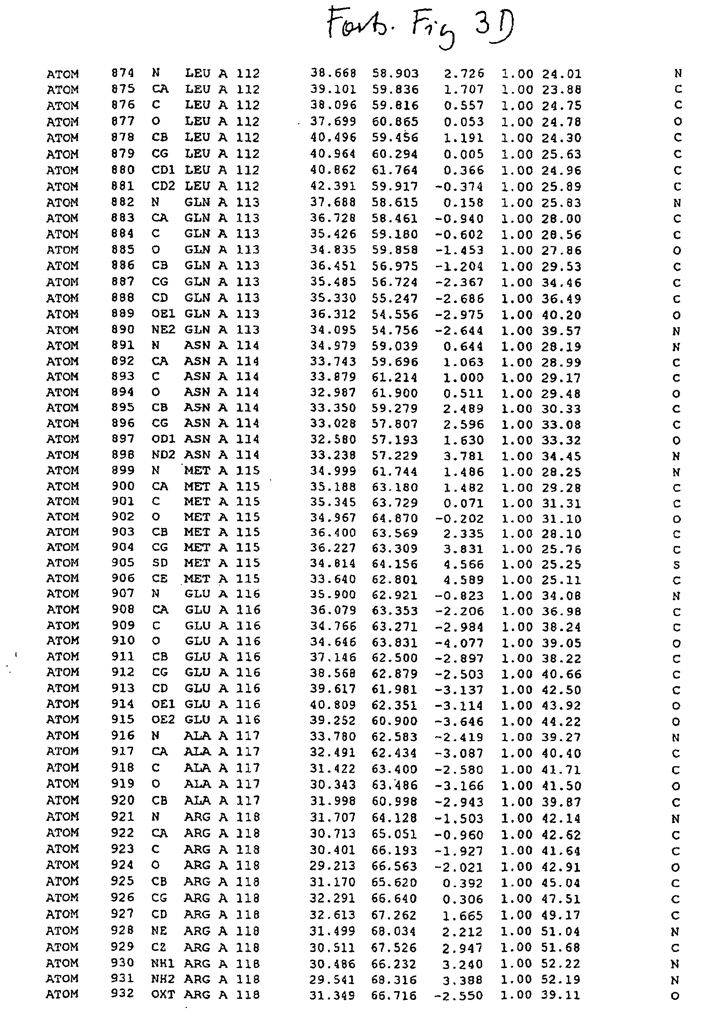

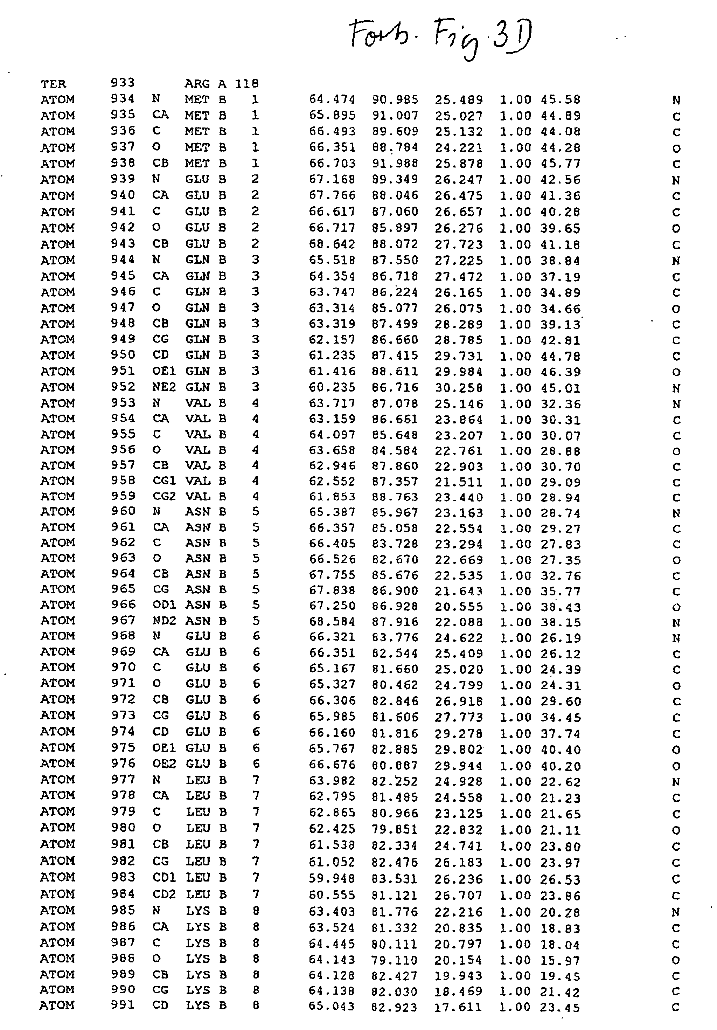

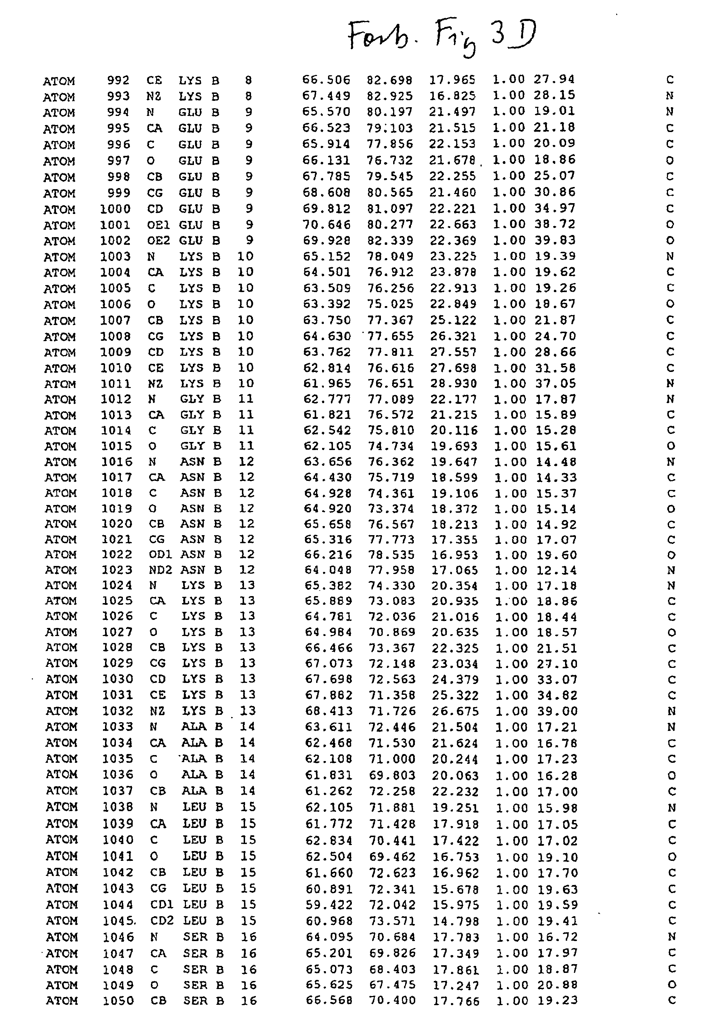

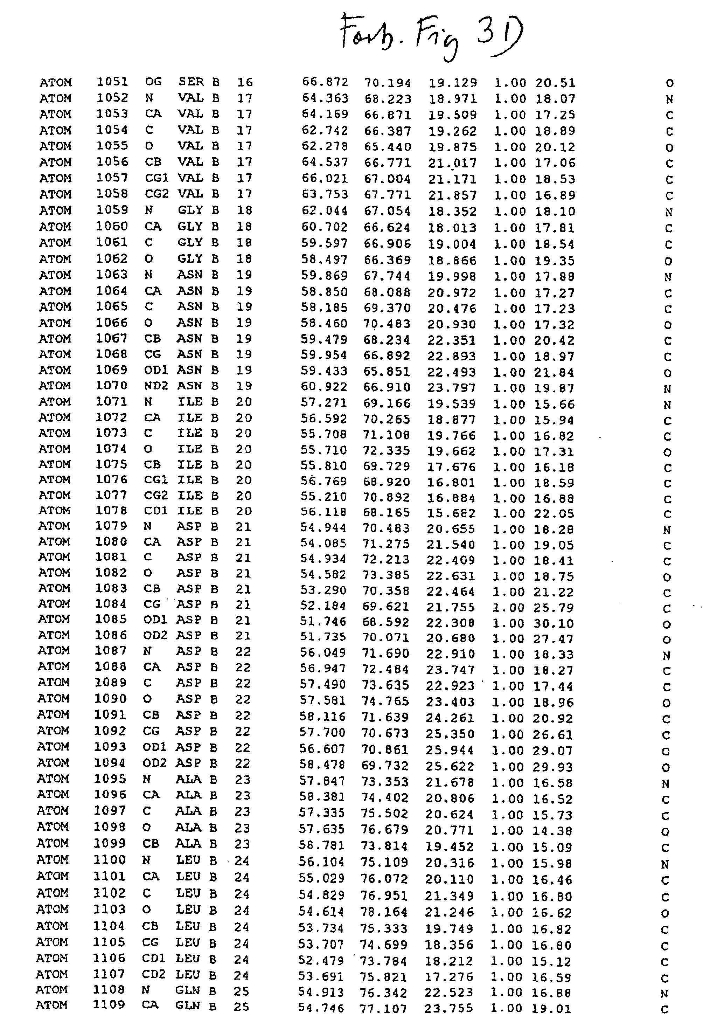

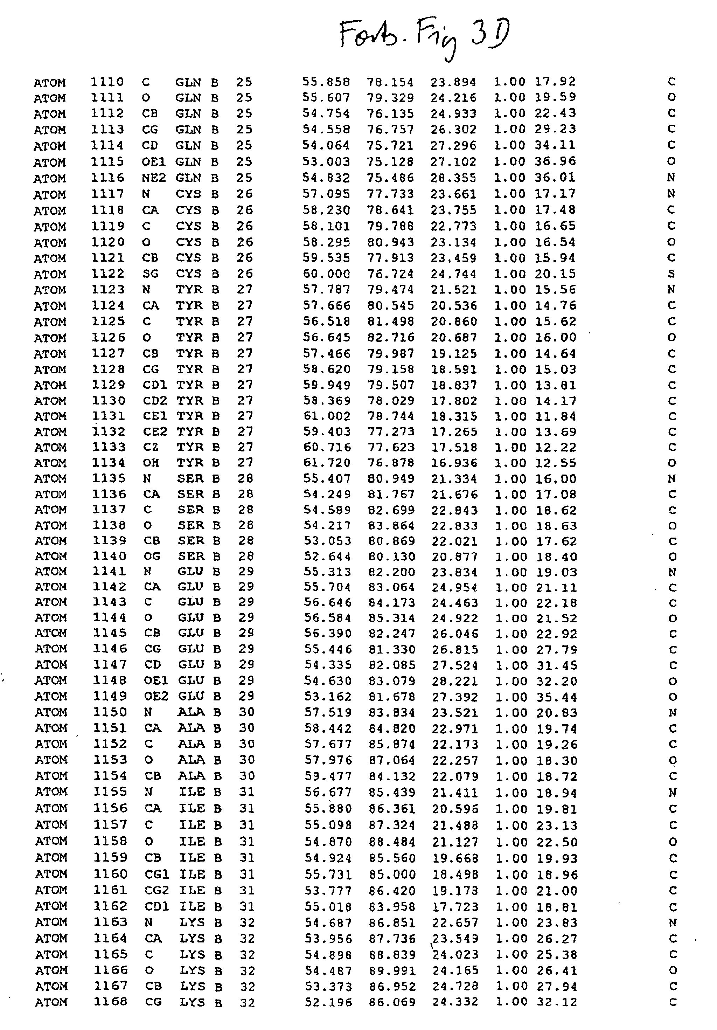

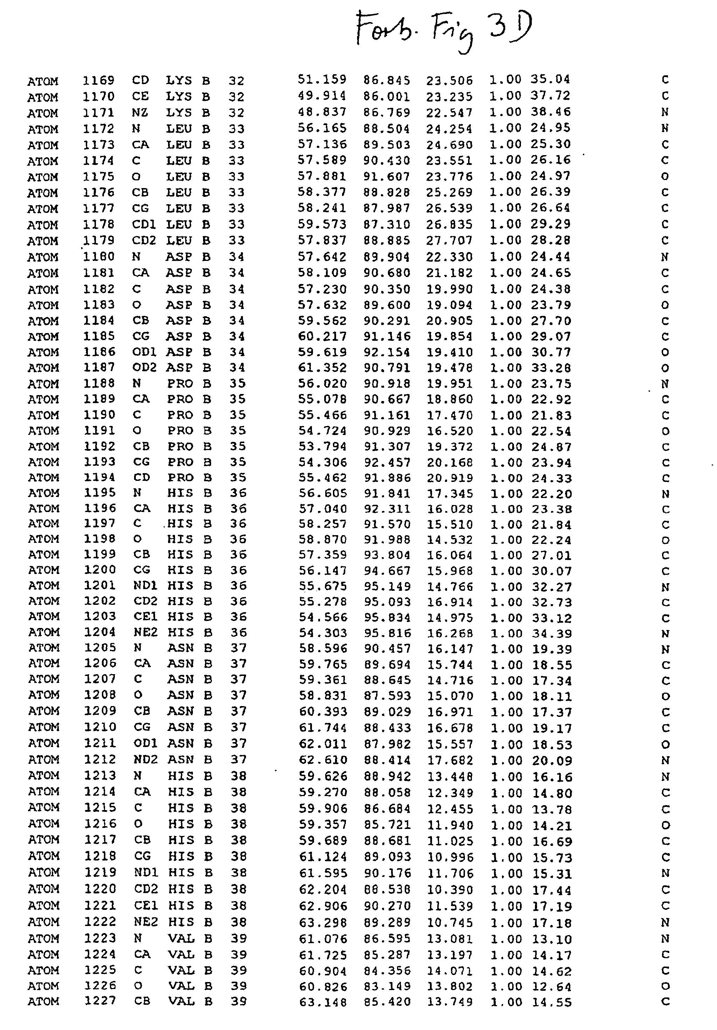

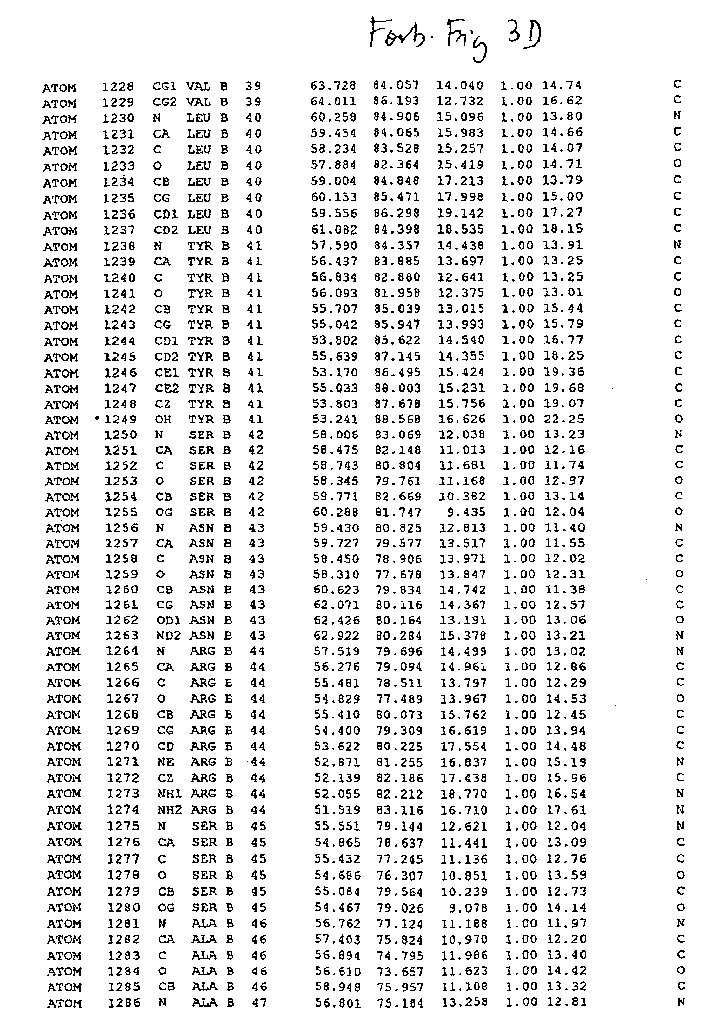

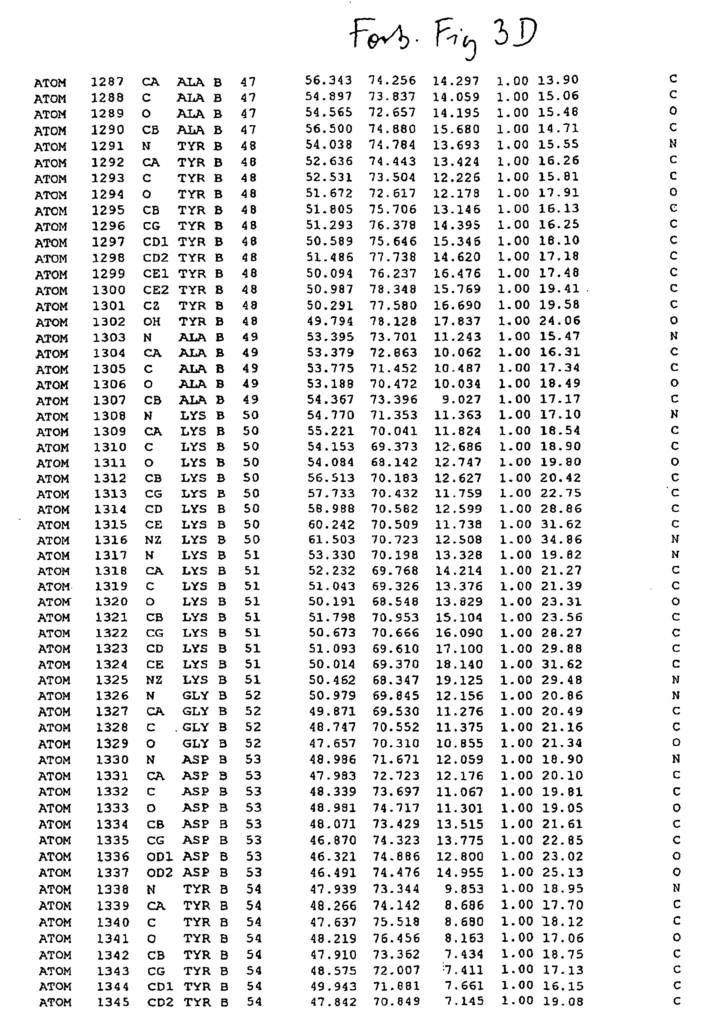

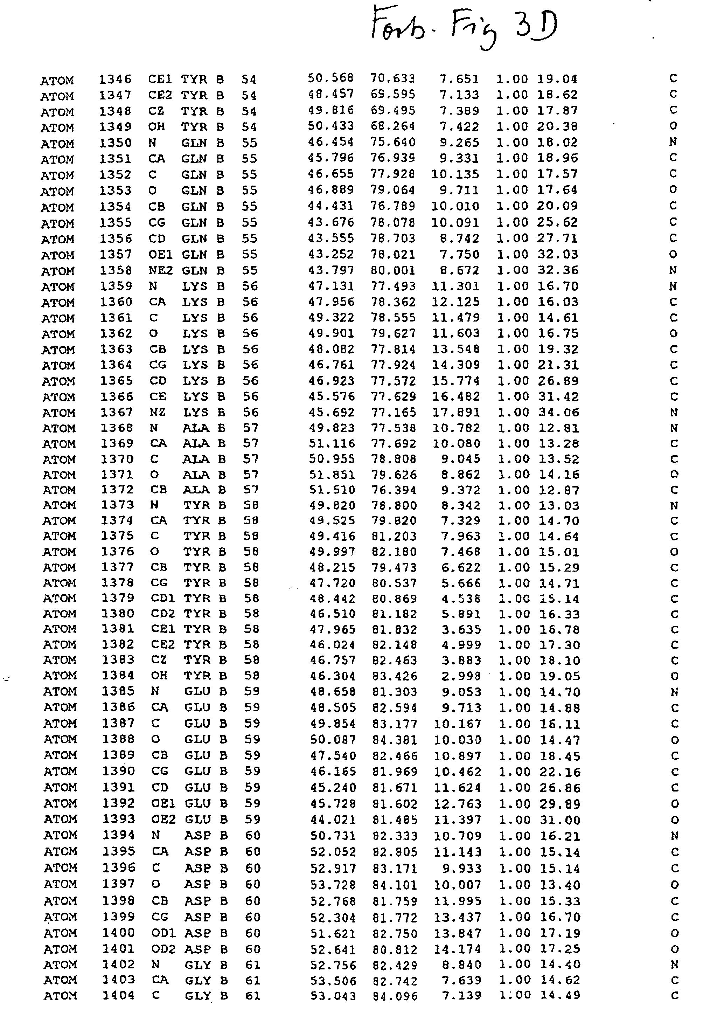

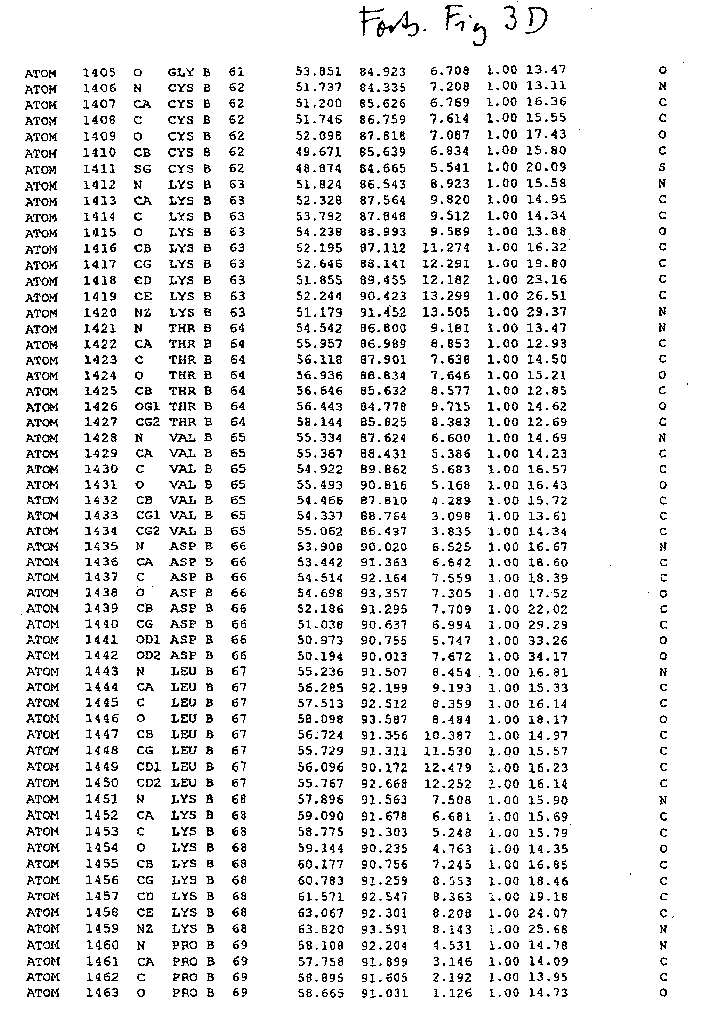

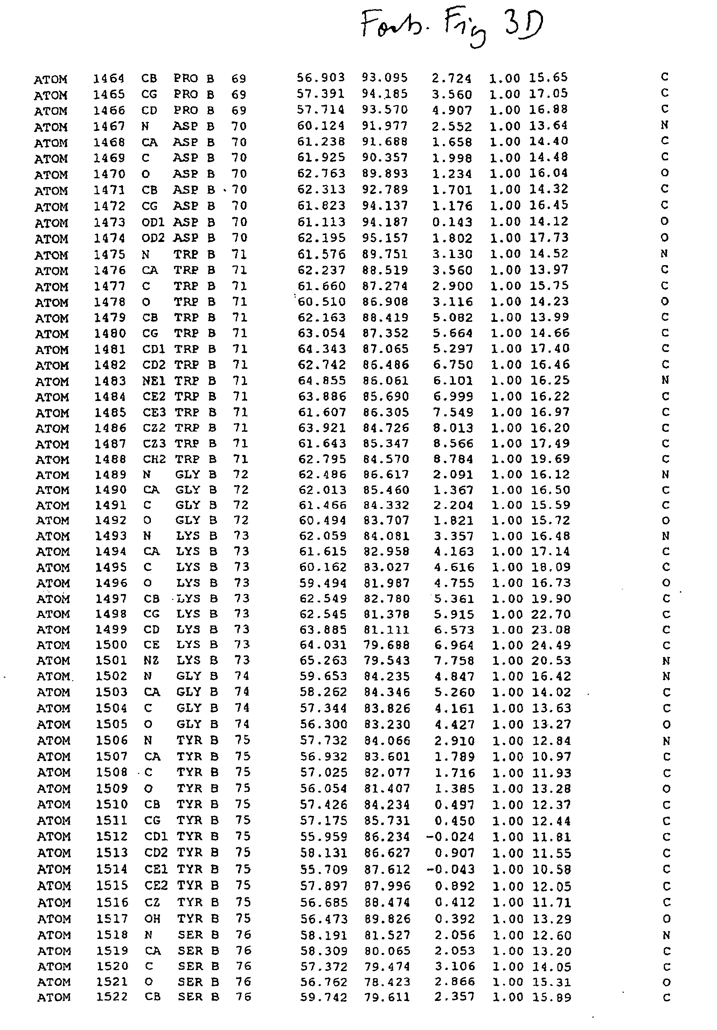

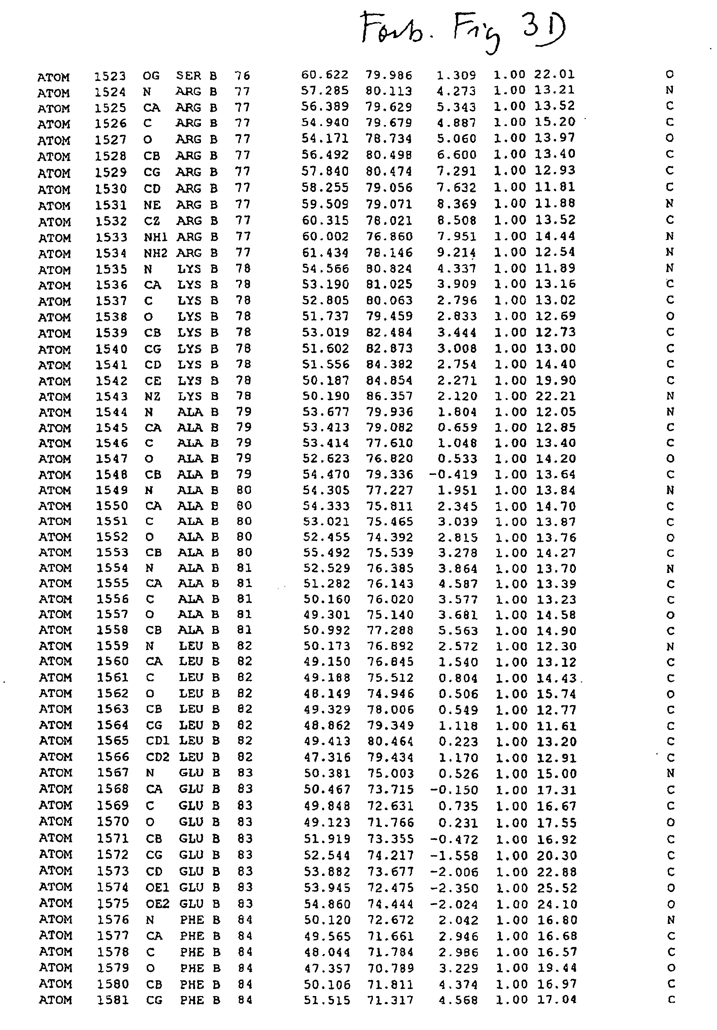

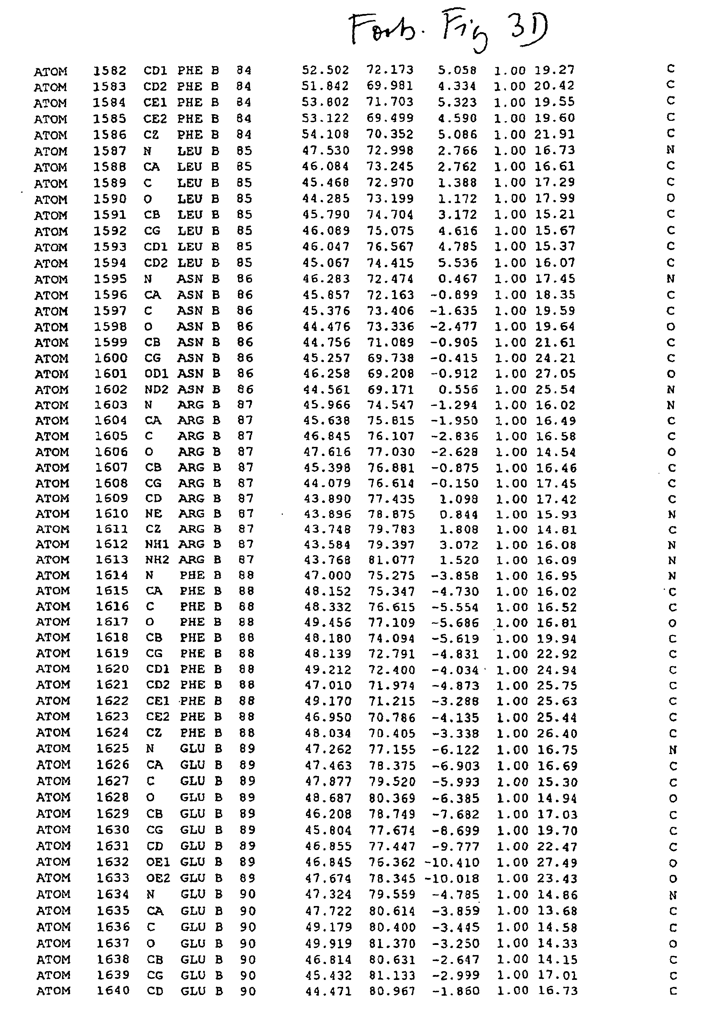

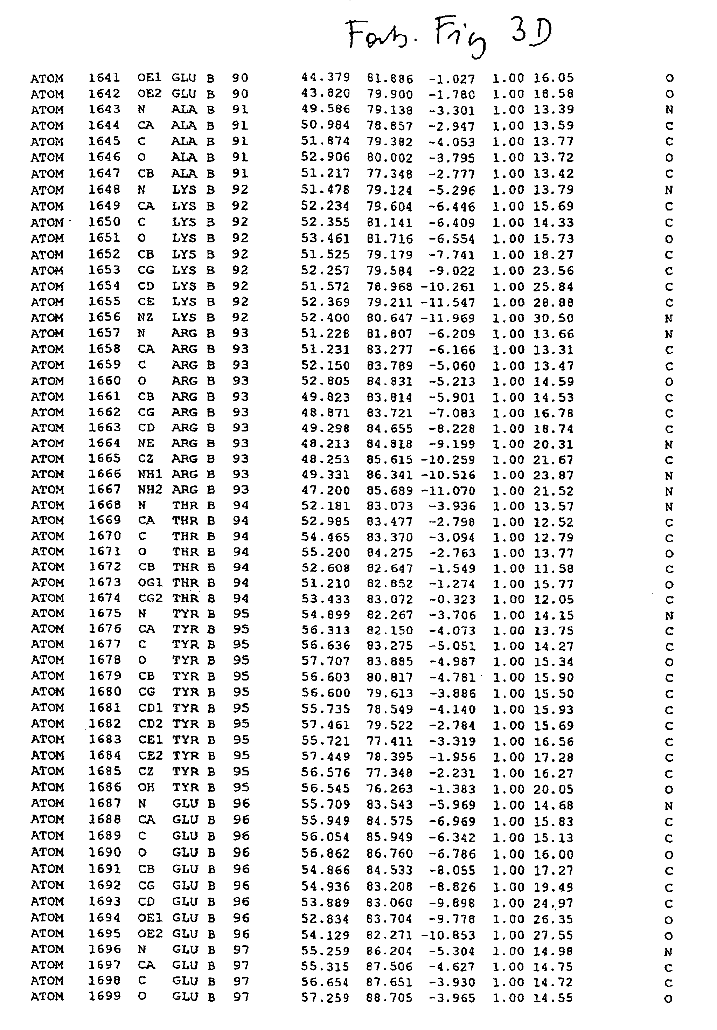

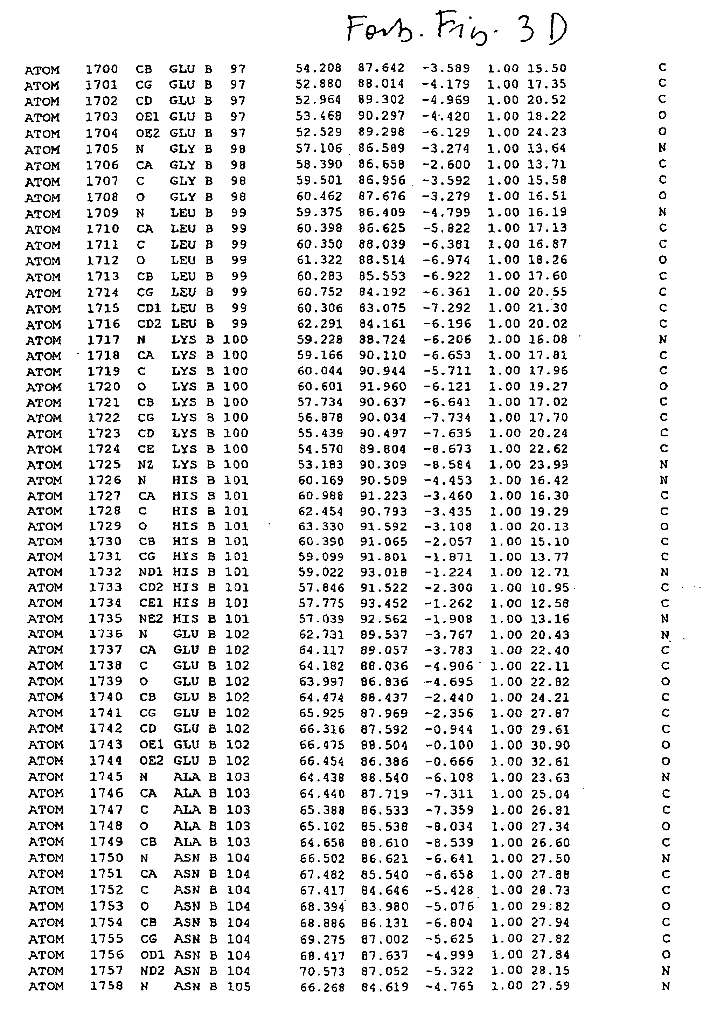

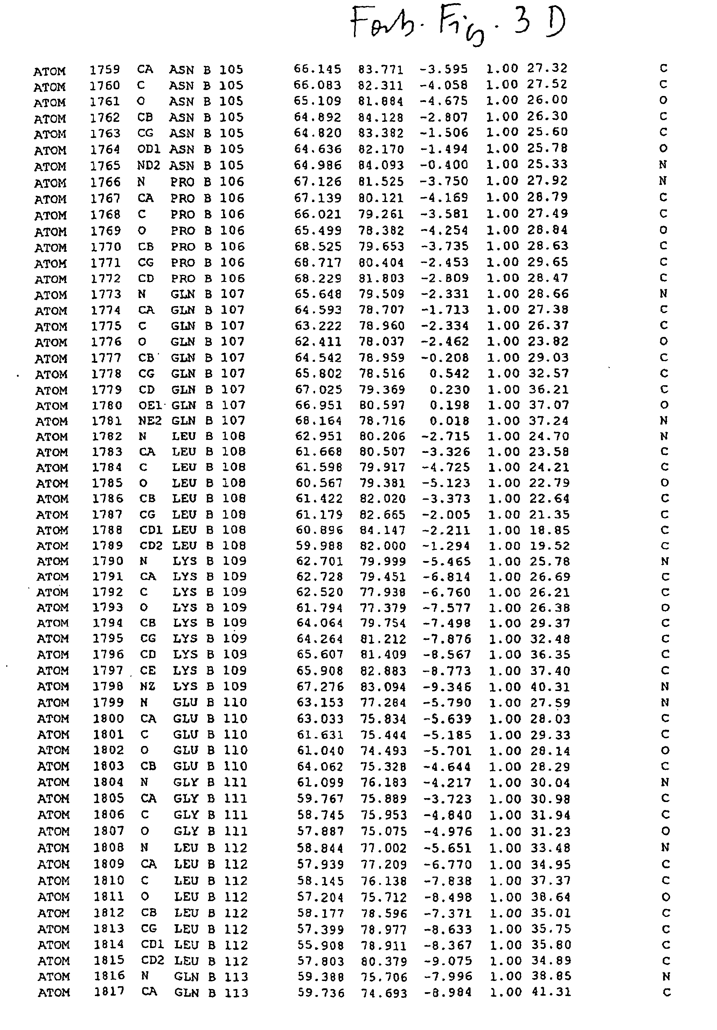

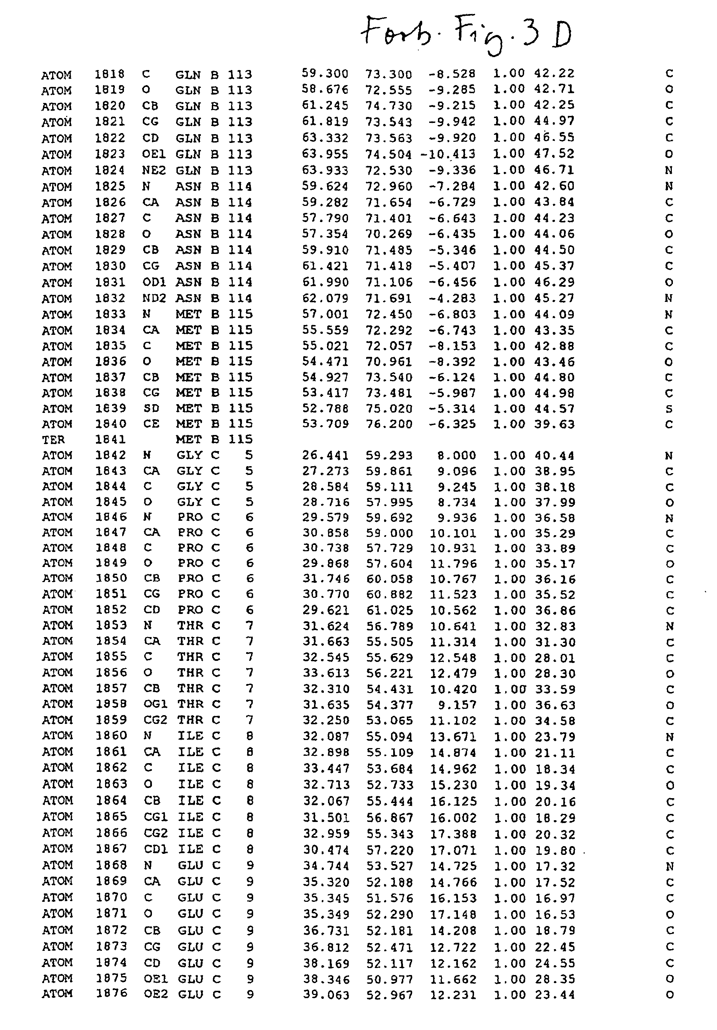

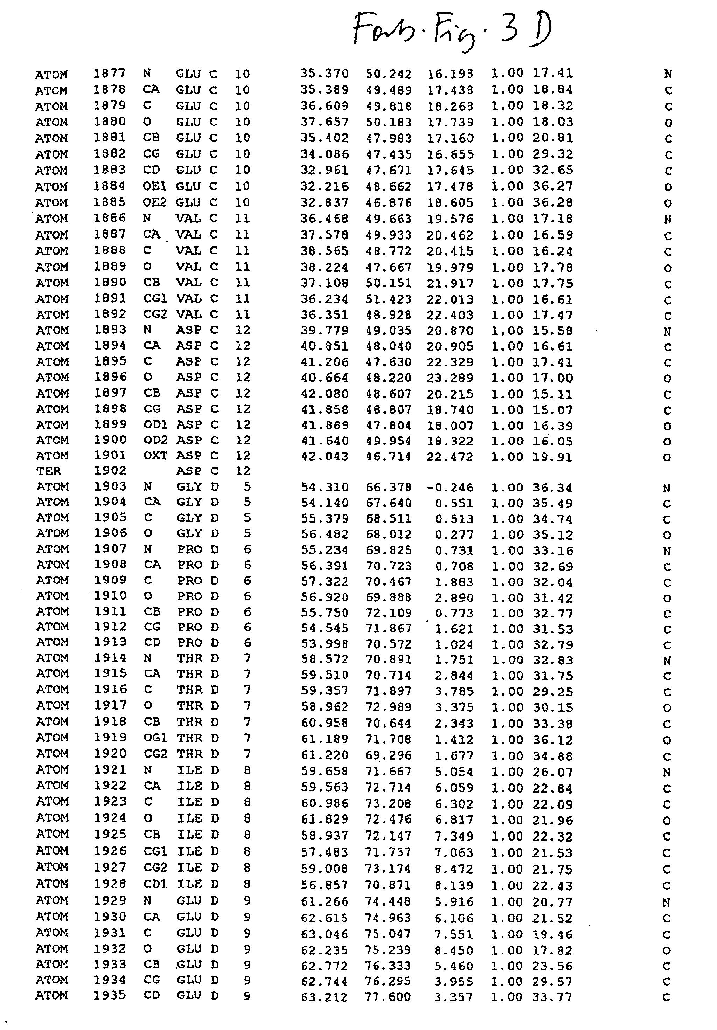

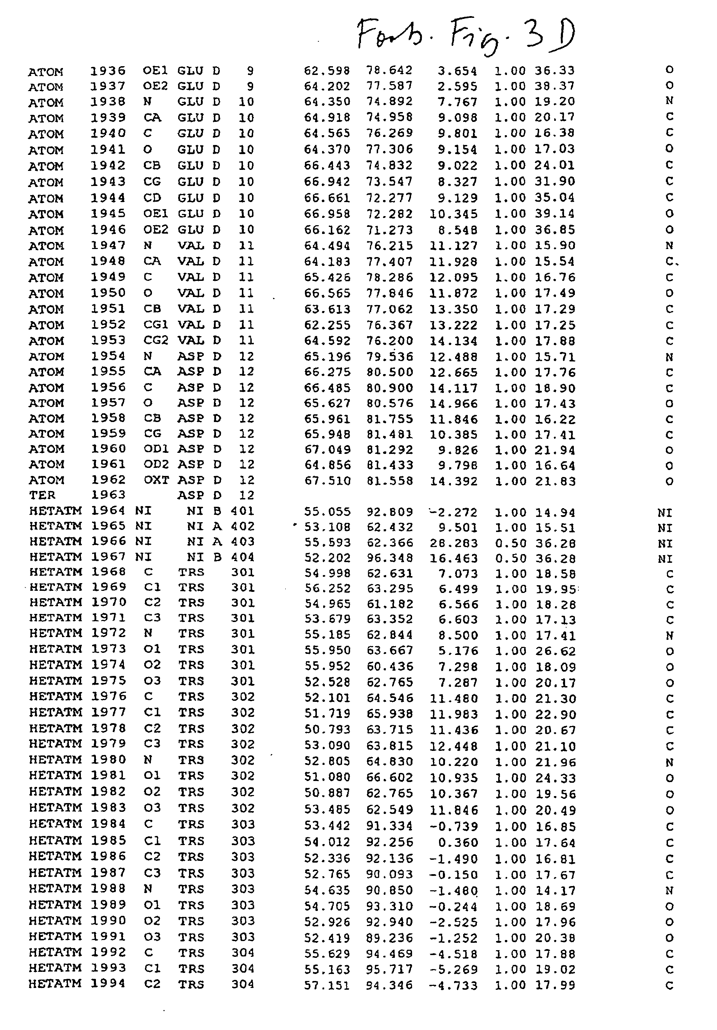

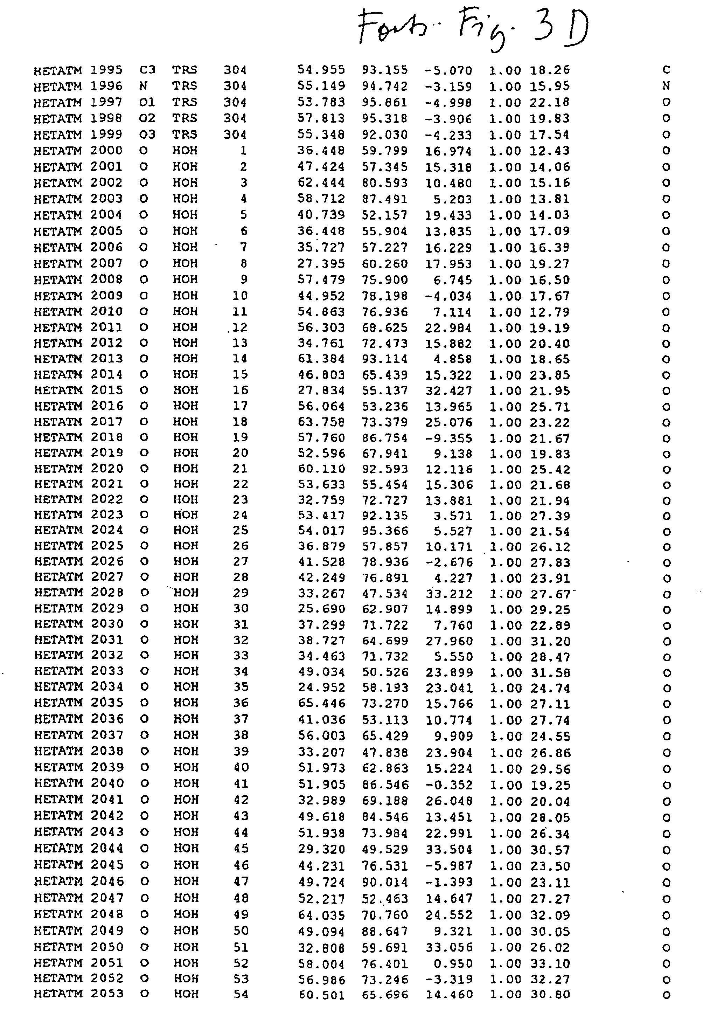

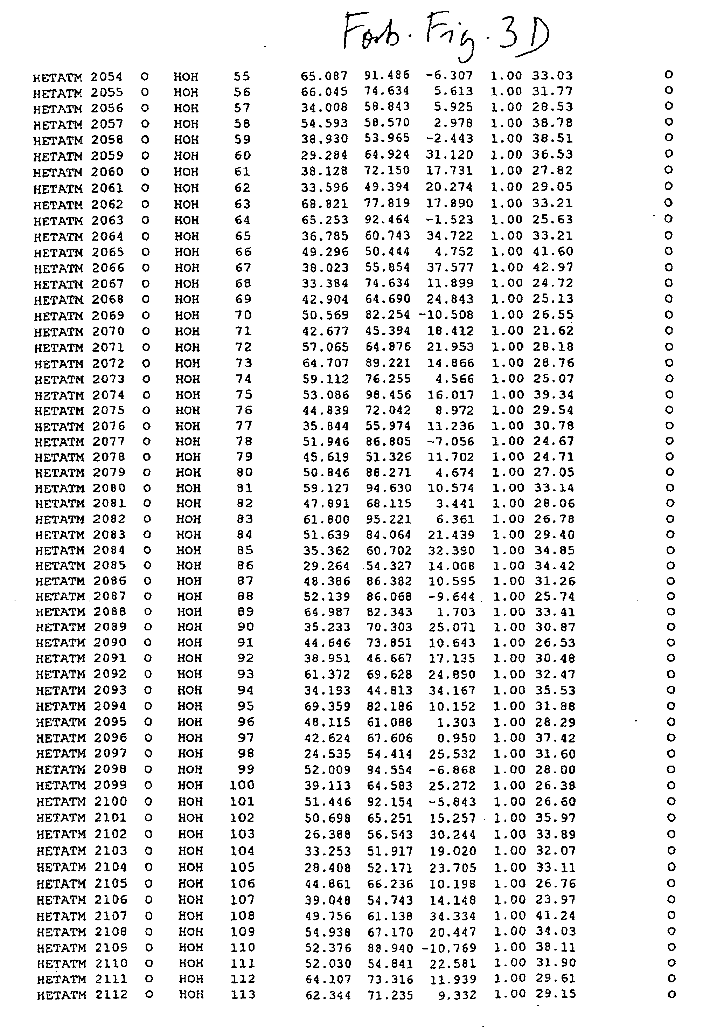

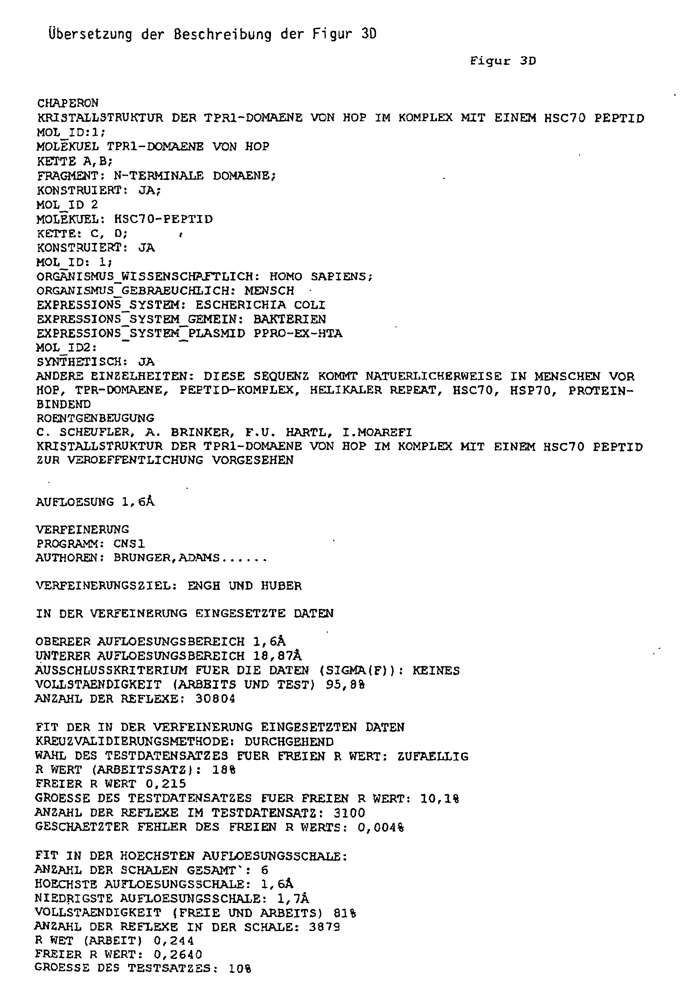

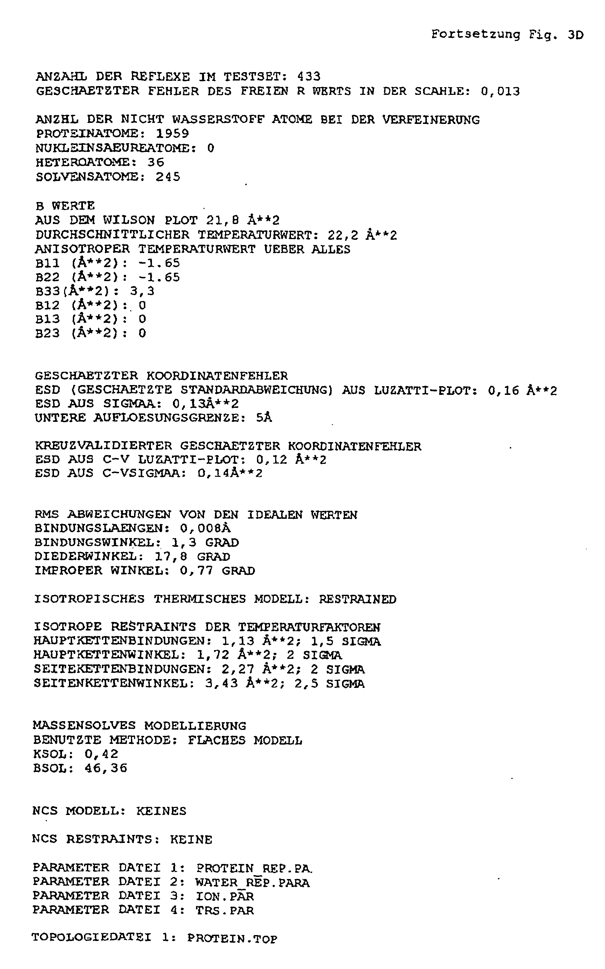

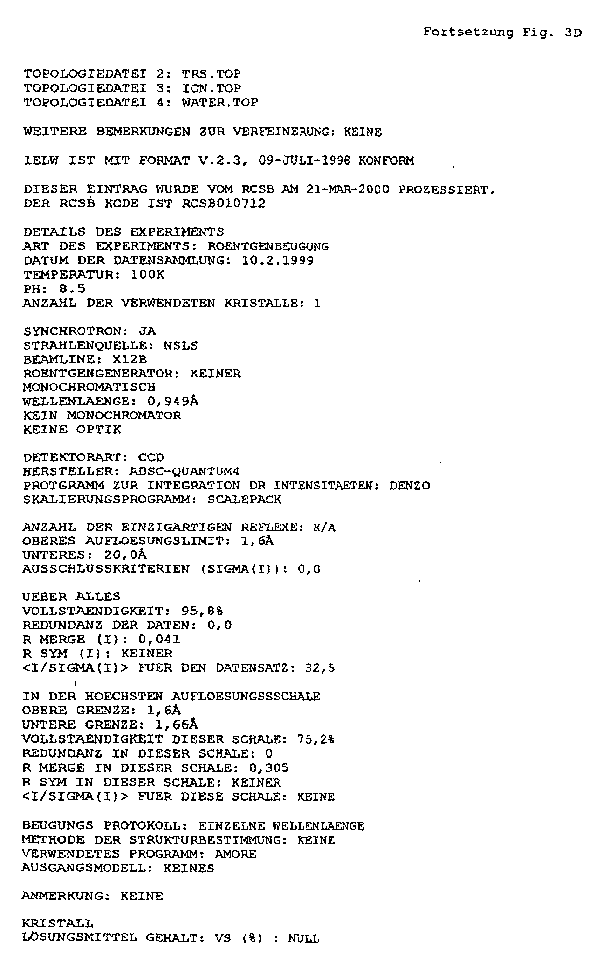

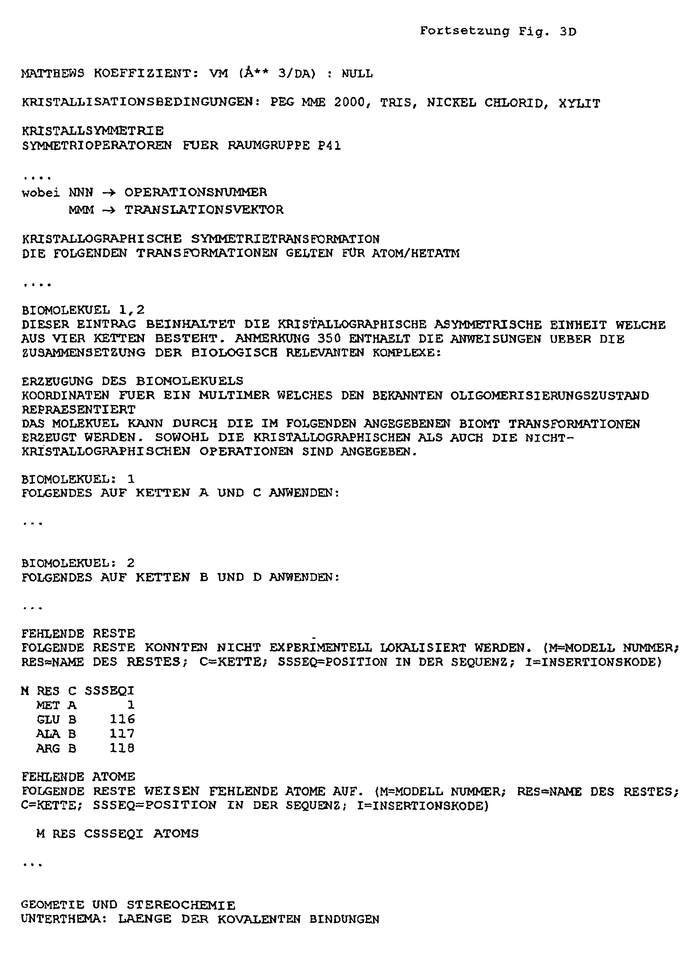

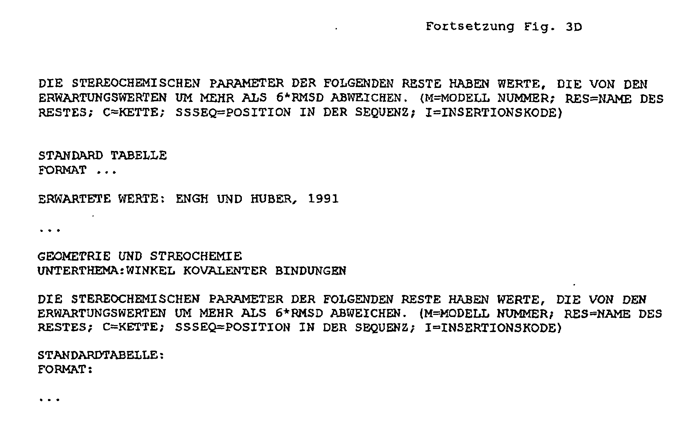

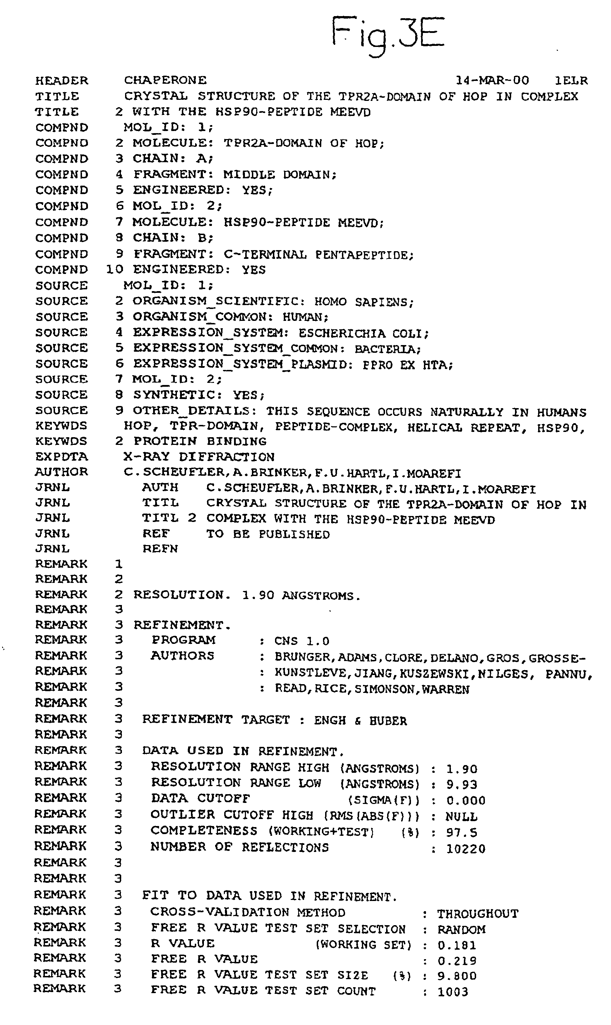

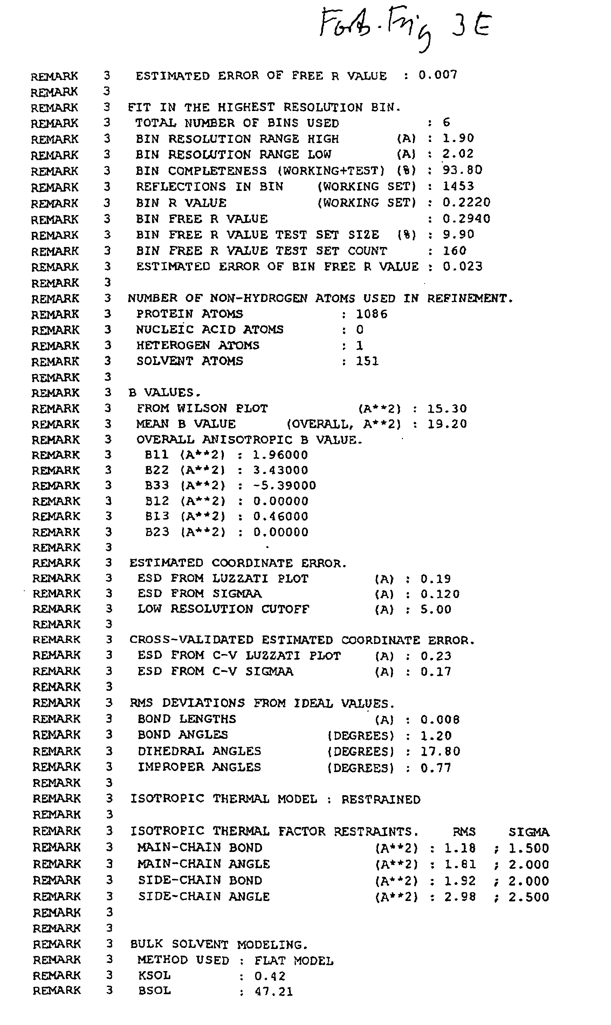

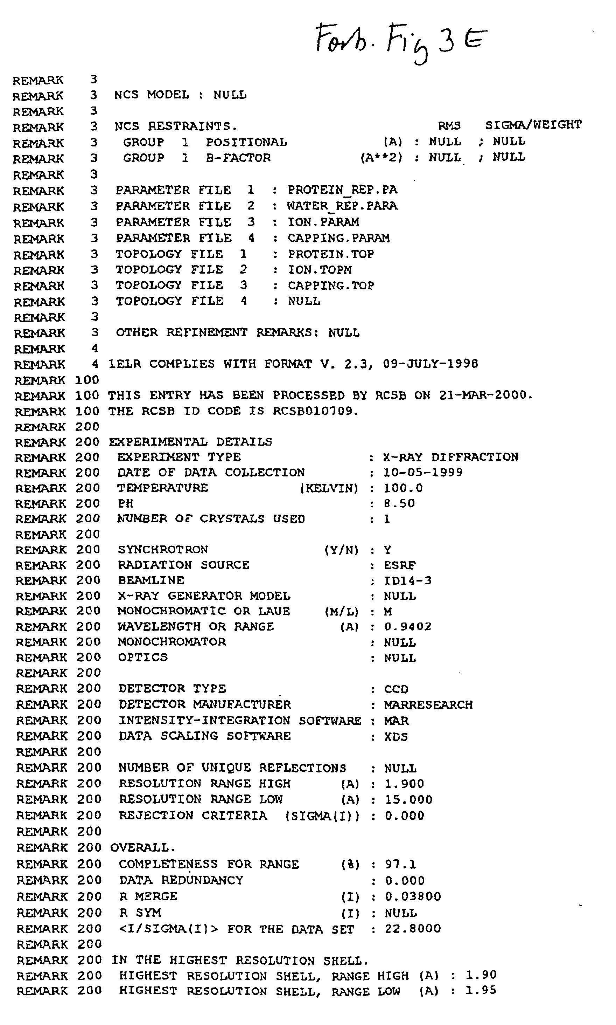

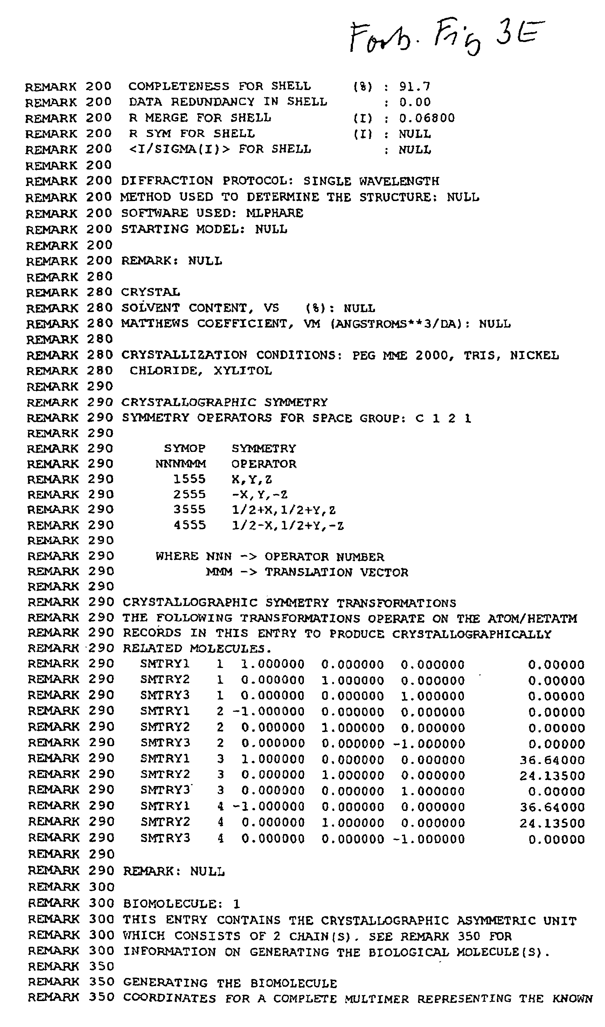

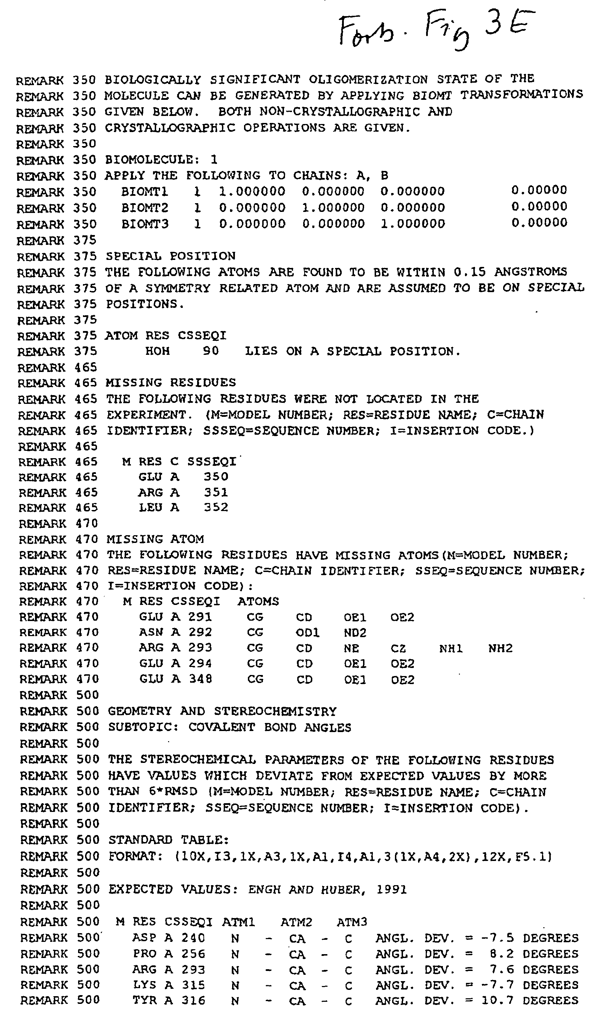

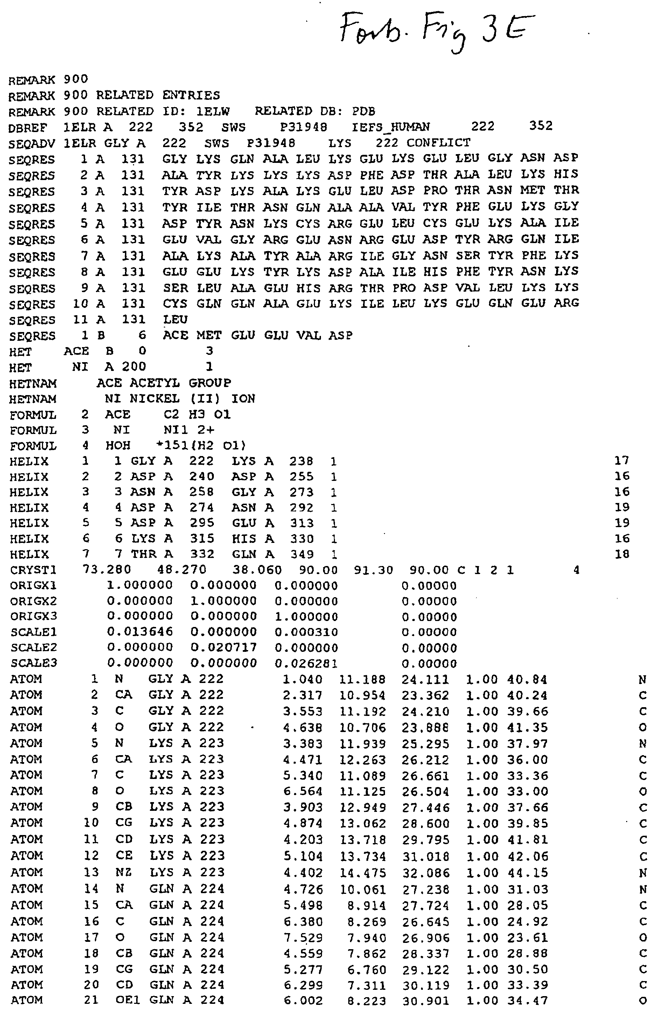

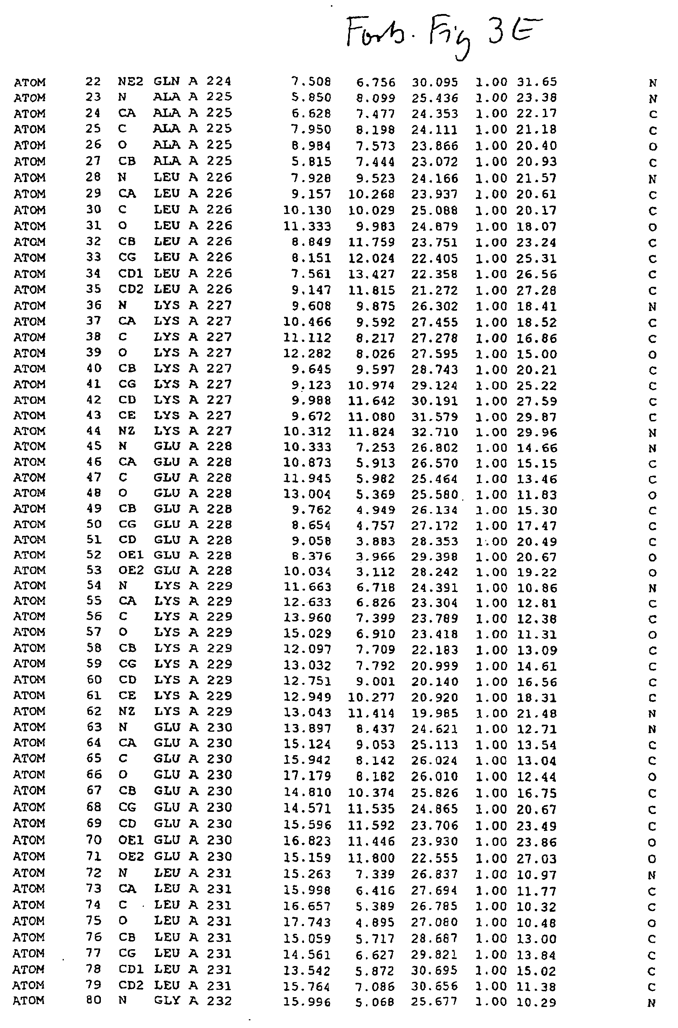

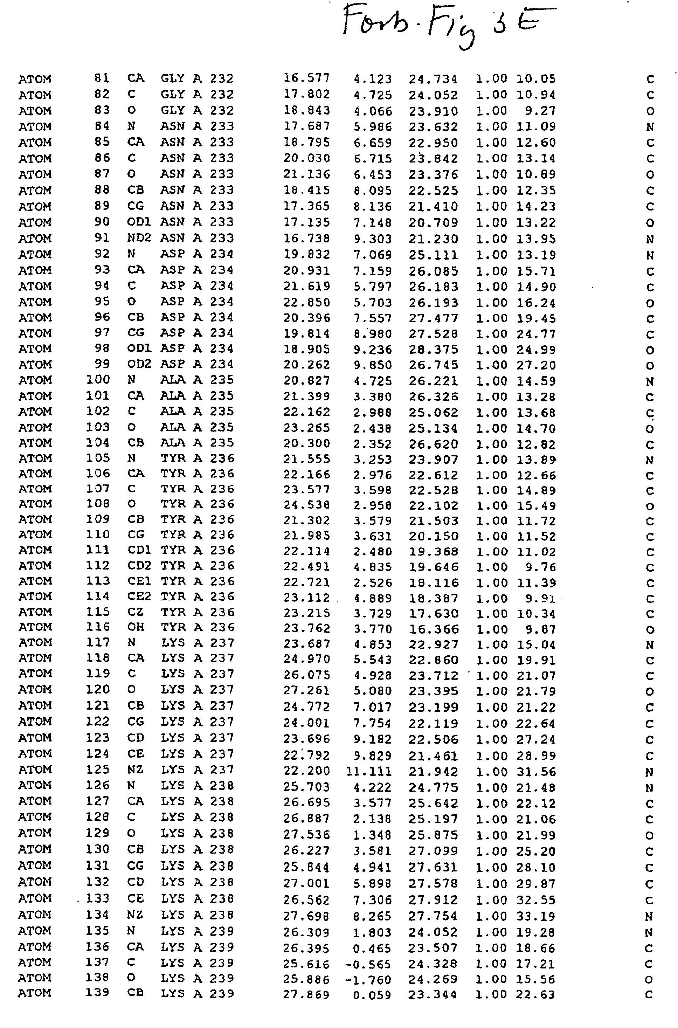

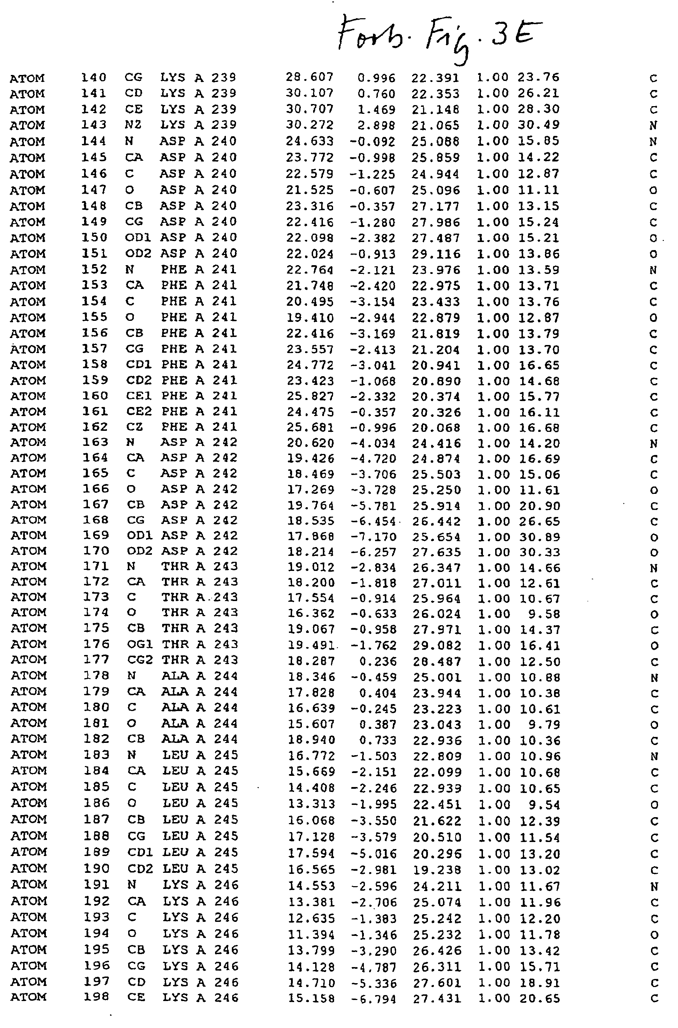

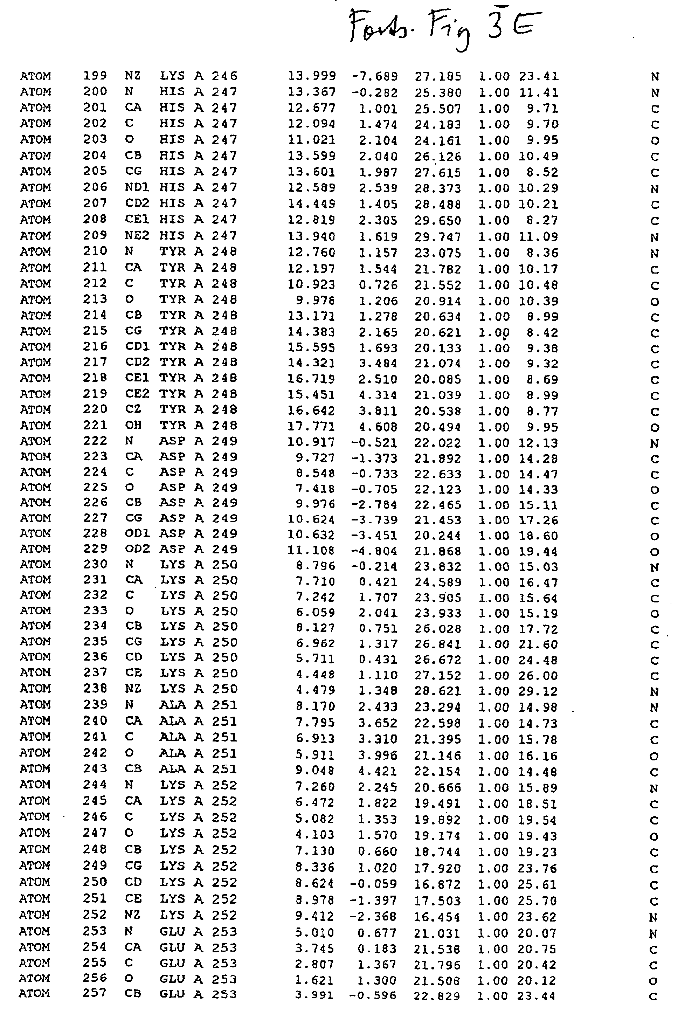

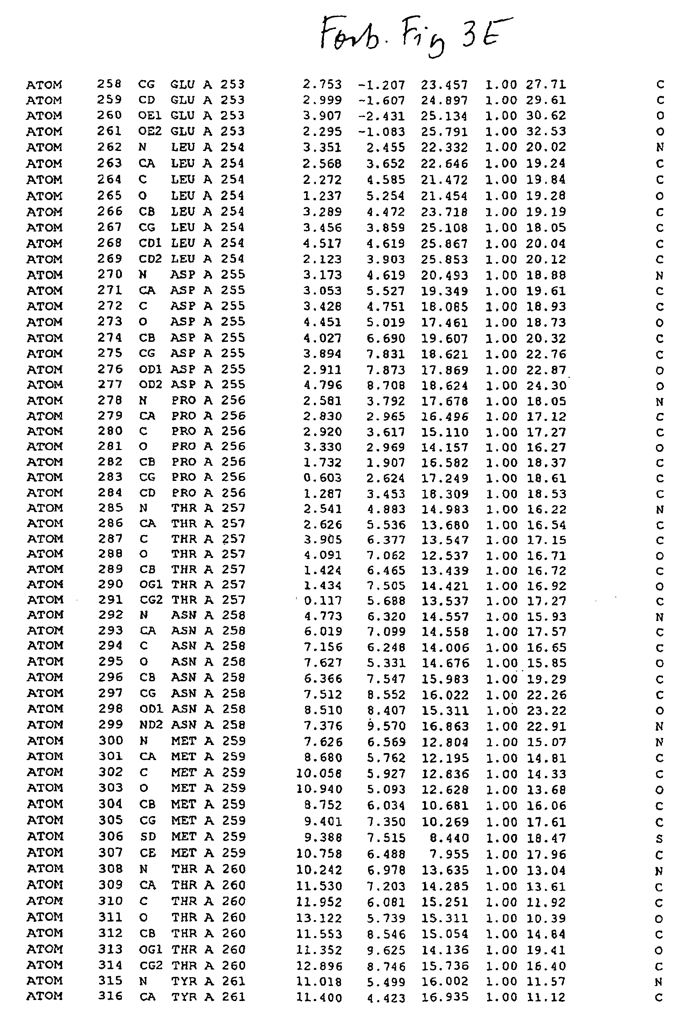

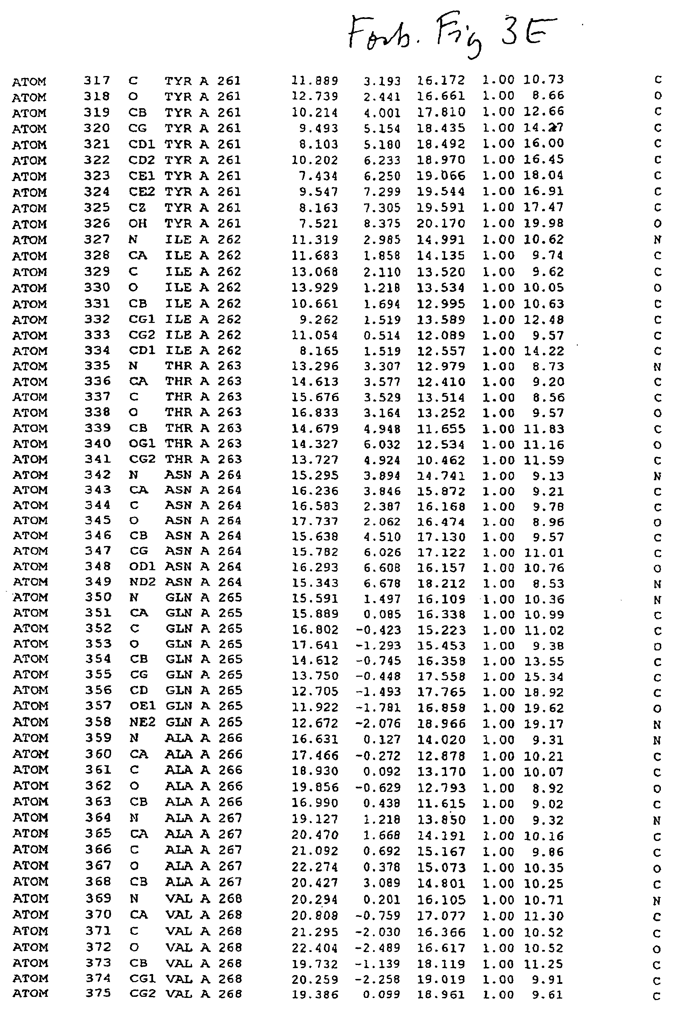

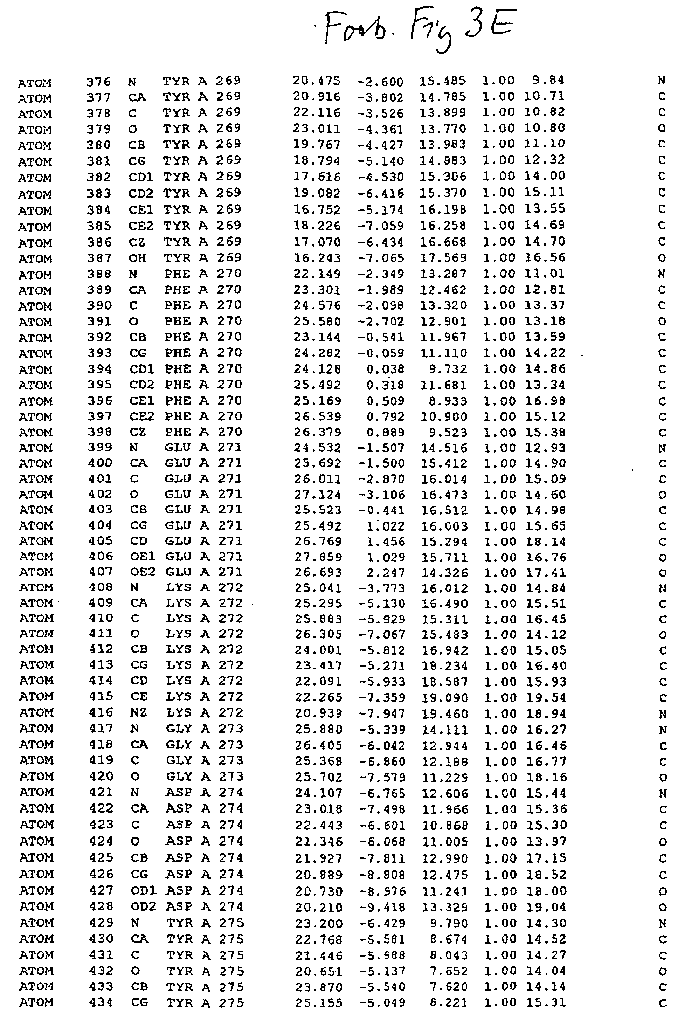

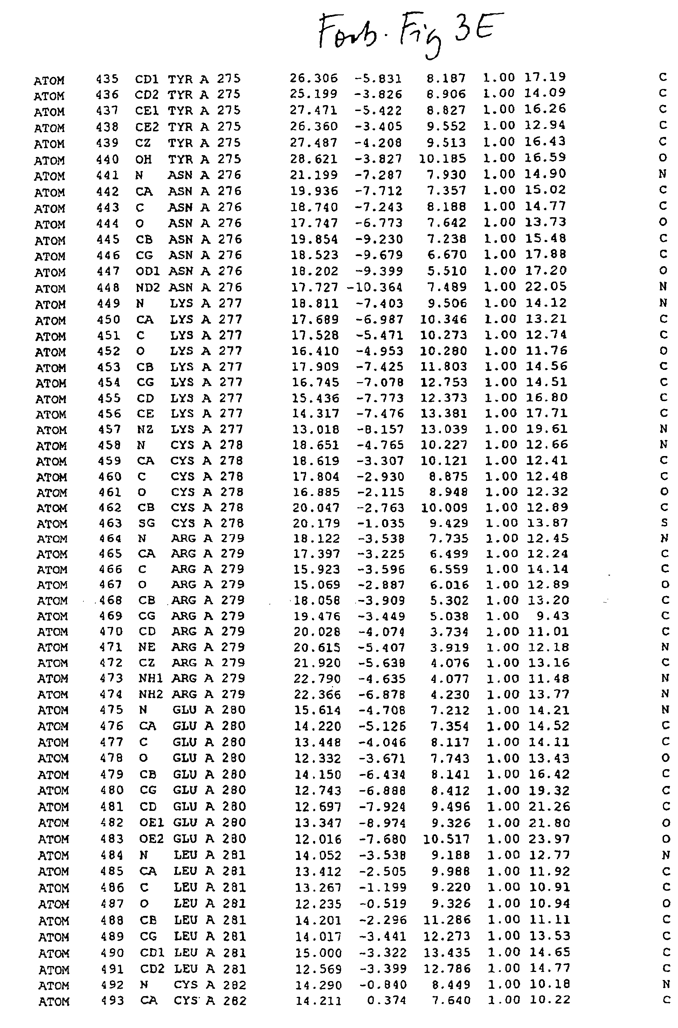

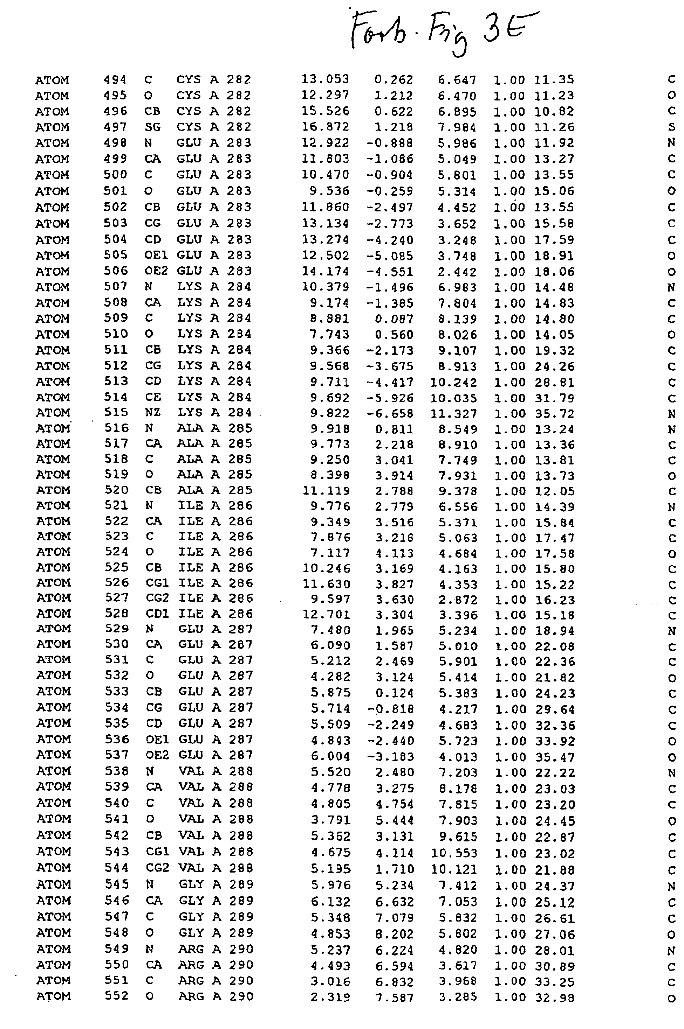

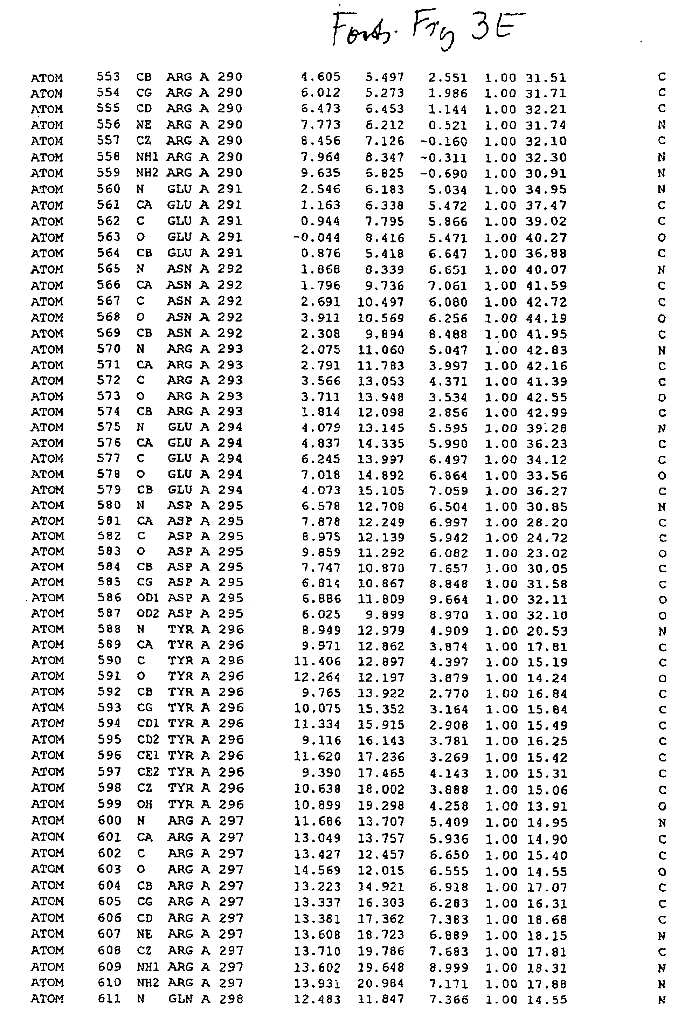

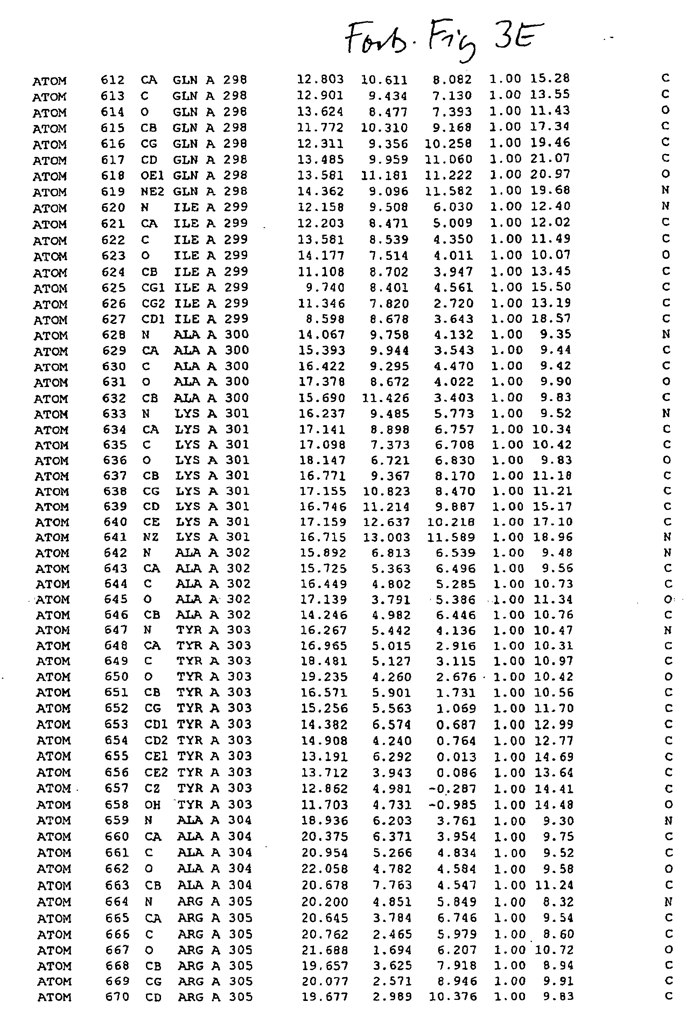

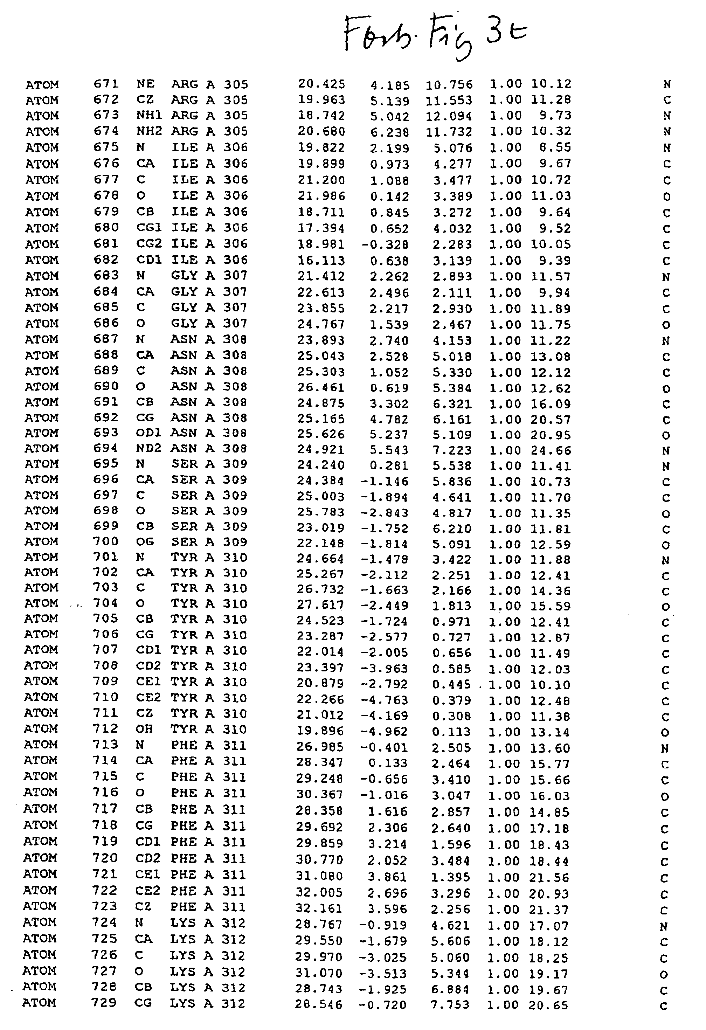

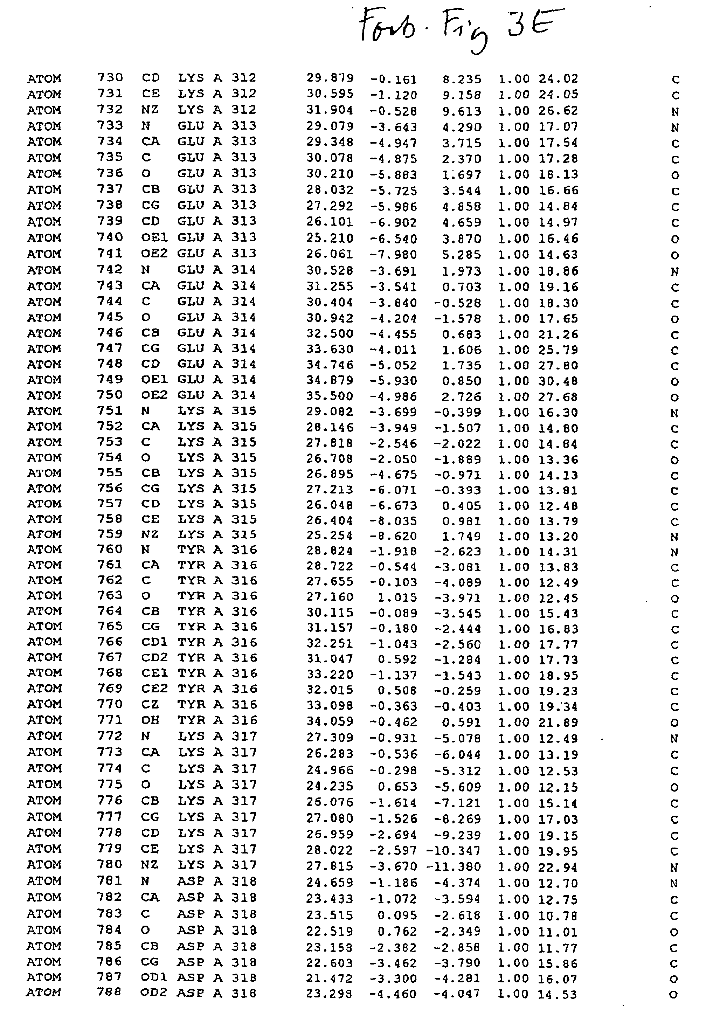

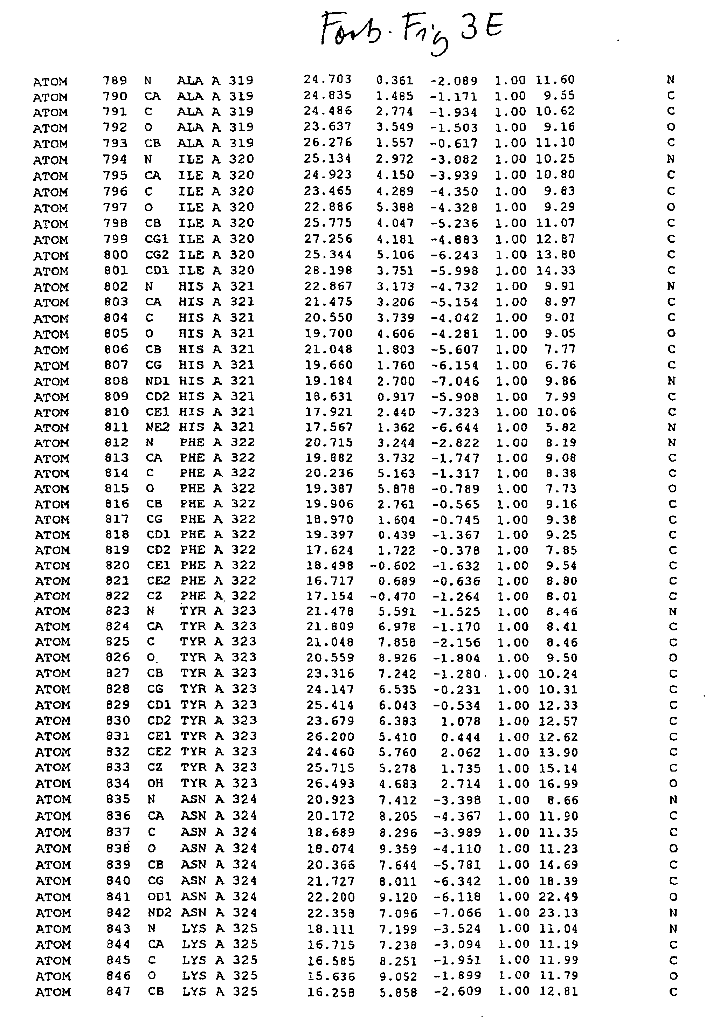

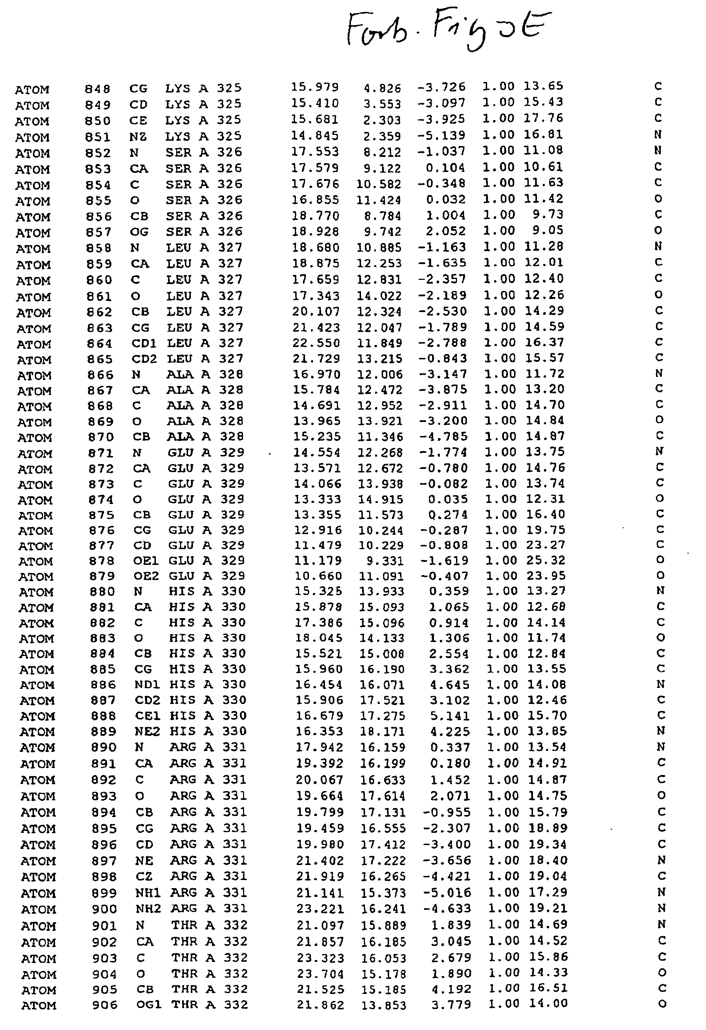

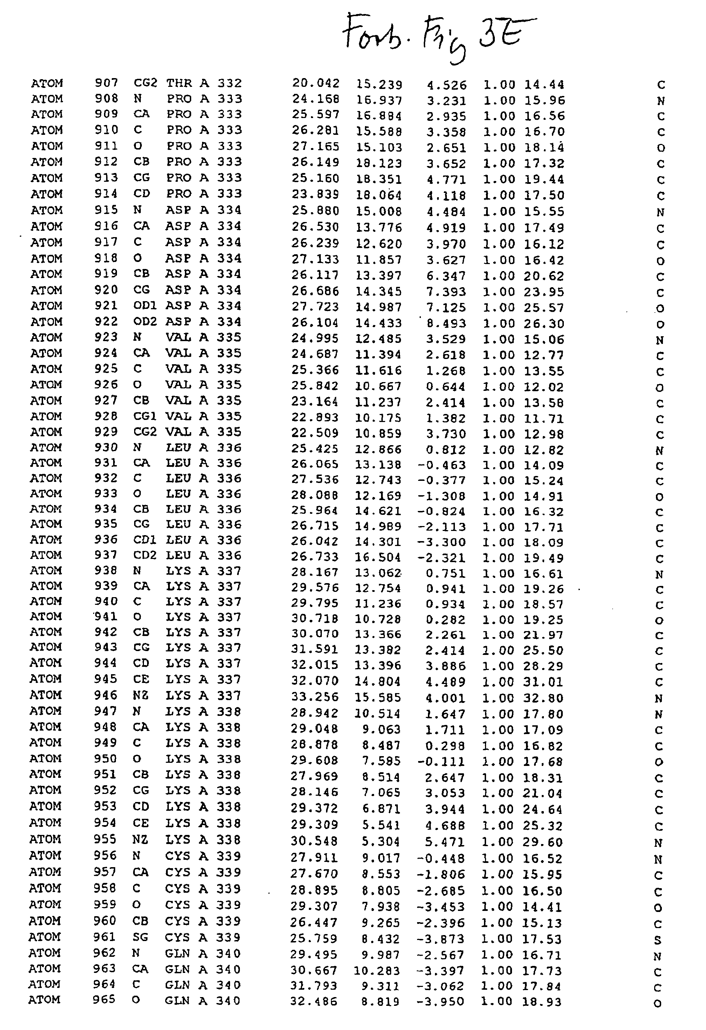

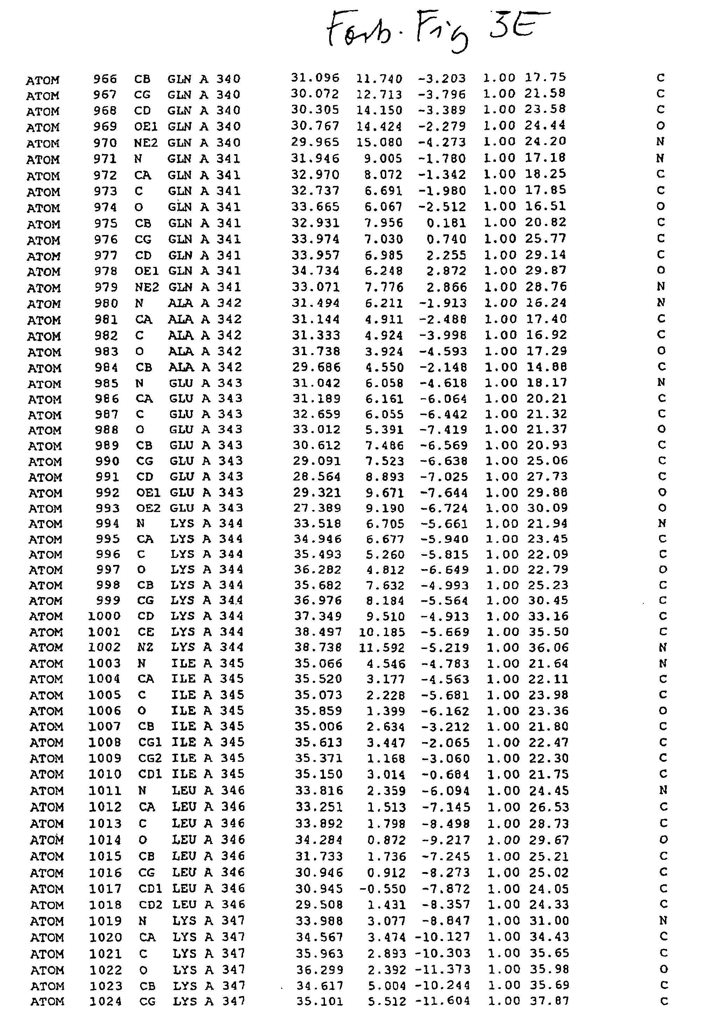

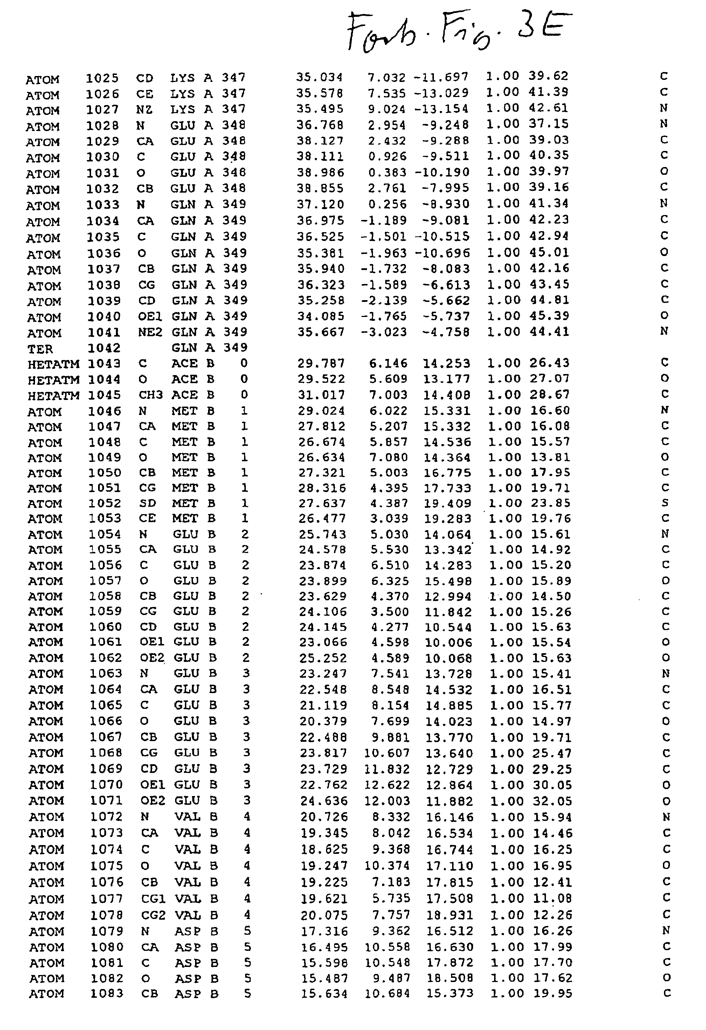

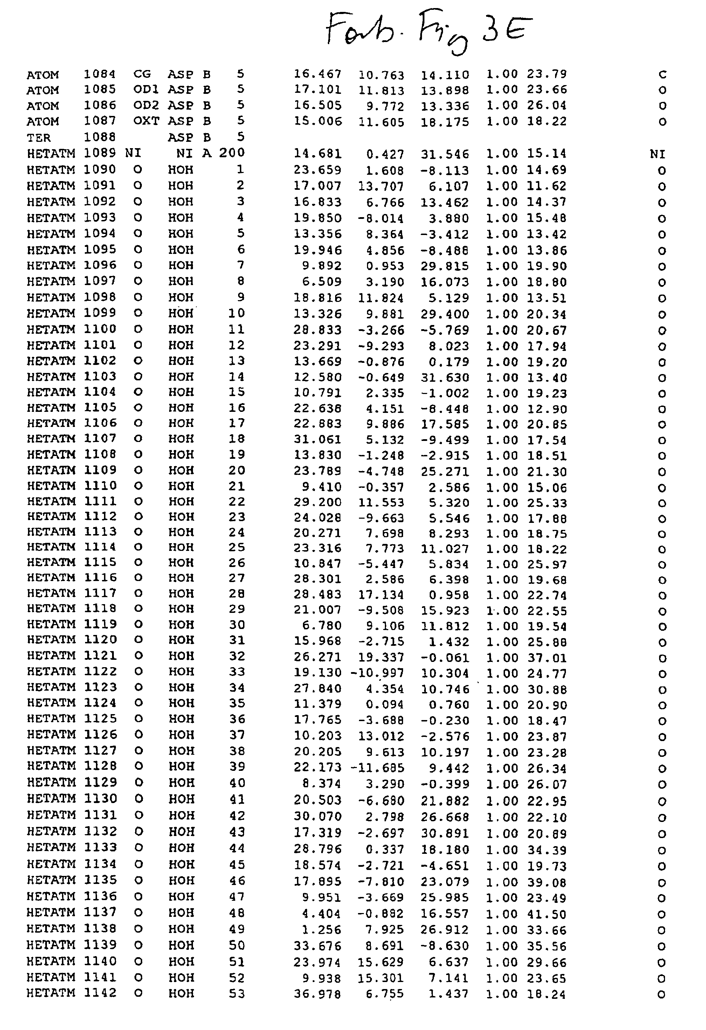

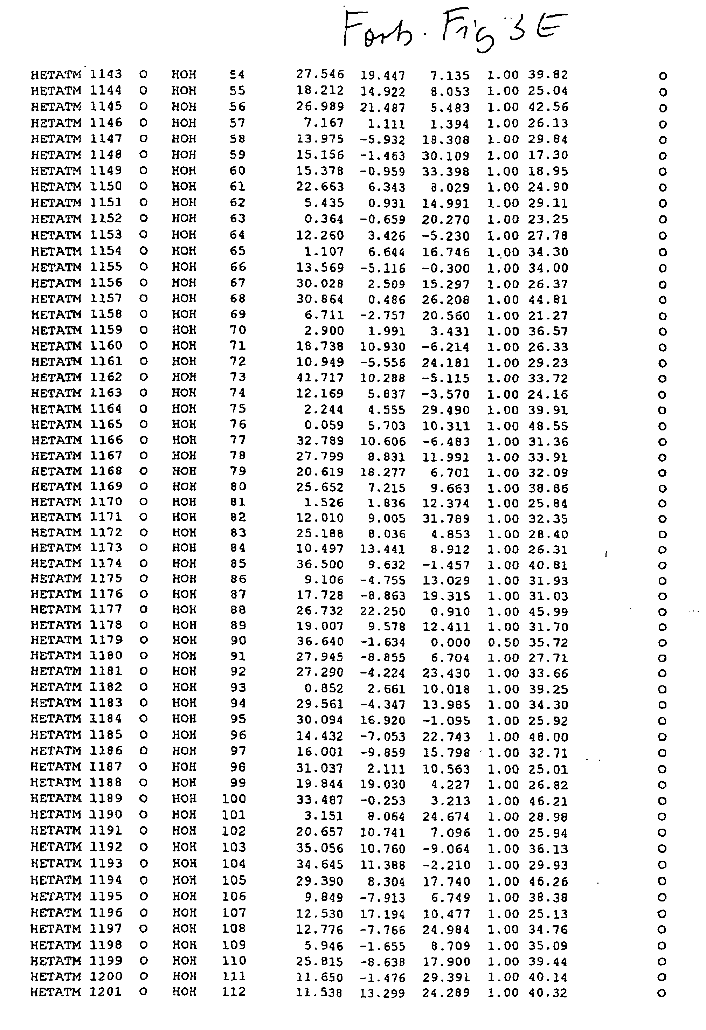

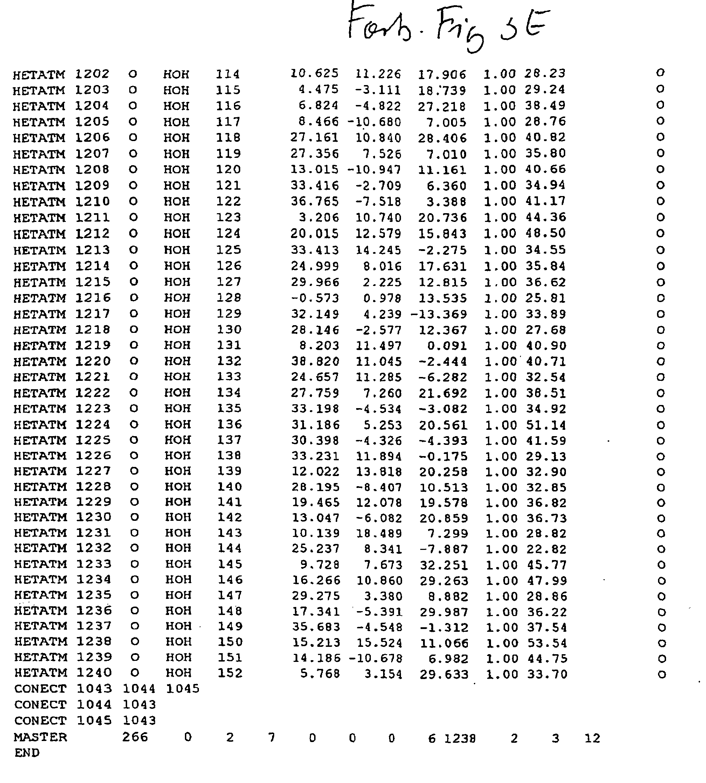

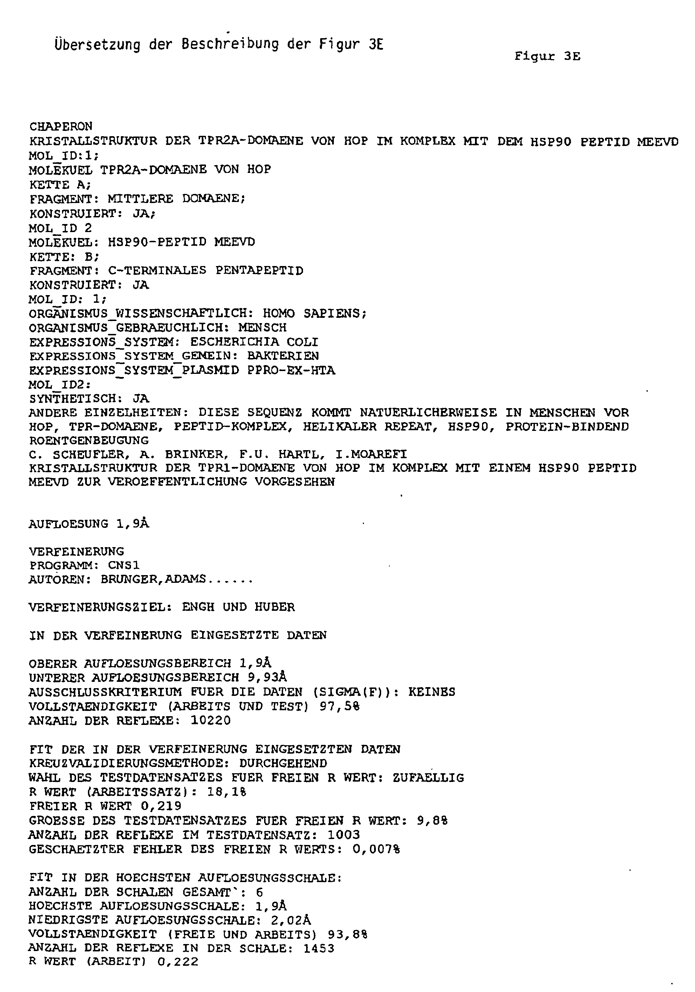

Unter dem Begriff Derivat eines TPR-Strukturmotivs oder einer TPR-Domäne eines Hop-Proteins werden solche Primärsequenzen verstanden, die die Raumform, d.h. also die Tertiärstruktur der TPR-Domäne oder des TPR-Strukturmotivs, wie sie gemäß Figuren 3C, 3D oder 3E ermittelt wurden, weitgehend aufrechterhält und nur lokale strukturelle Abweichungen erlaubt. Nach Überlagerung der Tertiärstruktur der Primärsequenz des Derivats mit einer der Ausgangsstrukturen gemäß Figur 3C, 3D oder 3E ist eine mittlere Standardabweichung für die Rückgrat-Koordinaten (rmsd) von weniger als 5 Å, insbesondere weniger als 3 und ganz besonders weniger als 2 Å bevorzugt.

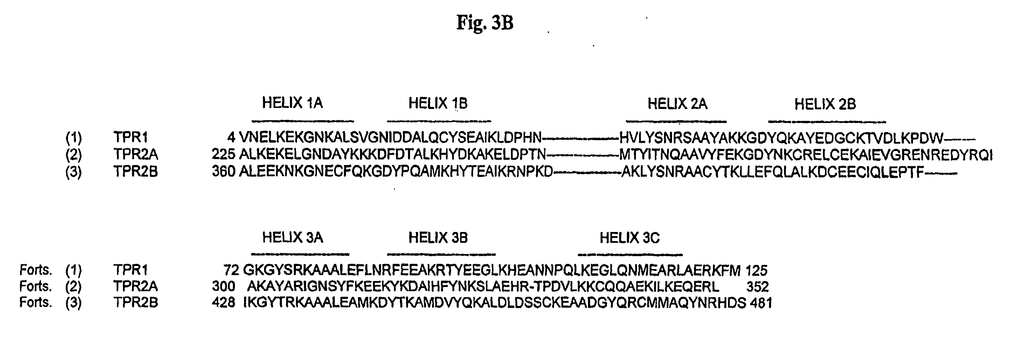

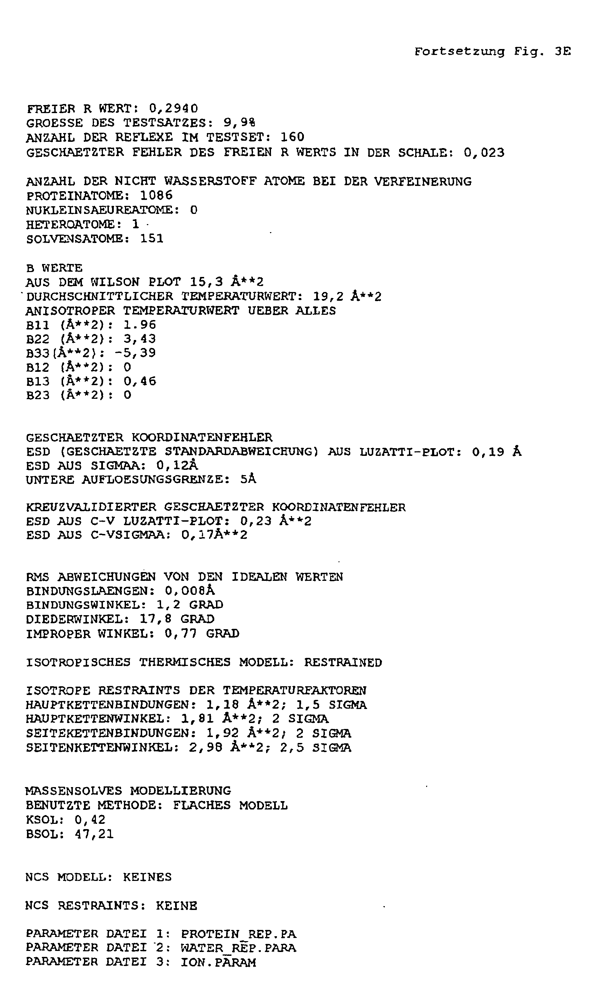

Insbesondere werden in diesem Zusammenhang solche Aminosäuresequenzen als Derivate bezeichnet, die nur konservative Substitutionen, d.h. den Austausch von bspw. polaren gegen polare Aminosäuren oder von hydrophoben gegen hydrophobe Aminosäuren (z.B. Leucin gegen Isoleucin oder Valin oder umgekehrt oder Serin gegen Threonin oder umgekehrt) haben, verstanden. Als Abschnitt oder Fragment eines TPR-Strukturmotivs oder einer TPR-Domäne eines Hop-Proteins werden solche Sequenzen verstanden, die im,Unterschied zur physiologischen Aminosäuresequenz insbesondere am N- oder C-Terminus Deletionen aufweisen, ggf. aber auch intrasequentielle Deletionen in den vorgenannten und bspw. in den Figuren 3A und 3B wiedergegebenen Sequenzen. Hierbei wird es sich typischerweise um mindestens eine Deletion handeln, wobei in jeder Deletion vorzugsweise weniger als zehn, insbesondere weniger als fünf, Aminosäuren, ganz besonders bevorzugt ein oder zwei Aminosäuren gegenüber der nativen Sequenz deletiert sein können.

In einer bevorzugten Ausführunqsform der vorliegenden Erfindung weist der Kristall eine Raumform eines Polypeptids auf, wobei das Polypeptid mindestens eine der Aminosäuresequenzen (1), (2) oder (3), wie in Figur 3B wiedergegeben, enthält. Weiterhin sind solche Kristalle mit Raumformen von Polypeptiden bevorzugt, die mindestens eine Aminosäuresequenz einer TPR-Domäne enthalten, wobei diese Sequenz(en) denjenigen Aminosäuresequenzen entsprechen, die bei Hop-Proteinen eukaryotischen Ursprungs auftreten. Gegebenenfalls kann es sich auch um Derivate bzw. Abschnitte oder Fragmente im vorgenannten Sinn derartiger Sequenzen von Hop-Proteinen eukaryotischen Ursprungs handeln.

Ganz besonders bevorzugt sind allerdings Kristalle mit Raumformen von Polypeptiden, die ein oder mehr TPR-Strukturmotiv/e oder TPR-Domäne/n eines Hop-Proteins humanen Ursprungs bzw. Abschnitte oder Derivate derselben aufweisen. Ganz besonders bevorzugt sind Kristalle mit Raumformen von Polypeptiden, wobei das Polypeptid die Aminosäuresequenz eines eukaryotischen, insbesondere humanen, Hop-Proteins, ggf. auch ein Derivat und/oder einen Abschnitt eines solchen eukaryotischen, vorzugsweise humanen, Hop-Proteins.

Weiterhin werden solche Kristalle mit Raumformen beansprucht, die sowohl ein Polypeptid, enthaltend mindestens ein TPR-Strukturmotiv eines TPR-Domänen enthaltenden Proteins, vor allem eines Hop-Proteins, als auch mindestens eine weitere Verbindung aufweisen. Hierbei wird es sich typischerweise um Verbindungen handeln, die als Liganden an das Polypeptid binden können, so daß die Raumform einen Komplex aus Polypeptid und mindestens einer weiteren auf kovalente oder nicht-kovalente Art gebundenen Verbindung darstellt. Hierbei kann es sich bei der Verbindung bzw. den Liganden um ein physiologisch auftretendes Molekül oder auch um ein nicht-physiologisch auftretendes Molekül handeln. Gegebenenfalls können, sofern mehr als ein Ligand in der Raumform auftritt, auch Kombinationen von physiologisch bzw. nicht-physiologisch auftretenden Molekülen in der Raumform enthalten sein. Bevorzugt sind dabei solche Liganden, die insbesondere unter physiologischen Bedingungen mit dem Polypeptid in Wechselwirkung treten und vorzugsweise hochaffines Bindungsverhalten zeigen (vorzugsweise K_{d}<30 µM). Hierbei kann es sich beispielsweise um Chaperon-Proteine oder Abschnitte oder Derivate derselben handeln, die mit den entsprechenden in einer erfindungsgemäßen Raumform auftretenden Polypeptiden, enthaltend TPR-Strukturmotive bzw. TPR-Domänen eines TPR-Domänen enthaltenden Proteins, vor allem eines Hop-Proteins, in Wechselwirkung treten.

Vorzugsweise wird erfindungsgemäß ein Kristall mit einer Raumform eines Polypeptids in Kombination mit Abschnitten von physiologischen Liganden offenbart. Dabei handelt es sich beispielsweise um die für die Wechselwirkung mit Hop-Proteinen relevanten Sequenzabschnitte bzw. Domänen von Chaperon-Proteinen, beispielsweise Hsp70 und/oder Hsp90. Typischerweise wird der Ligand, vorzugsweise ein physiologischer Ligand, an die in einem erfindungsgemäßen Raumform-Polypeptid enthaltene Sequenz eines TPR-Strukturmotivs binden bzw. insbesondere mit Aminosäuren einer im Polypeptid enthaltenen Aminosäuresequenz einer TPR-Domäne interagieren. Der Ligand kann selbst ein Polypeptid, ein Oligopeptid, ein Dipeptid oder ein synthetisch modifiziertes Derivat eines Poly-, Oligo- oder Dipeptids, insbesondere von physiologisch die Wechselwirkung vermittelnden Abschnitten von Chaperon-Proteinen, aber auch ein Peptidomimetikum oder ein organischchemisches Molekül mit einem Molekulargewicht von typischerweise <5000 sein.

Ganz besonders bevorzugt sind Kristalle mit Raumformen von Polypeptiden mit einem oder mehr Ligand/en, wobei der Ligand einen Abschnitt der C-terminalen Aminosäuresequenz eines Chaperon-Proteins, vorzugsweise von Hsp70 und/oder Hsp90, enthält oder ggf. aus diesem Abschnitt besteht. Der Ligand wird dann, wenn er nicht die gesamte C-terminale Domäne des Chaperon-Proteins aufweist, typischerweise die 5 bis 50, vorzugsweise 5 bis 25, ganz besonders bevorzugt 5 bis 12, C-terminalen Aminosäurereste eines solchen Chaperon-Proteins umfassen. Ganz besonders bevorzugt sind Kristalle mit Raumformen, die insbesondere an ein TPR-Strukturmotiv bzw. eine TPR-Domäne gebundene Liganden zeigen, wobei die Liganden vorzugsweise eine Bindungsaffinität von K_{d}<50 µM haben und typischerweise dadurch zumindest in vitro, vorzugsweise auch in vivo, die physiologische Funktion von Hop-Proteinen blockieren. Dabei wird sich der inhibitorische Charakter des Liganden typischerweise dadurch funktionell ergeben, daß die Bindungsstelle(n) von Hop-Proteinen für die Chaperon-Proteine mit dem Liganden besetzt sind, so daß das Hop-Protein seine physiologische Adapterfunktion in Hinblick auf das physiologische Zusammenspiel von Hsp70 und Hsp90 verliert. Ganz besonders bevorzugt sind daher Kristalle mit Raumformen von Polypeptiden, die mindestens ein Hop-TPR-Strukturmotiv oder eine TPR-Domäne enthalten, wobei der Ligand an das TPR-Strukturmotiv und/oder die TPR-Domäne andockt und gleichzeitig als Inhibitor der Interaktion zwischen den Proteinen Hop und Hsp70 und/oder den Proteinen Hop und Hsp90 wirkt. Typischerweise wird das Polypeptid in der erfindungsgemäßen Raumform sowohl Bindungsstellen für Hsp70 als auch für Hsp90 aufweisen und kann daher ggf. sowohl Liganden, die an die Hsp70-Bindungsstelle gebunden sind, als auch Liganden, die an die Hsp90-Bindungsstelle gebunden sind, aufweisen.

Bei der in erfindungsgemäßen Kristallen auftretenden Raumform eines Polypeptids der zuvor offenbarten Art, ggf. in Kombination mit einem oder mehreren Ligand/en, wird es sich um eine durch NMR-Strukturanalyse gewonnene Raumform (Wüthrich, NMR-Spectroscopy, 1986) oder aber um eine Kristallform handeln. Die Kristallform wird als Ergebnis nach Kristallisierung des Polypeptids und ggf. weiteren Komponenten, bspw. mindestens einem Liganden, mit nachfolgender röntgenkristallographischer Strukturaufklärung erhalten (Stout und Jensen, X-RAY Structure Determination, Wiley, 1989; die Offenbarung aus Stout und Jensen wird vollumfänglich in die vorliegende Offenbarung in Hinblick auf die Durchführung röntgenkristallographischer Experimente einbezogen) .

Kristallformen zeichnen sich auch dadurch aus, daß sie als dreidimensionale Struktur, charakterisiert durch Strukturkoordinaten für jedes einzelne, die Struktur aufbauende Atom, Bestandteil einer symmetrischen Anordnung in einem Kristall sind. Dabei ist es bevorzugt, daß eine Kristallform, die mindestens ein Polypeptid mit mindestens einem TPR-Strukturmotiv oder vorzugsweise mindestens einer TPR-Domäne, ganz besonders von einem Hop-Protein, enthält, nach Überlagerung mit den in den Fig. 3C, 3D oder 3E aufgelisteten Strukturkoordinaten für das mindestens eine an der Bindungsreaktion beteiligte TPR-Strukturmotiv oder die mindestens eine TPR-Domäne eine mittlere Standardabweichung (rmsd) von weniger als 2,5 Å, vorzugsweise von weniger als 2 Å, aufweist.

Liegt die Raumform als Kristallform vor, so wird ein die Kristallform aufweisender erfindungsgemäß Kristall typischerweise neben den Atomen des Polypeptids bzw. ggf. des/der Ligand/en (außer Wasserstoffatomen) weitere Metallionen enthalten, beispielsweise Erdalkalioder Alkalimetallionen, insbesondere Calciumionen, aber auch vor allem zur Phasenermittlung geeignete Schwermetallionen, wie z.B. Gold-, Nickel- oder Quecksilberionen.

Ganz besonders bevorzugt sind Kristalle mit Ramformen von Polypeptiden, die im Kristall als Kristallform vorliegen, dann, wenn die Kristallform des Polypeptids mindestens ein TPR-Strukturmotiv eines Hop-Proteins, und zwar insbesondere mindestens eine der Aminosäuresequenzen, die gemäß Figur 3A als Sequenzen (1), (2), (4), (5), (7) und/oder (8) bezeichnet werden, (oder deren Derivate oder Fragmente) enthält, wobei diese Sequenzen Strukturkoordinaten aufweisen, wie sie für die sequenzen (1), (4) und (7) in Figuren 3C bzw. 3D und für die Sequenzen (2), (5) und (8) in Figur 3E angegeben sind. Die Figuren 3C und 3D geben die Strukturkoordinaten für die TPR-Domäne TPR1 von Hop wieder, während Figur 3E die Strukturkoordinaten der TPR-Domäne TPR2A von Hop auflistet. Die vorgenannten Primärsequenzen (1) , (2), (4), (5), (7) und (8) gemäß Figur 3A können aufgrund ihrer Aminosäuresequenzen ihren jeweiligen Tertiärstrukturen, die in den Figuren 3C, 3D und 3E angegeben sind, ohne weiteres zugeordnet werden. Hierzu sind die Strukturkoordinaten der in den vorgenannten Sequenzen auftretenden Atome den Figuren 3C, 3D und 3E zu entnehmen. Die Auflistung der Atome in den Figuren 3C, 3D und 3E erfolgt jeweils sequentiell Aminosäure für Aminosäure vom N- zum C-Terminus.

Ganz besonders bevorzugt sind schließlich Kristalle mit Raumformen eines Polypeptids im Kristall als Kristallform, wenn das Polypeptid in der Kristallform mindestens eine TPR-Domäne eines Hop-Proteins (Struktur der Sequenzen (1) und (2) gemäß Figur 3B) mit allen entsprechenen Strukturkoordinaten, wie in Figur 3C und Figur 3D für die TPR-Domäne TPR1 und in Figur 3E für die TPR-Domäne TPR2A von Hop wiedergegeben, enthält.

Kristalle, die Raum- bzw. Kristallformen aufweisen, wie vorstehend beschrieben, sind nach symmetrischen Gesetzmäßigkeiten angeordnet. Hierunter fallen Kristalle all jener Kristallformen, die gemäß vorliegender Erfindung offenbart werden. Gemäß der vorliegenden Erfindung werden nämlich Kristalle beansprucht, die aus Einheitszellen aufgebaut sind, wobei die asymmetrische Einheit in der Einheitszelle des Kristalls mindestens ein Polypeptid und ggf. mindestens eine weitere Verbindung aufweist und wobei weiterhin das Polypeptid im Kristall eine Raumform als Kristallform einnimmt, wie vorhergehend offenbart.

Vorzugsweise wird der Kristall eine Raumgruppe aufweisen, die monoklin, tetragonal, orthorhombisch, kubisch, triklin, hexagonal oder trigonal/rhombohedral ist. Hierbei kann es sich um native Kristalle, Derivatkristalle oder auch Cokristalle handeln. Typischerweise wird dabei die Raumgruppe des eine Kristallform aufweisenden Kristalls die Raumgruppe P2₁, C2 oder P4₁ sein. Grundsätzlich können aber Kristalle mit Kristallformen in allen proteinkristallographisch möglichen Raumgruppen auftreten. Ganz besonders bevorzugt sind solche erfindungsgemäßen Kristalle, deren Einheitszelle Zellkonstanten von ungefähr a = 31,2 Å, b = 43,8 Å, c = 38,3 Å und β = 101,8° oder a = 75,5 Å und c = 42,9 Å aufweist.

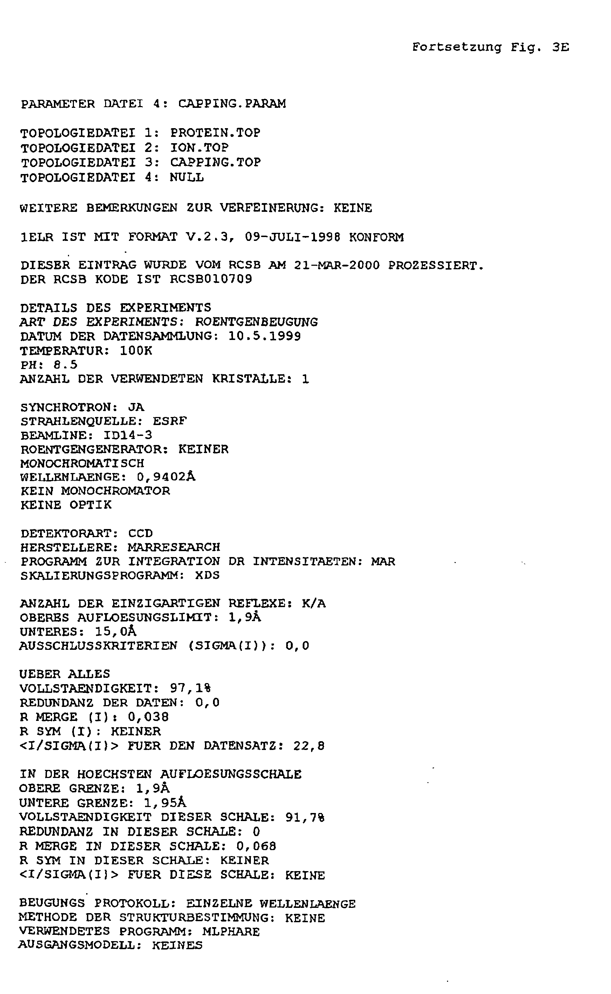

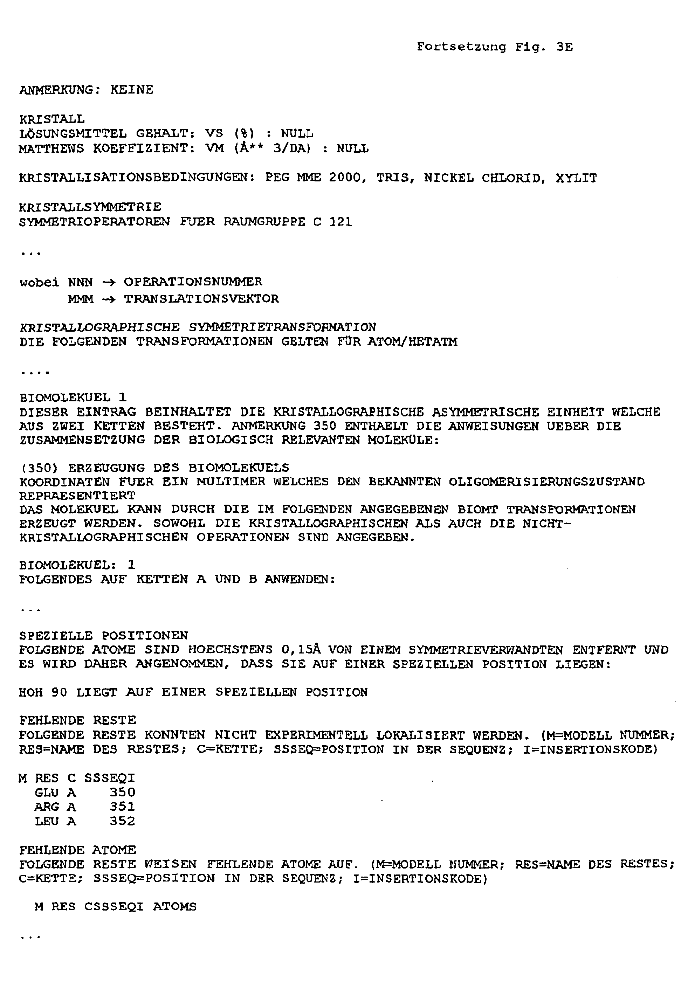

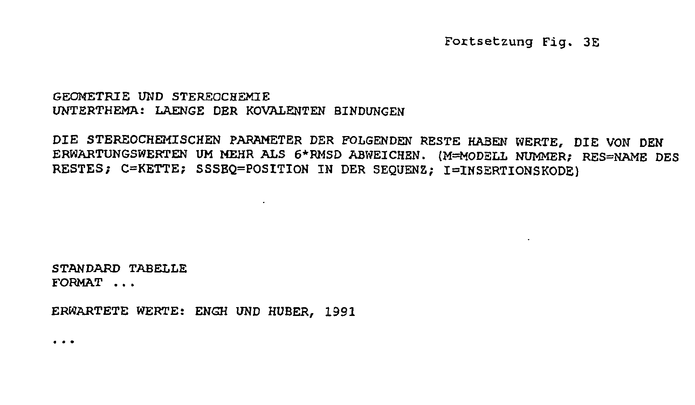

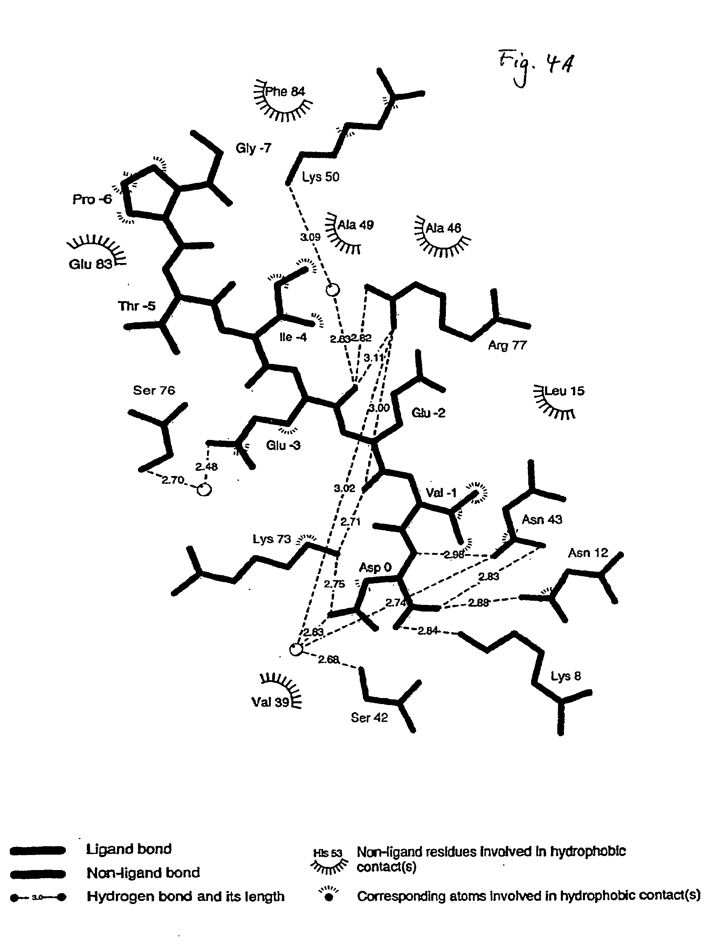

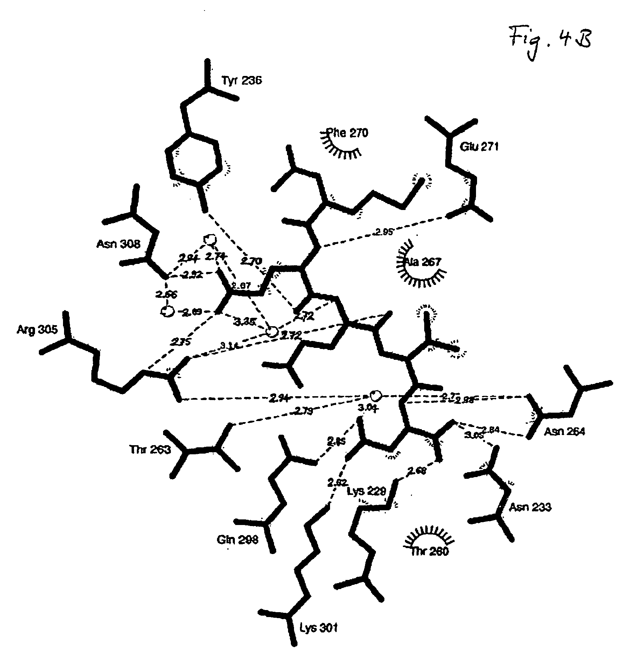

Als weiterer Erfindungsgegenstand werden Verbindungen offenbart, die als Liganden an TPR-Strukturmotive oder TPR-Domänen, die jeweils wiederum Bestandteile längerer Polypeptidketten sein können, vorzugsweise an TPR-Strukturmotive oder -Domänen von Hop-Proteinen binden können zur Anwendung als Arzneimittel. Als Proteine mit TPR-Domänen, an die erfindungsgemäße Liganden andocken können, kommen bspw. auch PP5, FKBP51, FKBO52, Cyp40, TOM34, TOM70, CNS1-sc, TTC1, TTC2, TTC3, TTC4, IRSP, SGT oder KIAA0719 in Betracht Erfindungsgemäße Verbindungen bilden nicht-kovalente Wechselwirkungen mit der Hauptkette und oder den Seitenketten von Aminosäuren, die Bestandteil einer TPR-Domäne, vorzugsweise einer TPR-Domäne eines Hop-Proteins sind oder eines der vorgehend genannten Proteine. Durch diese Bindung des Liganden wird vorzugsweise die physiologische Adapterfunktion des Hop-Proteins, das die Chaperon-Proteine Hsp70 und Hsp90 in mittelbaren Kontakt bringt, blockiert. Daher bindet in einer bevorzugten Ausführungsform die Verbindung mindestens irgendein TPR-Strukturmotiv aufweisenders Hop-Proteins so, daß die physiologische Bindung von Chaperon -Proteinen insbesondere Hsp70 und/oder Hsp90, an das TPR-Strukturmotiv bzw. die TPR-Strukturdomäne, blockiert wird. Die Liganden werden dabei vorzugsweise mit den Aminosäure Lys8, Asn12, Asn43, Lys73 und Arg77 (entsprechend der Zählweise im Hop-Protein) (bspw. Fig. 3) in Wechselwirkung treten. Ganz besonders bevorzugt .werden die Liganden mit der TPR Domäne einen Teil oder alle Wechselwirkungen ausbilden, die in den Figuren 4A und 4B schematisch für die jeweils gebundenen Peptide dargestellt sind. Insbesondere werden die Liganden jene Wasserstoffbrückenbindungen, hydrophoben Kontakte, van-der-Waals-Wechselwirkungen oder elektrostatischen Wechselwirkungen eingehen, die auch die Peptide mit den komplementären Aminosäureresten der TPR-Domänen eines Hop-Proteins eingehen. D.h. ein erfindungsgemäßer Ligand wird vorzugsweise an äquivalenten sterischen Positionen mit den gebundenen Peptiden äquivalente funktionelle Gruppen aufweisen. Insbesondere wird ein erfindungsgemäßer Ligand ebenfalls eine Doppel-Carboxylat-Funktion besitzen, um in der TPR-Domäne eines Hop-Proteins veranker werden zu können. Im Zusammenhang mit den potentiellen Wechselwirkungen des Liganden einer TPR-Domäne wird auf die Beschreibung der Figur 4 und auf die Darstellung der Ergebnisse bei der Beschreibung der Ergebnisse der Ausführungsbeispiele verwiesen. Ein erfindungsgemäßer Ligand wird mindestens einige, ggf. alle der dort beschriebenen Wechselwirkungen mit der TPR-Domäne ausbilden, so daß eine Bindungsaffinität Kd von weniger als 100 µM, vorzugsweise weniger als 50 µM, ganz besonders bevorzugt weniger 20 µM sichergestellt ist.

Insgesamt wird ein erfindungsgemäßer Ligand also vorzugsweise strukturelle Ausgestaltung haben, die dem Bindungsbereich einer TPR-Domäne, eines Hop-Proteins, vor allem den strukturellen Vorgaben aus den Figuren 3C, 3D oder 3E, komplementär ist. Bei diesen Liganden mit vorzugsweise Inhibitorfunktion für eine Chaperon- oder Co-Chaperon-Funktion kann es sich um modifizierte oder unmo difizierte Di-, Oligo- oder Polypeptide handeln. Peptidomimetikum eines Di- oder Oligopeptids ist denkbar. Bei den Peptidomimetika wird es sich vorzugsweise um solche Verbindungen handeln, deren Rückgrat keine amidartigen Bindungen, sondern andere chemische Brücken aufweisen, um die proteolytische Spaltung zu vermeiden. Eine erfindungsgemäße Inhibitorverbindung zur Anwendung als Araneimittel durch Strukturaufklärung mit Hilfe der eines Hop-Proteins bspw. ein modifiziertes oder unmodifiziertes. erfindungsgemäßes Peptid, das die Aminosäuren EEVD vorzugsweise am C-Terminus enthalten kann.

Besonders bevorzugt als erfindungsgemäße Inhibitoren zur Anwendung als Arzneimittel sind modifizierte oder unmodifizierte Oligopeptide, die vor zugsweise am C-Terminus die Aminosäuresequenzen GPXIEEVD (Ein-Buchstaben-Code) oder SXMEEVD enthält, wobei X für eine beliebige, natürlich auftretende Aminosäure steht.

Ein weiterer Gegenstand der vorliegenden Erfindung sind Verfahren zur Identifizierung einer Verbindung, die die Eigenschaft besitzt, als Inhibitor der Wechselwirkung zwischen Hop-Protein und dem Chaperon-Protein, insbesondere Hsp70 oder Hsp90, vor allem humanem Hsp70 und/oder Hsp90, zu wirken. Insbesondere bevorzugt ist ein solches Verfahren dann, wenn die Verbindung mit Ligandenfunktion an einen Strukturbereich einer TPR-Domäne, insbesondere im Bereich des aktiven Bindungszentrums, bindet. Ein solches Verfahren ist dadurch gekennzeichnet, daß (a) eine Raumform eines Kristalls nach den Ansprüchen 24 bis 26 durch Strukturaufklärung verhalten wird, (b) die Struktur der Raumform mit Hilfe der Strukturkoordinaten in drei Dimensionen dargestellt werden (c) sterische Eigenschaften und/oder funktionelle Gruppen einer Verbindung so gewählt werden, daß Wechselwirkungen zwischen der Verbindung und den Hauptund/oder Seitenketten des Polypeptids in dem Bindungsbereich ausgebildet oder ggf. optimiert werden. Nach Maßgabe dieser Wechselwirkungen werden erfindungsgemäß geeignete Liganden, insbesondere geeignete inhibitorische Liganden, die die Wechselwirkung zwischen Hop-Protein und Hsp70 und/oder Hsp90 blockieren, ermittelt.

Die Darstellung der Strukturkoordinaten einer erfindungsgemäßen Kristallform erfolgt vorzugsweise durch graphische Darstellung mit Hilfe entsprechender Computerprogramme auf einem Computerbildschirm. Anhand der, bezogen auf potentielle Liganden, komplementären Anordnung der Haupt- und Seitenketten der Kristallform, beispielsweise im Bindungsbereich einer TPR-Domäne eines Hop-Proteins kann nicht-automatisiert nach Erfahrung des Operators geeignete Liganden mit entsprechenden chemischen und/oder sterischen Eigenschaften identifiziert, am Bildschirm konstruiert und schließlich deren Bindungsverhalten simuliert werden.

Vorzugsweise aber erfolgt die Auswahl geeigneter Liganden jedoch automatisiert dadurch, daß Computerdatenbanken, die eine Vielzahl von Verbindungen enthalten, durchsucht werden. Die Suche wird auf die zuvor erfolgende Charakterisierung von geometrischen, chemischen und/oder physikalischen Eigenschaften für die gewünschten Liganden, beispielsweise Verbindungen mit struktureller und/oder funktioneller Ähnlichkeit zu Verbindungen, mit der Eigenschaft, als Ligand an Strukturbereiche eines Hop-Proteins oder strukturell mit einem Hop-Protein verwandter Proteine, insbesondere PP5, FKBP51, FKBP52, Cyp40, TOM34, TOM70, CNS1-sc, TTC1, TTC2, TTC3, TTC4, IRSP, SGT oder KIAA0719, zu binden, wobei die Verbindung nicht-kovalente Wechselwirkung mit der Hauptkette und/oder den Seitenketten von Aminosäuren, die Bestandteil einer TPR-Domäne eines Hop-Proteins oder eines strukturell mit einem Hop-Protein verwandten Proteins sind, eingeht, gestützt. Zu durchmusternde Datenbanken enthalten natürlich auftretende wie auch synthetische Verbindungen. Beispielsweise können die in der CCDC (Cambridge Crystal Data Center, 12 Union Road, Cambridge,.GB) gespeicherten Verbindungen für eine derartige Suche herangezogen werden. Aber auch die bei Tripos (s. Zitat a.a.O.) erhältlichen Datenbanken, nämlich Aldrich, Maybridge, Derwent World Drug Index, NCI und/oder Chapman & Hall können durchsuch werden. Die folgenden Computerprogramme können für eine derartige Durchmusterung eingesetzt werden: insbesondere das Programm "Unity", "FLEX-X" (Rarey et al. J. Mol. Biol. 261, 470-489, 1996), "Cscore" (Jones et al., J. Mol. Biol. 245, 43, 1995) aus der Sybyl Base-Umgebung des Tripos-Programmpakets.

Im folgenden wird die Durchführung eines erfindungsgemäßen Verfahrens zur Computer-gestützten Identifizierung potentieller Liganden näher beschrieben. Zunächst muß der gewünschte Bindungsbereich eines Liganden in einer erfindungsgemäßen Kristallform definiert werden. Bei dem Liganden wird es sich typischerweise um einen solchen mit inhibitorischen Eigenschaften handeln, aber auch Aktivatoren sind denkbar. Der Bindungsbereich wird durch entsprechende Parameter, beispielsweise Atomabstände, Wasserstoffbrücken-Bindungspotentiale, hydrophobe Bereiche und/oder Ladungen, charakterisiert und auf dieser Basis Randbedingungen für die chemischen, physikalischen und/oder geometrischen Eigenschaften des Liganden definiert. Ganz besonders bevorzugt sind an der Bindung mindestens eine der bereits zuvor spezifizierten Aminosäuren Lys8, Asn12, Asn43, Lys73 und Arg77, insbesondere mit den vorgenannten Seitenketten derselben, beteiligt. Daher wird auch für ein vorliegendes erfindungsgemäßes Verfahren zur Identifizierung von Verbindungen auf die vorangegangene Offenbarung zum Erfindungsgegenstand "Verbindung" vollinhaltlich Bezug genommen. Computerprogramme identifizieren in entsprechenden Datenbanken dann solche verbindungen, die die zuvor eingeführten Bedingungen erfüllen. Hierbei ist es besonders bevorzugt, das Programmpaket Sybil Base (Tripos, 1699 South Hanley Road, St. Louis, Missouri, USA) zu verwenden. Besonders bevorzugt ist es dabei, daß die zu durchsuchende Datenbank Verbindungen unter Angabe ihrer jeweiligen dreidimensionalen Strukturen zur Verfügung stellt. Sollte dies nicht gegeben sein, wird für ein erfindungsgemäßes Verfahren vorzugsweise in einem Verfahrensschritt (d) ein Computerprogramm eingesetzt werden, das vor der Prüfung, ob die vorgegebenen Randbedingungen von einem Liganden erfüllt sind, zunächst deren dreidimensionale Struktur berechnet (z.B. das Programm "CONCORD" aus der Sybyl-Umgebung von Tripos Inc.).

Typischerweise wird in einem Verfahrensschritt (e) das Wechselwirkungspotential zwischen einer identifizierten Verbindung, beispielsweise im Rahmen einer automatisierten Suche einer Verbindung aus einer Computerdatenbank, und dem gewünschten Bindungsbereich in einer Kristallform ermittelt. Ganz besonders bevorzugt ist ein erfindungsgemäßes Verfahren dann, wenn es zur Identifizierung von Verbindungen, die an eine Kristallform mit den Strukturkoordinaten der Fig. 3C, 3D oder 3E andocken sollen, dient. Die Stärke der gemäß Verfahrensschritt (e) ermittelten Wechselwirkung zwischen einer Verbindung aus einer Computerdatenbank und einer erfindungsgemäßen Kristallform geben Anhaltspunkte für deren Eignung, als Liganden eingesetzt werden zu können.

Nicht automatisiert gestaltet sich ein Verfahren zur Identifizierung geeigneter Verbindungen mit Ligandencharakter wie folgt. Eine Gerüstverbindung als Ausgangapunkt der Identifizierung wird in den durch die zu identifizierende Verbindung auszufüllenden Raum im Innern oder an der Oberfläche der Kristallform, z. B. in den Bindungsbereich einer TPR-Domäne in dem kristallisierten Polypeptid, manuell eingefügt. Für den nach Einfügung der Gerüststruktur noch verbliebenen Raum wird nach Fragmenten gesucht, die in Wechselwirkung mit der umgebenden Kristallform treten können und sich an die Gerüststruktur anlagern lassen. Diese Suche nach geeigneten Fragmenten erfolgt also nach Maßgabe der geometrischen und/oder physikochemischen Gegebenheiten der dreidimensionalen Struktur. Die Suche nach geeigneten Fragmenten kann beispielsweise als automatisierte Computersuche unter Vorgabe entsprechender Randbedingungen geführt werden. Etwaige durch den Operator und/oder durch die Computersuche ermittelten Fragmente werden nach Maßgabe chemischer Gesetzmäßigkeiten an die Ausgangsgerüststruktur des Startmodells graphisch angelagert und nach jedem derartigen Schritt das Wechselwirkungspotential mit dem Zielstrukturbereich in der Kristallform errechnet. Die Vorgehensweise erfolgt so lange, bis das Wechselwirkungspotential zwischen der zu identifizierenden Verbindung und dem Zielstrukturbereich optimiert ist.

Die Vorgehensweise der Schritte (c), (d) und (e) kann zyklische so lange wiederholt werden, bis eine Verbindung oder eine Verbindungsklasse in bezug auf ihr Bindungsverhalten, berechnet nach einem Wechselwirkungspotential, das dem jeweiligen Computerprogramm als Algorithmus zugrundeliegt, optimiert ist. Die durch eine relativ grobe Charakterisierung des Bindungebereichs der Kristallform zunächst erhaltene große Zahl an potentiell bindungsfähigen Verbindungen kann durch weitergehende Vorgaben physikalisch-chemischer oder sterischer Charakteristika an die gewünschte Zielverbindung zunehmend reduziert werden.

Insbesondere bietet sich hierfür auch eine sinnfällige Kombination der nicht-automatisierten und der automatisierten Suchverfahren nach geeigneten Verbindungen an. So etwa könnte eine zunächst durch automatisierte Computersuche aus Computerdatenbanken identifizierte Verbindung nicht-automatisiert durch Anlagerung von Fragmenten mit geeigneten funktionellen Gruppen verbessert werden.

Schließlich ist es im Rahmen der vorliegenden Erfindung bevorzugt, die durch derartige erfindungsgemäße Verfahren per automatisierter Computersuche erhaltenen Verbindungen zu synthetisieren oder, falls bereits synthetisiert und zugänglich, einer chemischen Bibliothek zu entnehmen und in einem geeigneten biologischen Testsystem in einem Verfahrensschritt (f) auf ihre biologische Wirksamkeit hin zu untersuchen. Abhängig vom Ergebnis des biologischen Testsystems, bei dem es sich z. B. um einen Ligandenbindungsassay handeln kann, können dann weitere chemische Modifikationen der zuvor ermittelten Verbindung oder der Verbindungsklase vorgenommen werden. Hierbei kann sich dann insbesondere die Anwendung von Programmpaketen zur Identifizierung geeigneter Fragmente, die gegen vorhandene Fragmente an der zuvor identifizierten Verbindung ausgetauscht oder an dieselbe zusätzlich angelagert werden könnten, als sinnvoll erweisen.

Ein weiterer Gegenstand der vorliegenden Erfindung sind gleichfalls Verfahren zur Identifizierung einer Verbindung mit der Eigenschaft, als Ligand, typischerweise als Inhibitor der Wechselwirkung zwischen einem Hop-Protein und einem Chaperon-Protein wirken zu können, wobei in einem solchen erfindungsgemäßen Verfahren in einem Verfahrensschritt (a) ein biologisches Testsystem vorangestellt wird, anhand dessen ein sog. "Screening" nach geeigneten Zielverbindungen durchgeführt wird. Auch hier können bevorzugt ein Bindungsassay als biologisches Testsystem dienen. In den weiteren Verfahrensschritten werden zunächst gemäß (b) solche Verbindungen (beispielsweise aus einer Bibliothek chemischer Verbindungen identifiziert, die ein positives Ergebnis im biologischen Test gezeigt haben. Diese Verbindungen, beispielsweise inhibierend oder gegebenenfalls auch aktivierend, werden in bezug auf ihre beispielsweise geometrischen und/oder chemischen Eigenschaften, insbesondere in bezug auf ihre dreidimensionale Struktur, charakterisiert (Verfahrensschritt (c)). Sofern die dreidimensionale Struktur der als Treffer im biologischen Test ermittelten Verbindungen nicht a priori bekannt ist, kann dieselbe durch Methoden der Strukturaufklärung, nämlich Röntgenkristallographie und/oder NMR-Spektroskopie, oder auch durch Modellierungen oder z.B. semi-quantenchemische Berechnungen ermittelt werden. In die gemäß Verfahrensschritt (d) als dreidimensionale Struktur (Kristallform) dargestellten atomaren Strukturkoordinaten eines erfindungsgemäße Kristalls werden dann gemäß (e) die im Rahmen der Verfahrensschritte (b) und (c) erhaltenen Verbindungen eingefügt. Hierbei kann es sich um Verbindungen handeln, die an einem für die physiologische Bindungsstelle relevanten Abschnitt oder aber auch an die Oberfläche des Polypeptids in der Kristallform binden. Das Einfügen der Verbindung in die Kristallform kann manuell nach der Erfahrung des Operators erfolgen oder aber auch automatisiert, indem mit Hilfe entsprechender Computerprogramme ("Dock" Kuntz et al., 1982, J. Mol. Biol. 161, 269-288, Sybyl/Base "FLEX-X", s. Zitat a.a.O.) eine Positionierung des Liganden mit der stärkstmöglichen Wechselwirkung zwischen Ligand und dem Zielstrukturbereich ermittelt wird (Verfahrensschritt (e)).

Indem eine derart erhaltene Verbindung graphisch in Kombination mit der in der Kristallform vorliegenden Struktur dargestellt wird, können weitere Verfahrensschritte erfolgen, die die Wirksamkeit der Zielverbindung verbessern. Insbesondere kann eine derart, bereits als geeignet identifizierte Verbindung als Basis ("template") für noch wirksamere Verbindungen, z. B. Verbindungen mit noch höherer Bindungskonstante, dienen. In diesem Zusammenhang können die bereits gemäß Ansprüchen 31 bis 36 beschriebenen Verfahren und Ansätze zum Einsatz kommen. Bevorzugt ist eine Vorgehensweise, die insoweit zyklisch ist, als nach dem "Screening" im biologischen Testsystem eine strukturelle Darstellung erfolgt und mit Hilfe von Computermethoden auf der Basis der im biologischen Testsystem erhaltenen Ergebnisse wirksamere Verbindungen ermittelt werden, die schließlich wiederum als Ausgangspunkt für den nächsten-Zyklus, an dessen Beginn ein biologisches Testsystem steht, dienen. Biologische Testsysteme (in vitro oder in vivo) können Aussagen über die Qualität der Verbindung, z.B. als Inhibitor der biologischen Reaktion, also des Bindungsereignisses, oder über die Bindungskonstante, die Toxizität oder die Metabolisierungseigenschaften oder gegebenenfalls über das Membrandurchtrittsvermögen der Verbindung etc., gemacht werden.

In einer alternativen Ausführungsform der vorliegenden Erfindung werden Verfahren zur Herstellung eines Kristalls mit mindestens einem Polypeptid offenbart, wobei in einem Verfahrensschritt (a) zunächst das Polypeptid in einem Expressionssystem überexprimiert, synthetisiert oder isoliert wird, (b) das gemäß (a) erhaltene Polypeptid in einem geeigneten Puffersystem gelöst wird und (c) die Kristallisierung, beispielsweise durch Dampfdiffusionsverfahren, eingeleitet wird. Typischerweise wird gemäß Verfahrensschritt (b) eine konzentrierte oder hochkonzentrierte Lösung des (der) Polypeptids/e vorliegen. Erfolgt die Kristallisierung des mindestens einen Polypeptids zu erfindungsgemäßen Kristallen, mit entsprechenden Kristallformen mit dem Ziel, die Kristalle nachfolgend zur Röntgenstrukturanalyse einzusetzen, so folgt nach der Kristallisierung die Sammlung von Röntgendiffraktionsdaten, die Bestimmung der Einheitszellkonstanten und der Symmetrie sowie die Berechnung der Elektronendichtekarten, in die das (die) Polypetid/e hineinmodelliert werden.

Ein weiterer Gegenstand der vorliegenden Erfindung sind Verfahren zur dreidimensionalen Darstellung einer Kristallform unbekannter Struktur mit mindestens einem Polypeptid, das mindestens ein an der Bindung an ein Chaperon-Protein, vorzugsweise ein Hsp70- und/oder Hsp90-Protein, beteiligtes TPR-Strukturmotiv oder vorzugsweise TPR-Strukturdomäne enthält. Ein solches Verfahren ist dadurch gekennzeichnet, daß die Kristallform mit unbekannter Struktur auf der Basis einer Kristallform eines erfindungsgemäßen Kristalls mit bekannter Struktur, beispielsweise auf Basis der in Fig. 3C. 3D oder 3E festgehaltenen Strukturkoordinaten, ermittelt wird. Hierbei gibt es verschiedene Möglichkeiten, bekannte Strukturkoordinaten von Kristallformen erfindungsgemäßer Kristalle zur Strukturaufklärung von Polypeptiden oder Polypeptidkomplexen mit bislang unbekannten 3D-Strukturen (Zielstruktur), die jedoch mit der bekannten Kristallform erfindungsgemäßer Kristalle gewisse Homologien in der Primärsequenz zeigen, einzusetzen.

Eine Möglichkeit ist in diesem Zusammenhang die Verwendung von Phaseninformation, die aus bekannten Ausgangsstrukturkoordinaten, beispielsweise den Strukturkoordinaten oder Teilen dieser Strukturkoordinaten gemäß Fig. 3C, 3D oder 3E, entnommen werden können. Hierzu wird die Phaseninformation, die im Falle einer bekannten 3D Struktur einer Kristallform eines erfindungsgemäßen Kristalls vorliegt bzw. berechnet werden kann, eingesetzt, um eine unbekannte Struktur, die sich vorzugsweise gegenüber der bekannten Struktur nur durch nicht wesentliche konformationelle Abweichungen unterscheidet (als Beispiele zu nennen wären Zielstrukturen, an die erstmals ein Ligand oder ein anderer Ligand als in der Ausgangsstruktur gebunden ist, oder Derivate, z.B. Zielstrukturen, die Mutanten der Ausgangsstruktur sind) zu lösen. Hierzu wird die Phaseninformation der gesamten oder eines Teils der bekannten Struktur mit den für die Kristallform unbekannter Struktur gesammelten Intensitäten der Reflexe kombiniert und aus dieser Kombination eine Elektronendichtekarte für die Kristallform unbekannter Struktur berechnet. Diese Methode wird als "molekulares Ersetzen" ("molecular replacement") bezeichnet. Vorzugsweise wird das molekulare Ersetzen mit dem Programmpaket X-PLORE (Brünger, Nature 355, 472-475, 1992) durchgeführt.

Eine weitere Möglichkeit, eine vorliegende erfindungsgemäße Kristallform eines erfindungsgemäßen Kristalls zur Strukturaufklarung von strukturell verwandten Sequenzen bzw. beim Vergleich der Primärstrukturen mit zumindest teilweise homologen Polypeptidketten einzusetzen, besteht erfindungsgemäß darin, daß (a) die Primärsequenz eines Polypeptids unbekannter 3D-Struktur mit einer Primärsequenz eines Polypeptids, das mindestens ein TPR-Strukturmotiv oder eine TPR-Domäne vorzugsweise eines Hop-Proteins, aufweist, verglichen wird und im Rahmen dieses Vergleichs homologe Abschnitte zwischen dem Polypeptid unbekannter Struktur und der Primärsequenz eines Polypeptids mit mindestens einem TPR-Strukturmotiv, vorzugsweise einer TPR-Bindungsdomäne eines Hop-Proteins geeignet für die Bindung an ein Chaperon-Protein, dessen Raum- oder vorzugsweise Kristallform bekannt ist, identifiziert werden, (b) die homologen Abschnitte in Anlehnung an die bekannte 3D-Struktur modelliert wird und schließlich gemäß Verfahrensschritt (c) mit Hilfe geeigneter Computerprogramme die modellierte 3D-Struktur des Polypeptids in Hinblick auf ihre sterischen Verhältnisse optimiert wird.

Das sog. "Alignment" der Primärsequenzen von zu vergleichenden Polypeptiden unbekannter bzw. bekannter 3D-Struktur gemäß (a) stellt eine zentrale Aufgabe für das Homologie-Modelling dar. Hierbei werden die ausgerichteten korrespondierenden Aminosäuren verschiedenen Kategorien zugeordnet, nämlich Positionen mit identischen, ähnlichen, entfernt ähnlichen oder unähnlichen Aminosäuren. Besondere Berücksichtigung müssen beim "Alignment" gegebenenfalls Insertionen oder Deletionen zwischen den zu vergleichenden Primärsequenzen finden. Die gemäß Verfahrensschritt (c) erfolgende Optimierung der auf der Grundlage der bekannten 3D-Struktur modellierten Zielstruktur kann durch die Methoden der Molekulardynamik-Simulation "molecular dynamics" oder durch Energieminimisierung (z.B. Sybil Base von Tripos, s. Zitat a.a.O.) erfolgen.

Besonders bevorzugt ist ein erfindungsgemäßes Verfahren zur Aufklärung von Kristallformen unbekannter Struktur dann, wenn es sich bei der Kristallform bekannter Struktur um ein Hop-Protein oder ein mit Hop verwandtes Protein, insbesondere eines der in Figur 3 genannten Proteine bzw. Proteinabschnitte, handelt und die Kristallform eines Hop-Isoproteins oder eines anderen verwandten Proteins z.B. durch "Molekulares Ersetzen" oder durch "Homologie-Modelling" aufgeklärt werden soll. Gleichfalls kann auf der Basis einer bekannten Hop-Kristallform des einen Wirtsorganismus eine Kristallform für einen Hop-Komplex, gegebenenfalls mit einem Liganden, von einem anderen Wirtsorganismus ermittelt werden.

Damit also können Strukturkoordinaten von erfindungsgemäßen Kristallformen durch Homologie-Modelling als Strukturmodelle für sequentiell homologe Polypeptide unbekannter 3D-Strukturen dienen. Im Rahmen des Homologie-Modellings kommen Programmpakete zum Einsatz, insbesondere kann mit dem Insight II Modellierungspaket (Molecular Simulations Inc.) ein derartiges Modelling durchgeführt werden. Die Beschreibung des Programmpakets Insight II wird vollumfänglich in die vorliegende Offenbarung, insbesondere in Hinblick auf das Homologie-Modelling, einbezogen.

Ein weiterer Gegenstand der vorliegende Erfindung sind Verfahren zur Identifizierung von Verbindungen, die die Wechselwirkung zwischen einem Polypeptid, enthaltend mindestens eine Aminosäuresequenz eines TPR-Strukturmotivs, vorzugsweise eines TPR-Strukturmotivs eines Hop-Proteins, oder eines Derivats einer solchen Aminosäuresequenz und einem Chaperon-Protein blockiert, wobei hierfür (a) die unbekannte 3D-Struktur eines derartigen Polypeptide, das einem Polypeptid in einer Raumform eines erfindungsgemäßen Kristalls entspricht, nach einem in den Ansprüchen 39 bis 41 beschriebenen Verfahren ermittelt wird und dann (b) mit Hilfe eines der in Zusammenhang mit den Ansprüchen 31 bis 38 beschriebenen Verfahren eine Verbindung mit der Fähigkeit, als Inhibitor der Wechselwirkung eines Chaperon-Proteins mit einem Polypeptid zunächst unbekannter und dann gemäß (a) aufgeklärter 3D-Struktur zu wirken, bestimmt wird. Hierbei wird es sich typischerweise um Verbindungen handeln, die entweder eine Bindung mit dem TPR-Domänen-Protein, vorzugsweise in dem für die Bindung mit dem physiologischen Bindungspartner maßgeblichen Bindungsbereich, eingehen, und so die Interaktion auf Grund ihrer Komplementarität zum TPR-Bindungsbereich blockieren, oder aber um Verbindungen, die den Bindungsbereich der TPR-Domäne simulieren und damit komplementär zur Bindungsstelle des Chaperon-Proteins sind.

Schließlich wird im Rahmen der vorliegenden Erfindung auch die Verwendung von Inhibitoren und/oder gegebenenfalls Aktivatoren der physiologischen Aktivität von Hop-Protein insbesondere von humanem Hop-Protein, nach einem der Ansprüche 27 bis 29 zur Herstellung eines Arzneimittels, zur Verwendung als Arzneimittel oder als Wirkstoff, der in einer pharmazeutischen Zusammensetzung enthalten ist, offenbart. In einer pharmazeutischen Zusammensetzung kann ein erfindungsgemäßer Inhibitor oder gegebenenfalls Aktivator mit mindestens einem weiteren Wirkstoff kombiniert sein und/oder die pharmazeutische Zusammensetzung bzw. der Inhibitor als Wirkstoff wird als Arzneimittel in eine dem Fachmann geläufigen Formulierung eingearbeitet sein. Die Formulierung wird dabei insbesondere vom Verabreichungsweg abhängen. Dieser kann oral, rektal, intranasal oder parenteral, insbesondere subkutan; intravenös, oder intramuskulär, sein. Pharmazeutische Zusammensetzung, die einen derartigen Inhibitor und/oder Aktivator enthalten, können die Verabreichungsform eines Puders, einer Suspension, einer Lösung, eines Sprays, einer Emulsion oder einer Creme haben.

Ein erfindungsgemäßer Inhibitor oder gegebenenfalls Aktivator kann mit einem pharmazeutisch akzeptablen Trägermaterial mit neutralem Charakter (wie z. B. wäßrigen oder nicht-wäßrigen Lösungsmitteln, Stabilisatoren, Emulgatoren, Detergentien und/oder Additiven und gegebenenfalls weiteren Farb- oder Geschmacksmitteln kombiniert sein.
Die Konzentration eines erfindungsgemäßen Inhibitors und/oder Aktivators in einer pharmazeutischen Zusammensetzung kann zwischen 0,1% und 100% variieren, abhängig insbesondere von dem Verabreichungsweg. Eine pharmazeutische Zusammensetzung oder ein Arzneimittel, enthaltend einen erfindungsgemäßen Inhibitor oder gegebenenfalls Aktivator kann zur Herstellung eines Arzneimittels zur Behandlung von Tumorerkrankungen, Autoimmunerkrankungen, Immunsuppresaion, Behandlung von Entzündungserkrankungen, GVHD oder zur Behandlung von Virusinfektionen dienen.

Anlagen A1 bis A4 sind Bestandteil der Offenbarung der vorliegenden Patentanmeldung.

Die vorliegende Erfindung wird durch die nachfolgenden Figuren näher erläutert.

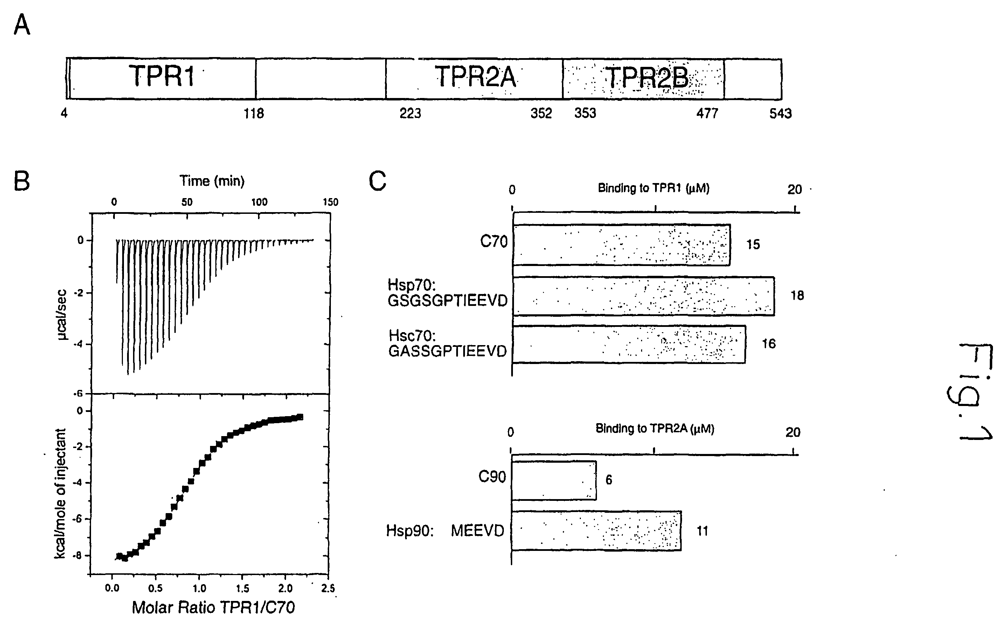

Die Figur 1 umfaßt die Figuren 1A, 1B und 1C. Figur 1A zeigt hierbei die sequenzielle Grobstruktur eines Hop-Proteins, und zwar unter Berücksichtigung der drei im Hop-Protein vorhandenen TPR-Domänen, nämlich TPR1, TPR2A und TPR2B. Figur iA gibt auch die Domänengrenzen wieder. Die Domäne TPR1 erstreckt sich definitionsgemäß bis zur Aminosäure 118, die Domäne TPR2A liegt zwischen Aminosäure 223 und Aminosäure 352 und die TPR-Domäne TPR2B zwischen Aminosäure 353 und 477.

Figur 1B zeigt eine Auftragung der kalorimetrischen isothermischen Titrationsmessungen zur Bestimmung der Wechselwirkung zwischen der TPR1-Domäne des Proteins Hop mit der C-terminalen 25kDa-Domäne von Hsp70 (C70). Im oberen Teil der Darstellung findet sich in inkrementeller Auftragung die Wärmefreisetzung nach Titration von C70 (180µM) mit TPR1 (3mM) bei 25°C. Im unteren Teil sind die kalorimetrischen Ergebnisse nach Integration und normalisiert auf die Menge von injiziertem TPR1 dargestellt. Die Kurve wurde an ein Bindungsmodell der Stöchiometrie 1:1 zwischen den Bindungspartnern angepaßt. Bestimmt wurden folgende Werte: K_{D} = 15µM, Stöchiometrie N = 0,87.

In Figur 1C sind die thermodynamischen Bindungskonstanten (K_{D}) bei der Wechselwirkung von TPR1 mit C70 und solchen Peptiden, die die letzten 12 Aminosäurereste von Hsp70 und Hsc70 (in der oberen Darstellung) umfassen, und für die Bindung von TPR2A an C90 und an ein Hsp90-Pentapeptid (in der unteren Darstellung) dargestellt. Die Titrationen wurden bei 25°C durch Injizieren von Proteinen oder Peptiden in eine Lösung, die TPR1 enthielt, durchgeführt.
Die stöchiometrischen Konstanten (N) lagen typischerweise zwischen 0,9 und 1,1. Die ITC-Messungen erwiesen sich als bestens reproduzierbar mit Standardabweichungen von ungefähr 5 bis 10% bei dreifacher Wiederholung der Messungen.

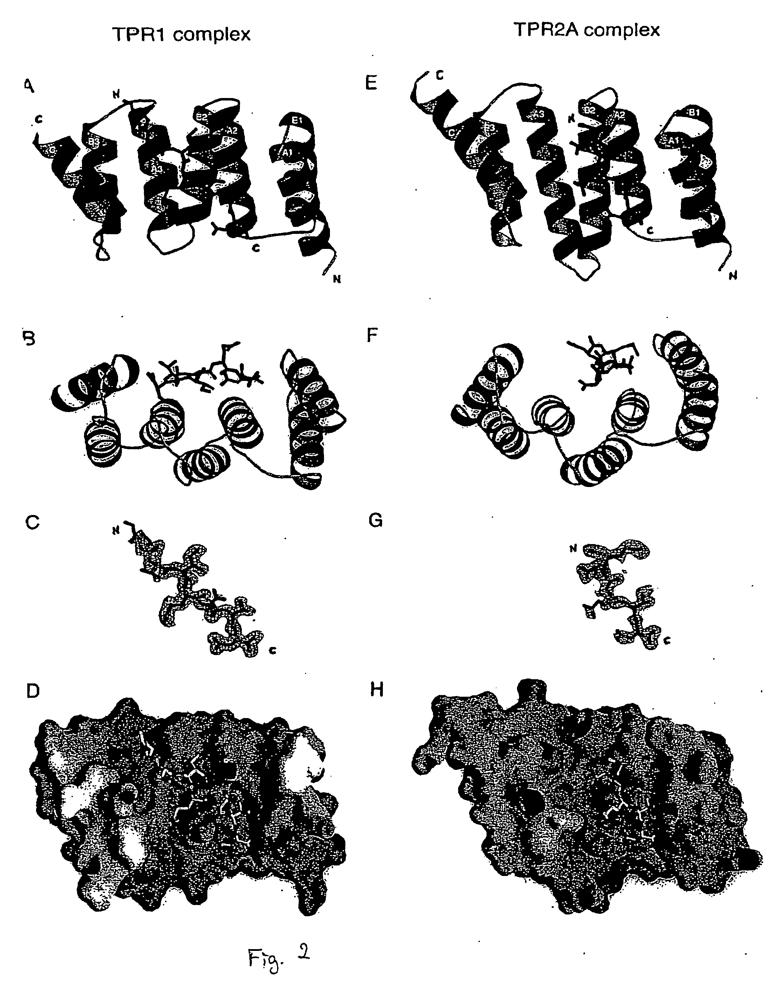

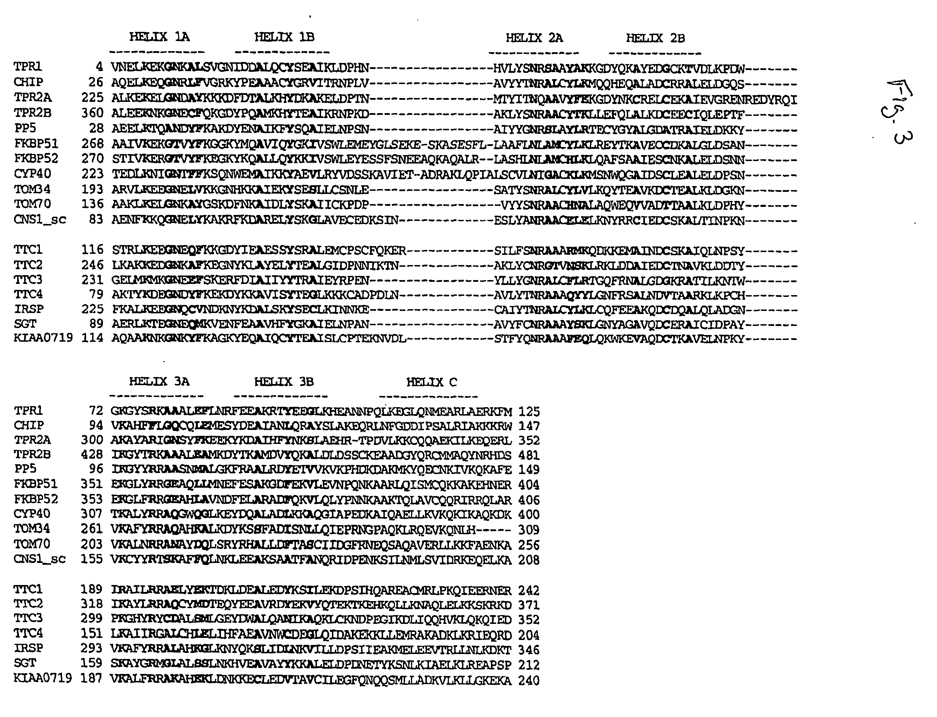

In Fig. 2 finden sich die Darstellungen A bis H, wobei es sich bei den Figuren A, B, E und F um schematisierte sog. "Ribbon"-Darstellungen handelt. Das Rückgrat der TPR-Domäne ist cyanofarben (TPR1) oder in Magenta (TPR2A) gehalten, wobei sowohl in Fig. 2A als auch in Fig. 2E die gebundenen Peptide als stäbchenförmige Modelldarstellungen in Pink angegeben sind. Die N- und C-Termini der TPR-Domänen bzw. der gebundenen Peptide sind mit den entsprechenden Buchstaben N und C markiert. Die in jedem TPR-Strukturmotiv auftretenden Helices A und B der drei aufeinanderfolgenden TPR-Strukturmotive sind ebenso dargestellt wie die flankierende Helix C. Dabei zeigen die Figuren 2A und 2E die wiegenförmigen TPR1- und TPR2-Domänen.

Die Figuren 2B und 2F haben denselben Darstellungsgegenstand wie die Figuren 2A und 2E, wobei in den Figuren 2B und 2F die Komplexe aus TPR1 mit gebundenem Peptid und TPR2A mit gebundenem Peptid um jeweils 90° gedreht sind. Nunmehr ist der aus den beiden Carboxylat-Gruppen bestehende Anker des Peptids in Richtung auf den Betrachter gerichtet. Die Figuren 2C und 2G stellen (2Fo-Fc)-Elektronendichtekarten dar, die für den Peptidbereich mit Hilfe des Endmodells berechnet wurden, wobei das Peptid hieraus weggelassen wurde. Die Figuren 2D und 2H sind Darstellungen des elektrostatischen Potentials von TPR1 und TPR2A, das auf die zugängliche molekulare Oberfläche so modelliert wurden, wie es sich mit Hilfe des Programms GRASP berechnen und visuell dargestellen ließ. Entsprechende nähere Erläuterungen finden sich bei Nicholls et al. (Biophysical Journal 64, A166). In rot sind die negativ geladenen, in blau die positiv geladenen und in grau die neutralen Bereiche gekennzeichnet. Die gebundenen Peptide finden sich in einer stabförmigen Darstellung ebenfalls dargestellt.

In Figur 3 wird ein sequenzspezifischer Vergleich ("Alignment") der TPR-Domänen von solchen Proteinen gezeigt, die entweder an Hsp70 oder Hsp90 binden. Konservierte Aminosäurereste, die an elektrostatischen Wechselwirkungen mit dem EEVD-Motiv des Liganden beteiligt sind, sind in blau markiert, während Aminosäurereste, die in hydrophober oder in van-der-Waals-Wechselwirkung mit dem Liganden stehen, rot gekennzeichnet sind. Jene Aminosäurereste, die im Fettdruck dargestellt sind, sind Bestandteil der TPR-Konsensus-Sequenz und sind auf Grund der Packung der verschiedenen α-Helices von benachbarten TPR-Strukturmotiven konserviert. Alle Sequenzen wurden aus der Datenbank GenBank entnommen und sind menschlichen Ursprungs, abgesehen von CNS1 und TOM70, die aus S. cerevisiae bzw. N. crassa stammen. Bei TPR1 und CHIP handelt es sich um Hsp70-bindende TPR-Domänen. Hsp90-bindende TPR-Domänen sind TPR2A, PP5, FKBP51, FKBP52, CYP40, TOM34, TOM70 und CNS1. Ein Ligand für die TPR-Domäne TPR2B des Hop-Proteins ist bislang nicht identifiziert worden.

Die weiteren Sequenzen TTC1 und TTC2 (diese treten in Wechselwirkung mit Neurofibromin), TTC3 (liegt in einer genomischen Region, die an der Pathologie des Down-Syndroms beteiligt ist), TTC4 (ist in einem Bereich der regelmäßig bei Sporadischem Brustkrebs deletiert ist), IRSP (ein Protein, das in Zusammenhang mit Infertilitätserscheinüngen steht) und SGT (kleines glutaminreiches TPR-Protein, das mit dem nicht-strukturellen NS1-Protein des Parvovirus H-1 interagiert) haben gleichfalls Sequenzen mit TPR-Domäneneigenschaften, die an Hsp70 und/oder Hsp90 binden können. Letzteres gilt auch für das menschliche Protein KIAA0719, dessen physiologische Funktion unbekannt ist.

Figur 3A gibt die Aminosäuresequenzen einer beispielhaften Auswahl von TPR-Strukturmotiven an, die in Polypeptiden auftreten können. Die in Figur 3A wiedergegebenen Aminosäuresequenzen treten in humanen Hop-Proteinen auf. Die Sequenzen (1), (4) und (7) sind Bestandteile der TPR-Domäne TPR1, die Sequenzen (2), (5) und (8) sind Bestandteile der TPR-Domäne TPR2A und die Sequenzen (3), (6) und (9) Bestandteile der TPR-Domäne TPR2B, jeweils vom Hop-Protein. Die räumlichen Strukturen der Sequenzen (1), (4) und (7) können der Figur 3C entnommen werden, die die Strukturkoordinaten der TPR-Domäne TPR1 ohne Komplexierung mit einem Liganden wiedergibt. Die TPR-Strukturmotive (1), (4) und (7) können in Figur 3C oder 3D anhand ihrer Sequenz (Strukturkoordinaten gelistet für Atome vom N-Terminus zum C-Terminus (Arg118), für die Elektronendichte beobachtet werden kann), zugeordnet werden (s. hierzu auch Figur 3). Analoges gilt für die räumlichen Strukturen der TPR-Strukturmotive (2), (5) und (8), die aus Figur 3E entnommen werden können.

Figur 3B stellt die Aminosäuresequenzen der drei in Hop-Proteinen auftretenden TPR-Domänen TPR1, TPR2A und TPR2B dar. Deren räumliche Struktur ist durch die atomaren Strukturkoordinaten gleichfalls den Figuren 3C, 3D und 3E zu entnehmen.

Figur 3C gibt die Strukturkoordinaten der TPR-Domäne TPR1 (ohne Komplexierung mit einem Liganden) wieder, soweit für die Aminosäuren der Sequenz Elektronendichte beobachtet werden konnte. In der Auflistung der atomaren Strukturkoordinaten sind die Strukturkoordinaten der Sequenzen (1), (4) und (7) gemäß Figur 3A enthalten. Angaben zur Strukturanalyse sind auf den Seiten 1 und 2 (von 39) der Figur 3C enthalten.

Figur 3D listet die Koordinaten für die Atome der TPR1-Domäne von Hop im Komplex mit dem Liganden GASSGPTIEEVD auf. Physikalische Angaben zur Durchführung der Röntgenstrukturanalyse ebenso wie zum kristallisierten Polypeptid sind auf den Seiten 1 bis 6 der Figur 3D wiedergegeben.

Figur 3E stellt die Koordinaten der TPR-Domäne TPR2A des Hop-Proteins im Komplex mit dem Liganden MEEVD dar. Die in Figur 3A bezeichneten TPR-Strukturmotive (Sequenzen (2), (5) und (8) sind gleichfalls in ihrer räumlichen Struktur in Fig. 3E enthalten). Die Seiten 1 bis 6 von Figur 3E geben Information zum kristallisierten Polypeptid und zur Durchführung der Röntgenstrukturanalyse wieder. Für die Figuren 3C, 3D und 3E gilt, daß die Struktur der terminalen, insbesondere C-terminalen Aminosäuren der TPR-Domänen, aufgrund der typischen Beweglichkeit dieser endständigen Aminosäuren nicht bestimmt werden kann.

Figur 4 ist eine schematische Darstellung der Wechselwirkung, die zwischen einer TPR-Domäne und einem komplementären Peptid bestehen können. Figur 4A stellt den TPR1/Peptid-Komplex dar, während Figur 4B den TPR2A/Peptid-Komplex zeigt. Die Farben für den Aminosäurerest von TPR1, TPR2A und der jeweiligen Peptide entsprechen der in Figur 2 dargestellten farblichen Kennzeichnung.

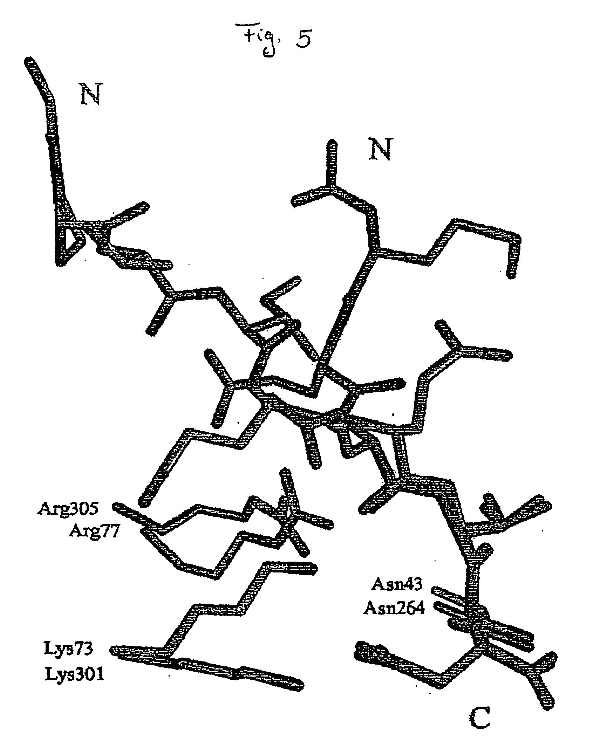

Figur 5 ist eine Überlagerung der beiden TPR/Peptid-Komplexe. Die Überlagerung der sog. Doppel-Carboxylat-Klammer der beiden TPR1/ und TPR2A/Peptid-Komplexe führt zu einer strukturellen Kongruenz der Seitenketten, die an den Peptidkontakten beteiligt sind und auch von Teilen der gebundenen Peptide. Der TPR1-Komp3ex ist in magenta gehalten, der TPR2A-Komplex ist in cyano dargestellt. Die Aminosäurereste der Doppel-Carboxylat-Klammer und die N-und C-Termini der Peptide sind markiert.

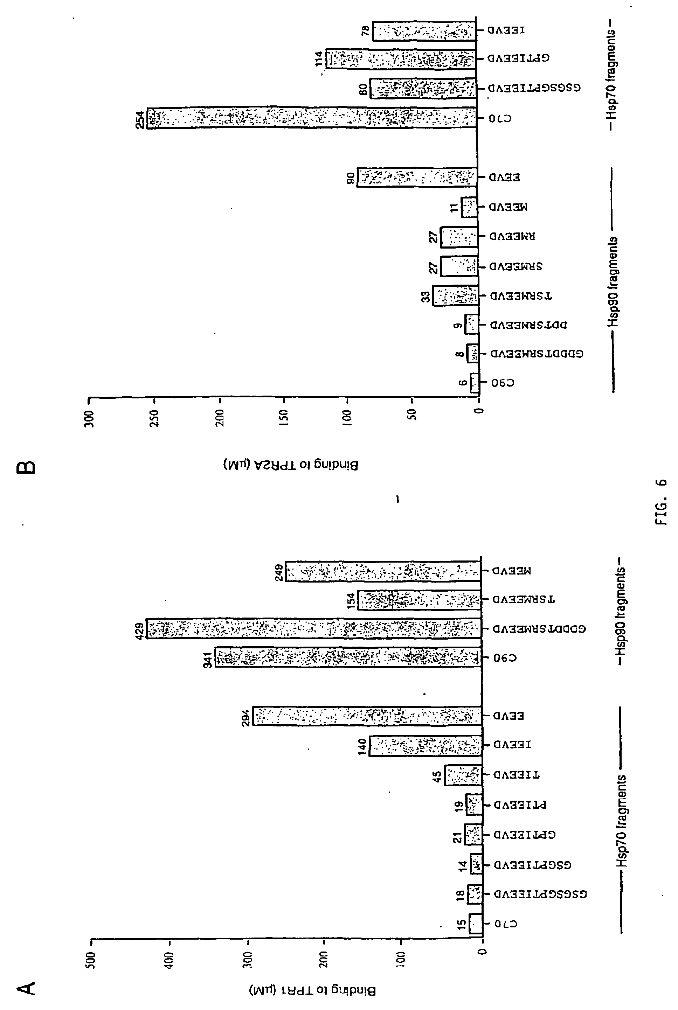

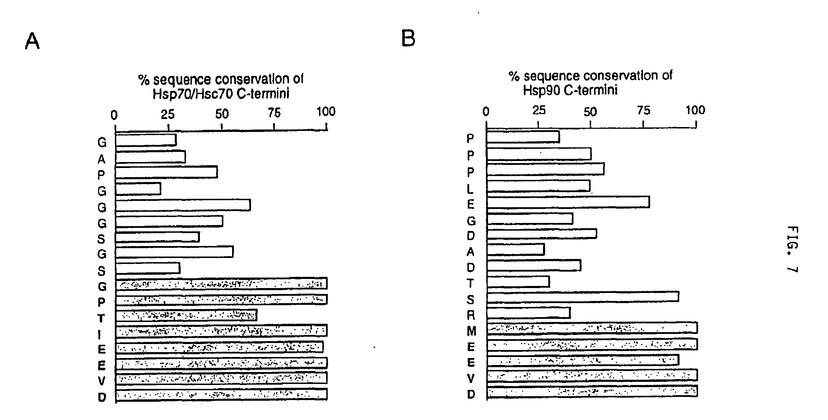

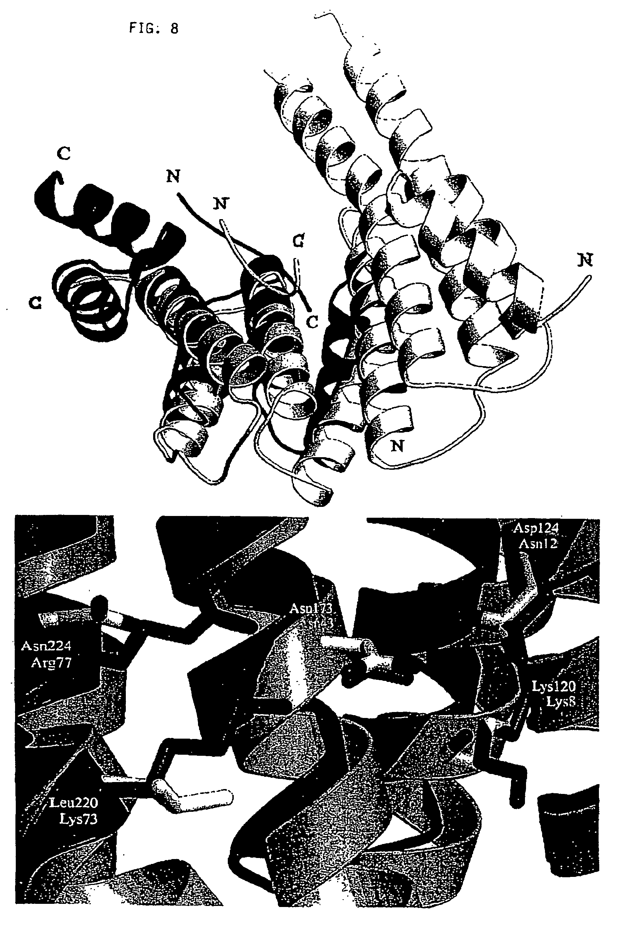

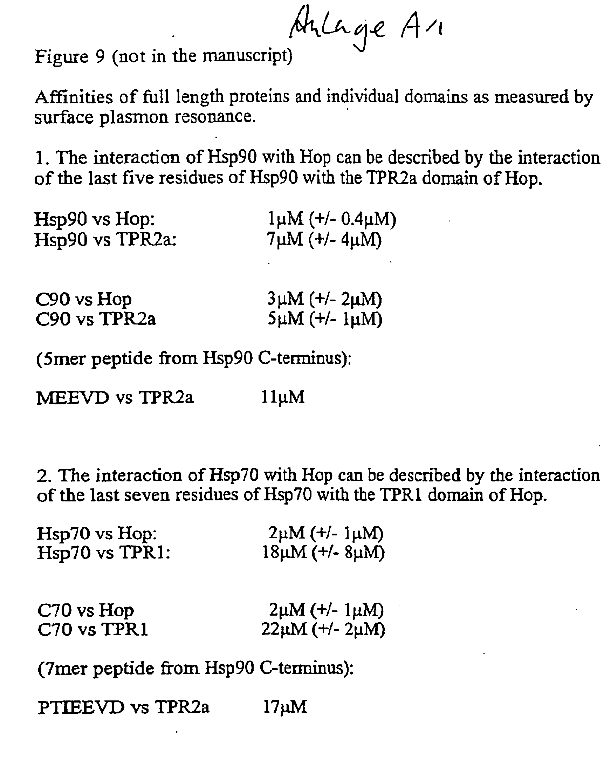

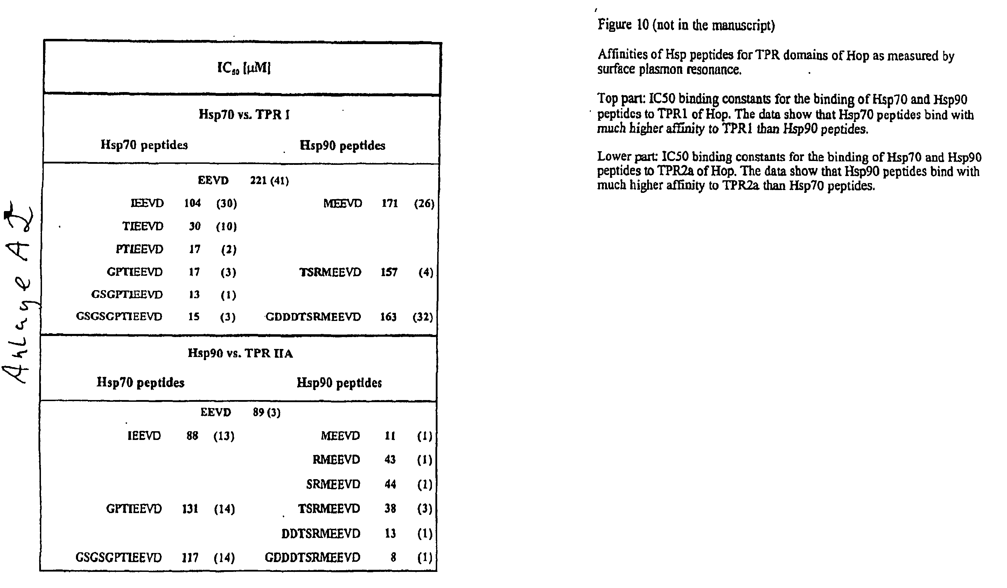

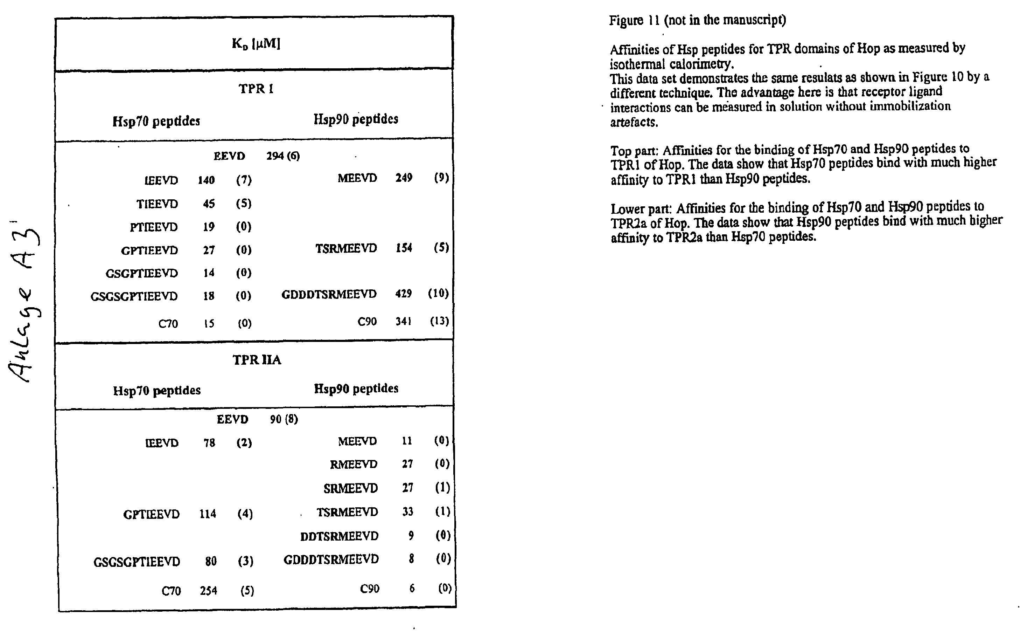

Figur 6 ist eine Auftragung der thermodynamischen Analyse der Wechselwirkung von TPR1 und TPR2A mit den C-terminalen Sequenzen von Hsp70 und Hsp90. Figur 6A stellt die Bindungskonstanten (K_{D}) für die Wechselwirkung von TPR1 mit EEVD-Peptiden dar, die mit Hilfe der ITC-Messungen (vgl. Fig. 1) ermittelt wurden. Figur 6B behandelt die Bindung von TPR2A an EEVD-Peptide. Die Sequenzfolge GSGSGPTIEEVD entspricht den 12 C-terminalen Aminosäureresten von Hsc70. GDDDTSRMEEVD entspricht den 12 C-terminalen Aminosäureresten von Hsp90. Die Titrationen wurden bei 25°C durch das Injizieren von Peptiden, die in Puffer G bei einer Konzentration von 7,5 mM bis 15 mM gelöst waren, in eine Lösung, die die TPR1-Domäne (450 µM - 950 µM) - im Puffer G enthielt; durchgeführt. Die Stöchiometrie-Konstanten (N) lagen typischerweise zwischen 0,9 und 1.1.

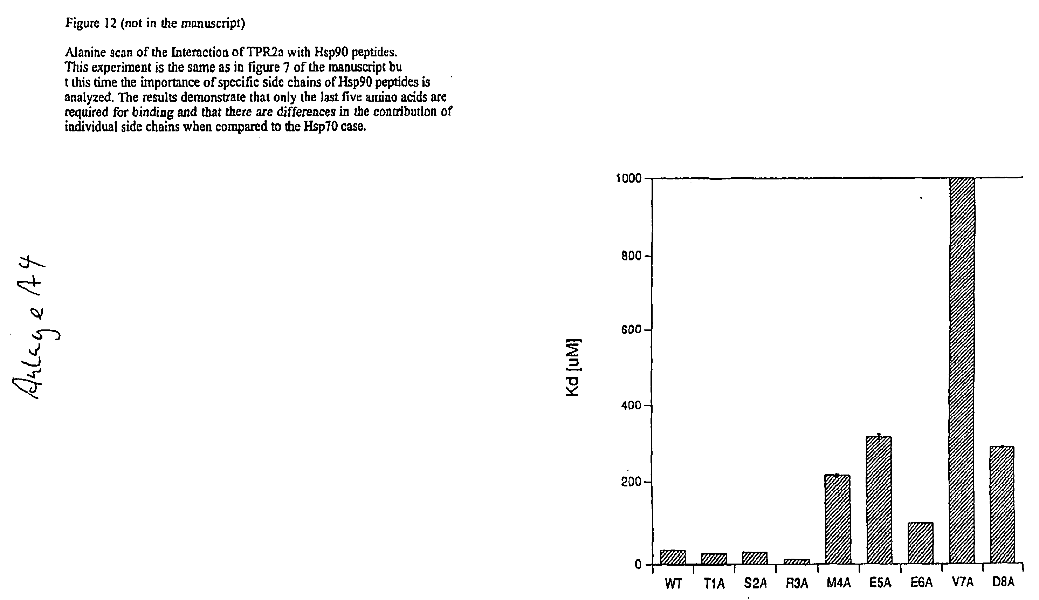

Figur 7 stellt die Sequenzkonservierung der C-Termini von Hsp70/Hsc70- und Hsp90-Chaperons dar. Figur 7A zeigt die Aminosäurekonservierung der cytosolischen Varianten von eukaryontischen Hsp70- und Hsc70-Protein, wobei 83 Sequenzen analysiert wurden. Die Konservierung der am häufigsten auftretenden Aminosäure an der jeweiligen Position ist in % angegeben. Jene Aminosäuren, die dem strukturell geordneten Peptidbereich angehören, sind im Fettdruck markiert. In Figur 7B wird in analoger Weise die Aminosäurekonservierung der cytosolischen Varianten des eukaryontischen Hsp90-Proteins dargestellt, wobei 138 Sequenzen analysiert wurden. Die letzten fünf Aminosäuren, die für die TPR-Bindung maßgeblich sind, sind im Fettdruck markiert.

Figur 8 zeigt die Überlagerung des TPR1/Peptids-Komplexes mit dem 14-3-3-Peptidkomplex. Figur 8A ist eine Darstellung des "Alignments" der korrespondierenden α-Helices und der gebundenen Peptide. Der TPR1-Komplex ist in magenta und der 14-3-3-Komplex ist in grau gehalten. N- und C-Termini der Domänen und der gebundenen Peptide sind markiert. Figur 8B stellt eine Überlagerung der sog. Doppel-Carboxylat-Klammer von TPR1 mit den entsprechenden Aminosäuren der 14-3-3-Domäne dar.

Die vorliegende Erfindung wird durch die nachfolgenden Ausführungsbeispiele näher erläutert.

Hierzu wurden die TPR-Domänen TPR1 und TPR2A des Hop-Proteins (Primärsequenzen unter der Bezeichnung (1) und (2) in Figur 3B angegeben) ohne Liganden (Figur 3C) bzw. im Komplex mit Polypeptidliganden kristallisiert und nachfolgend durch Röntgenkristallographie hochaufgelöste Kristallstrukturen der drei Kristalle erhalten.

### 1. Protein- und Peptidpräparation

Die Codons 1-118 (TPR1) und.223-352 (TPR2A) von humanem Hop-Protein wurden im richtigen Leseraster in die EheI-Schnittstelle des Plasmids pPROEx HTa (von der Fa. Life Sciences) zur Expression in E.coli BL21 (DE3) als Fusionsproteine mit abtrennbaren N-terminalem Hexahistidin-"Tags" kloniert. Die löslichen Proteine wurden mit Hilfe chromatographischer Verfahren auf Ni-NTA (von der Fa. Qiagen) und Superdex 200 (von der Fa. Pharmacia) gereinigt. Die His-Tags wurden unter Verwendung von TEV-Protease abgetrennt, wobei ein zusätzlicher Glycinrest vor dem N-Terminus verblieb. Die Proteine wurden durch Ultrafiltration bis zu einer Konzentration von 40 mg/ml aufkonzentriert. Für die Strukturbestimmung der Domäne TPR2A wurde eine Selenomethionin-markierte Form durch eine Expression im E.coli-Stamm B834 (DE3) hergestellt. Ein Selenomethionin-markiertes Peptid (die C-terminalen fünf Aminosäuren von Hsp90 mit der Sequenz MEEVD (Ein-Buchstaben-Code) ) wurde für die Co-Kristallisierung verwendet. Der Einbau der Methionine wurde durch Massenspektrometrie bestätigt. Das C-terminale Fragment von menschlichem Hsp70 (C70, Codons 382-641) wurde nach der gleichen Vorgehensweise kloniert, exprimiert und gereinigt. Die C-terminale Domäne von menschlichem Hsp90 (C90, Codons 625-732) wurde, wie bei Young et al. (1998, J. Biol. Chem. 273, 18007-18010) beschrieben, exprimiert und gereinigt.

Für die Synthese von Ac-(Se)Met-Glu-Glu-Val-Asp-OH wurde das L-Selenomethionin in Fmoc-(Se)Met-OH durch Reaktion mit Fmoc-OSu unter Standardbedingungen umgewandelt (Kristallisierung aus Ethylacetat/Hexan) . Das Tetrapeptid H-Glu(OtBu)-Glu(OtBu)-Val-Asp(OtBu)-Resin wurde nach Standard-Fmoc/HBTU/HOBt-Festphasenprotokollen auf Wang-Resin (von der Fa. Bachem, Bubendorf) synthetisiert. Das Fmoc-(Se)Met-OH wurde in einem zweifachen Überschuß an das Peptidresin mit HBTU/HOBt/DIEA (1:1:2) zweifach gekoppelt. Nach Piperidin-vermittelter Abspaltung der Fmoc-Schutzgruppe wurde die N-terminale Acetylierung mit einem achtfachen Überschuß von Ac20 in der Gegenwart von DIEA durchgeführt. Der Resinabtrennungs- und Deprotektionsschritt wurde mit Argon-gesättigtem TFA/TIS/H₂O (93,5:5:1,5) bei Raumtemperatur für 90 Minuten unter Argonatmosphäre durchgeführt. Das Resin wurde abfiltriert und das Produkt mit Methyltert-butyl-ether/Hexan präzipitiert. Das Rohpeptid wurde durch präparative RP-HPLC (Elution: linearer Gradient von 0,1% wäßriger TFA und 0,08% TFA in CH₃CN) gereinigt. Das Produkt wurde nach Lyophilisierung erhalten. Alle anderen Peptide wurden mit acetylierten N-Termini unter Verwendung von Festphasen-FMOC-Chemie synthetisiert.

### 2. Kristallisierung und Datensammlung

Die Kristalle des Polypeptids, das der TPR1-Domäne von Hop entspricht (Figur 3B, Sequenz (1)), im Komplex mit einem Peptid (GASSGPTIEEVD, C-terminales Dodekamer von Hsp70) wurden bei 20°C durch die Technik des "hängenden Tropfens" mit Hilfe von Dampfdiffusionsverfahren aufgezogen. Gleiche Volumina (1,5 µl) des Protein-Peptid-Komplexes (Protein:Peptid = 1:1,3) wurden bei einer Konzentration von 20 mg/ml in 15 mM HEPES (pH 7,5) mit einer Reservoir-Lösung gemischt, die 100 mM TRIS (pH 8,5), 24% (w/v) PEG MME 2000, 10 mM NiCl₂ und 15% (w/v) Xylitol enthielt. Die Kristallisierung wurde mit Hilfe des "Microseedings" verbessert, wobei die Kristalle ihre volle Größe (typischerweise 150x150x70 µm³) innerhalb von zwei Wochen erreichten. Die Kristalle wurden dann blitzartig in flüssigem Stickstoff gekühlt und bei 100 K während der Datensammlung konserviert. Hierfür wurde ein Oxford Cryostream-Gerät eingesetzt. Die Kristalle gehörten zur Raumgruppe P4₁ (a = 75,47 Å, c = 42,89 Å) mit zwei Molekülen pro asymmetrischer Einheit und einem Lösungsmittelanteil von 45%. Vier Nickelatome und vier TRIS-Moleküle wurden in der Struktur aufgefunden. Die hochaufgelösten Datensätze wurden an der Strahlenquelle X12B der national Synchroton Light Source in Brookhaven unter Verwendung eines ADSC Quantum-4 CCD-Detektors gesammelt. Weitere Einzelheiten der Datensammlung sind der Tabelle 1 zu entnehmen.

Die Kristalle von TPR2A (Aminosäuresequenz wie in Figur 3B für das mit (2) bezeichnete Polypeptid angegeben) wurden im Komplex mit einem Pentamer (MEEVD, entsprechend den fünf C-terminalen Aminosäuren des Chaperon-Proteins Hsp90) wie oben beschrieben aufgezogen. Hierzu wurden gleiche Mengen (1,8 µl) des Protein-Peptid-Komplexes (Protein:Peptid = 1:1,3) bei einer Konzentration von 15 mg/ml in 50 mM TRIS (pH 7,5), 2 mM DDT mit Reservoir-Lösung, die 100 mM TRIS (pH 8,5), 20% (w/v) PEG MME 2000, 5 mM NiCl₂ und 10% (w/v) Xylitol enthielt, gemischt. Die Kristalle waren nach einer Woche nachweisbar und erreichten ihre volle Größe (typischerweise 100x50x50 µm) innerhalb von drei Wochen. Es wurde festgestellt, daß die Nikkelionen und das Peptid für die Kristallisierung entscheidend waren. Ein in hohem Maße strukturell fixiertes Nickelion wurde in der Struktur aufgefunden, wobei es einen Kristallkontakt vermittelt. Es wurde für die Ermittlung der Phaseninformation verwendet. Die Kristalle gehörten zur Raumgruppe C2 mit einem Molekül pro asymmetrischer Einheit und einem Lösungsmittelanteil von 40%. Der native Datensatz wurde bis zu einer Auflösung von 1,9 Å an der Strahlenquelle ID14-3 beim ESRF unter Verwendung eines MarCCD-Detektors gesammelt. Die multiplen anisomorphen Diffraktionsdaten (MAD) von markiertem TPR2A-Peptid-Komplex wurden an der MPG/GBF-"Wiggler"-Strahlenquelle BW6/DORIS am DESY unter Verwendung eines MarCCD-Detektors aufgezeichnet. Die Datensätze wurden um die Absorptionskanten von Ni herum (Nil:peak, Ni2:inflection) und Se(Sel:peak, Se2:inflection) anhand eines einzigen Proteinkristall gesammelt. Alle Datensätze wurden mit Hilfe des HKL Software-Programmpakets (Otwinowski und Minor, 1997, Macromolecular Crystallography, Pt A, Seiten 307-326) prozessiert. Weitere Einzelheiten zur Datensammlung sind der Tabelle 2 zu entnehmen.

### 3. Strukturbestimmung und Strukturverfeinerung

Die Struktur des TPR1-Komplexes wurde durch das molekulare Ersetzen ("molecular replacement") unter Verwendung des Programms Amore, das Bestandteil der CCP4 Programmsuite (Bailey, 1994, Acta Crystallographica Section D-Biological Crystallography, 50, 760-763) ist, gelöst. Teile der zuvor bestimmten Kristallstruktur von TPR1L (Aminosäuren 1-140) dienten als Suchmodell. Der TPR1-Peptid-Komplex wurde mit dem Programm CNS (Brünger et al., 1998, Acta Crystallographica Section D, Biological Crystallography, 54, 905-921) unter Verwendung aller Diffraktionsdaten zwischen 20 und 1,6 Å Auflösung verfeinert, wobei 10% der Daten für die Kreuzvalidierung (s. Tabelle 1) ausgeschlossen wurden. Alle Verfeinerungsschritte wurden vollzogen, ohne von nichtkristallographischer Symmetrie Gebrauch zu machen. Blektronendichte konnte bei den beiden kristallographisch unabhängigen TPR1-Domänen in der asymmetrischen Einheit (Kette A und Kette B) für die Aminosäurereste von A2 bis A118 und B1 bis B115 beobachtet werden. Aufgrund der Beweglichkeit wurden die Reste A2, B1, B2 und B110 als Alanine modelliert. Die Positionen der Peptide wurden aus einer Differenzelektronendichtekarte ermittelt. In beiden Fällen konnten nur die letzten acht Aminosäuren GPTIEEVD des Peptids einer Elektronendichte zugeordnet werden. Die Differenzelektronendichtekarte erlaubte auch die Identifizierung der vier Nickelatome (zwei pro Monomer) und von vier TRIS-Molekülen (zwei pro Monomer). Zwei Nickelbindungsstellen wurden am Histidin 36 jeweils für die Ketten A und B lokalisiert. Die Okkupanz wurde manuell auf 0,5 eingestellt, um die Temperaturfaktoren an jene der gebundenen Histidine anzupassen. Zwei vollständig besetzte Positionen wurden am Histidin 101 (für die Ketten A und B) lokalisiert. Jedes Nickelatom, das an das Histidin gebunden ist, ist zusätzlich mit zwei TRIS-Molekülen komplexiert.

Das Endmodell, das 1960 Proteinatome, 36 Heteroatome und 245 Lösungsmittelmoleküle enthält, konvergierte bei einem R-Faktor R_{work} = 18,2% (R_{free} = 21,6%). Die mittlere Standardabweichung für die Bindungslängen beträgt 0,008 Å und für die Bindungswinkel 1,3°. In der vorhergehenden Beschreibung der Struktur wurde nur eine der beiden Ketten im Komplex mit dem gebundenen Peptid (Kette A bzw. Kette C für das Peptid) der beiden in der asymmetrischen Einheit enthaltenen Ketten A und B behandelt.

Für die Ermittlung der TPR2A-Struktur (Tabelle 2) wurden die MAD-Datensätze auf eine ungefähr absolute Skala gesetzt und als ein spezieller Fall von MIR-Daten behandelt, wobei verschiedene Programme aus der CCP4 Programmsuite (CCP4, 1994) eingesetzt wurden. Die Position eines Nickelatoms wurde durch Analyse von einer anomalen Differenz-Patterson-Karte aus den Daten bei einer Wellenlänge von maximal f_{NI}" (Ni1) bestimmt. Diese Nickelbindungsstelle wurde dann verwendet, um die MAD-Phasen mit Hilfe von MLPHARE (CCP4, 1994) zu berechnen. Diese Phasen wurden dann weiterverwendet, um die beiden Selen-Bindungsstellen aus der anomalen Differenz-Fourier-Karte, die mit Hilfe der bei maximal fₛₑ" (Se1) gewonnenen Daten berechnet wurden, zu identifizieren. Zur abschließenden Phasenermittlung wurde die Wellenlänge des Inflektionspunkts von Nikkel (Ni2) als nativer Datensatz verwendet und die drei anderen Wellenlängen (Nil, Se1, Se2) wurden als separate Derivate behandelt. Die Phasen wurden im Auflösungsbereich zwischen 17,0-2,1 Å berechnet und ihr anfänglicher "mean figure of merit" von 0,74 wurde durch die Methode des "solvent flattening" und "histogram matching" unter Verwendung des Programms DM (CCP4, 1994) verbessert. Die erhaltene Elektronendichtekarte entsprach hoher Qualität und erlaubte es, mit Hilfe von wARP (Perrakis et al, 1997) ungefähr 90% der Endstruktur zuzuordnen. Die Atommodelle wurden überprüft und unter Verwendung des Programms O (Jones et al., 1991, Acta Crystallographica, Section A 47, 110-119) weiter verbessert. Die Verfeinerungen wurden mit Hilfe des Programms CNS (Brünger et al, 1998, Acta Crystallographica, Section D, Biological Crystallography 54, 905-921) gegen den nativen Datensatz unter Verwendung aller Diffraktionsdaten zwischen 10 und 1,9 Å Auflösung (ausgeschlossen 10% der Daten für die Kreuzvalidierung) ausgeführt. Die Aminosäuren Lys223 bis Gln349 (TPR2A-Domäne) wurden der Elektronendichte zugeordnet, genauso wie die Aminosäuren Met-4 bis Asp0 des Pentapeptids. Aufgrund ihrer Beweglichkeit wurden die Reste A291 bis A294 und A348 als Alanine modelliert. Die Nickel-Bindungsstelle, die im Rahmen der Phasenermittlung verwendet wurde, erwies sich als Brücke eines Kristallkontakts von His247 zum His321 und Lys325 aus dem symmetrieverwandten Molekül.

Das Endmodell enthielt 1086 Proteinatome (44 für das Peptid) , 1 Heteroatom und 152 Lösungsmittelmoleküle, die bei einem R-Faktor von R_{work} = 18,1% (R_{free} = 21,9%) für alle Daten ohne irgendeinen Sigma-Grenzwert konvergierten. Die Standardabweichung für die Bindungslänge betrug 0,008 Å und für die Bindungswinkel 1,2°. Alle Reste sind in den favorisierten oder in den zumindest allgemein erlaubten Bereichen der Ramachandran-Auftragung angesiedelt, wie mit Hilfe des Programms PROCHECK (Laskowski et al., 1993) berechnet.

### 4. Isotherme Titrationskalorimetrie (ITC)

Die Bindung von Proteinfragmenten und Peptiden an die TPR-Domänen wurde mit Hilfe der isothermen Titrationskalorimetrie (Wiseman et al., 1989. Anal. Biochem. 179, 131-137) unter Verwendung eines MicroCal MCS-Titrationskalorimeters (MicroCal Inc., Northhampton, USA) gemessen. 40-50 Aliquots von 5 µl Peptidlösung (7,5mM - 15mM) wurden bei 25°C durch Injektion in 1,36 ml TPR1 (TPR2A)-Lösung (450 µM-950 µM) in die Kammer titriert. Alternativ wurde eine Lösung von 3 mM TPR1 (TPR2A) in 180 µM C70 (C90) -Lösung in der Kammer titriert. Die Peptide wurden aufgelöst in und die Proteine dialysiert gegen den Puffer G (25 mM HEPES, pH 7,5, 100 mM KAc, 5 mM MgAc₂). Die Injektionen wurden typischerweise über die Sättigungsgrenzen hinaus fortgesetzt, um die Wärme der Ligandenauflösung bestimmen zu können. Nach der Subtraktion der Auflösungswärme wurden die kalorimetrischen Daten analysiert, wobei eine Bewertungssoftware, die durch den Hersteller zur Verfügung gestellt wird (Version 2.9; MicroCal Software, Inc.), verwendet wurde.

### Ergebnisse der Ausführungsbeispiele

1.
   Durch beschränkte Proteolyse wurden drei definierte Domänen von menschlichem Hop-Protein identifiziert: TPR1, TPR2A und TPR2B (Figur 1A). Bei allen drei Domänen handelt es sich um TPR-Domänen.
2.
   Durch die isothermischen kalorimetrischen Messungen wurde gezeigt, daß auch ein 25kDa-Fragment von menschlichem Hsp70 (C70) weiterhin die Substratbindungsdomäne aufweist und daß der authentische C-Terminus von Hsp70, gebunden an die Domäne TPR1 mit einer Affinität von 15 µM und einem Stöchiometrie-Faktor (N) nahe bei 1 an die Domäne TPR1 bindet (Figuren 1B und 1C). Die gleichen Affinitäten wurden für ein Dodekamer mit Aminosäuresequenzen der C-Termini von Hsp70 und Hsc70, die nahezu identisch sind, als Liganden erhalten (Figur 1C). Ein 12kDa-Fragment von Hsp90 (C90), das die Dimerisierungsdomäne und den authentischen C-Terminus von Hsp90 (Aminosäuren 629-732) einschließt, bindet an die Domäne TPR2A mit einer Affinität von 6 µM und einem Stöchiometrie-Faktor nahe bei 1 (Figur 1C). Ein Peptid, das nur die letzten fünf Aminosäurereste von Hsp70 umfaßt, bindet immer noch an die Domäne TRP2A mit einer Affinität von 12 µM (Figur 1C) .
   Daraus ist zu schließen; daß die Bindung von TPR1 an C70 vollständig durch die Interaktion von TPR1 mit einem C-terminalen Dodekamer von Hsp70 beschrieben werden kann, während die Interaktion von TPR2A mit C90 im wesentlichen durch die fünf C-terminälen Aminosäuren von Hsp90, also ein Pentapeptid, vermittelt wird. Die gemessenen Affinitäten sind im übrigen vergleichbar mit jenen, die für die Interaktion von SH3-Domänen mit ihren Peptidliganden bestimmt wurden (Kuriyan und Cowburn, 1997, Annual Rev. Biophys. Biomol. Struct. *26*, 259-288).
3. Raumformen der Komplexe aus TPR-Domäne und den Peptidliganden
   Die beiden TPR-Domänen TPR1 und TPR2A von Hop wurden als Komplex mit ihrem jeweiligen Peptidliganden kristallisiert und deren hochaufgelöste Kristallstrukturen aufgeklärt. Ergebnis der Untersuchungen war eine als Kristallform vorliegende Raumform der beiden ggf. komplexierten Domänen. Beide TPR-Domänen bilden Mäander-förmige Strukturen aus sieben α-Helices (Figuren 2A und 2E), die in einer gegenläufigen Abfolge ("head to tail") ähnlich der Struktur der peptidfreien TPR-Domäne von PP5 (Das et al., 1998, EMBO J. 17, 1192-1199) angeordnet sind. Im Unterschied zur Sequenz von TPR1 weisen die TPR-Domänen PP5 und TPR2A eine Insertion zwisehen "repeat" 2 und 3 auf, was zu einer Verlängerung der Helices 2B und 3A um jeweils einen. "Turn" führt (Fig. 2E, Fig. 3). In beiden Strukturen erweist sich die Helix C, die C-terminal zu den drei TPR-Konsensusblöcken angeordnet ist, als integraler Bestandteil der TPR-Domäne (Figuren 2A, 2B, 2E und 2F).

Die TPR-Mäander bilden jetzt wiegenförmige ("cradle") Furchen, die die Peptide in einer ausgestreckten Konformation ("extended") aufnehmen (Figuren 2B, 2F, 2D und 2H). Die gebundenen Peptide stehen nur mit den Seitenketten der A-Helices der TPR-Domänen in Kontakt, die die innere Oberfläche der Wiege ergeben (Figuren 2B und 2F). Durch eine "simulated annealing" (2Fo-Fc)-Elektronendichtekarte des TPR1-gebundenen Peptids, die, ohne das Peptid-einzuschließen, berechnet wurde, zeigt sich, daß die letzten sieben Peptid-Aminosäurereste in dem Komplex mit der Domäne TPR1 sehr exakt definiert sind (Figur 2C). Eine ähnliche Elektronendichtekarte, die für das Peptid, das an die TPR2A-Struktur gebunden worden ist, berechnet wurde, zeigt, daß alle fünf Reste des gebundenen, Peptids gut strukturiert sind (Fig. 2G). Die ausgestreckte Konformation der Peptide stellt sicher, daß eine größtmögliche Oberfläche gegenüber den TPR-Domänen als Kontaktbereich zur Verfügung steht und daß derart die spezifische Erkennung von kurzen Aminosäurestücken mit ausreichender Affinität ermöglicht wird.

In der nachfolgenden Beschreibung der TPR-Peptid-Komplexe wird der C-terminale Asp-Rest der Peptide als Asp0 bezeichnet. Die vorhergehenden Reste werden in absteigender Reihenfolge mit Val-1, Glu-2, Glu-3, Ile-4, Thr-5, Pro-6 und Gly-7 für das Hsp70-Peptid (Figur 4A) bzw. als Met -4 für das Hsp90-Peptid (Figur 4B) numeriert. Nur die letzten acht Reste im Hsp70-Dodekamer-Peptid sind im TRP1-Komplex so geordnet, daß sie in der abschließenden Elektronendichtekarte erkennbar sind. Die Peptid-Seitenketten Pro-6, Ile-4, Val-1 und Asp0 sind an Wechselwirkungen mit der TPR1-Domäne beteiligt. Die übrigen Seitenketten im Peptidabschnitt mit geordneter Struktur, insbesondere die beiden nahezu absolut konservierten Glutaminsäure-Seitenketten an der Position -2 und -3, sind gegenüber dem Lösungsmittel exponiert (Fig. 2C; Fig. 4A). Im TPR2A-Komplex sind alle fünf Peptidreste in der abschließenden Elektronendichtekarte deutlich erkennbar. Mit Ausnahme von Glu-2 sind alle Seitenketten des Hsp90-Peptids an Wechselwirkungen mit der Domäne TPR2A beteiligt (Figur 4B). Alle elektrostatischen Kontakte zwischen den TPR-Domänen und den Peptiden sind ausschließlich für die Abschnitte des EEVD-Motivs zu verzeichnen, während die Abschnitte der gebundenen Peptide, die N-terminal zum EEVD-Motiv liegen, ausschließlich hydrophobe und van-der-Waals-Wechselwirkungen eingehen (Figuren 4A und 4B).

Drei Arten von Wasserstoffbrückenbindungswechselwirkungen sind an der Ausbildung der Peptidbindung an die Domänen TPR1 und TPR2A beteiligt: (a) sequenzunabhängige Interaktion mit dem Peptidrückgrat, (b) sequenzspezifische Interaktionen mit den Peptid-Seitenketten und (c) Kontakte mit dem C-terminalen Hauptketten-Carboxylat des letzten Peptidrestes Asp0 (Figuren 4A und 4B). Wie weiter unten beschrieben ist, bilden die zuletzt erwähnten Kontakte und die elektrostatischen Wechselwirkungen mit dem Hauptketten-Carboxylat von Asp0 eine sog. Doppel-Carboxylat-Klammer ("two-carboxylate clamp"), die hochkonserviert zwischen den beiden Komplexen ist.

Eine Vielzahl der direkten Wasserstoffbrückenbindungswechselwirkungen von den TPR-Domänen zu den Hsp-Peptiden richtet sich auf das Peptidrückgrat, so daß keine sequenzspezifischen Merkmale für die Bindung genutzt werden. Im TPR1-Komplex (Figur 4A) ist die Seitenketten-Carbonylgruppe von Asn43 (von TPR1) an direkten Rückgrat-Wechselwirkungen beteiligt, die mit dem Rückgrat-Amid von Asp0 des Hsp70-Peptids und der Amin-Seitenkette von Lys73 ausgebildet werden. Die Amin-Seitenkette von Lys73 bildet wiederum eine Wasserstoffbrückenbindung mit der Hauptketten-Carbonylgruppe des Peptidrestes Glu-2 aus. Arg77 der TPR1-Domäne spielt eine Schlüsselrolle in der Bindung des Rückgrats des Peptids. Ihre Guanidiniumgruppe geht drei direkte Wasserstoffbrückenbindungen mit den Peptid-Carbonylgruppen ein, und zwar eine an der -2 und zwei an der -3 Position. Eine zusätzliche Wasserstoffbrückenbindung an die Carbonylgruppe von Glu-3 wird durch ein fest gebundenes Wassermolekül, das durch Lys 50 in TPR1 positioniert wird, vermittelt (Figur 4A).

Im TPR2A-Komplex tritt die Carbonyl-Seitenkette von Asn264 in Kontakt mit dem Rückgrat-Amid von Asp0 des Hsp90-Peptids, die Guanidiniumgruppe von Arg305 bildet eine Wasserstoffbrücke mit der Hauptketten-Carbonylgruppe von Glu-2, die Hydroxylgruppe von Tyr236 eine Wasserstoffbrücke mit jener von Glu-3 und die Seitenkette von Glu271 tritt in Wechselwirkung mit der Amidgruppe von Glu-3. Zusätzlich ist ein Kontakt zwischen der Hydroxylgruppe von Tyr236 mit der Hauptketten-Carbonylgruppe von Glu-3 zu beobachten.

Die einzige Peptid-Seitenkette im TPR1-Komplex, die durch eine elektrostatische Wechselwirkung erkannt wird, ist die Seitenketten-Carboxylatgruppe von Asp0, die mit Lys73 interagiert (Figur 4A) . Ein zusätzlicher Seitenkettenkontakt wird durch ein geordnetes Wassermolekül vermittelt, das tetrahedral durch die Seitenketten-Carbonylgruppen von Asp0 und Asn43, die Guanidiniumgruppe von Arg77 und das Seitenketten-Hydroxyl von Ser42 koordiniert wird. Die elektrostatischen Wechselwirkungen von TPR2A mit dem Hsp90-Peptid sind jenen im TPR1-Komplex sehr ähnlich, mit Ausnahme einiger weniger Unterschiede.

TPR2A geht zwei Wasserstoffbrückenbindungen mit der Seitenkette von Asp0 via Lys301 einerseits und der Seitenketten-Amidgruppe von Gln298 andererseits ein. Genauso wie im TPR1-Komplex ist ein tetrahedral koordiniertes Wassermolekül in Kontakt mit der Asp0-Seitenkette, positioniert durch die Seitenketten-Carbonylgruppe von Asn264, die Guanidiniumgruppe von Arg355 und die Seitenketten-Amidgruppe von Asn233 (Figur 3C und Figur 4). Im Unterschied zum TPR1-Komplex ist im TPR2A-Komplex die Seitenkette von Glu-3 an einem festen Netzwerk aus Wasserstoffbrückenbindungen mit der TPR-Domäne beteiligt. Sie ist an direkten Kontakten zur Guanidiniumgruppe von Arg305 und der Seitenketten-Amidgruppe von Asn30B beteiligt, wobei sie zusätzlich in ein Netzwerk von indirekten Interaktionen, wobei geordnete Wassermoleküle involviert sind (Figur 4B), integriert ist.

In beiden TPR-komplexen wird das Hauptketten-Carboxylat von Asp0 in seiner Position durch drei zusätzliche starke Wasserstoffbrückenbindungen mit den Seitenketten-Aminen von Lys8 (Lys229), Asn12 (Asn233) und Asn43 (Asn264) der TPR1-bzw. TPR2A-Domäne fixiert. Über die zuvor beschriebenen elektrostatischen Interaktionen zwischen den TPR-Domänen und dem EEVD-Motiv der gebundenen Peptide hinaus ist der Peptidrest Val-1 an hydrophoben und van-der-Waals-Kontakten beteiligt, die ebenfalls im wesentlichen bei den beiden Komplexen konserviert sind (Figuren 4A und 4B).

Wie bereits beschrieben, bilden die fünf Aminosäurereste von TPR1, die an elektrostatischen Wechselwirkungen mit dem EEVD-Motiv beteiligt sind (Lys8, Asn12, Asn43, Lys73 und Arg77) eine Doppel-Carboxylat-Klammer ("two carboxylate clamp") , die fest mit dem endständigen Asp-Rest des gebundenen Peptids interagiert. Diese Reste zeigen deutliche strukturelle Gemeinsamkeiten mit den äquivalenten Resten von TPR2A (Lys229, Asn233, Asn264, Lys301 und Arg305), sobald ihre Cα-Atome übereinandergelagert werden (Figur 5). Die mittleren Standardabweichungen (rmsd) für die fünf Positionen der Doppel-Carboxylat-Klammer betragen 0,75 Å (verglichen mit 1,75 Å für die gesamten Domänen) . Außer Lys73 (Lys301) sind alle Seitenketten tatsächlich an der gleichen Position in den beiden Strukturen. Dieses "alignment" führt auch zu einer Überlagerung der beiden gebundenen Peptide. Dabei ist erkennbar, daß die Positionen Asp0 und Val-1 erstaunlich gut übereinander angeordnet sind, während die N-terminalen Abschnitte der beiden Peptide zunehmend vom C- zum N-Terminus divergieren, wobei jeweils von verschiedene Bereichen beim Eingehen von Wechselwirkungen mit der jeweiligen TPR-Domäne Gebrauch gemacht wird. Es ist zu bemerken, daß die Seitenkettenreste der Domänen TPR1 und TPR2A, die die Doppel-Carboxylat-Klammer bilden, bei allen TPR-Domänen, von denen bekannt sind, daß sie die C-terminalen Domänen von Hsp70/Hsc70 oder Hsp90 binden (Figur 3), hochkonserviert sind. Dies führt zu der Vermutung, daß diese TPR-Domänen an das C-terminale Carboxylat von Hsp70 oder Hsp90 durch ein sehr ähnliches Netzwerk von elektrostatischen Interaktionen binden (Figur 4).

Eine Protein-Datenbanksuche läßt erkennen, daß sieben zusätzliche TPR-Proteine, die ein C-terminales Aspartat via der Doppel-Carboxylat-Klammer zu binden in der Lage sein sollten, existieren, einschließlich mehrerer humaner Proteine, die an Krankheitsereignissen beteiligt sind (Figur 3). Insbesondere zu erwähnen sind die Proteine TTC1, TTC2, TTC3, TTC4, IRSP, SGT und KIAA0719, so daß erfindungsgemäß die Funktionalität dieser vorgenannten Proteine als TPR-Proteine mit Co-Chaperon-Fünktion für Hsp70 oder Hsp90 offenbart werden. Insbesondere werden daher erfindungsgemäß Verwendungen der vorgenannten und in Figur 3 aufgezählten Proteine als Co-Chaperone von Hsp70 und/oder Hsp90 offenbart.

Die Aminosäurereste, die stromaufwärts vom EEVD-Motiv gelegen sind, gewährleisten durch hydrophobe Interaktion die Hsp70-Spezifizität. Nach Maßgabe der erfindungsgemäßen Erkenntnisse sind die elektrostatischen Interaktionen der Domänen TPR1 und TPR2A mit dem EEVD-Motiv, wie oben beschrieben, nicht geeignet, zwischen den C-Termini von Hsp90 und Hsp70 eine Unterscheidung zu machen. Aus den Ergebnissen der Strukturaufklärung und den erfindungsgemäßen Raumformen der TPR/Peptid-Komplexe ist erkennbar, daß zusätzliche Kontakte mit den Peptidresten, die N-terminal zum EEVD-Motiv liegen, bestehen (Figuren 4A und 4B). Diese Wechselwirkungen sind entscheidend für die Peptidbindung, die mit einer physiologisch relevanten, hohen Affinität stattfinden muß (Figur 6A). Während das C-terminale Heptamerpeptid von Hsc70 an die TPR1-Domäne mit der gleichen Affinität wie die vollständige C-terminale Domäne von Hsp70/Hsc70 bindet, führt die Abspaltung der Aminosäurereste, die N-terminal zum EEVD-Motiv liegen, zu einem deutlichen Abfall der Affinität von 15-20 µM zu ungefähr 300 µM. Außerdem bindet das Peptid IEEVD, das bezüglich seiner Länge dem Hsp90-Peptid gleichkommt, mit einer Affinität von 140 µM signifikant schwächer als das Heptamer-Peptid (Figur 6A).

Der verborgene Oberflächenbereich für das strukturierte oktamerpeptid im TPR1-Komplex beträgt 1330 Å², aber nur 650 Å² für die vier Reste des IEEVD-Motivs. Die maßgeblichen Wechselwirkungen, die für die 20-fache Erhöhung der Affinität verantwortlich sind, beruhen demnach ausschließlich auf hydrophoben oder van-der-Waals-Interaktion von TPR1 mit den Seitenketten von Ile-4 und Pro-6 des Hsp70-Peptids (Fig. 4A). Jeder hydrophobe Kontakt des Peptids mit TPR1 ist im wesentlichen beschränkt auf eine spezifische A-Helix. Pro-6 ist in einer hydrophoben Tasche, die durch Glu83 und Phe84 der Helix A3 gebildet wird, angeordnet. Ile-4 bindet in eine Tasche, die durch die Aminosäurereste Ala46, Ala49 und Lys50 der Helix A2 (Fig. 4A) gebildet wird. Der hydrophobe Rest im EEVD-Motiv, nämlich Val-1, tritt in hydrophobe Wechselwirkungen mit Asn12 und Leu15 in der Helix A1 und mit Asn43 in der Helix A2 (Fig. 4A).

Das im TPR2A-Komplex gebundene Peptid ist deutlich kürzer als jenes, das im TPR1-Komplex gebunden ist. Der insgesamt verborgene Oberflächenbereich im TPR2A/MEEVD-Komplex beträgt 930Å² und 750Å² für die Reste EEVD. Nichtsdestotrotz liegen die für das Hsp90-Pentapeptid MEEVD gemessenen Affinitäten gegenüber TPR2A in der gleichen Größenordnung wie die Affinitäten des Hsp70-Peptids GPTIEEVD für TPR1, wobei längere Peptide wiederum nicht mit einer höheren Affinität binden (Fig. 6B). Die beiden zusätzlichen Wasserstoffbrückenbindungen, die durch TPR2A zum Glu-3 des Peptids eingegangen werden, scheinen die geringere hydrophobe Wechselwirkungsfläche auszugleichen. (Fig. 4B). Auch ist als Folge der stärkeren Wechselwirkung zwischen TPR2A und dem EEVD-Motiv festzustellen, daß der verborgene oberflächenbereich in diesem Fall ungefähr 100Å² größer ist als vergleichsweise für den verborgenen Oberflächenbereich, wie er sich für den Fall der Wechselwirkung von TPR1 in einem Komplex mit der Sequenz EEVD (ohne weitere N-terminale Aminosäuren) berechnet. Met-4 des Hsp90-Peptids ist an starken hydrophoben Interaktionen mit einer Tasche, die im wesentlichen durch die Seitenketten von Tyr236 und Glu271 gebildet wird, beteiligt. Val-1 des EEVD-Abschnitts bindet an eine hydrophobe Tasche, die durch Asn233, Asn264 und Ala267 gebildet wird.

Infolge mit den stärkeren Interaktionen in dem Hsp90-EEVD-Abschnitt führt eine Verkürzung des Pentapeptids MEEVD zu EEVD zwar zu einem signifikanten, aber gleichwohl mäßigen Bindungsaffinitätsverlust (von 11µm zu 90 µM), während die Domäne TPR1 das EEVD-Peptid nur mit einer Affinität von 300 µM bindet (Fig. 6A und Fig. 6B).

Die Abschnitte, die N-terminal zum EEVD-Motiv liegen, unterscheiden sich bei den C-Termini von Hsp70/Hsc70 und Hsp90-Proteinen in auffälliger Weise (Fig. 7A und B), was vermuten läßt, daß diese Sequenzen nicht nur für das Bindungsverhalten mit hoher Affinität wichtig sind, sondern auch für die TPR-Spezifität verantwortlich sind. Die acht C-terminalen Aminosäuren sind nahezu vollständig in allen cytosolischen Varianten von eukaryontischen Hsp70 und Hsc70-Proteinen (Konsensus: GPTIEEVD) konserviert. Außerdem handelt es sich bei diesen Aminosäureresten auch um jene, die in der Kristallstruktur des TPR1-Peptid-Komplexes strukturell gut definiert sind. Diese Sequenz ist mit großer Wahrscheinlichkeit Lösungsmittelexponiert, da sie mit der Peptidbindungsdomäne von Hsp70/Hsc70 über einen flexiblen "Linker"-Bereich von ungefähr neun Aminosäuren, bestehend aus einer Folge von Ala, Ser und Gly-Resten (Fig. 7A), verbunden ist. Der Sequenzvergleich ("Alignment") der C-Termini von cytosolischen varianten von eukaryontischen Hsp90 läßt vermuten, daß hier eine ähnliche Wechselwirkung von Hsp90-Proteinen mit ihren jeweiligen TPR-Bindungspartnern vorliegt (Fig. 7B). In Übereinstimmung mit den thermodynamischen Ergebnissen ergibt sich, daß ein hohes Maß an sequenzieller Konservierung für die letzten fünf Aminosäuren (Konsensussequenz: MEEVD) in Hinblick auf die TPR-Bindung wichtig ist.

Ein struktureller Vergleich (Fig. 5) erklärt auch, auf welche Weise die spezifische Bindung von Hsp70 und Hsp90 an die TPR-Domäne von Hop-Protein erzielt wird. Während die letzten beiden Aminosäurereste der Hsp70 und Hsp90-Peptide an vergleichbaren bzw. äquivalenten Stellen in ihren jeweiligen TPR-Domänen gebunden sind, ist für die weiter N-terminal gelegenen Abschnitte der Peptide eine Bindung an stark verschiedene TPR-Bereiche zu beobachten. Während der gänzlich konservierte VD-Teil der beiden Peptide in jeder der beiden TPR-Domänen durch elektrostatische Wechselwirkungen über die konservierte Doppel-Carboxylat-Klammer ("two-carboxylate clamp") angeordnet ist, verwenden die divergenten N-terminalen Bereiche der Peptide unterschiedliche hydrophobe Höhlen bzw. Aussparungen in den TPR-Domänen für die Selektivität der Bindung. Nur schwäche, nicht spezifische Bindung an TPR1 wurde für die C-terminale Domäne von Hsp90 (C90) und für von C90 abstammende Peptide beobachtet, wobei die Affinitäten in der gleichen Größenordnung liegen, wie sie für ein Peptid mit der vollständig konservierten EEVD-Sequenz allein beobachtet wurden (Fig. 6A). Zu ähnlichen Ergebnissen führt die Wechselwirkung der Domäne TPR2A mit C70 oder von C70 stammenden Peptiden aus der C-terminalen Bindungsdomäne von Hsp70 (Fig. 6B). In diesem Fall binden kurze C70-Peptide an die Domäne TPR2A mit einer Affinität, die auch für das Tetrapeptid EEVD beobachtet wurde, während die C70-Domäne mit einer signifikant reduzierten Affinität (250 µM) bindet. Dies ist darauf zurückzuführen, daß sterische Probleme zwischen der C70-Domäne und TPR2A bestehen.

**Tabelle 1**

| Statistische Angaben zur Datensammlung und der Verfeinerung für den TPR1-Komplex | |
|---|---|
| **Datensammlung** | |
| Röntgenquelle | NSLS, X12B^{b} λ = 0.949 Å, T = 100 K |
| | |
| Raumgruppe | P4₁ |
| Zellparameter | a = 75.47 Å, c = 42.89 Å |
| Zahl der Moleküe/asymmetr.Einheit | 2 |
| | |
| Zahl der Reflexe/Individualreflexe | 146181/30804 |
| Auflösung (Å) | 20.0 - 1.60 (1.66-1.60) |
| Vollständigkeit des Datensatzes (%)^{z} | 95.8 (75.2) |
| <I> / *<σ*_{*l*}*>* | 32.5 (3.3) |
| Rsym (%) | 4.1 (30.5) |

| **Model Refinement**^{**d**} | |
|---|---|
| Auflösung (Å) | 20.0 - 1.60 |
| Zahl der verwendeten Reflexe | 30804 |
| Zahl der Reflexe in R_{free} Satz | 3100 |
| R_{work} (%)^{e} | 18.2^{f} |
| R_{free} (%)^{e} | 21.6^{f} |
| Zahl der Protein-/Ligandenatome | 1840 / 120 |
| Zahl der Heteroatome | 36 |
| Zahl der Lösungsmittelmoleküle | 245 |
| R.m.s.d. Bindungslänge (Å) | 0.008 |
| R.m.s.d. Bindungswinkel (°) | 1.3 |

| | |
|---|---|
| ^{a}Values as defined in SCALEPACK (Otwinowski and Minor, 1997) ^{b}National Synchrotron Light Source in Brookhaven, beamtine X12B | |
| ^{c}Drop in higher resolution shell due to rectangular detector shape ^{d}Values as defined in SHELXL (Sheldrick 1997) for peptide free *I* CNS (Brünger et. al., 1998) for complex | |
| ^{e}No sigma cutoff | |
| ^{f}No NCS restrains | |

**Tabelle 2**

| Statistische Angaben zur Datensammlung und der Verfeinerung für den TPR2A-Komplex | | | | | |
|---|---|---|---|---|---|
| **Datensammlung** | | | | | |
| Raumgruppe Einheitszelle | C2 a = 73.28 Å, b = 48.27 Å, c = 38.06 Å, b = 91.30° | | | | |

| | Native^{a} | Ni1^{b} | Ni2^{b} | Se1^{b} | Se2^{b} |
|---|---|---|---|---|---|
| Röntgenquelle | ESRF, ID14-3^{c} | | DESY, BW6^{d} | | |
| Auflösung (Å) | 15-1.9 (1.95-1.90) | | 17-2.1 (2.14-2.10) | | |
| | | | | | |
| Wellenlänge (Å) | I = 0.9402 | I = 1.4828 | I = 1.4840 | I = 0.9793 | I = 0.9798 |
| | | | | | |
| **I/s**_{**l**} | 22.8 (9.0) | 22.3 (10.2) | 22.8 (10.1) | 23.8 (11.1) | 25.6 (10.6) |
| Vollständigkeit des Datensatzes (%) | 97.1 (91.7) | 93.0 (94.9) | 98.8 (98.6) | 96.8 (96.8) | 88.0(90.1) |
| R_{merge} (%)^{e} | 3.8(6.8) | 2.8 (8.3) | 3.3 (10.5) | 3.3(8.8) | 3.1 (9.0) |

| **MAD Phasing**^{**f**} | | | | | |
|---|---|---|---|---|---|
| Anomalous scatterer | | | 1 Ni, 2 Se | | |
| Auflösung (Å) | | | 17.0-2.1 | | |
| Figure of merit | | | 0.74 | | |
| | | | | | |
| R_{cullis_ano} | | 0.64 | 0.87 | 0.65 | 0.85 |
| Phasing power centric | | 0.48 | - | 1.51 | 1.30 |
| Phasing power acentric | | 0.65 | - | 2.46 | 2.27 |

| **Model Refinefement**^{**g**} | | | | | |
|---|---|---|---|---|---|
| Auflösung (Å) | 10-1.9 | Zahl der Protein-/Ligandenatome | | 1042 /44 | |
| Zahl der Reflexe für · R_{work} | 9217 | Zahl der Heteroatome (Ni) | | 1 | |
| Zahl der Reflexe für · R_{free} | 1003 | Zahl der Lösungsmittelmolküle | | 152 | |
| R_{work} (%)^{h} | 18.1 | R.m.s.d. Bindungslänge (Å) | | 0.008 | |
| R_{free} (%)^{h} | 21.9 | R.m.s.d. Bindungswinkel(°) | | 1.2 | |

| | | | | | |
|---|---|---|---|---|---|
| ^{a}values as defined in XDS (Kabsch. 1993), | | | | | |
| ^{b}values as defined in SCALEPACK (Otwinowski and Minor. 1997), | | | | | |
| ^{c}European Synchrotron Radiation Facility in Grenoble, beamline ID14-3, | | | | | |
| ^{d}Deutsches Elektronen Synchroton in Hamburg. beamline BW6, | | | | | |
| ^{e}Bijvoet pairs separated except for the native data set, | | | | | |
| ^{f}Unweighted values as defined In MLPHARE (CCP4. 1994), ^{g}Values as defined in CNS (Brünger et al., 1998), | | | | | |
| ^{h}No sigma cutoff | | | | | |

## Patentansprüche

1. Verfahren zur Herstellung eines Kristalls mit Einheitszellen, enthaltend in der asymmetrischen Einheit mindestens ein Polypeptid und ggf. mindestens eine weitere Verbindung, wobei das Polypeptid in der asymmetrischen Einheit mindestens eine Aminosäuresequenz eines TPR-Strukturmotivs eines Hop-Proteins enthält, **dadurch gekennzeichnet, dass**
(a) das Polypeptid in einem Expressionssystem überexprimiert wird,
(b) das überexprimierte Polypeptid gereinigt und aufkonzentriert wird,
(c) das gemäß (b) erhaltene Polypeptidkonzentrat in einem geeigneten Puffersystem, ggf. unter Hinzufügen mindestens einer weiteren Verbindung, gelöst wird und
(d) die Kristallisierung bei 20°C durch die Technik des "hängenden Tropfens" durch Dampfdiffusionsverfahren eingeleitet wird.

2. Kristall erhältlich nach einem Verfahren nach Anspruch 1.

3. Kristall nach Anspruch 2 enthaltend pro asymmetrischer Einheit in der Einheitszelle des Kristalls mindestens ein Polypeptid und gegebenenfalls mindestens eine weitere Verbindung, **dadurch gekennzeichnet, dass** die Polypeptide im Kristall eine Raumform einnehmen, wobei das Polypeptid in der Raumform mindestens eine Aminosäuresequenz eines TPR-Strukturmotivs eines Hop-Proteins enthält.

4. Kristall nach Anspruch 2 oder 3, **dadurch gekennzeichnet, dass** das Polypeptid in der Raumform mindestens eine der in Figur 3A wiedergegebenen Aminosäuresequenzen (1), (2), (3), (4), (5), (6), (7), (8) und/oder (9) enthält.

5. Kristall nach einem der vorgenannten Ansprüche 2 bis 4, **dadurch gekennzeichnet, dass** das Polypeptid in der Raumform mindestens eine der Aminosäuresequenzen der Domäne TPR1, TPR2A oder TPR2B eines Hop-Proteins oder eines Abschnitts/e derselben enthält.

6. Kristall nach einem der vorgenannten Ansprüche 2 bis 5, **dadurch gekennzeichnet, dass** das Polypeptid in der Raumform mindestens eine der Aminosäuresequenzen (1), (2) oder (3), wie in Figur 3B wiedergegeben, enthält.

7. Kristall nach einem der vorgenannten Ansprüche 2 bis 6, **dadurch gekennzeichnet, dass** die in dem Polypeptid enthaltene mindestens eine Aminosäuresequenz eines TPR-Struktucmotivs oder einer TPR-Domäne einer Aminosäuresequenz(en) eines Hop-Proteins eukaryotischen Ursprungs entspricht.

8. Kristall nach einem der vorgenannten Ansprüche 2 bis 7, **dadurch gekennzeichnet, dass** die in dem Polypeptid enthaltene Aminosäuresequenz eines TPR-Strukturmotivs oder einer TPR-Domäne einer Aminosäuresequenz(en) eines Hop-Proteins humanen Ursprungs entspricht.

9. Kristall nach einem der vorgenannten Ansprüche 2 bis 8, **dadurch gekennzeichnet, dass** das Polypeptid die Aminosäuresequenz eines eukaryotischen Hop-Proteins aufweist.

10. Kristall nach einem der vorgenannten Ansprüche 2 bis 9, **dadurch gekennzeichnet, dass** die Raumform das Polypeptid und mindestens eine weitere Verbindung aufweist.

11. Kristall nach einem der vorgenannten Ansprüche 2 bis 10, **dadurch gekennzeichnet, dass** die Raumform das Polypeptid und mindestens eine Verbindung aufweist, die als Ligand an das Polypeptid bindet.

12. Kristall nach Anspruch 10 oder 11, **dadurch gekennzeichnet, dass** die Verbindung oder der Ligand ein nicht physiologisch auftretendes Molekül ist.

13. Kristall nach einem der vorgenannten Ansprüche 2 bis 12, **dadurch gekennzeichnet, dass** die Raumform ein Polypeptid und mindestens einen physiologischen Liganden oder mindestens einen Abschnitt eines physiologischen Liganden für das Polypeptid aufweist

14. Kristall nach einem der vorgenannten Ansprüche 2 bis 13, **dadurch gekennzeichnet, dass** die Rautnform ein Polypeptid und mindestens einen Liganden oder einen Abschnitt eines Liganden für das Polypeptid aufweist, wobei der Ligand an die Aminosäuresequenz eines TPR-Strukturmotivs, insbesondere an die Aminosäuresequenz einer TPR-Domäne, bindet.

15. Kristall nach einem der vorgenannten Ansprüche 10 bis 14, **dadurch gekennzeichnet, dass** der Ligand ein Polypeptid, Oligopeptid, Dipeptid oder ein syntetisch modifiziertes Derivat eines Poly-, Oligo- oder Dipeptids ist.

16. Kristall nach einem der vorgenannten Ansprüche 10 bis 15, **dadurch gekennzeichnet, dass** der Ligand eine Aminosäuresequenz oder einen Abschnitt einer Aminosäuresequenz eines Proteins aus der Klasse der Chaperon-Proteine oder ein Derivat eines/r solchen Aminosäuresequenz oder eines solchen Abschnitts enthält.

17. Kristall nach einem der vorgenannten Ansprüche 10 bis 16, **dadurch gekennzeichnet, dass** der Ligand einen Abschnitt der C-terminalen Aminosäuresequenz eines Chaperon-Proteins aufweist.

18. Kristall nach einem der vorgenannten Ansprüche 10 bis 17, **dadurch gekennzeichnet, dass** der Ligand eine Aminosäuresequenz oder einen Abschnitt einer Aminosäuresequenz des Chaperon-Proteins Hsp70 und/oder Hsp90 aufweist

19. Kristall nach einem der vorgenannten Ansprüche 10 bis 18, **dadurch gekennzeichnet, dass** der Ligand ein Inhibitor der physiologischen Funktion, insbesondere der Adapterfunktion, eines Hop-Proteins ist.

20. Kristall nach einem der vorgenannten Ansprüche 10 bis 19, **dadurch gekennzeichnet, dass** der Ligand ein Inhibitor der Interaktionen zwischen den Proteinen Hop und Hsp70 und/oder den Proteinen Hop und Hsp90 ist

21. Kristall nach einem der vorgenannten Ansprüche 2 bis 20, **dadurch gekennzeichnet, dass** die Kristallform Metall-Ionen, insbesondere Schivermetall-Ionen, enthält.

22. Kristall nach einem der vorgenannten Ansprüche 2 bis 21, **dadurch gekennzeichnet**, - dass die Kristallform des Polypeptids mindestens ein TPR-Strukturmotiv eines Hop-Proteins (Struktur der Sequenzen (1), (2), (4), (5), (7) und/oder (8) gemäß Figur 3A) mit Strukturkoordinaten der entsprechenden Sequenzen, wie in Figur 3C und 3D bzw. Fig. 3E (für die Sequenzen (2), (5) und (8)) wiedergegeben, enthält.

23. Kristall nach einem der vorgenannten Ansprüche 2 bis 22, **dadurch gekennzeichnet, dass** die Kristallform des Polypeptids mindestens eine TPR-Domäne eines Hop-Proteins (Struktur der Sequenzen (1) und (2) gemäß Figur 3B) mit den entsprechenden Strukturkoordinaten, wie in Figur 3C und Figur 3D für TPR1 und Figur 3E für TPR2A wiedergegeben, enthält.

24. Kristall nach einem der vorgenannten Ansprüche 2 bis 23, **dadurch gekennzeichnet, dass** die Raumgruppe des die Kristallform aufweisenden Kristalls monoklin, tetragonal, orthorhombisch, kubisch, triklin, hexagonal oder trigonal/rhombohedcal ist.

25. Kristall nach einem der vorgenannten Ansprüche 2 bis 24, **dadurch gekennzeichnet, dass** die Raumgruppe des Kristalls P2₁, C2 oder P4₁ ist

26. Kristall nach einem der vorgenannten Ansprüche 2 bis 25, **dadurch gekennzeichnet, dass** die Einheitszelle des die Kzistallform enthaltenden. Kristalls Zellkonstanten von ungefähr a = 31,2 Å, b = 43,8 Å, c = 38,3 Å und β = 101,8° oder a = 75,5 Å und c = 42,9 Å aufweist.

27. Verbindung, **dadurch gekennzeichnet, dass** die Verbindung die Eigenschaft aufweist, als Ligand an Strukturbereiche eines Hop-Proteins zu binden, wobei die Verbindung nicht-kovalente Wechselwirkung mit der Hauptkette und/oder den Seitenketten von Aminosäuren, die Bestandteil einer TPR-Domäne eines Hop-Proteins sind, eingeht und wobei die Verbindung ein modifiziertes oder unmodifiziertes Oligopeptid ist, das die Aminosäuresequenz EEVD enthält, zur Anwendung als Arzneimittel

28. Verbindung nach Anspruch 27, **dadurch gekennzeichnet, dass** die Verbindung ein Oligopeptid ist, das die Aminosäuresequenz GPXIEEVD oder SXMEEVD enthält, wobei X für eine beliebige, natürlich auftretende Aminosäure steht, zur Anwendung als Arzneimittel.

29. Verbindung nach einem der Ansprüche 27 bis 28, **dadurch gekennzeichnet, dass** die Verbindung die in den Ansprüchen 27 oder 28 genannten Aminosäuresequenzen am C-Terminus aufweist.

30. Verwendung einer Verbindung nach einem der Ansprüche 27 bis 29 zur Herstellung eines Arzneimittels zur Behandlung von Tumorerkrankungen, zur Immunsuppression, insbesondere von Autoimmunerkrankungen oder anderen Entzündungserkrankungen, GvHD oder zur Behandlung von Virusinfektionen.

31. Verfahren zur Identifizierung einer Verbindung mit der Eigenschaft, als Inhibitor der Wechselwirkung zwischen einem Hop-Protein und einem Chaperon-Protein, insbesondere Hsp70 oder Hsp90, zu wirken, **dadurch gekennzeichnet, dass**
a) eine Raumform eines Kristalls nach einem der Ansprüche 2 bis 26 durch Strukturaufklärung erhalten wird,
b) die Struktur der Raumform mit Hilfe der Strukturkoordinaten dreidimensional dargestellt wird,
c) sterische Eigenschaften und/oder funktionelle Gruppen einer Verbindung so gewählt werden, dass Wechselwirkungen zwischen der Verbindung und der Hauptund/oder den Seitenketten des Polypeptids in dem Bindungsbereich ausgebildet oder ggf. optimiert werden.

32. Verfahren nach Anspruch 31, **dadurch gekennzeichnet, dass** in einem Verfahrensschritt (d) die dreidimensionale Struktur der gemäß (c) erhaltenen Verbindung ermittelt und in die Polppeptidstruktur modelliert wird.

33. Verfahren nach Anspruch 31 oder 32, **dadurch gekennzeichnet, dass** in einem Verfahrensschritt (e) die Stärke der Wechselwirkung zwischen der Verbindung und dem aufgefalteten Polypeptid gemäß einer Raumform eines Kristalls nach einem der Ansprüche 2 bis 26 bestimmt wird.

34. Verfahren nach einem der Ansprüche 31 bis 33, **dadurch gekennzeichnet, dass** gemäß Verfahrensschritt (b) alle oder ein Teil der Strukturkoordinaten aus Figur 3D, 3C und 3E dreidimensional dargestellt werden.

35. Verfahren nach einem der Ansprüche 31 bis 34, **dadurch gekennzeichnet, dass** die Verfahrensschritte (c) , (d) und (e) zyklisch so lange wiederholt werden, bis die gemäß (e) erhaltene Stärke der Wechselwirkung zwischen der Verbindung und der Polypeptidstruktur in einer Raumform mit einer Struktur eines Kristalls gemäß einem der Ansprüche 2 bis 26 optimiert wird.

36. Verfahren nach einem der Ansprüche 31 bis 35, **dadurch gekennzeichnet, dass** in einem Verfahrensschritt (f) die Eigenschaften, insbesondere die inhibitorischen Eigenschaften, der modellierten Verbindung in einem biologischen Testsystem ermittelt werden.

37. Verfahren zur Identifizierung einer Verbindung, mit der Eigenschaft, als Inhibitor der Wechselwirkung zwischen einem Hop-Protein und mindestens einem Chaperon-Protein, insbesondere Hsp70 oder Hsp90, zu wirken, **dadurch gekennzeichnet, dass**
(a) ein biologisches Testsystem für derartige potentielle Inhibitor-Verbindungen etabliert wird,
(b) als Inhibitoren wirkende Verbindungen durch ein biologisches Testsystem gemäß (a) ermittelt werden,
(c) die Konformation der Verbindung bestimmt wird,
(d) die Struktur eines Polypeptids aus einer Raumform eines Kristalls, wie nach einem der Ansprüche 2 bis 26 gegeben, durch deren Strukturkoordinaten 3-dimensional graphisch dargestellt wird und
(e) die gemäß (b) und (c) erhaltene Struktur der Verbindung in die gemäß (d) erhaltene Struktur des Polypeptids eingefügt wird.

38. Verfahren nach Anspruch 37, **dadurch gekennzeichnet, dass** in einem Verfahrensschritt (f) Art und/oder Stärke der Wechselwirkung zwischen der Verbindung und der Raumform eines Polypeptids bestimmt wird/werden.

39. Verfahren zur Darstellung einer 3-dimensionalen Struktur eines Polypeptids unbekannter Struktur, mit mindestens einem Polypeptid, das mindestens ein an der Bindung an ein Chaperon-Protein beteiligtes TPR-Strukturmotiv oder vorzugsweise TPR-Strukturdomäne enthält, **dadurch gekennzeichnet, dass** die unbekannte Struktur des Polypeptids auf der Basis einer bekannten Raumform eines Kristalls nach einem der Ansprüche 2 bis 26 ermittelt wird.

40. Verfahren nach Anspruch 39, **dadurch gekennzeichnet, dass** zur Aufklärung der unbekannten Struktur Strukturkoordinaten einer Raumform eines Kristalls nach einem der Ansprüche 2 bis 26, insbesondere Strukturkoordinaten wie in Figur 3C, 3D oder 3E dargestellt, eingesetzt werden.

41. Verfahren nach Anspruch 39 oder 40, **dadurch gekennzeichnet, dass**
(a) die Primärsequenz eines Polypeptids unbekannter 3D-Struktuz mit der Primärsequenz eines Polypeptids bekannter Struktur, das mindestens ein TPR-Strukturmotiv oder eine TPR-Domäne aufweist, verglichen wird,
(b) für gemäß (a) erhaltene homologe Abschnitte die 3D-Struktur des Polypeptids unbekannter Struktur in Anlehnung an die Struktur aus einer Raumform eines Kristalls nach einem der Ansprüche 2 bis 26 modelliert wird, und
(c) mit Hilfe entsprechender Computer-Programme energetische Optimierungen der gemäß (b) modellierten Struktur oder Strukturbereiche durchgeführt werden.

42. Verfahren zur Identifizierung einer Verbindung, die die Wechselwirkung zwischen einem Polypeptid, enthaltend mindestens eine Aminosäuresequenz eines TPR-Strukturmotivs eines Hop-Proteins oder ein Derivat einer solchen Aminosäuresequenz, insbesondere einem Hop-Protein, unbekannter 3D-Struktur und einem Chaperon-Protein, insbesondere Hsp70 und/oder Hsp90, inhibiert, **dadurch gekennzeichnet, dass**
(a) die unbekannte 3D-Struktur des Polypeptids nach einem Verfahren nach einem der Ansprüche 39 bis 41 ermittelt wird und
(b) mit Hilfe eines Verfahrens nach einem der Ansprüche 31 bis 38 eine Verbindung, mit der Eigenschaft als Inhibitor der Wechselwirkung eines Chaperon-Proteins mit einem Polypeptid unbekannter 3D-Struktur zu wirken, ermittelt wird.

## Claims

1. A method to produce a crystal with unit cells, which contain in the asymmetric unit at least one polypeptide and, optionally, at least one other compound, whereby the polypeptide in the asymmetric unit contains at least one amino acid sequence of a TPR structure motif of a Hop-Protein, **characterised in that**
a. the polypeptide is overexpressed in an expression system,
b. the overexpressed polypeptide is purified and re-concentrated,
c. the polypeptide concentrate which has been obtained according to (b) is dissolved in a suitable buffer system, possibly by adding at least one other compound and
d. crystallisation is initiated at 20° Cby the ''hanging drop technique", using a vapour diffusion procedure.

2. A Crystal obtainable according to a method of claim 1.

3. A crystal according to claim 2, containing per asymmetric unit in the unit cell of the crystal at least one polypeptide and, optionally, at least one other compound, **characterised in that** the polypeptides assume a spatial configuration in the crystal, whereby the polypeptide in the spatial configuration contains at least one amino acid sequence of a TPR structure motif of a Hop protein.

4. Crystal according to claim 2 or 3, **characterised in that** the polypeptide in its spatial configuration contains at least one of the amino acid sequences (1), (2), (3), (4), (5), (6), (7), (8) and/or (9) shown in figure 3A.

5. Crystal according to any one of claims 2 to 4, **characterised in that** the polypeptide in its spatial configuration contains at least one of the amino acid sequences of domain TPR1, TPR2A or TPR2B of a Hop protein or a fragment/fragments thereof.

6. Crystal according to any one of claims 2 to 5, **characterised in that** the polypeptide in its spatial configuration contains at least one of the amino acid sequences (1), (2) or (3), as shown in figure 3B.

7. Crystal according to any one of claims 2 to 6, **characterised in that** at least one amino acid sequence of a TPR-structure motif or a TPR-domain contained in the polypeptide corresponds to an amino acid sequence/sequences of a Hop protein of eukaryotic origin.

8. Crystal according to any one of claims 2 to 7, **characterised in that** the amino acid sequence of a TPR structure motif or a TPR domain contained in the polypeptide corresponds to an amino acid sequence/sequences of a Hop protein of human origin.

9. Crystal according to any one of claims 2 to 8, **characterised in that** the polypeptide has the amino acid sequence of a eukaryotic Hop protein.

10. Crystal according to any one of claims 2 to 9, **characterised in that** the spatial configuration comprises the polypeptide and at least one further compound.

11. Crystal according to any one of claims 2 to 10, **characterised in that** the spatial configuration comprises the polypeptide and at least one other compound which binds to the polypeptide as a ligand.

12. Crystal according to claim 10 or 11, **characterised in that** the compound or the ligand is not a molecule occurring physiologically.

13. Crystal according to any one of claims 2 to 12, **characterised in that** the spatial configuration comprises a polypeptide and at least one physiological ligand or at least one fragment of a physiological ligand for the polypeptide.

14. Crystal according to any one of claims 2 to 13, **characterised in that** the spatial configuration comprises a polypeptide and at least one ligand or a fragment of a ligand for the polypeptide, whereby the ligand binds to the amino acid sequence of a TPR structure motif, in particular to the amino acid sequence of a TPR domain.

15. Crystal according to any one of claims 10 to 14, **characterised in that** the ligand is a polypeptide, oligopeptide, dipeptide or a synthetically modified derivative of a poly-, oligo- or dipeptide.

16. Crystal according to any one of claims 10 to 15, **characterised in that** the ligand contains an amino acid sequence or a fragment of an amino acid sequence of a protein from the class of chaperone proteins or a derivative of such an amino acid sequence or fragment.

17. Crystal according to any one of claims 10 to 16, **characterised in that** the ligand comprises a fragment of a C-terminal amino acid sequence of a chaperone protein.

18. Crystal according to any one of claims 10 to 17, **characterised in that** the ligand comprises an amino acid sequence or a fragment of an amino acid sequence of the chaperone protein Hsp70 and/or Hsp90.

19. Crystal according to any one of claims 10 to 18, **characterised in that** the ligand is an inhibitor of the physiological function, in particular the adapter function, of a Hop protein.

20. Crystal according to any one of claims 10 to 19, **characterised in that** the ligand is an inhibitor of the interactions between the proteins Hop and Hsp70 and/or the proteins Hop and Hsp90.

21. Crystal according to any one of claims 2 to 20, **characterised in that** the crystal structure contains metal ions, in particular heavy metal ions.

22. Crystal according to any one of claims 2 to 21, **characterised in that** the crystal structure of the polypeptide contains at least one TPR structure motif of a Hop protein (structure of sequences (1), (2), (4), (5), (7) and/or (8) pursuant to figure 3A) with structure coordinates of the respective sequences as detailed in figure 3C and 3D or figure 3E (for the sequences (2), (5) and (8)).

23. Crystal according to any one of claims 2 to 22, **characterised in that** the crystal structure of the polypeptide contains at least one TPR domain of a Hop protein (structure of the sequences (1) and (2) pursuant to figure 3B) with the respective structure coordinates, as detailed in figure 3C and figure 3D for TPR1 and figure 3E for TPR2A.

24. The crystal according to any one of claims 2 to 23, **characterised in that** the space group of the crystal featuring the crystal structure is monoclinic, tetragonal, orthorhombic, cubic, triclinic, hexagonal or trigonal/rhombohedral.

25. The crystal according to any one of claims 2 to 24, **characterised in that** the space group of the crystal is P2₁, C2 or P4₁.

26. The crystal according to any one of claims 2 to 25, **characterised in that** the unit cell of the crystal, which contains the crystal structure, has cell constants of approximately a = 31.2 Å, b= 43.8 Å,c = 38.3 Å and β = 101.8° or a = 75.5 Å and c = 42.9 Å.

27. A compound, **characterised in that** the compound has the property to bind, as a ligand, to structure areas of a Hop protein, whereby the compound enters into non-covalent interaction with the main chain and/or the side chains of the amino acids, which are a component of a TPR domain of a Hop protein, and whereby the compound is a modified or unmodified oligopeptide which contains the amino acid sequence EEVD for use as a medicament.

28. The compound according to claim 27, **characterised in that** the compound is an oligopeptide, which contains the amino acid sequence GPXIEEVD or SXMEEVD, whereby X indicates any kind of naturally occurring amino acid, for use as a medicament.

29. The compound according to claims 27 or 28, **characterised in that** the compound comprises amino acid sequences at the C-terminus, which are defined in any one of claims 27 or 28.

30. The use of a compound according to any of claims 27 to 29 for the preparation of a medicament for the treatment of tumour disease states, immunosuppression, in particular autoimmune disease states or other inflammatory disease states, GvHD or for the treatment of viral infections.

31. A method for the identification of a compound with the characteristic of acting as an inhibitor to the interaction between a Hop protein and a chaperone protein, in particular Hsp70 or Hsp90, **characterised in that**
a. a spatial configuration of a crystal according to any one of claims 2 to 26 is obtained by a structure elucidation,
b. the structure of the spatial configuration is represented three-dimensionally by way of structure coordinates,
c. steric attributes and/or functional groups of a compound are selected in such a manner that interactions between the compound and the main chain and/or the side chains of the polypeptide at the binding site are formed or, optionally, optimised.

32. The method according to claim 31, **characterised in that** in step (d) the three-dimensional structure of the compound, obtained according to (c), is determined and modelled into the polypeptide structure.

33. The method according to claims 31 or 32, **characterised in that** in step (e) the strength of the interaction between the compound and the folded polypeptide in accordance with a spatial configuration of a crystal according to any one of claims 2 to 26 is determined.

34. The method according to any one of claims 31 to 33, **characterised in that** in step (b), all or a part of the structure coordinates of figure 3D, 3C and 3E are depicted three-dimensionally.

35. The method according to any one of claims 31 to 34, **characterised in that** the steps (c), (d) and (e) are iteratively repeated until the strength of the interaction obtained according to (e) between the compound and the polypeptide structure in a spatial configuration with a structure of a crystal according to any one of claims 2 to 26 is optimised.

36. The method according to any one of claims 31 to 35, **characterised in that** in step (f) the characteristics, in particular the inhibitory characteristics, of the modelled compound are ascertained in a biological test system.

37. The method for the identification of a compound with the characteristic to act as an inhibitor of the interaction between a Hop protein and at least one chaperone protein, in particular Hsp70 or Hsp90, **characterised in that**
a. a biological test system for such potential inhibitor compounds is set up,
b. compounds acting as inhibitors are ascertained by a biological test system according to (a),
c. the conformation of the compound is ascertained,
d. the structure of a polypeptide from a spatial configuration of a crystal, as defined in any one of the claims 2 to 26, is graphically depicted in 3D by its structure-coordinates and
e. the compound, obtained according to (b) and (c) is inserted into the structure of a polypeptide obtained according to (d).

38. The method according to claim 37, **characterised in that** in further step (f), the type and/or strength of the interaction between the compound and the spatial configuration of a polypeptide are/is determined.

39. A method to depict a three-dimensional structure of a polypeptide of unknown structure, having at least one polypeptide containing at least one TPR structure motif or preferably at least one TPR structure domain being involved in the binding to a chaperon protein, **characterised in that** the unknown structure of the polypeptide is determined on the basis of a known spatial configuration of a crystal according to any one of claims 2 to 26.

40. The method according to claim 39, **characterised in that** for the elucidation of an unknown structure, structure coordinates of a spatial configuration of a crystal according to any one of claims 2 to 26 are used, in particular structure coordinates as shown in figure 3C, 3D, or 3E.

41. The method according to claims 39 or 40, **characterised in that**
a. the primary sequence of a polypeptide with an unknown 3D structure is compared with the primary sequence of a polypeptide with a known structure, which comprises at least one TPR structure motif or at least one TPR domain,
b. for homologous fragments obtained according to (a), the 3D structure of the polypeptide with an unknown structure is modelled along the lines of the structure of a spatial configuration of a crystal according to any one of claims 2 to 26 and
c. by means of the relevant computer programs, energy optimisations of the structure or structure areas modelled according to (b) are undertaken.

42. The method for the identification of a compound which inhibits the interaction between a polypeptide, containing at least one amino acid sequence of a TPR structure motif of a Hop protein or a derivative of such an amino acid sequence, in particular a Hop protein, of unknown 3D structure and a chaperone protein, in particular Hsp70 and/or Hsp90, **characterised in that**
a. the unknown 3D structure of the polypeptide is determined according to a method defined in any one of claims 39 to 41 and
b. a compound with the characteristic of acting as an inhibitor to the interaction of a chaperone protein with a polypeptide with an unknown 3D structure is determined by way of a method according to any one of claims 31 to 38.

## Revendications

1. Procédé de fabrication d'un cristal ayant des mailles cristallines, contenant, dans la maille asymétrique, au moins un polypeptide et, le cas échéant, au moins un autre composé, le polypeptide dans la maille asymétrique contenant au moins une séquence d'acides aminés dun motif de structure TPR d'une protéine Hop, **caractérisé en ce que**
(a) on surexprime le polypeptide dans un système d'expression,
(b) on purifie le polypeptide surexprimé et on le concentre,
(c) on dissout le concentré de polypeptide obtenu selon (b) dans un système tampon approprié, le cas échéant en ajoutant au moins un autre composé, et
(d) on initie la cristallisation à 20°C selon la technique de la "goutte suspendue" avec un procédé de diffusion de la vapeur.

2. Cristal susceptible d'être obtenu selon un procédé selon la revendication 1.

3. Cristal selon la revendication 2 renfermant, par maille asymétrique dans la maille cristalline du cristal, au moins un polypeptide et, le cas échéant, au moins un autre composé, **caractérisé en ce que** les polypeptides adoptent, dans le cristal, une configuration spatiale, le polypeptide de configuration spatiale contenant au moins une séquence d'acides aminés d'un motif de structure TPR d'une protéine Hop.

4. Cristal selon la revendication 2 ou 3, **caractérisé en ce que** le polypeptide de configuration spatiale contient au moins l'une des séquences d'acides aminés (1), (2), (3), (4), (5), (6), (7), (8) et/ou (9) reproduites sur la figure 3A.

5. Cristal selon l'une quelconque des revendications précédentes 2 à 4, **caractérisé en ce que** le polypeptide de configuration spatiale contient au moins l'une des séquences d'acides aminés des domaines TPR1, TPR2A ou TPR2B d'une protéine Hop ou d'un segment(s) de celles-ci.

6. Cristal selon l'une quelconque des revendications précédentes 2 à 5, **caractérisé en ce que** le polypeptide de configuration spatiale contient au moins l'une des séquences d'acides aminés (1), (2) ou (3), comme représenté sur la figure 3B.

7. Cristal selon l'une quelconque des revendications précédentes 2 à 6, **caractérisé en ce que** la au moins une séquence d'acides aminés d'un motif de structure TPR ou d'un domaine TPR contenue dans le polypeptide correspond à une séquence(s) d'acides aminés d'une protéine Hop d'origine eucaryote.

8. Cristal selon l'une quelconque des revendications précédentes 2 à 7, **caractérisé en ce que** la séquence d'acides aminés d'un motif de structure TPR ou d'un domaine TPR contenue dans le polypeptide correspond à une séquence(s) d'acides aminés d'une protéine Hop d'origine humaine.

9. Cristal selon l'une quelconque des revendications précédentes 2 à 8, **caractérisé en ce que** le polypeptide présente la séquence d'acides aminés d'une protéine Hop eucaryote.

10. Cristal selon l'une quelconque des revendications précédentes 2 à 9, **caractérisé en ce que** la configuration spatiale présente le polypeptide et au moins un autre composé.

11. Cristal selon l'une quelconque des revendications précédentes 2 à 10, **caractérisé en ce que** la configuration spatiale présente le polypeptide et au moins un composé qui se lie au polypeptide en tant que ligand.

12. Cristal selon la revendication 10 ou 11, **caractérisé en ce que** le composé ou le ligand est une molécule qui n'apparaît pas physiologiquement.

13. Cristal selon l'une quelconque des revendications précédentes 2 à 12, **caractérisé en ce que** la configuration spatiale présente un polypeptide et au moins un ligand physiologique ou au moins un segment d'un ligand physiologique pour le polypeptide.

14. Cristal selon l'une quelconque des revendications précédentes 2 à 13, **caractérisé en ce que** la configuration spatiale présente un polypeptide et au moins un ligand ou un segment d'un ligand pour le polypeptide, le ligand se liant à la séquence d'acides aminés d'un motif de structure TPR, en particulier à la séquence d'acides aminés d'un domaine TPR.

15. Cristal selon l'une quelconque des revendications précédentes 10 à 14, **caractérisé en ce que** le ligand est un polypeptide, un oligopeptide, un dipeptide ou un dérivé, modifié par synthèse, d'un polypeptide, d'un oligopeptide ou d'un dipeptide.

16. Cristal selon l'une quelconque des revendications précédentes 10 à 15, **caractérisé en ce que** le ligand contient une séquence d'acides aminés ou un segment d'une séquence d'acides aminés d'une protéine de la famille des protéines chaperon ou un dérivé d'une telle séquence d'acides aminés ou d'un tel segment.

17. Cristal selon l'une quelconque des revendications précédentes 10 à 16, **caractérisé en ce que** le ligand présente un segment de la séquence d'acides aminés C-terminale d'une protéine chaperon.

18. Cristal selon l'une quelconque des revendications précédentes 10 à 17, **caractérisé en ce que** le ligand présente une séquence d'acides aminés ou un segment d'une séquence d'acides aminés de la protéine chaperon Hsp 70 et/ou Hsp90.

19. Cristal selon l'une quelconque des revendications précédentes 10 à 18, **caractérisé en ce que** le ligand est un inhibiteur de la fonction physiologique, en particulier de la fonction d'adaptateur, d'une protéine Hop.

20. Cristal selon l'une quelconque des revendications précédentes 10 à 19, **caractérisé en ce que** le ligand est un inhibiteur des interactions entre les protéines Hop et Hsp70 et/ou les protéines Hop et Hsp90.

21. Cristal selon l'une quelconque des revendications précédentes 2 à 20, **caractérisé en ce que** la forme cristalline contient des ions métalliques, en particulier des ions de métaux lourds.

22. Cristal selon l'une quelconque des revendications précédentes 2 à 21, **caractérisé en ce que** la forme cristalline du polypeptide contient au moins un motif de structure TPR d'une protéine Hop (structure des séquences (1), (2), (4), (5), (7) et/ou (8) de la figure 3A) avec des coordonnées structurelles des séquences correspondantes, comme représenté sur les figures 3C et 3D respectivement 3E (pour les séquences (2), (5) et (8)).

23. Cristal selon l'une quelconque des revendications précédentes 2 à 22, **caractérisé en ce que** la forme cristalline du polypeptide contient au moins un domaine TPR d'une protéine Hop (structure des séquences (1) et (2) selon la figure 3B) avec les coordonnées structurelles correspondantes, comme représenté sur les figures 3C et 3D pour TPR1 et la figure 3E pour TPR2A.

24. Cristal selon l'une quelconque des revendications précédentes 2 à 23, **caractérisé en ce que** le groupe spatial du cristal de forme cristalline est monoclinique, tétragonal, orthorhombique, cubique, triclinique, hexagonal ou trigonal/rhomboédrique.

25. Cristal selon l'une quelconque des revendications 2 à 24, **caractérisé en ce que** le groupe spatial du cristal est P2₁, C2 ou P4₁.

26. Cristal selon l'une quelconque des revendications précédentes 2 à 25, **caractérisé en ce que** la maille cristalline du cristal contenant la forme cristalline présente des paramètres de maille d'environ a = 31,2 Å, b = 43,8 Å, c = 38,3 Å et β = 101,8° ou a = 75,5 Å et c = 42,9 Å.

27. Composé, **caractérisé en ce que** le composé a la propriété de se lier en tant que liant à des domaines de structure d'une protéine Hop, le composé entrant en interaction non covalente avec la chaîne principale et/ou les chaînes latérales d'acides aminés qui font partie d'un domaine TPR d'une protéine Hop et le composé étant un oligopeptide modifié ou non modifié qui contient la séquence d'acides aminés EEVD, destiné à une utilisation en tant que médicament.

28. Composé selon la revendication 27, **caractérisé en ce que** le composé est un oligopeptide qui contient la séquence d'acides aminés GPXIEEVD ou SXMEEVD, X représentant un acide aminé quelconque présent à l'état naturel, destiné à une utilisation en tant que médicament.

29. Composé selon l'une quelconque des revendications 27 à 28, **caractérisé en ce que** le composé présente les séquences d'acides aminés indiquées dans les revendications 27 ou 28 à l'extrémité C.

30. Utilisation d'un composé selon l'une quelconque des revendications 27 à 29 pour la préparation d'un médicament destiné au traitement de maladies tumorales, à l'immunosuppression, en particulier de maladies auto-immunes ou d'autres maladies inflammatoires, de la réaction de greffe contre hôte ou pour traiter des infections virales.

31. Procéder d'identification d'un composé ayant la propriété d'agir en tant qu'inhibiteur de l'interaction entre une protéine Hop et une protéine chaperon, en particulier Hsp70 ou Hsp90, **caractérisé en ce que**
a) on obtient une configuration spatiale d'un cristal selon l'une quelconque des revendications 2 à 26 par exploration structurelle,
b) la structure de la configuration spatiale est représentée à l'aide de coordonnées structurelles en trois dimensions,
c) on choisit des propriétés stériques et/ou des groupes fonctionnels d'un composé de manière à créer ou, le cas échéant à optimiser des interactions entre le composé et la chaîne principale et/ou les chaînes latérales du polypeptide dans la zone de liaison.

32. Procédé selon la revendication 31, **caractérisé en ce que**, dans une étape (d) du procédé, on détermine la structure tridimensionnelle du composé obtenu selon (c) et on modélise la structure du polypeptide.

33. Procédé selon la revendication 31 ou 32, **caractérisé en ce que**, dans une étape (e) du procédé, on détermine la force de l'interaction entre le composé et le polypeptide déployé selon une configuration spatiale d'un cristal selon l'une quelconque des revendications 2 à 26.

34. Procédé selon l'une quelconque des revendications 31 à 33, **caractérisé en ce que**, selon l'étape (b) du procédé on représente tout ou partie des coordonnées structurelles de la figure 3D, 3C et 3E en trois dimensions.

35. Procédé selon l'une quelconque des revendications 31 à 34, **caractérisé en ce qu'**on répète les étapes (c), (d) et (e) du procédé de manière cyclique jusqu'à ce que la force, obtenue selon (e) de l'interaction entre le composé et la structure du polypeptide dans une configuration spatiale, soit optimisée avec une structure d'un cristal selon l'une quelconque des revendications 2 à 26.

36. Procédé selon l'une quelconque des revendications 31 à 35, **caractérisé en ce que**, dans une étape (f) du procédé, on détermine les propriétés, en particulier les propriétés inhibitrices, du composé modélisé dans un système de test biologique.

37. Procédé d'identification d'un composé ayant la propriété d'agir en tant qu'inhibiteur de l'interaction entre une protéine Hop et au moins une protéine chaperon, en particulier Hsp70 ou Hsp90, **caractérisé en ce que**
(a) on établit un système de test biologique pour de tels composés inhibiteurs potentiels,
(b) on détermine les composés agissant comme inhibiteurs avec un système de test biologique selon (a),
(c) on détermine la conformation du composé,
(d) on représente graphiquement la structure d'un polypeptide d'une configuration spatiale d'un cristal, comme indiqué selon l'une quelconque des revendications 2 à 26, en trois dimensions avec ses coordonnées structurelles et
(e) on insère la structure, obtenue selon (b) et (c) du composé dans la structure obtenue selon (d) du polypeptide.

38. Procédé selon la revendication 37, **caractérisé en ce que**, dans une étape (f) du procédé, on détermine la nature et/ou la force de l'interaction entre le composé et la configuration spatiale d'un polypeptide.

39. Procédé de représentation d'une structure en trois dimensions d'un polypeptide de structure inconnue, avec au moins un polypeptide qui contient au moins un motif de structure TPR, impliqué dans la liaison à une protéine chaperon, ou de préférence un domaine de structure TPR, **caractérisé en ce que** la structure inconnue du polypeptide est déterminée sur la base d'une configuration spatiale connue d'un cristal selon l'une quelconque des revendications 2 à 26.

40. Procédé selon la revendication 39, **caractérisé en ce qu'**on utilise des coordonnées structurelles d'une configuration spatiale d'un cristal selon l'une quelconque des revendications 2 à 26, en particulier des coordonnées structurelles comme représentées sur la figure 3C, 3D ou 3E, pour explorer la structure inconnue.

41. Procédé selon la revendication 39 ou 40, **caractérisé en ce que**
(a) on compare la séquence primaire d'un polypeptide de structure 3D inconnue avec la séquence primaire d'un polypeptide de structure connue qui présente au moins un motif de structure TPR ou un domaine TPR,
(b) on modélise, pour des segments homologues obtenus selon (a), la structure 3D du polypeptide de structure inconnue en s'appuyant sur la structure d'une configuration spatiale d'un cristal selon l'une quelconque des revendications 2 à 26 et
(c) on procède à des optimisations énergétiques de la structure ou des zones de structure modélisée(s) selon (b) à l'aide de programmes informatiques correspondants.

42. Procédé d'identification d'un composé qui inhibe l'interaction entre un polypeptide, contenant au moins une séquence d'acides aminés d'un motif de structure TPR d'une protéine Hop ou un dérivé d'une telle séquence d'acides aminés, en particulier d'une protéine Hop, de structure 3D inconnue et une protéine chaperon, en particulier Hsp 70 et/ou Hsp90, **caractérisé en ce que**
(a) on détermine la structure 3D inconnue du polypeptide selon un procédé selon l'une quelconque des revendications 39 à 41 et
(b) on détermine, à l'aide d'un procédé selon l'une quelconque des revendications 31 à 38, un composé ayant la propriété d'inhiber l'interaction d'une protéine chaperon avec un polypeptide de structure 3D inconnue.
